# EUROPEAN PATENT APPLICATION

(11) **EP 3 756 684 A1**
(43) Date of publication of application: **30.12.2020**
(21) Application number: 20184815.7
(22) Date of filing: 24.05.2010
(51) Int. Cl.: A61K 39/12

(54) **VACCINES AGAINST HERPES SIMPLEX VIRUS TYPE 2: COMPOSITIONS AND METHODS FOR ELICITING AN IMMUNE RESPONSE**

(30) Priority: 22.05.2009 US 18078409 P; 20.08.2009 US 23562809 P; 08.09.2009 US 24058709 P; 08.09.2009 US 24062609 P; 18.02.2010 US 30591810 P
(62) Document number(s) of the earlier application(s) in accordance with Art. 76 EPC: 19172066.3 / 3 590 533; 10778532.1 / 2 432 504
(71) Applicant: Genocea Biosciences, Inc., Cambridge, MA 02142 (US)
(72) Inventor: LONG, Deborah, Monroe, NY 10950 (US); FLECHTNER, Jessica, Sudbury, MA 01776 (US); SKOBERNE, Mojca, Cambridge, MA 02139 (US); SIBER, George, New York, NY 10023 (US)
(74) Representative: EIP

(57) **Abstract**

Herpes Simplex Virus-2 (HSV-2) infection is a major health concern. The present disclosure provides, *inter alia,* certain highly effective vaccines and immunogenic compositions against HSV-2. The antigens can be used therapeutically or prophylactically.

## Description

### Related Applications

This application claims the benefit of the filing date of U.S. Provisional Application No. 61/180,784, filed on May 22, 2009, U.S. Provisional Application No. 61/235,628, filed on August 20, 2009, U.S. Provisional Application No. 61/240,587, filed on September 8, 2009, U.S. Provisional Application No. 61/240,626, filed on September 8, 2009, and U.S. Provisional Application No. 61/305,918 filed on February 18, 2010. The entire teachings of the referenced applications are expressly incorporated herein by reference.

### I. Background

Herpes simplex virus type 2 (HSV-2) is the leading cause of genital herpes. HSV-2 is most often transmitted by sexual contact, and infection with the virus typically leads to recurring outbreaks of lesions on the genitals and perianal regions, combined with shedding of virus particles into the genital tract. Viral shedding can also occur in the absence of lesions or other symptoms. HSV-2 also establishes latency in sensory ganglia. HSV-2 infection causes physical discomfort and psychosexual morbidity in affected patients, and introduces additional health risks. In particular, patients infected with HSV-2 are at increased risk for contracting HIV, and pregnant mothers infected with HSV-2 can vertically transmit HSV-2 to their fetuses. In immunocompromised individuals or in neonates, HSV-2 infections can be fatal. Currently, there is no cure for HSV-2 infection.

HSV-2 infection is widespread, with one study estimating that nearly 20% of the population worldwide is infected (Looker et al., 2008, Bulletin of the World Health Organization, October 2008, 86(10)). More women than men are infected, and the prevalence of the disease increases with age. High numbers of adolescents diagnosed with HSV-2 indicate that the prevalence across the population will continue to rise, as HSV-2 infection is lifelong.

Treatment options for HSV-2 symptoms are limited. Antiviral therapy, using compounds such as famciclovir, valaciclovir, or aciclovir, limits the duration of symptoms and, in some cases, speeds healing of lesions and reduces incidence of viral shedding. Antiviral drugs are not curative, however, and do not prevent recurrence of outbreaks or clear the virus completely. In addition, use of antiviral drugs requires patients to recognize symptoms of HSV-2 infection, then obtain a confirmative diagnosis, and ultimately, comply with the antiviral regimen. These requirements may be untenable in regions of the world where antiviral drugs are not readily available. In addition, patients are often unaware that they are infected, either because they do not present symptoms, or because the symptoms of the initial infection subside, suggesting recovery from the disease.

To address the medical and social problems associated with HSV-2, it is highly desirable to develop pharmaceutical compositions to inhibit or counteract infection by HSV-2. An effective composition may be used to elicit an enhanced immune response against HSV-2, thereby preventing initial infection, blocking the ability of the virus to establish latency in sensory ganglia, eliminating recurrence of outbreaks, and/or preventing viral shedding. The immune system is known to mount a defense against HSV-2, as evidenced by recurrent infections which manifest with fewer, less intense symptoms and decreased frequency over time.

While the ultimate goal of an HSV vaccine would be long-lasting protection from viral infection, the suppression of disease symptoms would also provide significant health benefits. One of the current goals for either a prophylactic or therapeutic vaccine is to reduce clinical episodes and viral shedding from primary and latent infections. Three categories of prophylactic vaccines have been tested in clinical trials with disappointing results i) whole virus, ii) protein subunit and iii) gene-based subunit vaccines (Stanberry et al., Clinical Infect. Dis., 30(3):549-566, 2000). In the 1970s a number of killed virus vaccines were explored, none of which were efficacious. More recently an attenuated HSV was found to be poorly immunogenic. Subunit vaccines based on two recombinant glycoproteins have been clinically evaluated in combination with different adjuvant formulations. One developed by Chiron contains truncated forms of both glycoprotein D (gD2) and glycoprotein B (gB2) of HSV-2, purified from transfected Chinese Hamster Ovary (CHO) cells and formulated in the adjuvant MF59. Another developed by Glaxo-Smithkline (GSK) contains a truncated gD2 formulated with adjuvants alum and 3-O-deacylated monophosphoryl lipid A (MPL). Both vaccines were immunogenic and well tolerated in phase I/II trials. However in phase III analyses, the Chiron vaccine showed no overall efficacy against HSV-2 seroconversion and work was discontinued. The GSK vaccine showed significant efficacy (73-74%) in HSV-1, HSV-2 seranegative women volunteers but no efficacy in men.

While even limited vaccine efficacy would beneficially impact HSV sufferers, these trials are testing only a small number of vaccine possibilities. This is because the vaccine discovery has not been systematic. Pursuance of a whole-virus vaccine assumes that presentation of the pathogen itself to the immune system will generate optimal immunity. Indeed the breadth and duration of immune responses to whole pathogen vaccines historically have been better than subunit vaccines. However, pathogenicity of the vaccine strain must be considered. Subunit vaccines, to date, have been selected for vaccine testing based on their assumed importance in disease pathogenesis and immunogenicity during infection. These approaches have identified one candidate against HSV with limited efficacy in some but no efficacy in other formulations. Thus, new and improved methodologies for herpesvirus vaccine discovery are needed to protect against herpes diseases.

### -II. Summary of the Invention

Infection and transmission of HSV-2 is a major health concern. The present disclosure provides, *inter alia,* certain highly effective vaccines against HSV-2. Such vaccines can be used either therapeutically or prophylactically. The present disclosure also provides specific antigens and methods for using the antigens to elicit an immune response against HSV-2.

In one aspect, the present disclosure describes a vaccine formulation comprising a pharmaceutical-acceptable carrier and at least one polypeptide consisting of SEQ ID NOS: 2, 3, 4 and 5 or an immunogenic fragment thereof, and optionally further comprising SEQ ID NO:1 or an immunogenic fragment thereof. The vaccine formulation may comprise a first polypeptide consisting of one of the above SEQ ID NOS, and a second polypeptide consisting of another one of the above SEQ ID NOS.

Another aspect of the present invention provides a vaccine formulation comprising a pharmaceutically acceptable carrier, an adjuvant comprising one or more purified fractions of quillaja saponins, and at least one polypeptide comprising any of SEQ ID NOS: 2, 3, 4 and 5 or an immunogenic fragment thereof, and optionally further comprising SEQ ID NO:1 or an immunogenic fragment thereof.

A further aspect of the present invention provides a vaccine formulation comprising a pharmaceutically-acceptable carrier and a polypeptide consisting of SEQ ID NO: 2 or an immunogenic fragment thereof. Residues may be truncated from SEQ ID NO:2. The polypeptide may be glycosylated, or may be unglycosylated.

In still a further aspect, the present invention provides a vaccine formulation comprising a pharmaceutically-acceptable carrier and a polypeptide comprising SEQ ID NO:5, wherein the polypeptide lacks all or at least an 8 contiguous amino acid residue portion of the transmembrane domain spanning residues 341-363. Accordingly, one aspect of the present invention provides a vaccine formulation comprising a pharmaceutically-acceptable carrier and a polypeptide comprising SEQ ID NO:4. The polypeptide may be glycosylated, or may be unglycosylated.

Still another aspect of the present invention provides a vaccine formulation comprising a pharmaceutically-acceptable carrier, a polypeptide comprising SEQ ID NO:5. The polypeptide may be glycosylated, or may be unglycosylated.

Yet another aspect of the present invention provides a vaccine formulation comprising a pharmaceutically-acceptable carrier, a polypeptide comprising SEQ ID NO:3. The polypeptide may be glycosylated, or may be unglycosylated.

In some embodiments, polypeptides in the vaccine formulations that may be conjugated to an immunogenic carrier, for example keyhole limpet hemocyanin. In other embodiments, the vaccine formulations further comprise an adjuvant. The adjuvant may be one or more purified fractions of quillaja saponins.

The invention provides methods or treating a subject suffering from or susceptible to HSV-2 infection by administering an effective amount of a vaccine formulation disclosed herein. In some embodiments, the method inhibits HSV-2 symptoms, for example by reducing the number of herpetic lesions, reducing the number of days a subject experiences herpetic lesions, reducing infection by HSV-2 in an uninfected subject, increasing the IgG titer and/or T cell response to one or more HSV-2 antigens, and/or reducing the number of herpetic lesions at the onset of HSV-2 infection.

In another aspect, the present disclosure describes the results of a high-throughput system for in vitro screening of libraries of efficacious T cells to identify their specific target antigens from the complete proteome of HSV-2. This technology allowed the identification of individual antigens, likely to be effective in vivo, as either a prophylactic or therapeutic composition. In one aspect, herein are provided several critical protective T cell antigens that can be incorporated into protein-based compositions that elicit an immune response.

One aspect of the present invention provides pharmaceutical compositions comprising two or more isolated polypeptides selected from polypeptides having an amino acid sequence of at least one of SEQ ID NOS: 1-38, or an immunogenic fragment thereof.

In another aspect, the invention provides vaccine formulations that include a pharmaceutically-acceptable carrier and a polypeptide comprising at least one of SEQ ID NOS: 1-38, or an immunogenic fragment thereof. In certain embodiments, the polypeptide consists of at least one of SEQ ID NOS: 1-38.

Another aspect of the present invention provides a method of inducing an immune response in a subject, comprising administering to said subject an effective amount of a vaccine formulation or a pharmaceutical composition comprising an effective amount of two or more isolated polypeptides selected from polypeptides having an amino acid sequence of at least one of SEQ ID NOS: 1-38, or an immunogenic fragment thereof.

Yet another aspect of the present invention provides a method of reducing one or more symptoms of HSV-2 infection in a subject, comprising administering to said subject an effective amount of a vaccine formulation or a pharmaceutical composition comprising two or more isolated polypeptides selected from polypeptides having an amino acid sequence of at least one of SEQ ID NOS: 1-38, or an immunogenic fragment thereof. In some embodiments, the symptoms of HSV-2 infection comprise one or more of lesion formation, pain, irritation, itching, fever, malaise, headache, viral shedding, and prodrome.

A further aspect of the present invention provides a method of inhibiting the onset of HSV-2 infection, comprising administering an effective amount of a vaccine formulation or a composition comprising two or more isolated HSV polypeptides selected from polypeptides having an amino acid sequence of at least one of SEQ ID NOS: 1-38, or an immunogenic fragment thereof.

Applicants disclose another aspect of the present invention, which provides a method of inhibiting development of a latent HSV-2 infection in a subject exposed to HSV-2, comprising administering an effective amount of a vaccine formulation or a composition comprising two or more isolated HSV-2 polypeptides selected from polypeptides having an amino acid sequence of at least one of SEQ ID NOS: 1-38, or an immunogenic fragment thereof.

In a related aspect, the present invention provides a method of reducing viral shedding in a subject infected with HSV-2, comprising administering an effective amount of a vaccine formulation or a composition comprising two or more isolated HSV-2 polypeptides selected from polypeptides having an amino acid sequence of at least one of SEQ ID NOS: 1-38, or an immunogenic fragment thereof.

Further, an aspect of the present invention provides a method of reducing recurrence of outbreaks in a subject infected with HSV-2, comprising administering an effective amount of a vaccine formulation or a composition comprising two or more isolated HSV-2 polypeptides selected from polypeptides having an amino acid sequence of at least one of SEQ ID NOS: 1-38, or an immunogenic fragment thereof.

An additional aspect of the present invention provides a method of producing any of the pharmaceutical compositions described above, comprising expressing said two or more polypeptides; and isolating said two or more polypeptides.

Applicants further disclose an aspect of the present invention which provides a method for diagnosing severity of symptoms in a subjected infected with HSV-2, comprising (i) measuring activation of T cells in response to autologous antigen presenting cells (APC) pulsed with one or more isolated HSV-2 polypeptides selected from polypeptides set forth in SEQ ID NOS: 1-38, or an immunogenic fragment thereof, and (ii) comparing said levels to reference levels obtained from infected subjects experiencing frequent outbreaks; whereby a significant increase in said responses relative to reference levels indicates that said subject has less severe symptoms (e.g., the subject is asymptomatic). A significant increase in response can, for example, comprise a 1.5-fold or greater, 2-fold or greater, 3-fold or greater, 5-fold or greater, 10-fold or greater or even 20-fold or greater increase.

Another aspect of the present invention provides a method for diagnosing severity of symptoms in a subjected infected with HSV-2, comprising (i) measuring activation of T cells from naturally infected or virus-exposed subjects in response to APC presenting one or more isolated HSV-2 polypeptides selected from polypeptides set forth in SEQ ID NOS: 1-38, or an immunogenic fragment thereof, and (ii) comparing said levels to reference levels obtained from infected subjects experiencing frequent outbreaks; whereby a significant decrease in said activation relative to reference levels indicates that said subject has more severe symptoms (e.g., frequent outbreaks).

Another aspect of the present invention provides pharmaceutical compositions comprising an antibody that binds to one or more isolated HSV polypeptides selected from the list consisting of SEQ ID NOS: 1-38, or an immunogenic fragment thereof.

Moreover, a different aspect of the present invention provides a method of identifying immunogenic compositions for HSV-2 by testing two or more polypeptides selected from polypeptides having an amino acid sequence of any one of SEQ ID NOs. 1-38, or an immunogenic fragment thereof, for ability to promote cytokine production in a mammalian T cell, wherein an immunogenic composition is one that elevates levels of a cytokine significantly above the levels of that cytokine produced by a naive mammalian T cell. A significant increase in cytokine levels is typically one that is at least 1.5-fold, 2-fold, 3-fold, 5-fold, 10-fold or even 20-fold the level produced by a naive cell.

Still another aspect of the present invention provides a method of detecting HSV-2 in a sample from a subject, said method comprising (i) contacting said sample with one or more antibodies raised against one or more polypeptides having an amino acid sequence of SEQ ID NOS: 1-38 or an immunogenic fragment thereof, and (ii) detecting said one or more antibodies bound to said one or more HSV-2 polypeptide from the sample.

Finally, one aspect of the present invention provides pharmaceutical compositions comprising two or more isolated polynucleotides, selected from nucleotide SEQ ID NOS: 1-38, or fragments encoding immunogenic peptides thereof.

### III. Brief Description of the Drawings

Figures 1A and B are graphs showing, respectively, CD4⁺ and CD8⁺ T cell responses following immunization with gD2 full-length protein, gD2ΔTMR, or gD2 truncated immediately upstream of the transmembrane domain (denoted 306t).
Figures 2A and B are graphs showing, respectively, CD4⁺ and CD8⁺ T cell responses following immunization with pooled, overlapping peptides spanning gL2, ICP4.1, or ICP4 fragments encoded by RS1.1, RS1.3.1 and RS1.3.2.
Figure 3A and B are graphs showing, respectively, CD4⁺ and CD8⁺ T cell responses following immunization with gD2ΔTMR, or gD2ΔTMR and ICP4.2.

### IV. Detailed Description

This application describes vaccines and immunogenic compositions against HSV-2. Vaccine formulations may include a polypeptide comprising a sequence from Table 1 or an immunogenic fragment thereof, or a combination of at least two polypeptides comprising sequences from Table 1 or immunogenic fragments thereof. In certain embodiments, the polypeptide(s) of the vaccines comprise the entire sequence of at least one of SEQ ID NOS: 1-26 or consist of the entire sequence of any one of SEQ ID NOS: 1-26. Immunogenic compositions may include a polypeptide comprising a sequence from Table 1 or Table 2 or an immunogenic fragment thereof or a combination of at least two polypeptides comprising sequences from Table 1 or Table 2, or immunogenic fragments thereof. In certain embodiments, the polypeptide(s) of the immunogenic compositions comprise the entire sequence of any one of SEQ ID NOS: 1-38 or consist of the entire sequence of SEQ ID NO: 1-38. The polypeptides in Tables 1 or 2 may be encoded by SEQ ID NOS: 39-46 and 117-134 as indicated and/or by cDNA sequences publically available on http://www.ncbi.nlm.nih.gov/sites/entrez. cDNA and protein sequences may also be obtained from any known strains of HSV-2, including HG52, 333, and Strain G. Accordingly, cDNA sequences may be accessed by gene or protein name from genomic sequence at NC_001798.1, and may be approximately 97% conserved with sequences disclosed at NC_001798.1). As described herein, the polypeptides may be referred to by protein name, by SEQ ID NO, and/or by the name of the gene encoding the protein.

The polypeptides can be prepared in a variety of expression systems. Suitable expression systems include *E. coli* and Baculovirus-based expression systems (e.g., in insect cells). Polypeptides prepared using *E. coli* are typically full-length and unglycosylated, although truncated variants can be prepared. In certain embodiments, these truncated variants retain all or part of the signal domain. Polypeptides prepared using a Baculovirus system typically lack the N-terminal signal sequence, but are fully or partially glycosylated.

**Table 1. HSV-2 antigens for vaccines or immunogenic compositions**

| **Protein SEQ ID No.** | **DNA SEQ ID No.** | **Gene Name *Protein Name*** | **Gene ID No.** | **GenBank Accession Nos.** |
|---|---|---|---|---|
| 1 | 39 | RS1 *ICP4* | 1869897 | NP_044530.1 |
| 2 | 117 | RS1.2 *ICP4 internal fragment (ICP4.2)* | | RS1.2 corresponds to amino acid residues 383-766 or nucleotides 1150-2398 of the RS1 sequence |
| 3 | 118 | *gL2 cytoplasmic* | 1487292 | NP_044470.1 |
| 4 | 40 | US6ΔTMR *gD2 internal deletion (gDΔTMR)* | 9629336 | NP_044536.1 US6ΔTMR corresponds to gD2 will a deletion of amino acids 341-363 |
| 5 | | US6 *gD2* | | |
| 6 | 41 | RL1 *ICP34.5* | 9629329 | NP_044529.1 |
| 7 | 42 | RL2 *ICP0* | 109676722 | NP_044528.2 |
| 8 | 121 | RS1.1 *ICP4 internal fragments* | 1869897 | NP_044530.1 RS1.1 corresponds to residues 1-400 of RS1 |
| 9 | 122 | RS1.3.1 *ICP4 internal fragments* | 1869897 | NP_044530.1 RS1.3.1 corresponds to residues 750-1024 of RS1 |
| 10 | 123 | RS1.3.2 *ICP4 internal fragments* | 1869897 | NP_044530.1 RS1.3.2 corresponds to residues 1008-1319 of RS1 |
| 11 | 124 | RS1.3 *ICP4 internal fragments* | 1869897 | NP_044530.1 RS1.3 corresponds to residues 750-1319_of RS1 |
| 12 | 125 | RS1.4 *ICP4 internal fragments* | 1869897 | NP_044530.1 RS1.4 corresponds to residues 340-883 of RS1 |
| 13 | 126 | RS1.5 *ICP4 internal fragments* | 1869897 | NP_044530.1 RS1.5 corresponds to residues 775-1318 of RS1 |
| 14 | 127 | RS1.6 *ICP4 internal fragments* | 1869897 | NP_044530.1 RS1.6 corresponds to residues 209-1318 of RS1 |
| 15 | 128 | RS1.7 *ICP4 internal fragments* | 1869897 | NP_044530.1 RS1.7 has a deletion of residues 391-544 of RS1 |
| 16 | 129 | RS1.8 *ICP4 internal fragments* | 1869897 | NP_044530.1 RS1.8 has a deletion of residues 786-864 of RS1 |
| 17 | | UL2 *uracil DNA glycosylase* | | |
| 18 | | UL11 *myristylated* | | |
| | | *tegument protein* | | |
| 19 | 119 | UL1 *gL2 secreted* | 1487292 | NP_044470.1 |
| 20 | | UL19 *VP5* | | |
| 21 | 120 | UL19ΔTEV *VP5* | 9629288 | NP_044488.1 UL19ΔTEV is lacking the last 5 amino acids from the C-terminal end of UL19 |
| 22 | | UL36 *ICP 1*/*2* | | |
| 23 | 43 | UL36.3.4.1 *ICP 1*/*2 internal fragments* | 1487322 | NP_044506.1 UL 36.3.4.1 corresponds to residues 1318-2280 of UL36 |
| 24 | 44 | UL36.4.2.5 *ICP 1*/*2 internal fragments* | 1487322 | NP_044506.1 UL 36.4.2.5 corresponds to residues 2253-3122 of UL36 |
| 25 | | UL40 *ribonucleoside reductase* | | |
| 26 | 45 | US12 *ICP47* | 9629343 | NP_044543.1 |

**Table 2. Additional HSV-2 antigens for immunogenic compositions**

| **Protein SEQ ID No.** | **DNA SEQ ID** | **No. Gene Name *Protein Name*** | **Gene ID No.** | **GenBank Accession Nos.** |
|---|---|---|---|---|
| 27 | 134 | UL10 *gM2* | 9629279 | NP_044479.1 |
| 28 | | UL15 *DNA cleavage*/*packaging protein* | | |
| 29 | | UL26.5 *ICP35* | | |
| 30 | | UL30 *DNA-directed polymerase* | | |
| 31 | | UL5 | | |
| | | *DNA helicase*/*primase complex* | | |
| 32 | | UL8 *DNA helicase*/*primase complex* | | |
| 33 | | UL15.5 *unknown* | | |
| 34 | | UL32 *cleavage and packaging protein* | | |
| 35 | | UL36.4.2 *ICP 1*/*2 fragment* | | |
| 36 | | UL54 *ICP27* | | |
| 37 | 133 | UL49.5 *Membrane associated virion protein* | 1487337 | NP_044520.1 |
| 38 | 46 | US4 *gG2* | 9629334 | NP_044534.1 |

### A. Immunogenic HSV-2 polypeptides

Immunogenic polypeptides or polynucleotides as indicated in Table 1 and/or Table 2 may be used in pharmaceutical compositions. The invention provides pharmaceutical compositions containing immunogenic polypeptides or polynucleotides encoding these immunogenic polypeptides together with a pharmaceutical carrier. Antigens from HSV-2 may be identified by screening immune cells from patients infected with HSV-2. Briefly, a library of HSV-2 antigens was expressed by bacteria and mixed with antigen presenting cells (APCs). The APCs, in turn, processed and presented HSV-2-derived polypeptides to lymphocytes that had been isolated from human patients infected with HSV-2. The patients belonged to several populations: (1) exposed to HSV-2 but seronegative for infection, (2) infected with HSV-2 but asymptomatic, (3) infected with HSV-2 and experiencing infrequent outbreaks, (4) infected with HSV-2 and experiencing frequent outbreaks, (5) naive and (6) seronegative for HSV-2 (HSV-2⁻) but seropositive for HSV-1 (HSV-1⁺). Lymphocyte responses from each population were compared for reactivity to HSV-2-derived polypeptides, and the screen detected antigens that induced reactive lymphocytes with greater frequency in one patient population as compared to the others. Infected but asymptomatic, and exposed but seronegative patients may activate protective immune responses that patients who experience frequent outbreaks do not; in particular, exposed but seronegative patients are presumed to have mounted sterilizing immunity to HSV-2 infection. It is believed that a unique set of polypeptides will activate lymphocytes from these patient populations. Thus, the present invention contemplates compositions of the specific HSV-2 polypeptides that activate the lymphocytes of infected but asymptomatic, or exposed but seronegative patients or a combination of these polypeptides for inhibiting or counteracting infection by HSV-2.

Antigens identified on the basis of their immunogenicity in infected but asymptomatic, or exposed but seronegative patients are similarly expected to be immunogenic in any subject.

In some embodiments, a polypeptide may induce an innate immune response, a humoral immune response, or a cell-mediated immune response. The cell-mediated immune response may involve T_{H}1 cells, and in certain embodiments, the immune response involving T_{H}1 cells is an immune response in which T_{H}1 cells are activated. In some embodiments, an immunogenic polypeptide avoids induction of T_{H}2 cytokines. In some embodiments, the cell-mediated immune response may involve T_{H}17 cells, and in certain embodiments, the immune response involving T_{H}17 cells is an immune response in which T_{H}17 cells are activated.

Polypeptides (or immunogenic fragments thereof) in compositions of the invention may induce T cell responses in multiple individuals, regardless of the HLA haplotype of the individuals. Specifically, epitopes on the polypeptides may induce T cell responses in individuals with one or more of the following HLA supertypes: HLA-A2, -A3, -A24, -A1, -B7, -B8, -B27, -B44, -B58, and B62, and HLA-DQB01, -DQB02, -DQB03, -DQB-04, and -DQB05.

In some embodiments, one or more, e.g. two, three, four, or more polypeptides from Table 1 and/or Table 2 (or immunogenic fragments thereof) are provided in a composition of the invention. In some embodiments, two polypeptides from Table 1 and/or Table 2 are provided in a composition of the invention. In other embodiments, three polypeptides from Table 1 and/or Table 2 are provided in a composition of the invention.

In some embodiments, two, three, four, or more polypeptides from Table 1 and/or Table 2 (or immunogenic fragments thereof) are provided together as a conjugate. In some embodiments, two polypeptides from Table 1 and/or Table 2, or three polypeptides from Table 1 and/or Table 2, are provided as a conjugate. In some embodiments, two, three, four, or more polypeptides from Table 1 and/or Table 2 are covalently bound to each other, e.g., as a fusion protein. In some embodiments, two, three, four, or more polypeptides from Table 1 and/or Table 2 are covalently bound to each other, e.g., as a fusion protein. In some embodiments, two polypeptides from Table 1 and/or Table 2, or three polypeptides from Table 1 and/or Table 2, are covalently bound to each other, e.g. as a fusion protein.

In some embodiments, the compositions comprise two or more polypeptides selected from the group consisting of SEQ ID Nos. 1-38, and may contain or may not contain any other HSV-2 polypeptides.

In certain embodiments, Applicants provide polypeptides that are at least 70%, 80%, 90%, 95%, 96%, 97%, 98%, 99%, or 100% identical to a polypeptide encoded by a gene in Table 1 and/or Table 2, or a portion of said polypeptide. In certain embodiments, the homologous polypeptide is at least 8, 10, 15, 20, 30, 40, 50, 60, 80, 100, 120, 140, 160, 180, 200, 220, 240, 260, 280, 300, 350, 400, 450, or 500 amino acids in length. In some embodiments, such as those described immediately above, the polypeptide is no more than 300, 350, 400, 450, or 500 amino acids in length.

An immunogenic composition may also comprise portions of said polypeptides and genes, for example deletion mutants, truncation mutants, oligonucleotides, and peptide fragments. In some embodiments, the portions of said proteins are immunogenic.

The immunogenicity of a portion of a protein or a homolog thereof can be readily determined using the same assays that are used to determine the immunogenicity of the full-length protein. In some embodiments, the portion of the protein has substantially the same immunogenicity as the full-length proteins. In some embodiments, the immunogenicity is no more than 10%, 20%, 30%, 40%, or 50% less than that of the full-length protein. The protein fragments may be, for example, linear, circular, or branched. In some embodiments, a protein or protein fragment comprises one or more non-natural amino acids (e.g. an amino acid other than the 20 typically found in natural proteins). A non-natural amino acid may have an atypical side chain. In addition, peptidomimetics may be used; these may incorporate alterations to the peptide backbone.

Some embodiments of the polypeptide composition described herein include an immunogenic polypeptide that contains a membrane translocating sequence (MTS), to facilitate introduction of the polypeptide into the mammalian cell and subsequent stimulation of the cell-mediated immune response. Exemplary membrane translocating sequences include hydrophobic region in the signal sequence of Kaposi fibroblast growth factor, the MTS of α-synuclein, β-synuclein, or γ-synuclein, the third helix of the Antennapedia homeodomain, SN50, integrin β3 h-region, HIV Tat, pAntp, PR-39, abaecin, apidaecin, Bac5, Bac7, *P. berghei* CS protein, and those MTSs described in US Patents 6,248,558, 6,432,680 and 6,248,558.

In certain embodiments, the immunogenic polypeptide is conjugated (i.e. covalently bound) to another molecule. This may, for example, increase the half-life, solubility, bioavailability, or immunogenicity of the antigen. Molecules that may be conjugated to an immunogenic polypeptide include a carbohydrate, biotin, poly(ethylene glycol) (PEG), polysialic acid, N-propionylated polysialic acid, nucleic acids, polysaccharides, and PLGA. There are many different types of PEG, ranging from molecular weights of below 300 g/mol to over 10,000,000 g/mol. PEG chains can be linear, branched, or with comb or star geometries.

### B. Immunogenic HSV-2 polypeptides and nucleic acids for use in vaccines

In certain embodiments, one or more, e.g. two, three, four, or more immunogenic fragments or variants thereof are provided in a mixture. For example, a vaccine formulation may comprise any one or more of SEQ ID NOS: 1-26.

In certain embodiments, a vaccine formulation may comprise any one, two, or three of ICP4, ICP4.2, gL2, gD2ΔTMR and gD2 (SEQ ID NOS: 1-5), or immunogenic fragment(s) thereof. In certain embodiments, combinations contain polypeptides or immunogenic fragments from only one of ICP4 (SEQ ID NO 1) and ICP4.2 (SEQ ID NO 2). In other embodiments, combinations contain polypeptides or immunogenic fragments from only one of gD2ΔTMR (SEQ ID NO:4) and gD2 (SEQ ID NO:5).

Exemplary combinations of ICP4, ICP4.2, gL2, gD2ΔTMR and gD2 include:

| **Two antigen combinations** | |
|---|---|
| ICP4 SEQ ID NO: 1 | gL2 SEQ ID NO: 3 |
| ICP4 SEQ ID NO: 1 | gD2ΔTMR SEQ ID NO: 4 |
| ICP4 SEQ ID NO: 1 | gD2 SEQ ID NO: 5 |
| ICP4.2 SEQ ID NO: 2 | gL2 SEQ ID NO: 3 |
| ICP4.2 SEQ ID NO: 2 | gD2ΔTMR SEQ ID NO: 4 |
| ICP4.2 SEQ ID NO: 2 | gD2 SEQ ID NO: 5 |
| gL2 SEQ ID NO: 3 | gD2ΔTMR SEQ ID NO: 4 |
| gL2 SEQ ID NO: 3 | gD2 SEQ ID NO: 5 |

| **Three antigen combinations** | | |
|---|---|---|
| ICP4 SEQ ID NO: 1 | gL2 SEQ ID NO: 3 | gD2ΔTMR SEQ ID NO: 4 |
| ICP4.2 SEQ ID NO: 2 | gL2 SEQ ID NO: 3 | gD2ΔTMR SEQ ID NO: 4 |
| ICP4 SEQ ID NO: 1 | gL2 SEQ ID NO: 3 | gD2 SEQ ID NO: 5 |
| ICP4.2 SEQ ID NO: 2 | gL2 SEQ ID NO: 3 | gD2 SEQ ID NO: 5 |

The individual antigens and combinations described above can also include additional peptides from or derived from HSV-2, such as polypeptides comprising sequences selected from SEQ ID NO:6-26 or immunogenic fragments thereof.

### 1. ICP4 (SEQ ID NO: 1) encoded by RS1

RS1 encodes ICP4, a transcriptional transactivator that may interact with and recruit specific components of the general transcription machinery to viral promoters and stabilize their formation for transcription initiation. ICP4 contains distinct domains for transactivation/phosphorylation (approximately spanning acid residues 150-200 of SEQ ID NO:1), DNA binding (approximately spanning residues 380-540 of SEQ ID NO:1), nuclear localization (approximately spanning residues 630-730 of SEQ ID NO:1), and late regulatory transactivation (approximately spanning residues 1220-1319 of SEQ ID NO:1). The DNA and protein sequence of RS1 may be found by searching for RS1 in the publicly available database, Entrez Gene (on the NCBI NIH web site on the World Wide Web, at www.ncbi.nlm.nih.gov/sites/entrez?db=gene), in the Human herpesvirus 2 complete genome.

In some embodiments, vaccines against HSV-2 include a polypeptide containing at least 20 consecutive amino acid residues selected from residues 383-766 of ICP4 (SEQ ID NO: 1), but no more than 1000 amino acids of ICP4 (SEQ ID NO: 1). The polypeptide may also be a variant of the at least 20 residue fragment.

In certain embodiments, the polypeptide includes no more than 950, 900, 850, 800, 750, 700, 650, 600, 550, 500, 450 or even 400 consecutive amino acids from ICP4. Exemplary polypeptides correspond approximately to amino acids residues of full-length ICP4 as follows: 383-766 (RS1.2); 1-400 (RS1.1); 750-1024 (RS1.3.1); 1008-1319 (RS1.3.2); 750-1319 (RS1.3); 280-785 (RS1.4 comprising the full DNA binding region); 680-1319 (RS1.5 comprising the glycosylase/C-terminal region); 208-1319 (RS1.6 which may also comprise a Met residue at the N-term end); 1-380 plus 545-1319 (RS1.7, in which a region spanning approximately residues 381-544 is deleted, removing the DNA binding regions); 1-785 plus 870-1319 (RS1.8, in which a region spanning approximately residues 786-869 is deleted, removing the nuclear localization domain), or 1-766, 383-1318, 100-750, 400-1300, 250-766, 383-900 of ICP4 (SEQ ID NO.1) and the like.

### 2. ICP4 internal fragment ICP4.2 (SEQ ID NO: 2) encoded by RS1.2

RS1.2 encodes a 384 amino acid fragment corresponding to residues 383-766 of ICP4, (or nucleotides 1150-2398 of the RS1 sequence), denoted ICP4.2. The DNA and protein sequence of RS1.2 may be found by searching for RS1 in the publicly available database, Entrez Gene (on the NCBI NIH web site on the World Wide Web, at www.ncbi.nlm.nih.gov/sites/entrez?db=gene), in the Human herpesvirus 2 complete genome.

In specific embodiments, vaccines against HSV-2 include a polypeptide containing from 50 to all 384 amino acids residues of ICP4.2 (SEQ ID NO: 2), such as from 100 to 384, 200 to 384 or 250 to 350 residues. In particular embodiments, the polypeptide includes all of ICP4.2 (SEQ ID NO: 2) or is ICP4.2 (SEQ ID NO: 2) itself. These polypeptides may, for example, include the full length or fragments of ICP4.2 (SEQ ID NO:2) described herein with amino acids residues 1-382 or 767-1318 of ICP4 (SEQ ID NO. 1) or fragments thereof, which, in certain embodiments, are consecutive with the amino acid residues of ICP4.2 being used. Exemplary fragments that combine the residues of SEQ ID NO:2 with select residues from 1-382 or 767-1318 of SEQ ID NO:1 are described above.

An immunogenic fragment of ICP4.2 comprises at least one immunogenic portion, as measured experimentally or identified by algorithm. Peptides identified by such methods include the following:
GLAHVAAAV (SEQ ID NO:47)
FISGSVARA (SEQ ID NO:48)
QYALITRLL (SEQ ID NO:49)
RYDRAQKGF (SEQ ID NO:50)
GYAMAAGRF (SEQ ID NO:51)
PPHADAPRL (SEQ ID NO:52)
KPAAAAAPL (SEQ ID NO:53)
SEAAVAAV (SEQ ID NO:54)
FGWGLAHV (SEQ ID NO:55)
YALITRLLY (SEQ ID NO:56)
ALPRSPRLL (SEQ ID NO:57)
DLLFQNQSL (SEQ ID NO:58)
ADLLFQNQS (SEQ ID NO:59)
ARNSSSFIS (SEQ ID NO:60)
QACFRISGA (SEQ ID NO:61)
FVRDALVLM (SEQ ID NO:62)
FDGDLAAVP (SEQ ID NO:63)
GLGDSRPGL (SEQ ID NO:64)
WAPELGDAA (SEQ ID NO:65)
ECLAACRGI (SEQ ID NO:66)
RAWLRELRF (SEQ ID NO:67).

Thus, in some aspects, this application provides an immunogenic fragment of ICP4.2. The fragments, in some instances, are close in size to the full-length polypeptide. For example, they may lack at most one, two, three, four, five, ten, or twenty amino acids from one or both termini. In other embodiments, the fragment is 100-384 amino acids in length, or 150-384, or 200-384, or 250-384 amino acids in length. Other exemplary fragments are amino acid residues 1-350, 1-300, 1-250, 1-200, 1-150, 1-100, 1-50, 50-384, 50-350, 50-300, 50-250, 50-200, 50-150, 50-100, 100-384, 100-350, 100-300, 100-250, 100-200, 100-150, 150-383, 150-350, 150-300, 150-250, 150-200, 200-383, 200-350, 200-300, 200-250, 250-383, 250-350, 250-300, 300-383 and 350-383. The fragments described above or sub-fragments thereof (e.g., fragments of 8-50, 8-30, or 8-20 amino acid residues) preferably have one of the biological activities described below, such as increasing the T cell response by at least 1.5 fold or 2 fold. A fragment may be used as the polypeptide in the vaccines described herein or may be fused to another protein, protein fragment or a polypeptide.

In certain aspects, this application provides immunogenic polypeptides with at least 90%, 95%, 97%, 98%, 99%, or 99.5% identity to ICP4.2 or an immunogenic fragment thereof.

### 3. Glycoprotein L-2 (SEQ ID NO: 3) encoded by UL1

UL1 encodes Glycoprotein L-2 (gL2), a heterodimer glycoprotein that is required for the fusion of viral and cellular membranes and enables the virus to enter the host cell. The DNA and protein sequence of UL1 may be found by searching in the publicly available database, Entrez Gene (on the NCBI NIH web site on the World Wide Web, at ww.ncbi.nlm.nih.gov/sites/entrez?db=gene), in the Human herpesvirus 2 complete genome.

In some embodiments, vaccines against HSV-2 include a polypeptide containing at least 20 consecutive amino acid residues selected from residues 1-224 of gL2 (SEQ ID NO: 3), but no more than 224 amino acids of gL2 (SEQ ID NO: 3). The polypeptide may also be a variant of the at least 20 residue fragment.

In some embodiments, the polypeptide is at least 85% identical to a fragment of 200-250 amino acids of SEQ ID NO: 3.

In certain embodiments, the polypeptide includes no more than 200 or 100 consecutive amino acids from gL2. Exemplary polypeptides are amino acids residues 1-20, 21-40, 41-60, of 61-80, 81-100, 101-120, 121-140, 141-160, 161-180, 181-200, 201-221 of gL2 (SEQ ID NO. 3) and the like.

In other aspects, this application provides an immunogenic fragment of gL2. An immunogenic fragment of gL2 comprises at least one immunogenic portion, as measured experimentally or identified by algorithm. Peptides identified by such methods include the following:
AYLVNPFLF (SEQ ID NO: 100)
PFLFAAGFL (SEQ ID NO: 101)
TEYVLRSVI (SEQ ID NO: 102)
GSQATEYVL (SEQ ID NO: 103)
RIDGIFLRY (SEQ ID NO: 104)
FLEDLSHSV (SEQ ID NO: 105)
YVLRSVIAK (SEQ ID NO: 106)
YVLRSVIAK (SEQ ID NO: 107)
AYLVNPFLF (SEQ ID NO: 108)
ETTTRRALY (SEQ ID NO: 109)
RIDGIFLRY (SEQ ID NO: 110)
YLVNPFLFA (SEQ ID NO: 111)
FVCLFGLVV (SEQ ID NO: 112)
LYKEIRDAL (SEQ ID NO: 113)
GLDTFLWDR (SEQ ID NO: 114)
RVSPTRGRR (SEQ ID NO: 115)
YVLRSVIAK (SEQ ID NO: 115)
GLDTFLWDR (SEQ ID NO: 116)
DILRVPCMR (SEQ ID NO: 117)
DRHAQRAYL (SEQ ID NO: 118)

### 4. Glycoprotein D-2 (SEQ ID NO:118)encoded by US6 and internally-deleted Glycoprotein D-2 (SEQ ID NO:4) encoded by US6ΔTMR

US6 encodes envelope glycoprotein D-2 (gD2), an envelope glycoprotein that binds to host cell entry receptors and may trigger fusion of the virus with the host membrane. The gD2 protein has several distinct domains, including a signal domain (amino acid residues 1-25) which is cleaved from the mature protein, and a transmembrane domain (spanning approximately amino acids residues 340-363). The DNA and protein sequence of US6 may be found by searching in the publicly available database, Entrez Gene (on the NCBI NIH web site on the World Wide Web, at www.ncbi.nlm.nih.gov/sites/entrez?db=gene), in the Human herpesvirus 2 complete genome.

In some embodiments, vaccines against HSV-2 include a polypeptide comprising gD2 that is missing all or part of the transmembrane domain (which spans approximately amino acids residues 340-363 inclusive) as well as the signal sequence. In other embodiments, the deleted region may additionally include 5-10 amino acids of the sequence flanking the transmembrane domain. The deleted region may also comprise a portion of the transmembrane domain, for example at least 3 amino acids between residues 340-363. In some embodiments, at least one residue in the transmembrane domain has been modified, deleted or substituted, such that the transmembrane domain is no longer functional. For example, a variant may have its internal deletion begin at amino acid residue 336, 337, 338, 339, 340, 341, 342, 343, 344, 345 or 346 and end at amino acid residue 358, 359, 360, 361, 362, 363, 364, 365, 366, 367 or 368.

A construct encoding gD2 which is missing amino acid residues 341-363 (the transmembrane domain) is called US6ΔTMR (SEQ ID NO: 40). The corresponding protein is denoted gD2ΔTMR (SEQ ID NO:4). In other embodiments, an immunogenic fragment of gD2 or gD2ΔTMR may comprise a deletion in a portion of the transmembrane domain, and/or may comprise a deletion in the flanking sequence outside of the transmembrane domain.

In other aspects, this application provides an immunogenic fragment of gD2 or gD2ΔTMR. An immunogenic fragment of gD2 or gDΔTMR comprises at least one immunogenic portion, as measured experimentally or identified by algorithm. Peptides identified by such methods include the following:
ALAGSTLAV (SEQ ID NO.68)
LLEDPAGTV (SEQ ID NO.69)
VIGGIAFWV (SEQ ID NO.70)
TVYYAVLER (SEQ ID NO.71)
KYALADPSL (SEQ ID NO.72)
AFETAGTYL (SEQ ID NO.73)
APSNPGLII (SEQ ID NO.74)
IPITVYYAV (SEQ ID NO.75)
APPSHQPLF (SEQ ID NO.76)
FLMHAPAFE (SEQ ID NO.77)
FSAVSEDNL (SEQ ID NO.78)
VYYAVLER (SEQ ID NO.79)
IGMLPRFI (SEQ ID NO.80)
YTECPYNKS (SEQ ID NO.81)
FLMHAPAFE (SEQ ID NO.82)
NLGFLMHAP (SEQ ID NO.83)
VIGGIAFWV (SEQ ID NO.84)
GIAFWVRRR (SEQ ID NO.85)
SEDNLGFLM (SEQ ID NO.86)
RTQPRWSYY (SEQ ID NO.87)
IAFWVRRRA (SEQ ID NO.88)
LVIGGIAFW (SEQ ID NO.89)
FWVRRRAQM (SEQ ID NO.90)
PYTSTLLPP (SEQ ID NO.91)
VGTAALLVV (SEQ ID NO.92)
TAALLVVAV (SEQ ID NO.93)
TSTLLPPEL (SEQ ID NO.94)
GTVSSQIPP (SEQ ID NO.95)
TAGTYLRLV (SEQ ID NO.96)
GVTVDSIGM (SEQ ID NO.97)
AFWVRRRAQ (SEQ ID NO.98)
RVYHIQPSL (SEQ ID NO.99)

Thus, in some aspects, this application provides an immunogenic fragment of gD2 (SEQ ID NO:5) or gDΔTMR (SEQ ID NO: 4). The fragments, in some instances, are close in size to the full-length polypeptide. For example, they may lack at most one, two, three, four, five, ten, or twenty amino acids from one or both termini. In other embodiments, the fragment is 100-384 amino acids in length, or 150-384, or 200-384, or 250-384 amino acids in length. Other exemplary fragments are amino acid residues 1-350, 1-300, 1-250, 1-200, 1-150, 1-100, 1-50, 50-384, 50-350, 50-300, 50-250, 50-200, 50-150, 50-100, 100-384, 100-350, 100-300, 100-250, 100-200, 100-150, 150-383, 150-350, 150-300, 150-250, 150-200, 200-383, 200-350, 200-300, 200-250, 250-383, 250-350, 250-300, 300-383 and 350-383. The fragments described above or sub-fragments thereof (e.g., fragments of 8-50, 8-30, or 8-20 amino acid residues) preferably have one of the biological activities described below, such as increasing the T cell response by at least 1.5 fold or 2 fold. A fragment may be used as the polypeptide in the vaccines described herein or may be fused to another protein, protein fragment or a polypeptide.

In other embodiments, the polypeptide comprises the entire sequence of SEQ ID NO: 4 or SEQ ID NO:5, or consists of the entire sequence of SEQ ID NO: 4 or SEQ ID NO:5. In certain embodiments, an immunogenic fragment of gD2 retains all or part of the signal domain (amino acid residues 1-25) and/or the transmembrane domain (amino acids residues 339-363).

In certain embodiments, polypeptides have less than 20%, 30%, 40%, 50%, 60% or 70% homology with human autoantigens. Examples of such autoantigens include UL6 from HSV-1 and gK or UL53 from HSV-2.

In certain aspects, this application provides immunogenic polypeptides with at least 90%, 95%, 97%, 98%, 99%, or 99.5% identity to gDΔTMR, or an immunogenic fragment thereof.

### C. Additional features of HSV-2 polypeptides

Typically, the polypeptides present in the vaccine formulations or pharmaceutical compositions described herein are immunogenic, either alone or as a variant, which includes polypeptides fused to another polypeptide or mixed with or complexed to an adjuvant. Variants also include sequences with less than 100% sequence identity, as described herein. In addition, one may use fragments, precursors and analogs that have an appropriate immunogenicity.

These polypeptides may be immunogenic in mammals, for example, mice, guinea pigs, or humans. An immunogenic polypeptide is typically one capable of raising a significant immune response in an assay or in a subject. Alternatively, an immunogenic polypeptide may (i) induce production of antibodies, e.g., neutralizing antibodies, that bind to the polypeptide (ii) induce T_{H}1 immunity, (iii) activate the CD8+ CTL response, for example by increasing CD8+ T cells and/or increasing localization of CD8+ T cells to the site of infection or reinfection, (iv) induce T_{H}17 immunity, and/or (v) activate innate immunity. In some embodiments, an immunogenic polypeptide causes the production of a detectable amount of antibody specific to that antigen.

In certain embodiments, polypeptides have less than 20%, 30%, 40%, 50%, 60% or 70% homology with human autoantigens.

A polypeptide may comprise one or more immunogenic portions and one or more non-immunogenic portions. The immunogenic portions may be identified by various methods, including protein microarrays, ELISPOT/ELISA techniques, and/or specific assays on different deletion mutants (e.g., fragments) of the polypeptide in question. Immunogenic portions may also be identified by computer algorithms. Some such algorithms, like EpiMatrix (produced by EpiVax), use a computational matrix approach. Other computational tools for identifying antigenic epitopes include PEPVAC (Promiscuous EPitope-based VACcine, hosted by Dana Farber Cancer Institute on the world wide web at immunax.dfci.harvard.edu/PEPVAC), MHCPred (which uses a partial least squares approach and is hosted by The Jenner Institute on the world wide web at www.jenner.ac.uk/MHCPred), and Syfpeithi, hosted on the world wide web at www.syfpeithi.de/.

In some embodiments, the vaccine or pharmaceutical composition may comprise fusion proteins and/or fusion DNA constructs. The underlying DNA sequences above may be modified in ways that do not affect the sequence of the protein product. For instance, the DNA sequence may be codon-optimized to improve expression in a host such as *E. coli* or an insect cell line (e.g. using the baculovirus expression system) or mammalian (e.g. Chinese Hamster Ovary) cell line. In particular embodiments, such as when smaller related polypeptides, including those having a molecular weight less than about 5000 daltons, e.g., 1500 to 5000 daltons, are used, modification may be useful in eliciting the desired immune response. For example, the smaller polypeptides can be conjugated to an appropriate immunogenic carrier such as proteins from other pathogenic organisms or viruses (e.g.,tetanus toxoid), large proteins (e.g., keyhole limpet hemocyanin) or the like. Conjugation may be direct or indirect (e.g., via a linker). In other particular embodiments, a fusion protein may comprise a polypeptide disclosed above or an immunogenic fragment or variant thereof and a tag. A tag may be N-terminal or C-terminal. For instance, tags may be added to the nucleic acid or polypeptide to facilitate purification, detection, solubility, or confer other desirable characteristics on the protein or nucleic acid. For instance, a purification tag may be a peptide, oligopeptide, or polypeptide that may be used in affinity purification. Examples include His, GST, TAP, FLAG, myc, HA, MBP, VSV-G, thioredoxin, V5, avidin, streptavidin, BCCP, Calmodulin, Nus, S tags, lipoprotein D, and β galactosidase. In some embodiments, the fused portion is short. Thus, in some instances, the fusion protein comprises no more than 1, 2, 3, 4, 5, 10, 20, or 50 additional amino acids on one or both termini of a polypeptide described above, such as consecutive amino acids from any of the polypeptides in Table 1.

In some embodiments, tags, secretion signals, or other signal sequences may be added to the C-terminal end and/or to the N-terminal end of the polypeptide. Tags may be used to aid in purification of expresssed polypeptides. Exemplary tags include HHHHHH (SEQ ID NO: 130) and MSYYHHHHHH (SEQ ID NO: 131). Secretion signals may be optimized for use with non-mammalian cells, such as insect cells. An exemplary secretion signal is MKFLVNVALVFMVVYISYIYA (SEQ ID NO: 132).

A detection tag may be used to detect the tag and, consequently, any amino acid sequence fused to it. Detection tags include fluorescent proteins, proteins that bind a fluorescent label, and proteins that bind an electron-dense moeity. Examples of fluorescent proteins include dsRed, mRFP, YFP, GFP, CFP, BFP, and Venus. An example of a protein that binds a fluorescent or electron-dense label is FlAsH.

Another aspect disclosed herein is an antibody preparation generated against a composition of the invention (e.g., a composition comprising one or more or two or more of the polypeptides listed in Table 1). Any of a variety of antibodies are included. Such antibodies include, e.g., polyclonal, monoclonal, recombinant, humanized or partially humanized, single chain, Fab, and fragments thereof, etc. The antibodies can be of any isotype, e.g., IgA, IgG, various IgG isotypes such as IgGi, IgG₂, IgG₂ₐ, IgG_{2b}, IgG₃, IgG₄, etc.; and they can be from any animal species that produces antibodies, including goat, rabbit, mouse, chicken or the like. In some embodiments, Fab molecules are expressed and assembled in a genetically transformed host like *E. coli.* A lambda vector system is available thus to express a population of Fab's with a potential diversity equal to or exceeding that of subject generating the predecessor antibody. See Huse et al. (1989), Science 246, 1275-81.

### D. Components of vaccines and pharmaceutical compositions

In certain embodiments, the vaccines and pharmaceutical compositions comprise one or more of the polypeptides and nucleic acids described above and one or more of the following: an adjuvant, stabilizer, buffer, surfactant, controlled release component, salt, preservative, and an antibody specific to said antigen.

### 1. Adjuvants

The vaccine formulations and pharmaceutical compositions described herein may each include an adjuvant. Adjuvants can be broadly separated into two classes, based on their principal mechanisms of action: vaccine delivery systems and immunostimulatory adjuvants (see, *e.g.,* Singh et al., Curr. HIV Res. 1:309-20, 2003). Vaccine delivery systems are often particulate formulations, e.g., emulsions, microparticles, immune-stimulating complexes (ISCOMs), which may be, for example, particles and/or matrices, and liposomes. In contrast, immunostimulatory adjuvants are sometimes derived from pathogens and can represent pathogen associated molecular patterns (PAMP), *e.g*., lipopolysaccharides (LPS), monophosphoryl lipid (MPL), or CpG-containing DNA, which activate cells of the innate immune system.

Alternatively, adjuvants may be classified as organic and inorganic. Inorganic adjuvants include alum salts such as aluminum phosphate, amorphous aluminum hydroxyphosphate sulfate, and aluminum hydroxide, which are commonly used in human vaccines. Organic adjuvants comprise organic molecules including macromolecules. An example of an organic adjuvant is cholera toxin.

Adjuvants may also be classified by the response they induce, and adjuvants can activate more than one type of response. In some embodiments, the adjuvant induces the activation of CD4+ T cells. The adjuvant may induce activation of T_{H}1 cells and/or activation of T_{H}17 cells and/or activation of T_{H}2 cells. Alternately, the adjuvant may induce activation of T_{H}1 cells and/or T_{H}17 cells but not activation of T_{H}2 cells, or vice versa. In some embodiments, the adjuvant induces activation of CD8+ T cells. In further embodiments, the adjuvant may induce activation of Natural Killer T (NKT) cells. In some embodiments, the adjuvant induces the activation of T_{H}1 cells or T_{H}17 cells or T_{H}2 cells. In other embodiments, the adjuvant induces the activation of B cells. In yet other embodiments, the adjuvant induces the activation of antigen-presenting cells. These categories are not mutually exclusive; in some cases, an adjuvant activates more than one type of cell.

In certain embodiments, an adjuvant is a substance that increases the numbers or activity of antigen presenting cells such as dendritic cells. In certain embodiments, an adjuvant promotes the maturation of antigen presenting cells such as dendritic cells. In some embodiments, the adjuvant is or comprises a saponin. Typically, the saponin is a triterpene glycoside, such as those isolated from the bark of the Quillaja saponaria tree. A saponin extract from a biological source can be further fractionated (e.g., by chromatography) to isolate the portions of the extract with the best adjuvant activity and with acceptable toxicity. Typical fractions of extract from Quillaja saponaria tree used as adjuvants are known as fractions A and C. An exemplary saponin adjuvant is QS-21, which is available from Antigenics. QS-21 is an oligosaccharide-conjugated small molecule. Optionally, QS-21 may be admixed with a lipid such as 3D-MPL or cholesterol.

A particular form of saponins that may be used in vaccine formulations described herein is immunostimulating complexes (ISCOMs). ISCOMs are an art-recognized class of adjuvants, that generally comprise Quillaja saponin fractions and lipids (e.g., cholesterol and phospholipids such as phosphatidyl choline). In certain embodiments, an ISCOM is assembled together with a polypeptide or nucleic acid of interest. However, different saponin fractions may be used in different ratios. In addition, the different saponin fractions may either exist together in the same particles or have substantially only one fraction per particle (such that the indicated ratio of fractions A and C are generated by mixing together particles with the different fractions). In this context, "substantially" refers to less than 20%, 15%, 10%, 5%, 4%, 3%, 2% or even 1%. Such adjuvants may comprise fraction A and fraction C mixed into a ratio of 70-95 A: 30-5 C, such as 70 A : 30 C to 75 A : 5 C, 75 A : 5 C to 80 A : 20 C, 80 A : 20 C to 85 A : 15 C, 85 A : 15 C to 90 A : 10 C, 90 A : 10 C to 95 A : 5 C, or 95 A : 5 C to 99 A : 1 C. ISCOMatrix, produced by CSL, and AbISCO 100 and 300, produced by Isconova, are ISCOM matrices comprising saponin, cholesterol and phospholipid (lipids from cell membranes), which form cage-like structures typically 40-50 nm in diameter. Posintro, produced by Nordic Vaccines, is an ISCOM matrix where the immunogen is bound to the particle by a multitude of different mechanisms, e.g. electrostatic interaction by charge modification, incorporation of chelating groups or direct binding.

In some embodiments, the adjuvant is a TLR ligand. TLRs are proteins that may be found on leukocyte membranes, and recognize foreign antigens (including microbial antigens). An exemplary TLR ligand is IC-31, which is available from Intercell. IC31 comprises an anti-microbial peptide, KLK, and an immunostimulatory oligodeoxynucleotide, ODN1a. IC31 has TLR9 agonist activity. Another example is CpG-containing DNA, and different varieties of CpG-containing DNA are available from Prizer (Coley): VaxImmune is CpG 7909 (a (CpG)-containing oligodeoxy-nucleotide), and Actilon is TLR9 agonist, CpG 10101 (a (CpG)-containing oligodeoxy-nucleotide).

In some embodiments, the adjuvant is a nanoemulsion. One exemplary nanoemulsion adjuvant is Nanostat Vaccine, produced by Nanobio. This nanoemulsion is a high-energy, oil-in-water emulsion. This nanoemulsion typically has a size of 150-400 nanometers, and includes surfactants to provide stability. More information about Nanostat can be found in US Patents 6,015,832, 6,506,803, 6,559,189, 6,635,676, and 7,314,624.

Adjuvants may be covalently bound to antigens (e.g., the polypeptides described above). In some embodiments, the adjuvant may be a protein which induces inflammatory responses through activation of antigen-presenting cells (APCs). In some embodiments, one or more of these proteins can be recombinantly fused with an antigen of choice, such that the resultant fusion molecule promotes dendritic cell maturation, activates dendritic cells to produce cytokines and chemokines, and ultimately, enhances presentation of the antigen to T cells and initiation of T cell responses (see Wu et al., Cancer Res 2005; 65(11), pp 4947-4954). Other exemplary adjuvants that may be covalently bound to antigens comprise polysaccharides, synthetic peptides, lipopeptides, and nucleic acids.

The adjuvant can be used alone or in combination of two or more kinds. Adjuvants may be directly conjugated to antigens. Adjuvants may also be combined to increase the magnitude of the immune response to the antigen. Typically, the same adjuvant or mixture of adjuvants is present in each dose of a vaccine. Optionally, however, an adjuvant may be administered with the first dose of vaccine and not with subsequent doses (i.e. booster shots). Alternatively, a strong adjuvant may be administered with the first dose of vaccine and a weaker adjuvant or lower dose of the strong adjuvant may be administered with subsequent doses. The adjuvant can be administered before the administration of the antigen, concurrent with the administration of the antigen or after the administration of the antigen to a subject (sometimes within 1, 2, 6, or 12 hours, and sometimes within 1, 2, or 5 days). Certain adjuvants are appropriate for human patients, non-human animals, or both.

### 2. Additional components of vaccines and pharmaceutical compositions

In addition to the antigens and the adjuvants described above, a vaccine formulation or pharmaceutical composition may include one or more additional components.

In certain embodiments, the vaccine formulation or pharmaceutical composition may include one or more stabilizers such as sugars (such as sucrose, glucose, or fructose), phosphate (such as sodium phosphate dibasic, potassium phosphate monobasic, dibasic potassium phosphate, or monosodium phosphate), glutamate (such as monosodium L-glutamate), gelatin (such as processed gelatin, hydrolyzed gelatin, or porcine gelatin), amino acids (such as arginine, asparagine, histidine, L-histidine, alanine, valine, leucine, isoleucine, serine, threonine, lysine, phenylalanine, tyrosine, and the alkyl esters thereof), inosine, or sodium borate.

In certain embodiments, the vaccine formulation or pharmaceutical composition includes one or more buffers such as a mixture of sodium bicarbonate and ascorbic acid. In some embodiments, the vaccine formulation may be administered in saline, such as phosphate buffered saline (PBS), or distilled water.

In certain embodiments, the vaccine formulation or pharmaceutical composition includes one or more surfactants such as polysorbate 80 (Tween 80), Triton X-100, Polyethylene glycol tert-octylphenyl ether t-Octylphenoxypolyethoxyethanol 4-(1,1,3,3-Tetramethylbutyl)phenyl-polyethylene glycol (TRITON X-100); Polyoxyethylenesorbitan monolaurate Polyethylene glycol sorbitan monolaurate (TWEEN 20); and 4-(1,1,3,3-Tetramethylbutyl)phenol polymer with formaldehyde and oxirane (TYLOXAPOL). A surfactant can be ionic or nonionic.

In certain embodiments, the vaccine formulation or pharmaceutical composition includes one or more salts such as sodium chloride, ammonium chloride, calcium chloride, or potassium chloride.

In certain embodiments, a preservative is included in the vaccine. In other embodiments, no preservative is used. A preservative is most often used in multi-dose vaccine vials, and is less often needed in single-dose vaccine vials. In certain embodiments, the preservative is 2-phenoxyethanol, methyl and propyl parabens, benzyl alcohol, and/or sorbic acid.

In certain embodiments, the vaccine formulation or pharmaceutical composition is a controlled release formulation.

### E. DNA vaccines

In certain aspects, the vaccine comprises one of the nucleic acids disclosed herein. When a nucleic acid vaccine is administered to a patient, the corresponding gene product (such as a desired antigen) is produced in the patient's body. In some embodiments, nucleic acid vaccine vectors that include optimized recombinant polynucleotides can be delivered to a mammal (including humans) to induce a therapeutic or prophylactic immune response. The nucleic acid may be, for example, DNA, RNA, or a synthetic nucleic acid. The nucleic acid may be single stranded or double stranded.

Nucleic acid vaccine vectors (e.g., adenoviruses, liposomes, papillomaviruses, retroviruses, etc.) can be administered directly to the mammal for transduction of cells *in vivo.* The nucleic acid vaccines can be formulated as pharmaceutical compositions for administration in any suitable manner, including parenteral administration.

In determining the effective amount of the vector to be administered in the treatment or prophylaxis of an infection or other condition, the physician evaluates vector toxicities, progression of the disease, and the production of anti-vector antibodies, if any. Often, the dose equivalent of a naked nucleic acid from a vector is from about 1 µg to 1 mg for a typical 70 kilogram patient, and doses of vectors used to deliver the nucleic acid are calculated to yield an equivalent amount of therapeutic nucleic acid. Administration can be accomplished via single or divided doses. The toxicity and therapeutic efficacy of the nucleic acid vaccine vectors can be determined using standard pharmaceutical procedures in cell cultures or experimental animals.

A nucleic acid vaccine can contain DNA, RNA, a modified nucleic acid, or a combination thereof. In some embodiments, the vaccine comprises one or more cloning or expression vectors; for instance, the vaccine may comprise a plurality of expression vectors each capable of autonomous expression of a nucleotide coding region in a mammalian cell to produce at least one immunogenic polypeptide. An expression vector often includes a eukaryotic promoter sequence, such as the nucleotide sequence of a strong eukaryotic promoter, operably linked to one or more coding regions. The compositions and methods herein may involve the use of any particular eukaryotic promoter, and a wide variety are known; such as a CMV or RSV promoter. The promoter can be, but need not be, heterologous with respect to the host cell. The promoter used may be a constitutive promoter.

A vector useful in the present compositions and methods can be circular or linear, single-stranded or double stranded and can be a plasmid, cosmid, or episome. In a suitable embodiment, each nucleotide coding region is on a separate vector; however, it is to be understood that one or more coding regions can be present on a single vector, and these coding regions can be under the control of a single or multiple promoters.

Numerous plasmids may be used for the production of nucleic acid vaccines. Suitable embodiments of the nucleic acid vaccine employ constructs using the plasmids VR1012 (Vical Inc., San Diego Calif.), pCMVI.UBF3/2 (S. Johnston, University of Texas) or pcDNA3.1 (InVitrogen Corporation, Carlsbad, Calif.) as the vector. In addition, the vector construct can contain immunostimulatory sequences (ISS), such as unmethylated dCpG motifs, that stimulate the animal's immune system. The nucleic acid vaccine can also encode a fusion product containing the immunogenic polypeptide. Plasmid DNA can also be delivered using attenuated bacteria as delivery system, a method that is suitable for DNA vaccines that are administered orally. Bacteria are transformed with an independently replicating plasmid, which becomes released into the host cell cytoplasm following the death of the attenuated bacterium in the host cell.

An alternative approach to delivering the nucleic acid to an animal involves the use of a viral or bacterial vector. Examples of suitable viral vectors include adenovirus, polio virus, pox viruses such as alphaviruses, vaccinia, canary pox, and fowl pox, herpes viruses, including catfish herpes virus, adenovirus-associated vector, and retroviruses. Virus-like vectors include virosomes and virus-like particles. Exemplary bacterial vectors include attenuated forms of Salmonella, Shigella, Edwardsiella ictaluri, Yersinia ruckerii, and Listeria monocytogenes. In some embodiments, the nucleic acid is a vector, such as a plasmid, that is capable of autologous expression of the nucleotide sequence encoding the immunogenic polypeptide.

### F. Use of Vaccines

The vaccines described herein may be used for prophylactic and/or therapeutic treatment of herpes, including HSV-1 and particularly HSV-2. The subject receiving the vaccination may be a male or a female, and may be a child or adult. In some embodiments, the subject being treated is a human. In other embodiments, the subject is a non-human animal.

### 1. Prophylactic use

In prophylactic embodiments, the HSV-2 vaccine is administered to a subject to induce an immune response that can help protect against the establishment of HSV-2.

In some embodiments, the vaccine compositions of the invention confer protective immunity, allowing a vaccinated individual to exhibit delayed onset of symptoms or reduced severity of symptoms (e.g., reduced number of lesions at the onset of infection), as the result of his/her exposure to the vaccine (e.g., a memory response). In certain embodiments, the reduction in severity of symptoms is at least 25%, 40%, 50%, 60%, 70%, 80% or even 90%. Some vaccinated individuals may display no symptoms upon contact with HSV-2 or even no infection by HSV-2. Protective immunity is typically achieved by one or more of the following mechanisms: mucosal, humoral, or cellular immunity. Mucosal immunity is primarily the result of secretory IgA (sIGA) antibodies on mucosal surfaces of the respiratory, gastrointestinal, and genitourinary tracts. The sIGA antibodies are generated after a series of events mediated by antigen-processing cells, B and T lymphocytes, that result in sIGA production by B lymphocytes on mucosa-lined tissues of the body. Humoral immunity is typically the result of IgG antibodies and IgM antibodies in serum. For example, the IgG titer can be raised by 1.5-fold, 2-fold, 3-fold, 4-fold, 5-fold, 10-fold, 20-fold, 50-fold, or even 100-fold or more following administration of a vaccine formulation described herein. Cellular immunity can be achieved through cytotoxic T lymphocytes or through delayed-type hypersensitivity that involves macrophages and T lymphocytes, as well as other mechanisms involving T cells without a requirement for antibodies. In particular, cellular immunity may be mediated by T_{H}1 cells or T_{H}17 cells. Activation of T_{H}1 cells can be measured by secretion of IFN-γ, relative to the level of IFN-γ released in response to a polypeptide that does not generate an immunologic response. In certain embodiments, the amount of IFN-γ released in 1.5-fold, 2-fold, 3-fold, 4-fold, 5-fold, 10-fold, 20-fold, 50-fold or even 100-fold greater. The primary result of protective immunity is the destruction of HSV-2 viral particles or inhibition of HSV-2's ability to replicate. In some embodiments, the protective immunity conferred by presentation of antigen before exposure to HSV-2 will reduce the likelihood of seroconversion to an HSV-2-positive status.

The duration of protective immunity is preferably as long as possible. In certain embodiments, vaccine formulations produce protective immunity lasting six months, one year, two years, five years, ten years, twenty years or even a lifetime.

### 2. Therapeutic use

In therapeutic applications, the vaccine comprising a polypeptide or nucleic acid of the invention may be administered to a patient suffering from HSV-2, in an amount sufficient to treat the patient. Treating the patient, in this case, may refer to delaying or reducing symptoms of HSV-2 in an infected individual. In some embodiments, treating the patient refers to reducing the duration of lesions, reducing the number of lesions, reducing the duration of symptoms per episode, and/or otherwise reducing the intensity of symptoms per episode. In certain embodiments, the vaccine reduces the duration or severity of mild symptoms; in some embodiments, the vaccine reduces the duration or severity of serious symptoms. In some embodiments, the vaccine reduces viral shedding and therefore the transmissibility of HSV-2 from the vaccinated patient. In certain embodiments, the reductions described above are at least 25%, 30%, 40%, 50%, 60%, 70%, 80% or even 90%. In certain embodiments, the reductions described above include the complete cessation of symptoms, viral shedding and/or future outbreaks (e.g., by blocking the ability of the virus to establish latency in sensory ganglia).

In therapeutic embodiments, the HSV-2 vaccine is administered to an individual post-infection. The HSV-2 vaccine may be administered shortly after infection, e.g. before symptoms manifest, or may be administered during or after manifestation of symptoms. In some embodiments, the HSV-2 may prevent endogenous reactivation of earlier infection. In some embodiments, a postinfection vaccine could be administered to patients in high-risk groups.

The duration of therapeutic effects of a vaccine formulation disclosed herein is preferably as long as possible. In certain embodiments, vaccine formulations produce therapeutic effects lasting one month, two months, three months, six months, one year, two years, five years, ten years, twenty years or even a lifetime.

### 3. Assaying vaccination efficacy

The efficacy of vaccination with the vaccines disclosed herein may be determined in a number of ways.

Vaccine efficacy may be assayed in various model systems. Suitable model systems used to study HSV-2 include a guinea pig model and a mouse model, as described in the examples below. Briefly, the animals are vaccinated and then challenged with HSV-2 or the vaccine is administered to already-infected animals. The response of the animals to the HSV-2 challenge or the vaccine is then compared with control animals, using one of the measures described above. A similar assay could be used for clinical testing of humans. The treatment and prophylactic effects described above represent additional ways of determining efficacy of a vaccine.

In addition, efficacy may be evaluated by *in vitro* immunization of naive human peripheral blood mononuclear cells (PBMC), where APCs are exposed to the vaccine and then the APCs are co-cultured with naive T cells from the same donor to evaluate the primary response to immunization in a test tube. An activation of the T-cells by 1.5 fold, 2-fold, 5-fold, 10-fold, 20-fold, 50-fold or 100-fold or more relative to activation of T-cells using APCs not exposed to a vaccine, in certain embodiments, is considered an adequate response.

Vaccine efficacy may further be determined by viral neutralization assays. Briefly, animals are immunized and serum is collected on various days post-immunization. Serial dilutions of serum are pre-incubated with virus during which time antibodies in the serum that are specific for the virus will bind to it. The virus/serum mixture is then added to permissive cells to determine infectivity by a plaque assay. If antibodies in the serum neutralize the virus, there are fewer plaques compared to the control group.

### G. Uses of Pharmaceutical Compositions

### 1. Defense against HSV infection

The pharmaceutical compositions of the present disclosure are designed to elicit an immune response against HSV-2. Compositions described herein may stimulate an innate immune response, an antibody response or a cell-mediated immune response, or a combination of these responses, in the subject to which it is administered. In some embodiments, the composition stimulates immune cells at the peripheral site of infection or sensory ganglia, such as neutrophils, macrophages, and NK cells. The composition may stimulate infiltration by macrophages; production of antiviral compounds, such including nitric oxide, TNF-α, interferons (IFN), and interleukin 12 (IL-12) by neutrophils; and/or stimulation of NK cells to produce IFN-γ. IL-2, IFN-α and IFN-β production may also be triggered by the polypeptides of the present composition, and are believed to aid in controlling infection.

In some embodiments, the composition comprises antigens that stimulate production of neutralizing antibodies. Neutralizing antibodies may target the glycoproteins of the viral envelope, which mediate the interaction of virions with host cell and are responsible for attachment, binding, and entry of HSV-2 into cells. Accordingly, an exemplary composition comprises one or more glycoproteins described above or encoded by nucleic acids described above. Immunogenic antigens and/or epitopes as described herein may be administered separately, in series, or in combination with one another.

In some embodiments, the composition elicits a cell-mediated response, which may involve CD4+ T cells, CD8+ T cells and/or production of antiviral cytokines. The composition may trigger IFN-γ secretion, for example through the activation of the innate immune response, and mediate CD8+ T cell clearing of the virus. IFN-γ is also secreted by T_{H}1 cells, (T_{H}17 cells?) Tc cells, dendritic cells, and NK cells, and the composition may trigger IFN-γ secretion by any of these cell types. Such activity of CD8+ T cells may be cytolytic, or, alternately, may be regulated by inhibitor molecules on the surface of the neurons which prevent neuronal killing. CD4+ and/or CD8+ T cells may play a role in maintaining latency of the virus, thus preventing reactivation. In some embodiments, the composition boosts a CD4+ T cell response and/or a CD8+ T cell response that prevents reactivation of the virus from its latent state.

In some embodiments, the composition blocks the ability of HSV to evade the host immune response, or, alternately, boosts immune responses normally evaded by HSV. In some embodiments, the composition inhibits HSV-2 from shifting the immunological balance towards tolerance of HSV antigens. HSV-2 may mediate tolerance through T_{H}2 cells. First, HSV-2 may induce suppressor T cells, such as CD4+ CD25+ cells and Tr1 cells that secrete IL-10, a T_{H}2 cytokine. T_{H}2 cytokines downregulate costimulatory molecules and inhibit the maturation and function of antigen-presenting dendritic cells. In addition, infection with HSV-2 inhibits the maturation and migration of dendritic cells, which are essential for efficient CTL priming. Notably, T_{H}2 cytokines are produced during recurrence of HSV-2 infection, in contrast to T_{H}1 cytokines, which are produced during recurrence-free episodes. Thus, in certain embodiments, the compositions of the invention repress suppressor T cells and/or induce maturation or migration or both of dendritic cells.

In some embodiments, methods of inducing an immune response against HSV-2 in a mammal comprise administering the compositions described above. The composition may be used to induce an immune response at different time points, such as before exposure to HSV-2, after initial infection with HSV-2, before or after HSV-2 has established latency, before or after HSV-2 shedding occurs, and/or before or after recurrent outbreaks occur. In some embodiments, an immune response against HSV-2 may be induced at one or more of the timepoints above. The composition may induce a T_{H}1 response and/or a T_{H}17 response but not a T_{H}2 response, or may activate the responses at the same time or at different times.

In some embodiments, administration of the composition reduces symptoms associated with initial infection, latency, or recurrent infection with HSV. Such a composition may reduce incidence and/or severity of lesions, sores, pain, irritation, itching, fever, malaise, headache, viral shedding, or prodromes associated with HSV infection or outbreak.

In some embodiments, one or more antibodies to antigens of HSV-2 may be administered to individuals in order to produce passive immunity. Passive immunity results from the transfer of active humoral immunity in the form of ready-made antibodies, from one individual to another. Passive immunization may be used when there is a high risk of infection and insufficient time for the body to develop its own immune response, or to reduce the symptoms of ongoing or immunosuppressive diseases. Adoptive transfer of T cells may provide another method of eliciting an immune response to HSV-2 antigens in patients. In one embodiment, autologous T cells may be expanded on APCs presenting the antigens derived from the polypeptides described above. Subsequently, the expanded HSV-2-specific T cells are transferred back into the patient from which the T cells were derived.

### 2. Diagnostic uses

This application provides, *inter alia,* a rapid, inexpensive, sensitive, and specific method for detection of HSV-2 in patients. In this respect it should be useful to hospitals and physicians examining and treating patients with or at risk for HSV-2 infection. As used herein, "patient" refers to an individual (such as a human) that either has an HSV-2 infection or has the potential to contract an HSV-2 infection.

In some embodiments, one may use an antibody against one of the polypeptides described herein, such as those of Table 1 and/or Table 2, to detect HSV-2 in an individual. The instant disclosure also provides a method of phenotyping biological samples from patients suspected of having a HSV-2 infection that involves: (a) rendering a biological sample amenable to immunoassay, if necessary; (b) contacting the sample with an appropriate HSV-2-specific antibody or antigen-binding portion thereof under conditions that allow for binding of the antibody or antigen-binding portion to an epitope of HSV-2; and (c) determining if the sample shows the presence of HSV-2 as compared to a control tissue; where if the test tissue shows the presence of HSV-2, the patient is identified as likely having a HSV-2 infection.

Alternatively, one may use the polypeptides described above to detect anti-HSV-2 antibodies in an individual. The instant disclosure also provides a method of phenotyping biological samples from patients suspected of having a HSV-2 infection: (a) rendering a biological sample amenable to an affinity assay such as ELISA, if necessary; (b) contacting the sample with a HSV-2-specific antigen or portion thereof under conditions that allow for binding of the antigen to any host antibodies present in the sample; and (c) determining if the sample shows the presence of HSV-2 as compared to a control tissue; wherein if the test tissue shows the presence of HSV-2, the patient is identified as likely having a HSV-2 infection. The aforementioned test may be appropriately adjusted to detect other viral infections, for instance by using a homolog (from another viral species) of the proteins described above, such as in Table 1 and/or Table 2.

A number of methods for measuring antibody-antigen binding are known in the art, including ELISA (enzyme-linked immunosorbent assay), Western blotting, competition assay, and spot-blot. The detection step may be, for instance, chemiluminescent, fluorescent, or colorimetric. One suitable method for measuring antibody-protein binding is the Luminex xMAP system, where peptides are conjugated to a dye-containing microsphere. Certain systems, including the xMAP system, are amenable to measuring several different markers in multiplex, and could be used to measure levels of antibodies at once. In some embodiments, other systems are used to assay a plurality of markers in multiplex. For example, profiling may be performed using any of the following systems: antigen microarrays, bead microarrays, nanobarcodes particle technology, arrayed proteins from cDNA expression libraries, protein in situ array, protein arrays of living transformants, universal protein array, lab-on-a-chip microfluidics, and peptides on pins. Another type of clinical assay is a chemiluminescent assay to detect antibody binding. In some such assays, including the VITROS Eci anti-HCV assay, antibodies are bound to a solid-phase support made up of microparticles in liquid suspension, and a surface fluorometer is used to quantify the enzymatic generation of a fluorescent product.

In other embodiments, one may use the polypeptides described above, such as those of Table 1 and/or Table 2, to detect T cells that are specific to HSV-2. The instant disclosure provides a method of phentoyping biological samples from patients suspected of having a HSV-2 infection, involving (a) rendering a biological sample amendable to an assay for activation of T cells, if necessary, (b) contacting the sample with a HSV-2-specific polypeptide or portion thereof under conditions that allow APCs to process the polypeptide, and (c) determining activation of the T cells in response to the HSV-2-specific polypeptide, where an elevated T cell activation relative to an uninfected patient indicates HSV-2 infection. This diagnostic assay is intended to detect the presence of HSV-2-specific T cells in any patients, including those patients who have been exposed to HSV-2 but have not seroconverted to produce detectable levels of anti-HSV-2 antibodies.

T cell activation may be measured using many proliferation assays, including cytokine-specific ELISA, cell proliferation measured by tritiated thymidine incorporation or membrane intercolating (PKH-67) or cytoplasmic (CFSE) dyes, ELISPOT, flow cytometry, and bead arrays. In addition, one may measure the T cell response in T cell lines or in T cell hybridomas from mice or humans that are specific for the antigens. Readouts for activated T cells include proliferation, cytokine production, or readout of a surrogate enzyme expressed by the hybridoma that is induced when the T cell or T cell hybridoma is activated in response to an antigen. For example, activation of a T cell response may be detected by T cell hybridoma that is engineered to produce β-galactosidase. β-galactosidase may be detected through the use of colorimetric β-galactosidase substrates such as chlorophenyl red β-D galactopyranoside (CPRG).

Infection with HSV-2 may be acute or latent. In some embodiments, if the biological sample shows the presence of HSV-2, one may administer a therapeutically effective amount of the compositions and therapies described herein to the patient. The biological sample may comprise, for example, blood, semen, urine, vaginal fluid, mucus, saliva, feces, urine, cerebrospinal fluid, or a tissue sample. In some embodiments, the biological sample is an organ intended for transplantation. In certain embodiments, before the detection step, the biological sample is subject to culture conditions that promote the growth of HSV-2.

The diagnostic tests herein may be used to detect HSV-2 in a variety of samples, including samples taken from patients and samples obtained from other sources. For example, the diagnostic tests may be used to detect HSV-2 on objects such as medical instruments. In some embodiments, the tests herein may be performed on samples taken from animals such as agricultural animals (cows, pigs, chickens, goats, horses and the like), companion animals (dogs, cats, birds, and the like), or wild animals. In certain embodiments, the tests herein may be performed on samples taken from cell cultures such as cultures of human cells that produce a therapeutic protein, cultures of bacteria intended to produce a useful biological molecule, or cultures of cells grown for research purposes.

The invention also includes a method of determining the location of a HSV-2 infection in a patient comprising: (a) administering a pharmaceutical composition comprising a labeled HSV-2 antibody or antigen-binding portion thereof to the patient, (b) detecting the label, and (c) determining if the patient has HSV-2 compared to a control. In certain embodiments, the method further comprises, if the patient has an HSV-2 infection, administering a therapeutically effective amount of a composition described herein to the patient. The method may further comprise determining the infected cell types and/or volume of the HSV-2 in the patient. This method may be used to evaluate the spread of HSV-2 in the patient and determine whether a localized therapy is appropriate.

In some embodiments, the polypeptides described herein may be used to make a prognosis of the course of infection. In some embodiments, T cell or antibody responses specific for the polypeptides herein may be detected in a sample taken from a patient. If antibodies or T cells are present at normal levels, it would indicate that the patient has raised an effective immune response against the pathogen. If antibodies or T cells are absent, or present at reduced levels, it would indicate that the patient is failing to raise a sufficient response against the pathogen, and a more aggressive treatment would be recommended. In some embodiments, antibody or T cells present at reduced levels refers to responses that are present at less than 50%, 20%, 10%, 5%, 2%, or 1% the typical level in a patient with a protective immune response. T cell responses may be detected by methods known in the art such as T cell proliferation, ELISPOT or ELISA, and antibodies may be detected by affinity for any of the antigens described herein, using methods known in the art such as ELISA.

In some embodiments, detection of T cells specific for HSV-2 antigens may be used to predict the progress and symptoms of HSV-2 infection in a patient. After infection with HSV-2, some patients remain asymptomatic, although the virus may establish latency. Other patients exhibit symptoms of HSV-2 infection, and may experience recurrent outbreaks. The HSV-2 antigens found in asymptomatic patients may differ from those antigens found in patients who present symptoms and/or recurrent outbreaks. Accordingly, the detection methods of the present invention may be used to distinguish between subgroups within the population of patients infected with HSV-2. Subgroups may be further divided into patients who experience frequent outbreaks and those who infrequently or never experience outbreaks, or patients who shed high levels of virus and those who shed low levels or do not shed. The categorization of patients, based on the presence and levels of T cell responses to certain HSV-2 antigens but not others, may help health care practitioners to determine appropriate treatment regimens. Similarly, differences in the magnitude of T cell responses and/or differences in the combination and levels of cytokines produced by T cells may also be used to predict the progress and symptoms of HSV-2 infection in a patient. Thus, an infected patient whose complement of HSV-2 antigens to which T cells respond predicts severe symptoms, frequent outbreaks, and/or high levels of viral shedding may require more intensive antiviral therapy and/or a longer course of therapeutic treatment than a patient whose complement of HSV-2 antigens predicts an asymptomatic infection.

It will be understood by one of skill in the art that the methods herein are not limited to detection of HSV-2. Other embodiments include the detection of related viruses including viruses with proteins homologous to the proteins described above, such as those in Table 1 and/or Table 2. Such related viruses include, for example, other members of the *Herpesviridae* family. Depending on the homology, these related viruses may also include viruses that are not members of the *Herpesviridae* family.

### 3. Use in groups with increased risk for infection by HSV-2

Essentially any individual has a certain risk of infection with HSV-2. However, certain sub-populations have an increased risk of infection. In some embodiments, patients receiving the composition for HSV-2 are immunocompromised.

An immunocompromising condition arising from a medical treatment is likely to expose the individual in question to a higher risk of infection. It is possible to treat an infection prophylactically in an individual having the immunocompromised condition before or during treatments known to generate such a condition. By prophylactically treating with the antigen before or during a treatment known to generate such a condition it is possible to prevent a subsequent infection or to reduce the risk of the individual contracting an infection due to the immunocompromised condition. Should the individual contract an infection, e.g., following a treatment leading to an immunocompromised condition, it is also possible to treat the infection by administering to the individual an antigen composition.

In certain embodiments, the compositions are administered to children or adult patients. In other embodiments, compositions are appropriate for pregnant women who were infected before becoming pregnant, or who became infected during pregnancy, such as to inhibit infection of a fetus or baby. The compositions may also be administered to neonates and infants who became infected in utero or during delivery.

### H. Doses and Routes of Administration

### 1. Dosage amounts and timing

The amount of antigen in each vaccine dose is selected as an effective amount, which induces an prophylactic or therapeutic response, as described above, in either a single dose or over multiple doses. Preferably, the dose is without significant adverse side effects in typical vaccinees. Such amount will vary depending upon which specific antigen is employed. Generally, it is expected that a dose will comprise 1-1000 µg of protein, in some instances 2-100 µg, for instance 4-40 µg. An optimal amount for a particular vaccine can be ascertained by standard studies involving observation of antibody titers, T cell activation levels, and other responses in subjects. In some embodiments, the appropriate amount of antigen to be delivered will depend on the age, weight, and health (e.g. immunocompromised status) of a subject. When present, typically an adjuvant will be present in amounts from 1 µg - 250 µg per dose, for example 50-150 µg, 75-125µg or 100 µg.

In some embodiments, only one dose of the vaccine is administered to achieve the results described above. In other embodiments, following an initial vaccination, subjects receive one or more boost vaccinations, for a total of two, three, four or five vaccinations. Advantageously, the number is three or fewer. A boost vaccination may be administered, for example, about 1 month, 2 months, 4 months, 6 months, or 12 months after the initial vaccination, such that one vaccination regimen involves administration at 0, 0.5-2 and 4-8 months. It may be advantageous to administer split doses of vaccines which may be administered by the same or different routes.

The pharmaceutical compositions described herein may take on a variety of dosage forms. In certain embodiments, the composition is provided in solid or powdered (e.g., lyophilized) form; it also may be provided in solution form. In certain embodiments, a dosage form is provided as a dose of lyophilized composition and at least one separate sterile container of diluent.

In some embodiments, the antigen is delivered to a patient at an amount of 1µmol per dose. In some embodiments, the antigen is delivered at a dose ranging from 10 nmol to 100 nmol per dose. The appropriate amount of antigen to be delivered may be determined by one of skill in the art. In some embodiments, the appropriate amount of antigen to be delivered will depend on the age, weight, and health (e.g., immunocompromised status) of a subject.

Pharmaceutical compositions disclosed herein are (in some embodiments) administered in amounts sufficient to elicit production of antibodies as part of an immunogenic response. In some embodiments, the composition may be formulated to contain 5 mcg/0.5 mL or an amount ranging from 10 mcg/1mL to 200mcg/1 mL of an antigen. In other embodiments, the composition may comprise a combination of antigens. The plurality of antigens may each be the same concentration, or may be different concentrations.

In some embodiments, the composition will be administered in a dose escalation manner, such that successive administrations of the composition contain a higher concentration of composition than previous administrations. In some embodiments, the composition will be administered in a manner such that successive administrations of the composition contain a lower concentration of composition than previous administrations.

In therapeutic applications, compositions are administered to a patient suffering from a disease in an amount sufficient to cure or at least partially arrest the disease and its complications.

Therapeutic applications of a composition described herein include reducing transmissibility, slowing disease progression, reducing viral shedding, or eliminating recurrent infections in patients that have been infected with HSV-2, such as by 90%, 80%, 70%, 60%, 50%, 40%, 30%, 20% or 10% of the levels at which they would occur in individuals who are not treated with the composition. The composition may also reduce the quantity of HSV-2 shed by infected individuals, inhibit the expression of proteins required for reactivation of HSV-2 from the latent stage in infected patients, and/or inhibit replication of HSV-2 in neurons of infected patients, such as by 90%, 80%, 70%, 60%, 50%, 40%, 30%, 20%, or 10% of the levels at which they would occur in individuals not treated with the composition.

In prophylactic embodiments, compositions are administered to a human or other mammal to induce an immune response that can inhibit the establishment of an infectious disease or other condition. In some embodiments, a composition may partially block the virus from establishing latency or reduce the efficiency with which latency is established.

In some embodiments, only one dose (administration) of the composition is given. In other embodiments, the composition is administered in multiple doses. In various embodiments, the composition is administered once, twice, three times, or more than three times. The number of doses administered to a subject is dependent upon the antigen, the extent of the disease or the expected exposure to the disease, and the response of a subject to the composition.

In some embodiments, the compositions are administered in combination with antimicrobial molecules. Antimicrobial molecules may include antiviral molecules. Many antiviral molecules are currently known in the art, and target one or more stage of the viral life cycle, including viral attachment to host cells, release of viral genes and/or enzymes into the host cell, replication of viral components using host-cell machinery, assembly of viral components into complete viral particles, and release of viral particles to infect new hosts.

### 2. Routes of administration

The vaccine formulations and pharmaceutical compositions herein can be delivered by administration to an individual, typically by systemic administration (e.g., intravenous, intraperitoneal, intramuscular, intradermal, subcutaneous, transdermal, subdermal, intracranial, intranasal, mucosal, anal, vaginal, oral, sublingual, buccal route or they can be inhaled) or they can be administered by topical application.

In some embodiments, the composition may be administered directly to the likely sites of infection. In female patients, the composition may be applied topically to mucosal membranes, or delivery vaginally or rectally using devices and methods known in the art. The vaginal and rectal routes of delivery permits extended, continuous or pulsed delivery and administration of composition dosages, and may be administered either before or after exposure to HSV, depending on the use of a prophylactic or therapeutic composition. In male patients, the composition may be applied topically to the skin or mucosal membranes, or delivered rectally. In both patient populations, the composition may also be targeted to the sensory ganglia.

An HSV-2 vaccine or pharmaceutical composition is often administered via the intramuscular route. Typically, in this route, the vaccine is injected into an accessible area of muscle tissue. Intramuscular injections are, in some embodiments, given in the deltoid, vastus lateralis, ventrogluteal or dorsogluteal muscles. The injection is typically given at an approximately 90° angle to the surface of the skin, so the vaccine penetrates the muscle.

An HSV-2 vaccine may also be administered subcutaneously. The injection is typically given at a 45° angle to the surface of the skin, so the vaccine is administered to the subcutis and not the muscle.

In some embodiments, the HSV-2 vaccine is administered intradermally. Intradermal administration is similar to subcutaneous administration, but the injection is not as deep and the target skin layer is the dermis. The injection is typically given at a 10-15° angle to the surface of the skin, so the vaccine is delivered just beneath the epidermis.

### 3. Formulations

The vaccine formulation may be suitable for administration to a human patient, and vaccine preparation may conform to USFDA guidelines. In some embodiments, the vaccine formulation is suitable for administration to a non-human animal. In some embodiments, the vaccine is substantially free of either endotoxins or exotoxins. Endotoxins include pyrogens, such as lipopolysaccharide (LPS) molecules. The vaccine may also be substantially free of inactive protein fragments. In some embodiments, the vaccine has lower levels of pyrogens than industrial water, tap water, or distilled water. Other vaccine components may be purified using methods known in the art, such as ion-exchange chromatography, ultrafiltration, or distillation. In other embodiments, the pyrogens may be inactivated or destroyed prior to administration to a patient. Raw materials for vaccines, such as water, buffers, salts and other chemicals may also be screened and depyrogenated. All materials in the vaccine may be sterile, and each lot of the vaccine may be tested for sterility. Thus, in certain embodiments the endotoxin levels in the vaccine fall below the levels set by the USFDA, for example 0.2 endotoxin (EU)/kg of product for an intrathecal injectable composition; 5 EU/kg of product for a non-intrathecal injectable composition, and 0.25-0.5 EU/mL for sterile water.

In some embodiments, the vaccine comprising a polypeptide contains less than 5%, 2%, 1%, 0.5%, 0.2%, 0.1% of other, undesired unpolypeptides, relative to the amount of desired polypeptides. In some embodiments, the vaccine contains less than 5%, less than 2%, less than 1%, less than 0.5%, less than 0.2%, or less than 0.1% DNA and/or RNA.

It is preferred that the vaccine has low or no toxicity, within a reasonable risk-benefit ratio.

The formulations suitable for introduction of the pharmaceutical composition vary according to route of administration. Formulations suitable for parenteral administration, such as, for example, by intraarticular (in the joints), intravenous, intramuscular, intradermal, intraperitoneal, intranasal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampoules and vials.

Injection solutions and suspensions can be prepared from sterile powders, granules, and tablets of the kind previously described. Cells transduced by the packaged nucleic acid can also be administered intravenously or parenterally.

Formulations suitable for oral administration can consist of (a) liquid solutions, such as an effective amount of the polypeptides or packaged nucleic acids suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the active ingredient, carriers known in the art. The pharmaceutical compositions can be encapsulated, e.g., in liposomes, or in a formulation that provides for slow release of the active ingredient.

The antigens, alone or in combination with other suitable components, can be made into aerosol formulations (e.g., they can be "nebulized") to be administered via inhalation. Aerosol formulations can be placed into pressurized acceptable propellants, such as dichlorodifluoromethane, propane, nitrogen, and the like.

Suitable formulations for vaginal or rectal administration include, for example, suppositories, which consist of the polypeptides or packaged nucleic acids with a suppository base. Suitable suppository bases include natural or synthetic triglycerides or paraffin hydrocarbons. In addition, it is also possible to use gelatin rectal capsules which consist of a combination of the polypeptides or packaged nucleic acids with a base, including, for example, liquid triglycerides, polyethylene glycols, and paraffin hydrocarbons. The formulation may be suitable for administration to a human patient, and the preparation may conform to US FDA guidelines. In some embodiments, the formulation is suitable for administration to a non-human animal. In some embodiments, the composition is substantially free of either endotoxins or exotoxins. Endotoxins may include pyrogens, such as lipopolysaccharide (LPS) molecules. The composition may also be substantially free of inactive protein fragments which may cause a fever or other side effects. In some embodiments, the composition contains less than 1%, less than 0.1%, less than 0.01%, less than 0.001%, or less than 0.0001% of endotoxins, exotoxins, and/or inactive protein fragments. In some embodiments, the composition has lower levels of pyrogens than industrial water, tap water, or distilled water. Other components may be purified using methods known in the art, such as ion-exchange chromatography, ultrafiltration, or distillation. In other embodiments, the pyrogens may be inactivated or destroyed prior to administration to a patient. Raw materials for compositions, such as water, buffers, salts and other chemicals may also be screened and depyrogenated. All materials in the composition may be sterile, and each lot of the composition may be tested for sterility. Thus, in certain embodiments the endotoxin levels in the composition fall below the levels set by the USFDA: 0.2 endotoxin (EU)/kg of product for an intrathecal injectable composition; 5 EU/kg of product for a non-intrathecal injectable composition, and 0.25-0.5 EU/mL for sterile water.

In certain embodiments, the preparation comprises less than 50%, 20%, 10%, or 5% (by dry weight) contaminating protein. In certain embodiments, the desired molecule is present in the substantial absence of other biological macromolecules, such as other proteins (particularly other proteins which may substantially mask, diminish, confuse or alter the characteristics of the component proteins either as purified preparations or in their function in the subject reconstituted mixture). In certain embodiments, at least 80%, 90%, 95%, 99%, or 99.8% (by dry weight) of biological macromolecules of the same type present (but water, buffers, and other small molecules, especially molecules having a molecular weight of less than 5000, can be present).

It is preferred that the composition has low or no toxicity, within a reasonable risk-benefit ratio. In certain embodiments, the composition comprises ingredients at concentrations that are less than LD₅₀ measurements for the animal being treated with the composition. LD₅₀ measurements may be obtained in mice or other experimental model systems, and extrapolated to humans and other animals. Methods for estimating the LD₅₀ of compounds in humans and other animals are well-known in the art. A composition, and any component within it, might have an LD₅₀ value in rats of greater than 100 g/kg, greater than 50g/kg, greater than 20 g/kg, greater than 10 g/kg, greater than 5 g/kg, greater than 2 g/kg, greater than 1 g/kg, greater than 500 mg/kg, greater than 200 mg/kg, greater than 100 mg/kg, greater than 50 mg/kg, greater than 20 mg/kg, or greater than 10 mg/kg. In some embodiments, the therapeutic index of the composition (measured as the toxic dose for 50% of the population (TD₅₀) divided by the minimum effective dose for 50% of the population (ED₅₀)), is greater than 1, greater than 10, or greater than 100.

### I. Preparation and Storage of Vaccines Formulations and Immunogenic Compositions

The HSV-2 vaccines described herein may be produced using a variety of techniques. For example, a polypeptide may be produced using recombinant DNA technology in a suitable host cell. A suitable host cell may be bacterial, yeast, mammalian, or other type of cell. The host cell may be modified to express an exogenous copy of one of the relevant polypeptide genes. Typically, the gene is operably linked to appropriate regulatory sequences such as a strong promoter and a polyadenylation sequence. In some embodiments, the promoter is inducible or repressible. Other regulatory sequences may provide for secretion or excretion of the polypeptide of interest or retention of the polypeptide of interest in the cytoplasm or in the membrane, depending on how one wishes to purify the polypeptide. The gene may be present on an extrachromosomal plasmid, or may be integrated into the host genome. One of skill in the art will recognize that it is not necessary to use a nucleic acid 100% identical to the naturally-occurring sequence. Rather, some alterations to these sequences are tolerated and may be desirable. For instance, the nucleic acid may be altered to take advantage of the degeneracy of the genetic code such that the encoded polypeptide remains the same. In some embodiments, the gene is codon-optimized to improve expression in a particular host. The nucleic acid may be produced, for example, by PCR or by chemical synthesis.

Once a recombinant cell line has been produced, a polypeptide may be isolated from it. The isolation may be accomplished, for example, by affinity purification techniques or by physical separation techniques (e.g., a size column).

In a further aspect of the present disclosure, there is provided a method of manufacture comprising mixing one or more polypeptides or an immonogenic fragment or variant thereof with a carrier and/or an adjuvant. In some embodiments, the adjuvant is one that stimulates a T_{H}1 cell response.

In some embodiments, antigens for inclusion in compositions of the invention may be produced in cell culture. One method comprises providing one or more mammalian expression vectors and cloning nucleotides encoding two or more polypeptides selected from polypeptides having an amino acid sequence of any one of SEQ ID NOS: 1-38, then expressing and isolating the polypeptides.

The immunogenic polypeptides described herein, and nucleic acid compositions that express the polypeptides, can be packaged in packs, dispenser devices, and kits for administering nucleic acid compositions to a mammal. For example, packs or dispenser devices that contain one or more unit dosage forms are provided. Typically, instructions for administration of the compounds will be provided with the packaging, along with a suitable indication on the label that the compound is suitable for treatment of an indicated condition, such as those disclosed herein.

### V. Examples

### Example 1. Identification of HSV-2 antigens.

A library of HSV-2 antigens (from HSV-2 Strain G, Lot # 7C0013, from Advanced Biotechnologies Inc, Maryland) was prepared and screened with peripheral blood mononuclear cells (PBMC) from human donors. Briefly, a library of HSV antigens was expressed by bacteria and mixed with antigen presenting cells (APCs). The APCs, in turn, presented HSV-derived peptides to lymphocytes that had been isolated from human patients infected with HSV-2. The patients belonged to several populations, as described below. Lymphocyte responses from each population were compared for reactivity to each expressed protein, and the screen detected antigens that induced reactive lymphocytes with greater frequency in one patient population as compared to the others. Infected but asymptomatic, and exposed but seronegative patients may activate protective immune responses that patients who experience frequent outbreaks do not; in particular, exposed but seronegative patients are presumed to have mounted sterilizing immunity to HSV-2 infection. It is believed that a unique set of polypeptides will activate lymphocytes from these patient populations.

The release of IFN-γ from CD4⁺ T cells and CD8⁺ T cells from each population was measured by ELISA following exposure to candidate antigens. Antigens were selected on the basis of the fold increase of IFN-γ released, relative to the level of IFN-γ released by frequent recurrers who experience more than four outbreaks per year.

### A. Identification of antigens encoded by UL10, UL19, UL40, US4, US6, RS1 (RS1.1, RS1.2, RS1.3), UL 36 (UL36.3, UL36.4, UL36.5), UL32, and RL2

Lymphocytes were isolated from patients belonging to several populations: asymptomatic (n=40), exposed (n=40), frequent recurrers who experience 4 or more outbreaks per year (n=43), less-frequent recurrers who experience less than 4 outbreaks per year (n=19), naive (n=10), and HSV-2⁻/HSV-1⁺ (n=10). Table 3 shows the frequency analysis for thirteen HSV-2 antigens encoded by UL10, UL19, UL40, US4, US6, RS1 (RS1.1, RS1.2, RS1.3), UL36 (UL36.3, UL 36.4, UL36.5), UL32, and RL2 in the exposed patient cohort compared to recurrers with 2 or more outbreaks per year.

**Table 3. Frequency analysis for antigens encoded by UL10, UL19, UL40, US4, US6, RS1 (RS1.1, RS1.2, RS1.3), UL36 (UL36.3, UL36.4, UL36.5), UL32 and RL2**

| **HSV-2 Gene** | **Protein Name** | **Frequency Analysis (HSV-1/HSV-2 seronegative)** | |
|---|---|---|---|
| | | **% response from exposed donors** | **fold increase over recurrer response** |
| **UL10** | gM | 23% | 1.4 |
| **UL19** | VP5 | - | - |
| **UL40** | ribonucleotide reductase | 36% | 3.0 |
| **Us4** | gG | 24% | 1.6 |
| **Us6** | gD | 27% | 1.9 |
| **RS1** | ICP4 | | |
| **RS1.1** | | 54% | 3.0 |
| **RS1.2** | | 46% | 2.3 |
| **RS1.3** | | 23% | 1.2 |
| **UL36** | Major tegument | | |
| **UL36.3** | protein | 46% | 2.3 |
| **UL36.4** | | 46% | 4.2 |
| **UL36.5** | | 31% | 1.9 |
| **UL32** | DNA cleavage & packaging proteiin | - | - |
| **RL2** | ICP0 | 45% | 1.6 |

### B. Identification of antigens encoded by UL1, UL49.5, and UL54

Lymphocytes were isolated from patients belonging to several populations: asymptomatic (n=40), exposed (n=40), frequent recurrers who experience 4 or more outbreaks per year (n=43), less-frequent recurrers who experience less than 4 outbreaks per year (n=19), naïive (n=10), and HSV-2⁻/HSV-1⁺ (n=10).

Table 4 shows the frequency analysis for three HSV-2 antigens encoded by UL1, UL49.5 and UL54, in the exposed patient cohort compared to recurrers with 2 or more outbreaks per year.

**Table 4. Frequency analysis for antigens encoded by UL1, UL49.5, and UL54**

| **HSV-2 Gene** | **Protein Name** | **Frequency Analysis (HSV-1/HSV-2 seronegative)** | |
|---|---|---|---|
| | | % response from exposed donors | fold increase over recurrer response |
| **UL1** | gL2 | 64% | 2.7 |
| **UL49.5** | (virion p) | 37% | 2.1 |
| **UL54** | ICP27 | 22% | 5.8 |

### C. Identification of antigens encoded by RL1, Ul1, and UL11

Lymphocytes were isolated from patients belonging to several populations: asymptomatic (n=40), exposed (n=40), frequent recurrers who experience 4 or more outbreaks per year (n=43), less-frequent recurrers who experience less than 4 outbreaks per year (n=19), naïive (n=10), and HSV-2⁻/HSV-1⁺ (n=10).

Table 5 shows the frequency analysis for three HSV-2 antigens encoded by RL1, Ul1, and UL11 in the exposed patient cohort compared to recurrers with 2 or more outbreaks per year.

**Table 5. Frequency analysis for HSV-2 antigens encoded by RL1, Ul1, and UL11**

| **HSV-2 Gene** | **Protein Name** | **Frequency Analysis (HSV-1/HSV-2 seronegative)** | |
|---|---|---|---|
| | | % response from exposed donors | fold increase over recurrer response |
| **RL1** | ICP34.5 | 45% | 1.3 |
| **UL2** | DNA glycosylase | 23% | 1.4 |
| **UL11** | tegument protein | 21% | <1.0 |

### Example 2. In vivo data

### A. [Protocol A] Guinea pig therapeutic vaccination protocol

Female Hartley guinea pigs were challenged intravaginally with HSV-2 strain MS at 5x10⁵ pfu to establish a genital tract infection. Animals were monitored for infection by vaginal swab on day 1 post-infection, and acute disease between days 3 and 14 post-infection. On day 14, after resolution of primary disease, the animals were randomized into groups of 12 and immunized subcutaneously with antigen (HSV-2 polypeptide at 15 µg dose) plus adjuvant (50 µg dose of an ISCOM matrix with a 91:9 mixture of Quillaja saponin fractions A and C). Each group received a total of 3 vaccinations, on days 14, 21, and 34 post-infection. Genital swabs were collected during the vaccination period to monitor viral shedding, and daily observations were recorded. Symptoms were scored on a scale from 0 to 4 based upon severity, 0 = no symptoms; 1= redness or swelling; 2 = a few small vesicles; 3 = several large vesicles; 4 = several large vesicles with maceration. In addition, animals with lesions intermediate in severity between the above scores were given a score of 0.5, 1.5, 2.5, or 3.5.

### 1. Results of therapeutic vaccination studies with ICP4.2, gD2ΔTMR, and gD2

The results of the studies are presented below in Tables 6-10. The IgG titer was determined at day 41 post-infection and 7 days after third immunization using an average of 4 out of the 12 animals in each group. The mean recurrent lesion scores and mean lesion days were each determined from day 15 to day 63 post-infection. The lesion scores represent total lesions for each group from day 15 to 60 and then a mean was calculated. Mean lesion days represent the mean number of days post-infection that immunized or non-immunized animals had herpetic lesions present. Vaginal-swab samples were collected from all animals for 12 days between days 20-59 post-infection and stored at -80°C until assayed for virus shedding titers by quantitative real-time PCR.

**Table 6. Results of therapeutic vaccination studies with ICP4.2 (SEQ ID NO: 2): lesions**

| **Groups N=12** | **Dose** | **gD2 IgG** Titer | **Mean Recurrent Lesion Score** | **% Reduction** | **Mean Lesion Days** | **% Reduction** |
|---|---|---|---|---|---|---|
| Phosphate-Buffered Saline | - | 1:263 | 8.1 | - | 9.0 | - |
| adjuvant only | 50 µg x 3 | 1:331 | 7.1 | 14 | 8.5 | 6 |
| ICP4.2 + adjuvant | 15 µg x 3 | 1:1079 | 4.3 | 47 | 5.1 | 44 |

**Table 7. Results of therapeutic vaccination studies with ICP4.2 (SEQ ID NO: 2): viral shedding**

| **Groups** | **No. of animals/total with no detectable viral shedding** | **Mean number of days viral shedding detected ±SEM** | **% Reduction** | **P value*** |
|---|---|---|---|---|
| Phosphate-Buffered Saline | 0/11 | 4.5 ± 0.8 | - | - |
| Adjuvant only | 0/12 | 4.4 ± 0.7 | 2 | 0.971 |
| ICP4.2 + adjuvant | 5/11 | 1.5 ± 0.5 | 67 | 0.004 |

**Table 8. Results of therapeutic vaccination studies with gD2ΔTMR (SEQ ID NO:4): lesions**

| **Groups** | **Mean Recurrent Lesion Score** | **% Reduction** | **Mean Lesion Days** | **% Reduction** |
|---|---|---|---|---|
| Adjuvant only | 8.7 | - | 11.7 | - |
| gD2ΔTMR | 5.7 | 34 | 8.6 | 26 |

**Table 9. Results of therapeutic vaccination studies with gD2 (SEQ ID NO: 5): lesions**

| **Groups N=12** | **Dose** | **gD2 IgG Titer** | **Mean Recurrent Lesion Score** | **% Reduction** | **Mean Lesion Days** | **% Reduction** |
|---|---|---|---|---|---|---|
| Phosphate-Buffered | - | 1:263 | 8.1 | - | 9.0 | - |
| Saline | | | | | | |
| Adjuvant only | 50 µg x 3 | 1:331 | 7.1 | 14 | 8.5 | 6 |
| gD2 + adjuvant | 15 µg x 3 | >1:6400 | 4.0 | 51 (p=0.04) | 5.0 | 45 |

**Table 10. Results of therapeutic vaccination studies with gD2 (SEQ ID NO: 5): viral shedding**

| **Groups** | **No. of animals/total with no detectable viral shedding** | **Mean number of days viral shedding detected ±SEM** | **% Reduction** | **P value*** |
|---|---|---|---|---|
| Phosphate-Buffered Saline | 0/11 | 4.5 ± 0.8 | - | - |
| Adjuvant only | 0/12 | 4.4 ± 0.7 | 2 | 0.971 |
| gD2 + adjuvant | 4/12 | 2.4 ± 0.6 | 47 | 0.047 |

### B. [Protocol B] Murine prophylactic vaccination protocol

Female C57BL/6 mice from 6 to 8 weeks of age were immunized subcutaneously with antigen (HSV-2 polypeptide) plus adjuvant (12 µg dose of an ISCOM matrix with a 82:18 mixture of Quillaja saponin fractions A and C) on day 0 and day 9. On day 11, estrous cycles were synchronized with depo provera and then the mice were challenged on day 16 via intravaginal deposition of 10 times the LD₅₀ of HSV-2 strain 333 while under anaesthesia. All animals were monitored for morbidity (clinical score) and mortality, and body weights and vaginal swabs were collected between days 17 and 28 post-infection. Clinical scores were recorded using the following scale: 0 = no symptoms, 1 = vaginal erythema, 2 = vaginal erythema and edema, 3 = vaginal herpetic lesions, 4 = unilateral paralysis or severe genital ulceration, and 5 = bilateral paralysis or death.

### 1. Results of murine prophylactic vaccination studies with ICP4.2, VP5, gD2ΔTMR and gD2ΔTMR and ICP4.2

In the experimental group, mice were immunized subcutaneously with either 5 µg or 10 µg of antigen plus adjuvant (12 µg dose of an ISCOM matrix with a 82:18 mixture of Quillaja saponin fractions A and C) on day 0 and day 9. Control animals received phosphate buffered saline (PBS) only, or adjuvant only.

Mice receiving PBS only or adjuvant only all died by day 9 post-challenge (no survivors). In contrast, mice receiving antigen largely survived to day 9, and 20-75% survived to day 12 post-challenge. The severity of disease symptoms (genital and neurological disease) were also scored at either day 9 or 10 post-challenge. Mice immunized with ICP4.2, VP5, gD2ΔTMR, or gD2ΔTMR and ICP4.2 with ISCOM adjuvant showed a significant decrease in disease symptoms compared to the PBS only or adjuvant only groups.

**Table 11. Results of murine prophylactic vaccination studies**

| **Groups** | **Mean Disease Score Day 10** | **% Reduction** | **P value*** | **% Survival Day 12** |
|---|---|---|---|---|
| PBS only/adjuvant only | 5.00/4.81 | - | - | % |
| ICP4.2 | 3.6 | 28 | - | 2.0% |
| VP5 + adjuvant | 3.13 | 35 | .146 | 3.8% |
| gD2ΔTMR + adjuvant | 1.44 | 70 | .023 | 7.5% |
| gD2ΔTMR + ICP4.2 + adjuvant | 0.75 | 84 | .020 | 8.8% |

| | | | | |
|---|---|---|---|---|
| * Student's t test | | | | |

### C. [Protocol C] Guinea pig prophylactic vaccination protocol

Female Hartley guinea pigs from 250-350 grams (weight) were immunized subcutaneously with 15 µg of antigen plus adjuvant (50 µg dose of an ISCOM matrix with a 91:9 mixture of Quillaja saponin fractions A and C) on day 0 and day 14-21. Sera were collected by toenail clip 2-3 weeks after the boost and then the guinea pigs were challenged via intravaginal deposition of 5 x 10⁵ PFU of HSV-2 strain MS. Vaginal-swab samples were collected from all animals on days 30 and 32 and stored at -80°C until assayed for virus titers by quantitative real-time PCR. Guinea pigs were evaluated daily (day 1-14), and primary genital skin disease was quantified using a lesion severity score scale from 1-4. Numerical scores were assigned to specific disease signs as follows: 0, no disease; 1, redness or swelling; 2, a few small vesicles; 3, several large vesicles; 4, several large vesicles with maceration. At the end of the study, the guinea pigs were euthanized , and the dorsal root ganglia (DRG) were harvested, stored at -80°C until they were processed for quantitative real-time PCR analysis.

**Table 12. Results of guinea pig prophylactic vaccination studies with gD2ΔTMR and VP5**

| Groups | Viral titer, PFU/ml Day 2 | Total mean acute lesion score | % Reduction | Copies HSV-2 DNA/ 1 µg DRG DNA | % Reduction |
|---|---|---|---|---|---|
| Adjuvant | 2.3 x 10⁶ | 22.6 | - | 959 | - |
| only | | | | | |
| gD2ΔTMR + Adjuvant | 1.7 x 10⁶ | 7.7 | 66% | 274 | 71% |
| VP5 + adjuvant | 5.9 x 10⁵ | 18.2 | 17% | 283 | 70% |

### D. [Protocol D] Immunogenicity assay I (standard)

Mice were immunized subcutaneously in the scruff of the neck with a 100 µl injection of 5 µg antigen plus adjuvant (12 µg dose of an ISCOM matrix with a 82:18 mixture of Quillaja saponin fractions A and C) in saline. The mice received one or two injections, 7 days apart. Analysis of the immunogenicity of the injection occurred 7 days after the final injection.

The immunogenicity assay was an *ex vivo* IFN-γ ELISPOT. CD4⁺ and CD8⁺ T cells were enriched from the spleen and analyzed separately. For the ELISPOT assay, membrane plates were prepared by coating them overnight with capture antibody and subsequently blocked by supplemented medium for a minimum of 2 hours at 37 °C. The mice were euthanized and their spleens harvested. The T cells were then prepared by sorting the splenocytes for CD4⁺ and CD8⁺ T cells using magnetic beads. The blocking solution was washed out from ELISPOT plates and the T cells were plated out onto the blocked plates. The plates were returned to the incubator to allow the T cells to settle. APCs were prepared by pulsing naïive T-depleted splenocytes with antigen for 2 hours at 37°C. For CD4⁺ ELISPOTs, APCs were pulsed with whole protein. For CD8⁺ ELISPOTs, APCs were pulsed with E. *coli* expressing protein plus cLLO. A medium control was APCs incubated for 2 hours at 37 °C with no additional antigen. The pulsed APCs were irradiated, washed and adjusted to 2 x 10⁶ cells/ml. The APCs were added to appropriate wells of plates containing T cells. Then phorbol myristate acetate (PMA) and ionomycin were added to control wells as a positive control. The plates were allowed to incubate for 18 hours at 37°C under 5% CO₂. The plates were then developed using a secondary biotinylated antibody, horseradish peroxidase (HRP) and 3-amino-9-ethylcarbazole (AEC) substrate.

### 1. Results of immunogenicity assay I with ICP4.2

The immunogenicity assay I showed a robust immunogenic response for both the one and two injection regimens with ICP4.2. For the one injection regimen, the number of IFN-γ spots per 200,000 T cells were 8 and 101 for CD4⁺ and CD8⁺ cells, respectively. For the two injection regimen, there were 50 and 70 spots, respectively. In contrast, less than 15 spots were observed for media or adjuvant alone in either CD4⁺ or CD8⁺ cells.

### 2. Results of immunogenicity assay I with gD2ΔTMR and gD2

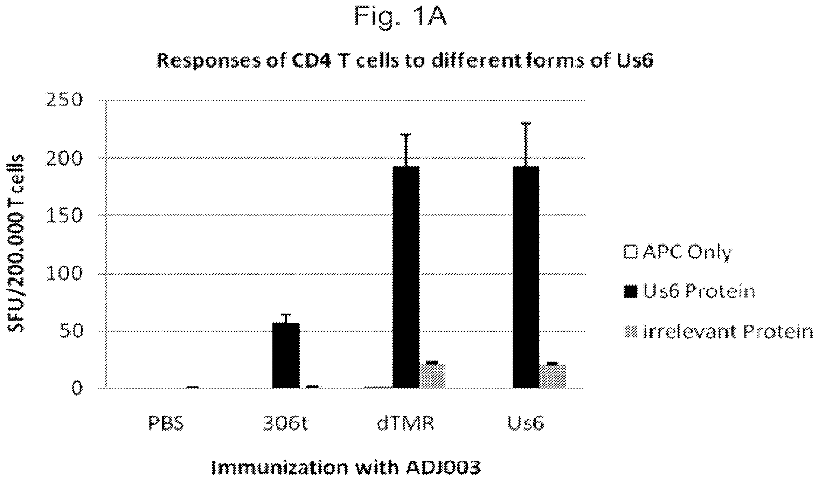

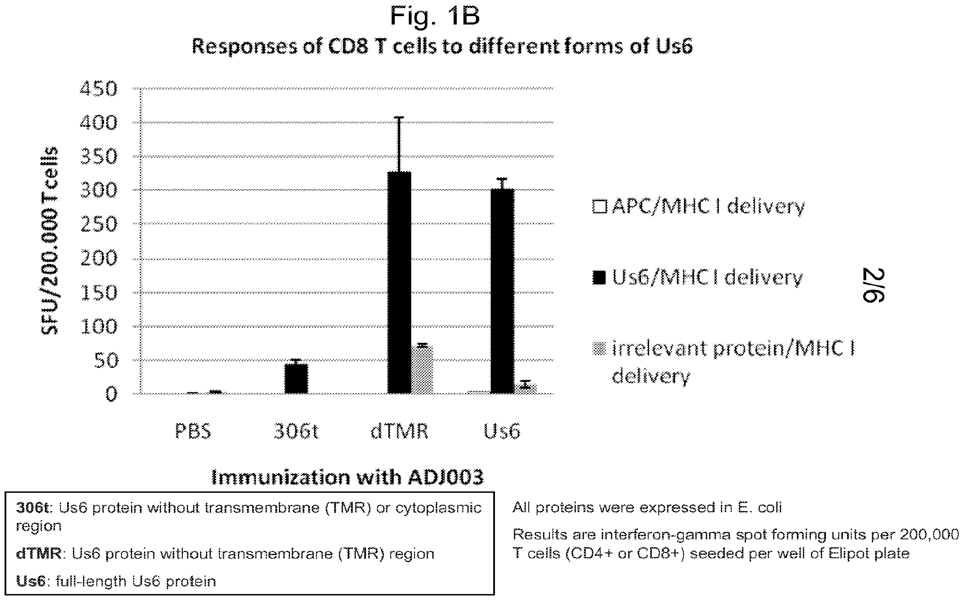

Results of immunogenicity assay I are shown in Figure 1A and B. Robust CD4⁺ and CD8⁺ T cell responses were obtained for both full-length gD2 and for gD2ΔTMR. In contrast, gD2 antigen truncated immediately upstream of the transmembrane domain (denoted 306t in Figure 1) showed significantly reduced responses.

### E. [Protocol E] Immunogenicity assay II (rapid)

Recombinant *E*. *coli* from Genocea's proprietary library of HSV-2 orfeome were induced to express gL2 or fragments of ICP4 protein (ICP4.2, and polypeptides encoded by RS1.1, RS1.3.1 and RS 1.3.2). The protein was retained within bacterial cells. The bacteria were then fixed with PFA, washed extensively with PBS and stored at -80C until used for immunization.

Three mice per group were immunized with 1x10⁸ bacteria in PBS per mouse by intraperitoneal injection. Mice received 1-2 additional boosters at 1 week intervals. Seven days after last boost, sera were collected and analyzed in an HSV-2 neutralization assay. Five-fold serial dilutions were prepared for plasma or serum samples in a 96-well round-bottom plate, followed by the addition of 50 PFUs HSV-2 (strain 333) to each well. The plates were covered and incubated at 37°C for 1 hour. 200µl of virus-serum dilution was transferred in duplicate to Vero cells grown in a 48-well tissue culture plate and incubated for 1 hour at 37°C. 300µl of DMEM containing 2% FBS was then added to each well and the plates were incubated for 48 hours at 37°C. To visualize virus plaques the plates were stained with crystal violet.

**Table 13. Results of HSV-2 neutralization assay with gL2, ICP4.2, and polypeptides encoded by RS1.1, RS1.3.1 and RS1.3.2**

| Immunogen | HSV-2 Neutralization IgG Titer* |
|---|---|
| E coli//gL2 | 1:50 |
| Ecoli//RS 1.1 | <1:20 |
| Ecoli//ICP4.2 | <1:20 |
| E.coli/RS 1.3.1 | 1:100 |
| E.coli//RS 1.3.2 | <1:20 |
| Positive control (DL11 Mab) | 1:2500 |
| Negative control (Naive mouse serum) | <1:20 |

| | |
|---|---|
| * Serum dilution that inhibits 50% of virus control | |

### F. [Protocol F] Immunogenicity assay III (overlapping peptide pools)

Mice were immunized with 2 µg/mouse of pooled, overlapping peptides (OLP) spanning the entire sequence of gL2, ICP4, and ICP4 fragments encoded by RS1.3.1 and RS1.3.2. OLPs were formulated in TiterMax adjuvant (Alexis Biochemical) in a total volume of 100 µl per mouse where adjuvant represented 1/3 of the subcutaneous dose. Mice were immunized on day 0, boosted on day 6 and spleens and blood were collected on day 11. Single cell suspensions were prepared from spleens and erythrocytes were lysed. The splenocyte suspensions were then divided into halves. The first half was separated into antigen presenting cells, CD4⁺ and CD8⁺ T cells; 200,000 T cells were seeded per well of IFN-gamma ELISPOT plate and stimulated with 100, 000 APCs and OLP pool corresponding to immunization, irrelevant peptide, positive and negative control. Cells were incubated in plates overnight after which the plates were developed and spots per well were counted. The second half of each splenocyte suspension was run as unseparated splenocytes (400,000/well), pulsed with peptides, and assayed as described above.

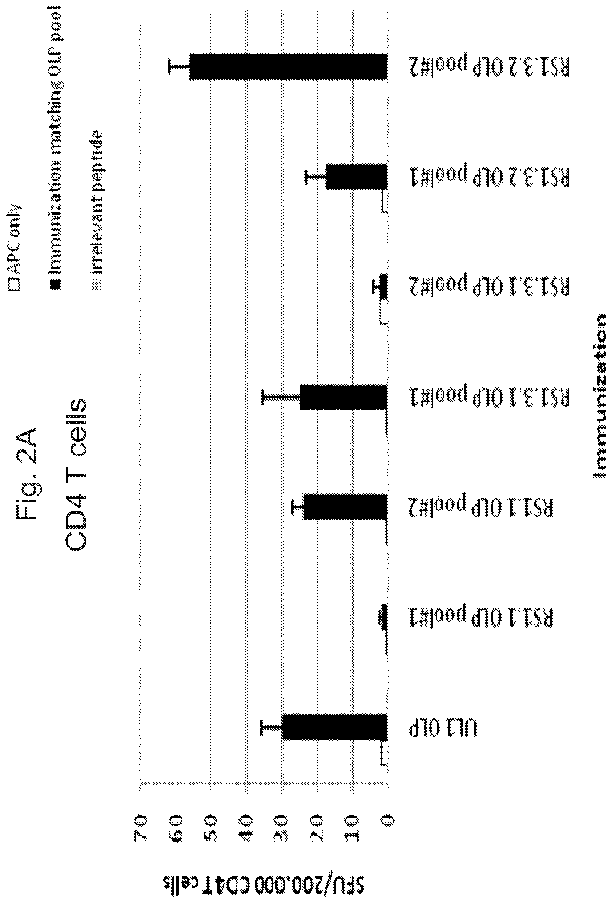

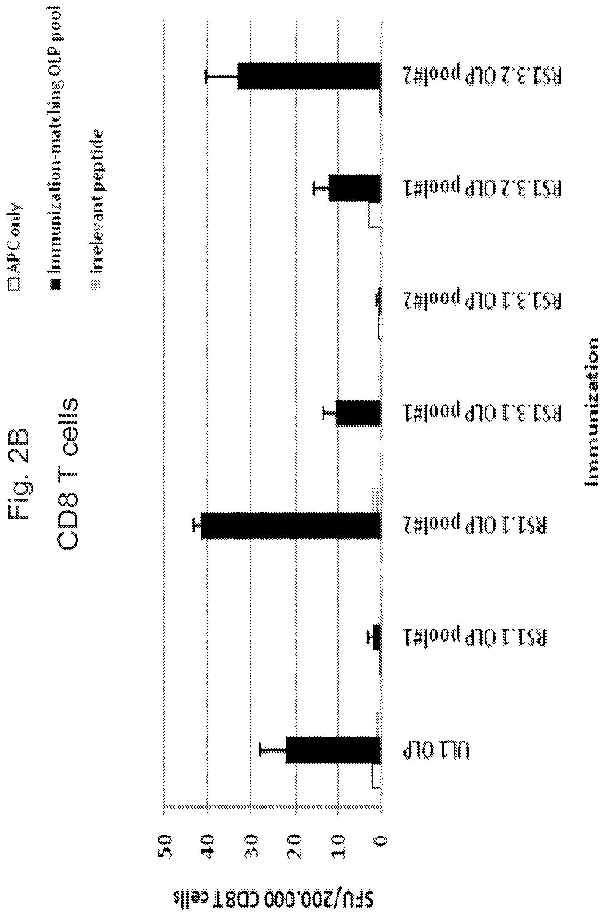

Results are shown in Figure 2A and B as magnitude of response per immunization group.

### G. [Protocol G] Vaccination with at least two antigens

### Example 1. Immunogenicity of gD2ΔTMR and ICP4 or ICP4.2 in C57BL/6 mice

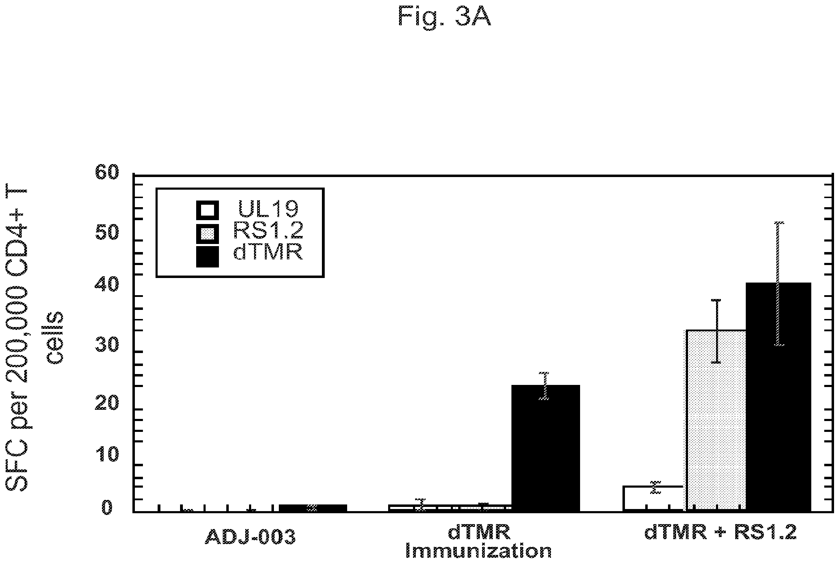

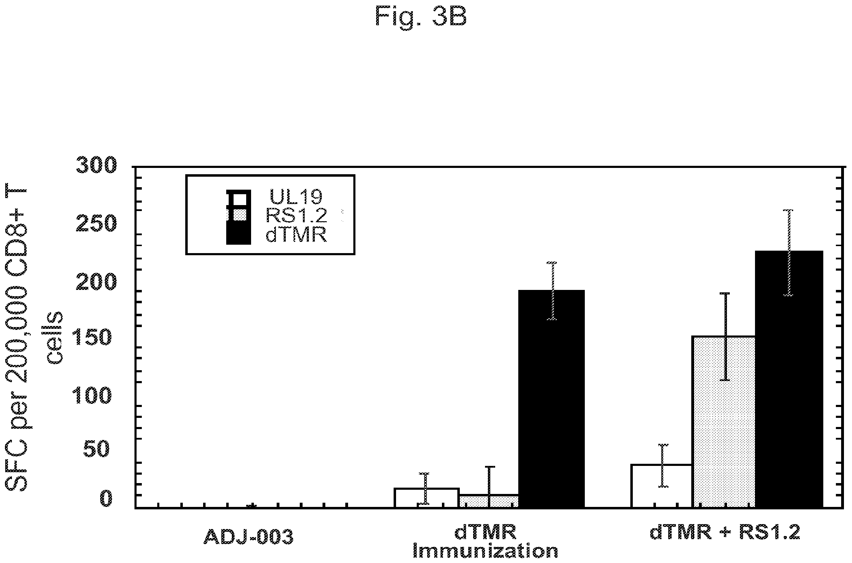

Purified protein was mixed with adjuvant and immunized into naive mice to evaluate the ability to make CD4⁺ and CD8⁺ T cell responses to the protein antigens. Briefly, antigen alone (gD2ΔTMR (5µg)) or combinations of antigens (gD2ΔTMR and ICP4.2 (10µg)) were mixed with adjuvant (12µg dose of an ISCOM matrix with a 82:18 mixture of Quillaja saponin fractions A and C) and administered subcutaneously to mice, twice, 9 days apart. Seven days after the second immunization, mice were euthanized and spleens were harvested for *ex vivo* IFNγ ELISPOT assays. CD4⁺ and CD8⁺ T cells were sorted out of the splenocyte population using antibody-coated magnetic beads and then co-cultured on IFNγ-specific antibody-coated membranes in 96-well plates with naive splenocytes that were pulsed with specific or non-specific antigens (as described) and irradiated with an x-ray irradiator. After 18 hours of incubation, captured IFNγ was detected with a biotinylated secondary IFNy-specific antibody and visualized with horseradish peroxidase and 3-amino-9-ethylcarbazole substrate. Data are reported as the number of IFN-γ spot forming units per 2x10⁵ T cells ± standard deviation of three mice per group. Figure 3 shows the number of IFN-γ spot forming units per 2x10⁵ CD4⁺ or CD8⁺ T cells ± standard deviation of three mice per group. As seen in Figures 3A and B, the number of IFN-γ spot forming units per CD4⁺ T cells or CD8⁺ T cells is increased in mice immunized with gD2ΔTMR antigen combined with ICP4.2 compared to gD2ΔTMR antigen alone.

### Example 2. Combinations of gD2 and ICP4.2 plus adjuvant immunization reduced disease symptoms and mortality in mice.

The ability to trigger protective immunity after immunization with the ICP4.2 protein in combination with gD2 plus adjuvant was evaluated in a lethal HSV-2 challenge mouse model. Briefly, eight C57BL/6 mice per group were immunized with either gD2 (2µg) or ICP4.2 (10µg) plus adjuvant individually or with both antigens mixed together plus adjuvant. Formulations were administered subcutaneously in the scruff of the neck twice, 9 days apart. Estrus cycles were synchronized with depo provera 5 days prior to virus infection, and animals were challenged intravaginally 7 days after the second immunization with 20 times the LD₅₀ of HSV-2 strain 333. Disease symptoms were scored post-infection, and survival monitored. Disease severity scores were as follows: 0= no symptoms, 1= redness, 2= redness and swelling, 3= herpetic lesions, 4=severe ulceration or unilateral paralysis, and 5= bilateral paralysis or death.

**Table 14. Effect of HSV-2 proteins gD2 and ICP4.2 on disease symptoms, viral replication and mortality**

| **Antigen (+ adjuvant) N=8** | **Mean disease score Day** 7 | **Reduction in disease score** | **P value**** | **Reduction in virus titer** | **% Survival Day 11** |
|---|---|---|---|---|---|
| PBS | 3.5 ± 0.3 | - | - | - | 0% |
| gD2* (2ug) | 2.5 ± 0.2 | 29% | 0.016 | 0% | 25% |
| ICP4.2 (10ug) | 1.7 ± 0.4 | 51% | 0.005 | 0% | 13% |
| gD2 (2ug) + ICP4.2 (10ug) | 1.3 ± 0.3 | 63% | 0.0004 | 20% | 50% |

| | | | | | |
|---|---|---|---|---|---|
| *EC; **Student's t-test | | | | | |

### Example 3. Combinations of gD2ΔTMR and ICP4.2 plus adjuvant immunization reduced disease symptoms and mortality in mice.

Mice immunized with a combination of gD2ΔTMR and ICP4.2 antigens showed a lower mean disease score at ten days after virus challenge compared to animals receiving the individual antigen with adjuvant.

**Table 15. Effect of HSV-2 proteins gD2ΔTMR and ICP4.2 on disease symptoms and survival rate in mice**

| Groups | Mean Disease Score Day 10 | % Reduction | P value* | % Survival Day 12 |
|---|---|---|---|---|
| Adjuvant only | 4.81 | - | - | 0% |
| gD2ΔTMR + adjuvant | 1.44 | 70 | 0.023 | 75% |
| gD2ΔTMR + ICP4.2 + adjuvant | 0.75 | 84 | 0.020 | 88% |

### Example 4. Combination of gD2 and ICP4.2 plus adjuvant immunization reduces severity of recurrent lesions when administered therapeutically to HSV-2 infected guinea pigs

The ability to affect HSV-2 reactivation in infected guinea pigs after therapeutic immunization with antigens plus adjuvant was evaluated. Briefly, guinea pigs were infected intravaginally with 5 x 10⁵ pfu of HSV-2 strain MS, monitored for primary disease for 14 days, and then randomized into immunization groups (N=15). Animals were immunized three times subcutaneously on day 14, 21, and 35 post-infection with antigen (15µg) plus adjuvant (50µg) or adjuvant alone, or vehicle control and scored daily for local disease severity. The scoring system was as follows: 0= no symptoms, 1= redness, 2=single lesions, 3= large or fused lesions, 4=severe ulceration or unilateral paralysis, and 5= bilateral paralysis or death.
Table 16 shows the data as the mean recurrent lesion score for each week after the guinea pigs recovered from their acute disease. The guinea pigs treated with a combination of gD2 and ICP4.2 antigens showed a reduction in the mean lesion score at 7 (day 42) and 14 (day 49) days after their last immunization compared to animals receiving the individual antigens with adjuvant.

**Table 16. Effect of HSV-2 proteins gD2 and ICP4.2 vaccine on recurrent genital skin disease**

| Mean Recurrent Lesion Score Post HSV-2 Infection | | | | | |
|---|---|---|---|---|---|
| Antigen + Adjuvant | Day 15-21 | Day 22-28 | Day 29-35 | Day 36-42 | Day 43-49 |
| PBS | 2.00 ± 0.45 | 1.17 ± 0.35 | 1.50 ± 0.50 | 0.87 ± 0.28 | 1.33 ± 0.33 |
| gD2 | 1.00 ± 0.30 | 0.67 ± 0.24 | 0.80 ± 0.19 | 0.83 ± 0.26 | 0.77 ± 0.28 |
| ICP4.2 | 1.97 ± 0.38 | 1.07 ± 0.29 | 1.03 ± 0.33 | 0.53 ± 0.16 | 0.83 ± 0.29 |
| gD2 & ICP4.2 | 1.43 ± 0.32 | 0.80 ± 0.27 | 1.07 ± 0.33 | 0.43 ± 0.19 | 0.70 ± 0.27 |

### Sequences

SEQ ID NO: 1 = ICP4, full-length

SAEQRKKKKTTTTTQGRGAEVAMADEDGGRLRAAAETTGGPGSPDPADGPPPTPNPDR
RPAARPGFGWHGGPEENEDEADDAAADADADEAAPASGEAVDEPAADGVVSPRQLALLASMVDEAVRT
IPSPPPERDGAQEEAARSPSPPRTPSMRADYGEENDDDDDDDDDDDRDAGRWVRGPETTSAVRGAYPD
PMASLSPRPPAPRRHHHHHHRRRRAPRRRSAASDSSKSGSSSSASSASSSASSSSSASASSSDDDDD
DDAARAPASAADHAAGGTLGADDEEAGVPARAPGAAPRPSPPRAEPAPARTPAATAGRLERRRARAAV
AGRDATGRFTAGRPRRVELDADAASGAFYARYRDGYVSGEPWPGAGPPPPGRVLYGGLGDSRPGLWGA
PEAEEARARFEASGAPAPVWAPELGDAAQQYALITRLLYTPDAEAMGWLQNPRVAPGDVALDQACFRI
SGAARNSSSFISGSVARAVPHLGYAMAAGRFGWGLAHVAAAVAMSRRYDRAQKGFLLTSLRRAYAPLL
ARENAALTGARTPDDGGDANRHDGDDARGKPAAAAAPLPSAAASPADERAVPAGYGAAGVLAALGRLS
AAPASAPAGADDDDDDDGAGGGGGRRAEAGRVAVECLAACRGILEALAEGFDGDLAAVPGLAGARPA
APPRPGPAGAAAPPHADAPRLRAWLRELRFVRDALVLMRLRGDLRVAGGSEAAVAAVRAVSLVAGALG
PALPRSPRLLSSAAAAAADLLFQNQSLRPLLADTVAAADSLAAPASAPREARKRKSPAPARAPPGGAP
RPPKKSRADAPRPAAAPPAGAAPPAPPTPPPRPPRPAALTRRPAEGPDPQGGWRRQPPGPSHTPAPSA
AALEAYCAPRAVAELTDHPLFPAPWRPALMFDPRALASLAARCAAPPPGGAPAAFGPLRASGPLRRAA
AWMRQVPDPEDVRVVILYSPLPGEDLAAGRAGGGPPPEWSAERGGLSCLLAALGNRLCGPATAAWAGN
WTGAPDVSALGAQGVLLLSTRDLAFAGAVEFLGLLAGACDRRLIVVNAVRAADWPADGPVVSRQHAYL
ACEVLPAVQCAVRWPAARDLRRTVLASGRVFGPGVFARVEAAHARLYPDAPPLRLCRGANVRYRVRTR
FGPDTLVPMSPREYRRAVLPALDGRAAASGAGDAMAPGAPDFCEDEAHSHRACARWGLGAPLRPVYVA
LGRDAVRGGPAELRGPRREFCARALLEPDGDAPPLVLRDDADAGPPPQIRWASAAGRAGTVLAAAGGG
VEVVGTAAGLATPPRREPVDMDAELEDDDDGLFGE

SEQ ID NO: 2 = ICP4 internal fragment

MVLYGGLGDSRPGLWGAPEAEEARARFEASGAPAPVWAPELGDAAQQYALITRLLYTP
DAEAMGWLQNPRVAPGDVALDQACFRISGAARNSSSFISGSVARAVPHLGYAMAAGRFGWGLAHVAAA
VAMSRRYDRAQKGFLLTSLRRAYAPLLARENAALTGARTPDDGGDANRRDGDDARGKPAAAAAPLPSA
AASPADERAVPAGYGAAGVLAALGRLSAAPASAPAGADDDDDDDGAGGGGGGGGGGRRAEAGRVA
VECLAACRGILEALAEGFDGDLAAVPGLAGARPAAPPRPGPAGAAAPPHADAPRLRAWLRELRFVRDA
LVLMRLRGDLRVAGGSEAAVAAVRAVSLVAGALGPALPRSPRLLSSAAAAAADLLFQNQSL

SEQ ID NO: 3 = gL2 cytoplasmic

MGFVCLFGLVVMGAWGAWGGSQATEYVLRSVIAKEVGDILRVPCMRTPADDVSWRYEA

PSVIDYARIDGIFLRYHCPGLDTFLWDRHAQRAYLVNPFLFAAGFLEDLSHSVFPADTQETTTRRALY

KEIRDALGSRKQAVSHAPVRAGCVNFDYSRTRRCVGRRDLRPANTTSTWEPPVSSDDEASSQSKPLAT
QPPVLALSNAPPRRVSPTRGRRRHTRLRRN

SEQ ID NO: 4 = gD2 internal deletion

NRWKYALADPSLKMADPNRFRGKNLPVLDQLTDPPGVKRVYHIQPSL

EDPFQPPSIPITVYYAVLERACRSVLLHAPSEAPQIVRGASDEARKHTYNLTIAWYRMGDNCAIPITV

MEYTECPYNKSLGVCPIRTQPRWSYYDSFSAVSEDNLGFLMHAPAFETAGTYLRLVKINDWTEITQFI

LEHRARASCKYALPLRIPPAACLTSKAYQQGVTVDSIGMLPRFIPENQRTVALYSLKIAGWHGPKPPY

TSTLLPPELSDTTNATQPELVPEDPEDSALLEDPAGTVSSQIPPNWHIPSIQDVAPHHAPAAPSNPRR

RAQMAPKRLRLPHIRDDDAPPSHQPLFY

SEQ ID NO: 5 = predicted gD2 sequence

MGRLTSGVGTAALLVVAVGLRVVCAKYALADPSLKMADPNRFRGKNLPVLDQLTDPPGVKRVYHIQPS

LEDPFQPPSIPITVYYAVLERACRSVLLHAPSEAPQIVRGASDEARKHTYNLTIAWYRMGDNCAIPIT

VMEYTECPYNKSLGVCPIRTQPRWSYYDSFSAVSEDNLGFLMHAPAFETAGTYLRLVKINDWTEITQF

ILEHRARASCKYALPLRIPPAACLTSKAYQQGVTVDSIGMLPRFIPENQRTVALYSLKIAGWHGPKPP

YTSTLLPPELSDTTNATQPELVPEDPEDSALLEDPAGTVSSQIPPNWHIPSIQDVAPHHAPAAPSNPG

LIIGALAGSTLAVLVIGGIAFWVRRRAQMAPKRLRLPHIRDDDAPPSHQPLFY

SEQ ID NO: 6 = ICP34.5

MSRRRGPRRRGPRRRPRPGAPAVPRPGAPAVPRPGALPTADSQMVPAYDSGTAVESAPAASSLLRRWL

LVPQADDSDDADYAGNDDAEWANSPPSEGGGKAPEAPHAAPAAACPPPPPRKERGPQRPLPPHLALRL

RTTTEYLARLSLRRRRPPASPPADAPRGKVCFSPRVQVRHLVAWETAARLARRGSWARERADRDRFRR

RVAAAEAVIGPCLEPEARARARARARAHEDGGPAEEEEAAAAARGSSAAAGPGRRAV

SEQ ID NO: 7 = ICP0

MEPRPGTSSRADPGPERPPRQTPGTQPAAPHAWGMLNDMQWLASSDSEEETEVGISDD
DLHRDSTSEAGSTDTEMFEAGLMDAATPPARPPAERQGSPTPADAQGSCGGGPVGEEEAEAGGGGDVC
AVCTDEIAPPLRCQSFPCLHPFCIPCMKTWIPLRNTCPLCNTPVAYLIVGVTASGSFSTIPIVNDPRT
RVEAEAAVRAGTAVDFIWTGNPRTAPRSLSLGGHTVRALSPTPPWPGTDDEDDDLADVDYVPPAPRRA
PRRGGGGAGATRGTSQPAATRPAPPGAPRSSSSGGAPLRAGVGSGSGGGPAVAAVVPRVASLPPAAGG
GRAQARRVGEDAAAAEGRTPPARQPRAAQEPPIVISDSPPPSPRRPAGPGPLSFVSSSSAQVSSGPGG
GGLPQSSGRAARPRAAVAPRVRSPPRAAAAPVVSASADAAGPAPPAVPVDAHRAPRSRMTQAQTDTQA
QSLGRAGATDARGSGGPGAEGGPGVPRGTNTPGAAPHAAEGAAARPRKRRGSDSGPAASSSASSSAAP

RSPLAPQGVGAKRAAPRRAPDSDSGDRGHGPLAPASAGAAPPSASPSSQAAVAAASSSSASSSSASSSS
SASSSSASSSSASSSSASSSSASSSSAGGAGGSVASASGAGERRETSLGPRAAAPRGPRKCARKTRHAE
GGPEPGARDPAPGLTRYLPIAGVSSVVALAPYVNKTVTGDCLPVLDMETGHIGAYVVLVDQTGNVADL
LRAAAPAWSRRTLLPEHARNCVRPPDYPTPPASEWNSLWMTPVGNMLFDQGTLVGALDFHGLRSRHPW
SREQGAPAPAGDAPAGHGE

SEQ ID NO: 8 = ICP4 internal fragments (RS1.1, #1-400)

msaeqrkkkktttttqgrgaevamadedggrlraaaettggpgspdpadgppptpn

pdrrpaarpgfgwhggpeenedeaddaaadadadeaapasgeavdepaadgvvspr

qlallasmvdeavrtipsppperdgaqeeaarspspprtpsmradygeendddddd

dddddrdagrwvrgpettsavrgaypdpmaslsprppaprrhhhhhhrrrraprr

rsaasdssksgssssassasssasssssasasssddddddaarapasaadhaagg

tlgaddeeagvparapgaaprpsppraepapartpaatagrlerrraraavagrda

tgrftagrprrveldadaasgafyaryrdgyvsgepwpgagppppgrvlygglgds

rpglwgap

SEQ ID NO: 9 = ICP4 internal fragments (RS1.3.1, #750-1024)

ssaaaaaadllfqnqslrplladtvaaadslaapasaprearkrkspaparappgg

aprppkksradaprpaaappagaappapptppprpprpaaltrrpaegpdpqggwr

rqppgpshtpapsaaaleaycapravaeltdhplfpapwrpalmfdpralaslaar

caapppggapaafgplrasgplrraaawmrqvpdpedvrvvilysplpgedlaagr

agggpppewsaergglscllaalgnrlcgpataawagnwtgapdvsalgaq

SEQ ID NO: 10 = ICP4 internal fragments (RS1.3.2, #1008-1319)

wagnwtgapdvsalgaqgvlllstrdlafagaveflgllagacdrrlivvnavraa

dwpadgpvvsrqhaylacevlpavqcavrwpaardlrrtvlasgrvfgpgvfarve

aaharlypdapplrlcrganvryrvrtrfgpdtlvpmspreyrravlpaldgraaa

sgagdamapgapdfcedeahshracarwglgaplrpvyvalgrdavrggpaelrgp

rrefcarallepdgdapplvlrddadagpppqirwasaagragtvlaaaggvevv

gtaaglatpprrepvdmdaeleddddglfge

SEQ ID NO: 11 = ICP4 internal fragments (RS1.3, #750-1319)

Ssaaaaaadllfqnqslrplladtvaaadslaapasaprearkrkspaparappgg
aprppkksradaprpaaappagaappapptppprpprpaaltrrpaegpdpqggwr
rqppgpshtpapsaaaleaycapravaeltdhplfpapwrpalmfdpralaslaar
caapppggapaafgplrasgplrraaawmrqvpdpedvrvvilysplpgedlaagr
agggpppewsaergglscllaalgnrlcgpataawagnwtgapdvsalgaqgvlll
strdlafagaveflgllagacdrrlivvnavraadwpadgpvvsrqhaylacevlp
avqcavrwpaardlrrtvlasgrvfgpgvfarveaaharlypdapplrlcrganvr
yrvrtrfgpdtlvpmspreyrravlpaldgraaasgagdamapgapdfcedeahsh
racarwglgaplrpvyvalgrdavrggpaelrgprrefcarallepdgdapplvlr
ddadagpppqirwasaagragtvlaaagggvevvgtaaglatpprrepvdmdaele
ddddglfge

SEQ ID NO: 12 = ICP4 internal fragments (RS1.4, #340-883)

tagrprrveldadaasgafyaryrdgyvsgepwpgagppppgrvlygglgdsrpgl

wgapeaeeararfeasgapapvwapelgdaaqqyalitrllytpdaeamgwlqnpr

vapgdvaldqacfrisgaarnsssfisgsvaravphlgyamaagrfgwglahvaaa

vamsrrydraqkgflltslrrayapllarenaaltgartpddggdanrhdgddarg

kpaaaaaplpsaaaspaderavpagygaagvlaalgrlsaapasapagaddddddd

gaggggggrraeagrvaveclaacrgilealaegfdgdlaavpglagarpaapprp

gpagaaapphadaprlrawlrelrfvrdalvlmrlrgdlrvaggseaavaavravs

lvagalgpalprsprllssaaaaaadllfqnqslrplladtvaaadslaapasapr

earkrkspaparappggaprppkksradaprpaaappagaappapptppprpprpa

altrrpaegpdpqggwrrqppgpshtpapsaaaleayca

SEQ ID NO: 13 = ICP4 internal fragments (RS1.5, #775-1318)

aaadslaapasaprearkrkspaparappggaprppkksradaprpaaappagaap

papptppprpprpaaltrrpaegpdpqggwrrqppgpshtpapsaaaleaycapra

vaeltdhplfpapwrpalmfdpralaslaarcaapppggapaafgplrasgplrra

aawmrqvpdpedvrvvilysplpgedlaagragggpppewsaergglscllaalgn

rlcgpataawagnwtgapdvsalgaqgvlllstrdlafagaveflgllagacdrrl

ivvnavraadwpadgpvvsrqhaylacevlpavqcavrwpaardlrrtvlasgrvf

gpgvfarveaaharlypdapplrlcrganvryrvrtrfgpdtlvpmspreyrravl
paldgraaasgagdamapgapdfcedeahshracarwglgaplrpvyvalgrdavr
ggpaelrgprrefcarallepdgdapplvlrddadagpppqirwasaagragtvla
aagggvevvgtaaglatpprrepvdmdaeledddglfge

SEQ ID NO: 14 = ICP4 internal fragments (RS1.6, #209-1318)

hrrrraprrrsaasdssksgssssassasssasssssasasssdddddd
aarapasaadhaaggtlgaddeeagvparapgaaprpspprae papartpaatagr
lerrraraavagrdatgrftagrprrveldadaasgafyaryrdgyvsgepwpgag
ppppgrvlygglgdsrpglwgapeaeeararfeasgapapvwapelgdaaqqyali
trllytpdaeamgwlqnprvapgdvaldqacfrisgaarnsssfisgsvaravphl
gyamaagrfgwglahvaaavamsrrydraqkgflltslrrayapllarenaaltga
rtpddggdanrhdgddargkpaaaaplpsaaaspaderavpagygaagvlaalgr
lsaapasapagaddddddgagggggrraeagrvaveclaacrgilealaegfdg
dlaavpglagarpaapprpgpagaaapphadaprlrawlrelrfvrdalvlmrlrg
dlrvaggseaavaavravslvagalgpalprsprllssaaaaadllfqnqslrpl
ladtvaaadslaapasaprearkrkspaparappggaprppkksradaprpaaapp
agaappapptppprpprpaaltrrpaegpdpqggwrrqppgpshtpapsaaaleay
capravaeltdhplfpapwrpalmfdpralaslaarcaapppggapaafgplrasg
plrraaawmrqvpdpedvrvvilysplpgedlaagragggpppewsaergglscll
aalgnrlcgpataawagnwtgapdvsalgaqgvlllstrdlafagaveflgllaga
cdrrlivvnavraadwpadgpvvsrqhaylacevlpavqcavrwpaardlrrtvla
sgrvfgpgvfarveaaharlypdapplrlcrganvryrvrtrfgpdtlvpmsprey
rravlpaldgraaasgagdamapgapdfcedeahshracarwglgaplrpvyvalg
rdavrggpaelrgprrefcarallepdgdapplvlrddadagpppqirwasaagra
gtvlaaagggvevvgtaaglatpprrepvdmdaeledddglfge

SEQ ID NO: 15 = ICP4 internal fragments (RS1.7, deletion of 391-544)

msaeqrkkkktttttqgrgaevamadedggrlraaaettggpgspdpadgppptpn

pdrrpaarpgfgwhggpeenedeaddaaadadadeaapasgeavdepaadgvvspr

qlallasmvdeavrtipsppperdgaqeeaarspspprtpsmradygeendddddd

ddddddrdagrwvrgpettsavrgaypdpmaslsprppaprrhhhhhhrrrraprr
rsaasdssksgssssassasssasssssasasssddddddaarapasaadhaagg
tlgaddeeagvparapgaaprpsppraepapartpaatagrlerrraraavagrda
tgrftagrprrveldadaasgafyaryrdgyvsgepwpgagppppgrvlygglgar
tpddggdanrhdgddargkpaaaaplpsaaaspaderavpagygaagvlaalgrl
saapasapagaddddddgagggggrraeagrvaveclaacrgilealaegfdgd
laavpglagarpaapprpgpagaaapphadaprlrawlrelrfvrdalvlmrlrgd
lrvaggseaavaavravslvagalgpalprsprllssaaaaaadllfqnqslrpll
adtvaaadslaapasaprearkrkspaparappggaprppkksradaprpaaappa
gaappapptpppprpprpaaltrrpaegpdpqggwrrqppgpshtpapsaaaleayc
apravaeltdhplfpapwrpalmfdpralaslaarcaapppggapaafgplrasgp
lrraaawmrqvpdpedvrvvilysplpgedlaagragggpppewsaergglsclla
algnrlcgpataawagnwtgapdvsalgaqgvlllstrdlafagaveflgllagac
drrlivvnavraadwpadgpvvsrqhaylacevlpavqcavrwpaardlrrtvlas
grvfgpgvfarveaaharlypdapplrlcrganvryrvrtrfgpdtlvpmspreyr
ravlpaldgraaasgagdamapgapdfcedeahsracarwglgaplrpvyvalgr
davrggpaelrgprrefcarallepdgdapplvlrddadagpppqirwasaagrag
tvlaaagggvevvgtaaglatpprrepvdmdaeledddglfge

SEQ ID NO: 16 = ICP4 internal fragments (RS1.8, deletion of 786-864)

msaeqrkkkktttttqgrgaevamadedggrlraaaettggpgspdpadgppptpnpdrrpaarpgfg

whggpeenedeaddaaadadadeaapasgeavdepaadgvvsprqlallasmvdeavrtipsppperd

gaqeeaarspsspprtpsmradygeenddddddddddrdagrwvrgpettsavrgaypdpmaslsprp

paprrhhhhhhrrrraprrrsaasdssksgssssassasssasssssasasssddddddaarapas

aadhaaggtlgaddeeagvparapgaaprpsppraepapartpaatagrlerrraraavagrdatgrf

tagrprrveldadaasgafyaryrdgyvsgepwpgagppppgrvlygglgdsrpglwgapeaeearar

feasgapapvwapelgdaaqqyalitrllytpdaeamgwlqnprvapgdvaldqacfrisgaarnsss

fisgsvaravphlgyamaagrfgwglahvaaavamsrrydraqkgflltslrrayapllarenaaltg

artpddggdanrhdgddargkpaaaaaplpsaaaspaderavpagygaagvlaalgrlsaapasapag

adddddddgaggggggrraeagrvaveclaacrgilealaegfdgdlaavpglagarpaapprpgpag

aaapphadaprlrawlrelrfvrdalvlmrlrgdlrvaggseaavaavravslvagalgpalprsprl

lssaaaaaadllfqnqslrplladtvaaadslaapastpapsaaaleaycapravaeltdhplfpapw

rpalmfdpralaslaarcaapppggapaafgplrasgplrraaawmrqvpdpedvrvvilysplpged

laagragggpppewsaerggglscllaalgnrlcgpataawagnwtgapdvsalgaqgvlllstrdlaf

agaveflgllagacdrrlivvnavraadwpadgpvvsrqhaylacevlpavqcavrwpaardlrrtvl
asgrvfgpgvfarveaaharlypdapplrlcrganvryrvrtrfgpdtlvpmspreyrravlpaldgr
aaasgagdamapgapdfcedeahshracarwglgaplrpvyvalgrdavrggpaelrgprrefcaral
lepdgdapplvlrddadagpppqirwasaagragtvlaaagggvevvgtaaglatpprrepvdmdael
eddddglfge

SEQ ID NO: 17 = predicted sequence for uracil DNA glycosylase (encoded by UL2)

MFSASTTPEQPLGLSGDATPPLPTSVPLDWAAFRRAFLIDDAWRPLLEPELANPLTARLLAEYDRRCQ

TEEVLPPREDVFSWTRYCTPDDVRVVIIGQDPYHHPGQAHGLAFSVRADVPVPPSLRNVLAAVKNCYP

DARMSGRGCLEKWARDGVLLLNTTLTVKRGAAASHSKLGWDRFVGGVVQRLAARRPGLVFMLWGAHAQ

NAIRPDPRQHYVLKFSHPSPLSKVPFGTCQHFLAANRYLETRDIMPIDWSV

SEQ ID NO: 18 = predicted sequence for tegument protein encoded by UL11

MGLAFSGARPCCCRHNVITTDGGEVVSLTAHEFDVVDIESEEEGNFYVPPDVRVVTRAPGPQYRRASD

PPSRHTRRRDPDVARPPATLTPPLSDSE

SEQ ID NO: 19 = gL2 secreted

NRWGFVCLFGLVVMGAWGAWGGSQATEYVLRSVIAKEVGDILRVPCM

RTPADDVSWRYEAPSVIDYARIDGIFLRYHCPGLDTFLWDRHAQRAYLVNPFLFAAGFLEDLSHSVFP

ADTQETTTRRALYKEIRDALGSRKQAVSHAPVRAGCVNFDYSRTRRCVGRRDLRPANTTSTWEPPVSS

DDEASSQSKPLATQPPVLALSNAPPRRVSPTRGRRRHTRLRRN

SEQ ID NO: 20 = predicted sequence for VP5 encoded by UL19

DYDIPTTENLYFQGMAAPARDPPGYRYAAAMVPTGSILSTIEVASHRRLFDFFARVRS
DENSLYDVEFDALLGSYCNTLSLVRFLELGLSVACVCTKFPELAYMNEGRVQFEVHQPLIARDGPHPV
EQPVHNYMTKVIDRRALNAAFSLATEAIALLTGEALDGTGISLHRQLRAIQQLARNVQAVLGAFERGT
ADQMLHVLLEKAPPLALLLPMQRYLDNGRLATRVARATLVAELKRSFCDTSFFLGKAGHRREAIEAWL
VDLTTATQPSVAVPRLTHADTRGRPVDGVLVTTAAIKQRLLQSFLKVEDTEADVPVTYGEMVLNGANL
VTALVMGKAVRSLDDVGRHLLEMQEEQLEANRETLDELESAPQTTRVRADLVAIGDRLVFLEALEKRI
YAATNVPYPLVGAMDLTFVLPLGLFNPAMERFAAHAGDLVPAPGHPEPRAFPPRQLFFWGKDHQVLRL
SMENAVGTVCHPSLMNIDAAVGGVNHDPVEAANPYGAYVAAPAGPGADMQQRFLNAWRQRLAHGRVRW
VAECQMTAEQFMQPDNANLALELHPAFDFFAGVADVELPGGEVPPAGPGAIQATWRVVNGNLPLALCP
VAFRDARGLELGVGRHAMAPATIAAVRGAFEDRSYPAVFYLLQAAIHGSEHVFCALARLVTQCITSYW

NNTRCAAFVNDYSLVSYIVTYLGGDLPEECMAVYRDLVAHVEALAQLVDDFTLPGPELGGQAQAELNH
LMRDPALLPPLVWDCDGLMRHAALDRHRDCRIDAGEHEPVYAAACNVATADFNRNDGRLLHNTQARAA
DAADDRPHRPADWTVHHKIYYYVLVPAFSRGRCCTAGVRFDRVYATLQNMVVPEIAPGEECPSDPVTD
PAHPLHPANLVANTVNAMFHNGRVVVDGPAMLTLQVLAHNMAERTTALLCSAAPDAGANTASTANMRI
FDGALHAGVLLMAPQHLDHTIQNGEYFYVLPVHALFAGADHVANAPNFPPALRDLARHVPLVPPALGA
NYFSSIRQPVVQHARESAAGENALTYALMAGYFKMSPVALYHQLKTGLHPGFGFTVVRQDRFVTENVL
FSERASEAYFLGQLQVARHETGGGVSFTLTQPRGNVDLGVGYTAVAATATVRNPVTDMGNLPQNFYLG
RGAPPLLDNAAAVYLRNAVVAGNRLGPAQPLPVFGCAQVPRRAGMDHGQDAVCEFIATPVATDINYFR
RPCNPRGRAAGGVYAGDKEGDVIALMYDHGQSDPARPFAATANPWASQRFSYGDLLYNGAYHLNGASP
VLSPCFKFFTAADITAKHRCLERLIVETGSAVSTATAASDVQFKRPPGCRELVEDPCGLFQEAYPITC
ASDPALLRSARDGEAHARETHFTQYLIYDASPLKGLSL

SEQ ID NO: 21 = VP5 encoded by UL19ΔTEV

MAAPARDPPGYRYAAAMVPTGSILSTIEVASHRRLFDFFARVRSDENSLYDVEFDALL
GSYCNTLSLVRFLELGLSVACVCTKFPELAYMNEGRVQFEVHQPLIARDGPHPVEQPVHNYMTKVIDR
RALNAAFSLATEAIALLTGEALDGTGISLHRQLRAIQQLARNVQAVLGAFERGTADQMLHVLLEKAPP
LALLLPMQRYLDNGRLATRVARATLVAELKRSFCDTSFFLGKAGHRREAIEAWLVDLTTATQPSVAVP
RLTHADTRGRPVDGVLVTTAAIKQRLLQSFLKVEDTEADVPVTYGEMVLNGANLVTALVMGKAVRSLD
DVGRHLLEMQEEQLEANRETLDELESAPQTTRVRADLVAIGDRLVFLEALEKRIYAATNVPYPLVGAM
DLTFVLPLGLFNPAMERFAAHAGDLVPAPGHPEPRAFPPRQLFFWGKDHQVLRLSMENAVGTVCHPSL
MNIDAAVGGVNHDPVEAANPYGAYVAAPAGPGADMQQRFLNAWRQRLAHGRVRWVAECQMTAEQFMQP
DNANLALELHPAFDFFAGVADVELPGGEVPPAGPGAIQATWRVVNGNLPLALCPVAFRDARGLELGVG
RHAMAPATIAAVRGAFEDRSYPAVFYLLQAAIHGSEHVFCALARLVTQCITSYWNNTRCAAFVNDYSL
VSYIVTYLGGDLPEECMAVYRDLVAHVEALAQLVDDFTLPGPELGGQAQAELNHLMRDPALLPPLVWD
CDGLMRHAALDRHRDCRIDAGEHEPVYAAACNVATADFNRNDGRLLHNTQARAADAADDRPHRPADWT
VHHKIYYYVLVPAFSRGRCCTAGVRFDRVYATLQNMVVPEIAPGEECPSDPVTDPAHPLHPANLVANT
VNAMFHNGRVVVDGPAMLTLQVLAHNMAERTTALLCSAAPDAGANTASTANMRIFDGALHAGVLLMAP
QHLDHTIQNGEYFYVLPVHALFAGADHVANAPNFPPALRDLARHVPLVPPALGANYFSSIRQPVVQHA
RESAAGENALTYALMAGYFKMSPVALYHQLKTGLHPGFGFTVVRQDRFVTENVLFSERASEAYFLGQL
QVARHETGGGVSFTLTQPRGNVDLGVGYTAVAATATVRNPVTDMGNLPQNFYLGRGAPPLLDNAAAVY
LRNAVVAGNRLGPAQPLPVFGCAQVPRRAGMDHGQDAVCEFIATPVATDINYFRRPCNPRGRAAGGVY
AGDKEGDVIALMYDHGQSDPARPFAATANPWASQRFSYGDLLYNGAYHLNGASPVLSPCFKFFTAADI
TAKHRCLERLIVETGSAVSTATAASDVQFKRPPGCRELVEDPCGLFQEAYPITCASDPALLRSARDGE
AHARETHFTQYLIYDASPLKGLSL

SEQ ID NO: 22 = predicted sequence for ICP1/2 encoded by UL36

MIPAALPHPTMKRQGDRDIVVTGVRNQFATDLEPGGSVSCMRSSLSFLSLLFDVGPRDVLSAEAIEGC
LVEGGEWTRAAAGSGPPRMCSIIELPNFLEYPAARGGLRCVFSRVYGEVGFFGEPTAGLLETQCPAHT
FFAGPWAMRPLSYTLLTIGPLGMGLYRDGDTAYLFDPHGLPAGTPAFIAKVRAGDVYPYLTYYAHDRP
KVRWAGAMVFFVPSGPGAVAPADLTAAALHLYGASETYLQDEPFVERRVAITHPLRGEIGGLGALFVG
VVPRGDGEGSGPVVPALPAPTHVQTPGADRPPEAPRGASGPPDTPQAGHPNRPPDDVWAAALEGTPPA
KPSAPDAAASGPPHAAPPPQTPAGDAAEEAEDLRVLEVGAVPVGRHRARYSTGLPKRRRPTWTPPSSV
EDLTSGERPAPKAPPAKAKKKSAPKKKAPVAAEVPASSPTPIAATVPPAPDTPPQSGQGGGDDGPASP
SSPSVLETLGARRPPEPPGADLAQLFEVHPNVAATAVRLAARDAALAREVAACSQLTINALRSPYPAH
PGLLELCVIFFFERVLAFLIENGARTHTQAGVAGPAAALLDFTLRMLPRKTAVGDFLASTRMSLADVA
AHRPLIQHVLDENSQIGRLALAKLVLVARDVIRETDAFYGDLADLDLQLRAAPPANLYARLGEWLLER
SRAHPNTLFAPATPTHPEPLLHRIQALAQFARGEEMRVEAEAREMREALDALARGVDSVSQRAGPLTV
MPVPAAPGAGGRAPCPPALGPEAIQARLEDVRIQARRAIESAVKEYFHRGAVYSAKALQASDSHDCRF
HVASAAVVPMVQLLESLPAFDQHTRDVAQRAALPPPPPLATSPQAILLRDLLQRGQPLDAPEDLAAWL
SVLTDAATQGLIERKPLEELARSIHGINDQQARRSSGLAELQRFDALDAALAQQLDSDAAFVPATGPA
PYVDGGGLSPEATRMAEDALRQARAMEAAKMTAELAPEARSRLRERAHALEAMLNDARERAKVAHDAR
EKFLHKLQGVLRPLPDFVGLKACPAVLATLRASLPAGWTDLADAVRGPPPEVTAALRADLWGLLGQYR
EALEHPTPDTATALAGLHPAFVVVLKTLFADAPETPVLVQFFSDHAPTIAKAVSNAINAGSAAVATAS
PAATVDAAVRAHGALADAVSALGAAARDPASPLSFLAVLADSAAGYVKATRLALEARGAIDELTTLGS
AAADLVVQARRACAQPEGDHAALIDAAARATTAARESLAGHEAGFGGLLHAEGTAGDHSPSGRALQEL
GKVIGATRRRADELEAAVADLTAKMAAQRARGSSERWAAGVEAALDRVENRAEFDVVELRRLQALAGT
HGYNPRDFRKRAEQALAANAEAVTLALDTAFAFNPYTPENQRHPMLPPLAAIHRLGWSAAFHAAAETY
ADMFRVDAEPLARLLRIAEGLLEMAQAGDGFIDYHEAVGRLADDMTSVPGLRRYVPFFQHGYADYVEL
RDRLDAIRADVHRALGGVPLDLAAAAEQISAARNDPEATAELVRTGVTLPCPSEDALVACAAALERVD
QSPVKNTAYAEYVAFVTRQDTAETKDAVVRAKQQRAEATERVMAGLREALAARERRAQIEAEGLANLK
TMLKVVAVPATVAKTLDQARSVAEIADQVEVLLDQTEKTRELDVPAVIWLEHAQRTFETHPLSAARGD
GPGPLARHAGRLGALFDTRRRVDALRRSLEEAEAEWDEVWGRFGRVRGGAWKSPEGFRAMHEQLRALQ
DTTNTVSGLRAQPAYERLSARYQGVLGAKGAERAEAVEELGARVTKHTALCARLRDEVVRRVPWEMNF
DALGGLLAEFDAAAADLAPWAVEEFRGARELIQYRMGLYSAYARAGGQTGAGAESAPAPLLVDLRALD
ARARASSSPEGHEVDPQLLRRRGEAYLRAGGDPGPLVLREAVSALDLPFATSFLAPDGTPLQYALCFP
AVTDKLGALLMRPEAACVRPPLPTDVLESAPTVTAMYVLTVVNRLQLALSDAQAANFQLFGRFVRHRQ
ATWGASMDAAAELYVALVATTLTREFGCRWAQLGWASGAAAPRPPPGPRGSQRHCVAFNENDVLVALV
AGVPEHIYNFWRLDLVRQHEYMHLTLERAFEDAAESMLFVQRLTPHPDARIRVLPTFLDGGPPTRGLL
FGTRLADWRRGKLSETDPLAPWRSALELGTQRRDVPALGKLSPAQALAAVSVLGRMCLPSAALAALWT
CMFPDDYTEYDSFDALLAARLESGQTLGPAGGREASLPEAPHALYRPTGQHVAVLAAATHRTPAARVT
AMDLVLAAVLLGAPVVVALRNTTAFSRESELELCLTLFDSRPGGPDAALRDVVSSDIETWAVGLLHTD
LNPIENACLAAQLPRLSALIAERPLADGPPCLVLVDISMTPVAVLWEAPEPPGPPDVRFVGSEATEEL
PFVATAGDVLAASAADADPFFARAILGRPFDASLLTGELFPGHPVYQRPLADEAGPSAPTAARDPRDL
AGGDGGSGPEDPAAPPARQADPGVLAPTLLTDATTGEPVPPRMWAWIHGLEELASDDAGGPTPNPAPA
LLPPPATDQSVPTSQYAPRPIGPAATARETRPSVPPQQNTGRVPVAPRDDPRPSPPTPSPPADAALPP

PAFSGSAAAFSAAVPRVRRSRRTRAKSRAPRASAPPEGWRPPALPAPVAPVAASARPPDQPPTPESAP
PAWVSALPLPPGPASARGAFPAPTLAPIPPPPAEGAVVPGGDRRRGRRQTTAGPSPTPPRGPAAGPPR
RLTRPAVASLSASLNSLPSPRDPADHAAAVSAAAAAVPPSPGLAPPTSAVQTSPPPLAPGPVAPSEPL
CGWVVPGGPVARRPPPQSPATKPAARTRIRARSVPQPPLPQPPLPQPPLPQPPLPQPPLPQPPLPQPP
LPQPPLPQPPLPQPPLPQPPLPPVTRTLTPQSRDSVPTPESPTHTNTHLPVSAVTSWASSLALHVDSA
PPPASLLQTLHISSDDEHSDADSLRFSDSDDTEALDPLPPEPHLPPADEPPGPLAADHLQSPHSQFGP
LPVQANAVLSRRYVRSTGRSALAVLIRACRRIQQQLQRTRRALFQRSNAVLTSLHHVRMLLG

SEQ ID NO: 23 = ICP1/2 internal fragments encoded by UL36.3.4.1

AAQRARGSSERWAAGVEAALDRVENRAEFDVVELRRLQALAGTHGYNPRDFRKRAEQA
LAANAEAVTLALDTAFAFNPYTPENQRHPMLPPLAAIHRLGWSAAFHAAAETYADMFRVDAEPLARLL
RIAEGLLEMAQAGDGFIDYHEAVGRLADDMTSVPGLRRYVPFFQHGYADYVELRDRLDAIRADVHRAL
GGVPLDLAAAAEQISAARNDPEATAELVRTGVTLPCPSEDALVACAAALERVDQSPVKNTAYAEYVAF
VTRQDTAETKDAVVRAKQQRAEATERVMAGLREALAARERRAQIEAEGLANLKTMLKVVAVPATVAKT
LDQARSVAEIADQVEVLLDQTEKTRELDVPAVIWLEHAQRTFETHPLSAARGDGPGPLARHAGRLGAL
FDTRRRVDALRRSLEEAEAEWDEVWGRFGRVRGGAWKSPEGFRAMHEQLRALQDTTNTVSGLRAQPAY
ERLSARYQGVLGAKGAERAEAVEELGARVTKHTALCARLRDEVVRRVPWEMNFDALGGLLAEFDAAAA
DLAPWAVEEFRGARELIQYRMGLYSAYARAGGQTGAGAESAPAPLLVDLRALDARARASSSPEGHEVD
PQLLRRRGEAYLRAGGDPGPLVLREAVSALDLPFATSFLAPDGTPLQYALCFPAVTDKLGALLMRPEA
ACVRPPLPTDVLESAPTVTAMYVLTVVNRLQLALSDAQAANFQLFGRFVRHRQATWGASMDAAAELYV
ALVATTLTREFGCRWAQLGWASGAAAPRPPPGPRGSQRHCVAFNENDVLVALVAGVPEHIYNFWRLDL
VRQHEYMHLTLERAFEDAAESMLFVQRLTPHPDARIRVLPTFLDGGPPTRGLLFGTRLADWRRGKLSE
TDPLAPWRSALELGTQRRDVPALGKLSPAQALAAVSVLGRMCLPSAALAALWTCMFPDDYTEYDSFDA
LLAARLESGQTLGPAGGREASL

SEQ ID NO: 24 = ICP1/2 internal fragments encoded by UL36.4.2.5

EYDSFDALLAARLESGQTLGPAGGREASLPEAPHALYRPTGQHVAVLAAATHRTPAAR

VTAMDLVLAAVLLGAPVVVALRNTTAFSRESELELCLTLFDSRPGGPDAALRDVVSSDIETWAVGLLH

TDLNPIENACLAAQLPRLSALIAERPLADGPPCLVLVDISMTPVAVLWEAPEPPGPPDVRFVGSEATE

ELPFVATAGDVLAASAADADPFFARAILGRPFDASLLTGELFPGHPVYQRPLADEAGPSAPTAARDPR

DLAGGDGGSGPEDPAAPPARQADPGVLAPTLLTDATTGEPVPPRMWAWIHGLEELASDDAGGPTPNPA

PALLPPPATDQSVPTSQYAPRPIGPAATARETRPSVPPQQNTGRVPVAPRDDPRPSPPTPSPPADAAL

PPPAFSGSAAAFSAAVPRVRRSRRTRAKSRAPRASAPPEGWRPPALPAPVAPVAASARPPDQPPTPES

APPAWVSALPLPPGPASARGAFPAPTLAPIPPPPAEGAVVPGGDRRRGRRQTTAGPSPTPPRGPAAGP

PRRLTRPAVASLSASLNSLPSPRDPADHAAAVSAAAAAVPPSPGLAPPTSAVQTSPPPLAPGPVAPSE

PLCGWVVPGGPVARRPPPQSPATKPAARTRIRARSVPQPPLPQPPLPQPPLPQPPLPQPPLPQPPLPQ

PPLPQPPLPQPPLPQPPLPQPPLPQPPLPPVTRTLTPQSRDSVPTPESPTHTNTHLPVSAVTSWASSLALHVD

SAPPPASLLQTLHISSDDEHSDADSLRFSDSDDTEALDPLPPEPHLPPADEPPGPLAADHLQSPHSQF
GPLPVQANAVLSRRYVRSTGRSALAVLIRACRRIQQQLQRTRRALFQRSNAVLTSLHHVRMLLG

SEQ ID NO: 25 = predicted sequence for reductase encoded by UL40

MDPAVSPASTDPLDTHASGAGAAPIPVCPTPERYFYTSQCPDINHLRSLSILNRWLET

ELVFVGDEEDVSKLSEGELGFYRFLFAFLSAADDLVTENLGGLSGLFEQKDILHYYVEQECIEVVHSR

VYNIIQLVLFHNNDQARRAYVARTINHPAIRVKVDWLEARVRECDSIPEKFILMILIEGVFFAASFAA

IAYLRTNNLLRVTCQSNDLISRDEAVHTTASCYIYNNYLGGHAKPEAARVYRLFREAVDIEIGFIRSQ

APTDSSILSPGALAAIENYVRFSADRLLGLIHMQPLYSAPAPDASFPLSLMSTDKHTNFFECRSTSYA

GAVVNDL

SEQ ID NO: 26 = ICP47 encoded by US12

MSWALKTTDMFLDSSRCTHRTYGDVCAEIHKREREDREAARTAVTDPELPLLCPPDVRSDPASRNPTQ

QTRGCARSNERQDRVLAP

SEQ ID NO: 27 = gM2 encoded by UL10

MGRRAPRGSPEAAPGADVAPGARAAWWVWCVQVATFIVSAICVVGLLVLASVFRDRFPCLYAPATSYA

KANATVEVRGGVAVPLRLDTQSLLATYAITSTLLLAAAVYAAVGAVTSRYERALDAARRLAAARMAMP

HATLIAGNVCAWLLQITVLLLAHRISQLAHLIYVLHFACLVYLAAHFCTRGVLSGTYLRQVHGLIDPA

PTHHRIVGPVRAVMTNALLLGTLLCTAAAAVSLNTIAALNFNFSAPSMLICLTTLFALLVVSLLLVVE

GVLCHYVRVLVGPHLGAIAATGIVGLACEHYHTGGYYVVEQQWPGAQTGVRVALALVAAFALAMAVLR

CTRAYLYHRRHHTKFFVRMRDTRHRAHSALRRVRSSMRGSRRGGPPGDPGYAETPYASVSHHAEIDRY

GDSDGDPIYDEVAPDHEAELYARVQRPGPVPDAEPIYDTVEGYAPRSAGEPVYSTVRRW

SEQ ID NO: 28 = predicted sequence for cleavage/package protein encoded by
UL15
MFGQQLASDVQQYLERLEKQRQQKVGVDEASAGLTLGGDALRVPFLDFATATPKRHQTVVPGVGTLHD
CCEHSPLFSAVARRLLFNSLVPAQLRGRDFGGDHTAKLEFLAPELVRAVARLRFRECAPEDAVPQRNA
YYSVLNTFQALHRSEAFRQLVHFVRDFAQLLKTSFRASSLAETTGPPKKRAKVDVATHGQTYGTLELF
QKMILMHATYFLAAVLLGDHAEQVNTFLRLVFEIPLFSDTAVRHFRQRATVFLVPRRHGKTWFLVPLI
ALSLASFRGIKIGYTAHIRKATEPVFDEIDACLRGWFGSSRVDHVKGETISFSFPDGSRSTIVFASSH
NTNGIRGQDFNLLFVDEANFIRPDAVQTIMGFLNQANCKIIFVSSTNTGKASTSFLYNLRGAADELLN
VVTYICDDHMPRVVTHTNATACSCYILNKPVFITMDGAVRRTADLFLPDSFMQEIIGGQARETGDDRP
VLTKSAGERFLLYRPSTTTNSGLMAPELYVYVDPAFTANTRASGTGIAVVGRYRDDFIIFALEHFFLR

ALTGSAPADIARCVVHSLAQVLALHPGAFRSVRVAEGNSSQDSAVAIATHVHTEMHRILASAGANGP
GPELLFYHCEPPGGAVLYPFFLLNKQKTPAFEYFIKKFNSGGVMASQELVSVTVRLQTDPVEYLSEQL
NNLIETVSPNTDVRMYSGKRNGAADDLMVAVIMAIYLAAPTGIPPAFFPITRTS

SEQ ID NO: 29 = predicted sequence for ICP35 encoded by UL26.5

MNPVSASGAPAPPPPGDGSYLWIPASHYNQLVTGQSAPRHPPLTACGLPAAGTVAYGHPGAGPSPHYP

PPPAHPYPGMLFAGPSPLEAQIAALVGAIAADRQAGGLPAAAGDHGIRGSAKRRRHEVEQPEYDCGRD

EPDRDFPYYPGEARPEPRPVDSRRAARQASGPHETITALVGAVTSLQQELAHMRARTHAPYGPYPPVG

PYHHPHADTETPAQPPRYPAKAVYLPPPHIAPPGPPLSGAVPPPSYPPVAVTPGPAPPLHQPSPAHAH

PPPPPPGPTPPPAASLPQPEAPGAEAGALVNASSAAHVNVDTARAADLFVSQMMGSR

SEQ ID NO: 30 = predicted sequence for polymerase encoded by UL30

MFCAAGGPASPGGKPAARAASGFFAPHNPRGATQTAPPPCRRQNFYNPHLAQTGTQPKALGPAQRHTY

YSECDEFRFIAPRSLDEDAPAEQRTGVHDGRLRRAPKVYCGGDERDVLRVGPEGFWPRRLRLWGGADH

APEGFDPTVTVFHVYDILEHVEHAYSMRAAQLHERFMDAITPAGTVITLLGLTPEGHRVAVHVYGTRQ

YFYMNKAEVDRHLQCRAPRDLCERLAAALRESPGASFRGISADHFEAEVVERADVYYYETRPTLYYRV

FVRSGRALAYLCDNFCPAIRKYEGGVDATTRFILDNPGFVTFGWYRLKPGRGNAPAQPRPPTAFGTSS

DVEFNCTADNLAVEGAMCDLPAYKLMCFDIECKAGGEDELAFPVAERPEDLVIQISCLLYDLSTTALE

HILLFSLGSCDLPESHLSDLASRGLPAPVVLEFDSEFEMLLAFMTFVKQYGPEFVTGYNIINFDWPFV

LTKLTEIYKVPLDGYGRMNGRGVFRVWDIGQSHFQKRSKIKVNGMVNIDMYGIITDKVKLSSYKLNAV

AEAVLKDKKKDLSYRDIPAYYASGPAQRGVIGEYCVQDSLLVGQLFFKFLPHLELSAVARLAGINITR

TIYDGQQIRVFTCLLRLAGQKGFILPDTQGRFRGLDKEAPKRPAVPRGEGERPGDGNGDEDKDDDEDG

DEDGDEREEVARETGGRHVGYQGARVLDPTSGFHVDPVVVFDFASLYPSIIQAHNLCFSTLSLRPEAV

AHLEADRDYLEIEVGGRRLFFVKAHVRESLLSILLRDWLAMRKQIRSRIPQSTPEEAVLLDKQQAAIK

VVCNSVYGFTGVQHGLLPCLHVAATVTTIGREMLLATRAYVHARWAEFDQLLADFPEAAGMRAPGPYS

MRIIYGDTDSIFVLCRGLTAAGLVAMGDKMASHISRALFLPPIKLECEKTFTKLLLIAKKKYIGVICG

GKMLIKGVDLVRKNNCAFINRTSRALVDLLFYDDTVSGAAAALAERPAEEWLARPLPEGLQAFGAVLV

DAHRRITDPERDIQDFVLTAELSRHPRAYTNKRLAHLTVYYKLMARRAQVPSIKDRIPYVIVAQTREV

EETVARLAALRELDAAAPGDEPAPPAALPSPAKRPRETPSHADPPGGASKPRKLLVSELAEDPGYAIA

RGVPLNTDYYFSHLLGAACVTFKALFGNNAKITESLLKRFIPETWHPPDDVAARLRAAGFGPAGAGAT

AEETRRMLHRAFDTLA

SEQ ID NO: 31 = predicted sequence for helicase/primase complex encoded by
UL5

MAASGGEGSRDVRAPGPPPQQPGARPAVRFRDEAFLNFTSMHGVQPIIARIRELSQQQLDVTQVPRLQ
WFRDVAALEVPTGLPLREFPFAAYLITGNAGSGKSTCVQTLNEVLDCVVTGATRIAAQNMYVKLSGAF
LSRPINTIFHEFGFRGNHVQAQLGQHPYTLASSPASLEDLQRRDLTYYWEVILDITKRALAAHGGEDA
RNEFHALTALEQTLGLGQGALTRLASVTHGALPAFTRSNIIVIDEAGLLGRHLLTTVVYCWWMINALY
HTPQYAGRLRPVLVCVGSPTQTASLESTFEHQKLRCSVRQSENVLTYLICNRTLREYTRLSHSWAIFI
NNKRCVEHEFGNLMKVLEYGLPITEEHMQFVDRFVVPESYITNPANLPGWTRLFSSHKEVSAYMAKLH
AYLKVTREGEFVVFTLPVLTFVSVKEFDEYRRLTQQPTLTMEKWITANASRITNYSQSQDQDAGHVRC
EVHSKQQLVVARNDITYVLNSQVAVTARLRKMVFGFDGTFRTFEAVLRDDSFVKTQGETSVEFAYRFL
SRLMFGGLIHFYNFLQRPGLDATQRTLAYGRLGELTAELLSLRRDAAGASATRAADTSDRSPGERAFN
FKHLGPRDGGPDDFPDDDLDVIFAGLDEQQLDVFYCHYALEEPETTAAVHAQFGLLKRAFLGRYLILR
ELFGEVFESAPFSTYVDNVIFRGCELLTGSPRGGLMSVALQTDNYTLMGYTYTRVFAFAEELRRRHAT
AGVAEFLEESPLPYIVLRDQHGFMSVVNTNISEFVESIDSTELAMAINADYGISSKLAMTITRSQGLS
LDKVAICFTPGNLRLNSAYVAMSRTTSSEFLHMNLNPLRERHERDDVISEHILSALRDPNVVIVY

SEQ ID NO: 32 = predicted sequence for helicase/primase complex encoded by
UL8
MEAPGIVWVEESVSAITLYAVWLPPRTRDCLHALLYLVCRDAAGEARARFAEVSVGSSDLQDFYGSPD
VSAPGAVAAARAATAPAASPLEPLGDPTLWRALYACVLAALERQTGRWALFVPLRLGWDPQTGLVVRV
ERASWGPPAAPRAALLDVEAKVDVDPLALSARVAEHPGARLAWARLAAIRDSPQCASSASLAVTITTR
TARFAREYTTLAFPPTRKEGAFADLVEVCEVGLRPRGHPQRVTARVLLPRGYDYFVSAGDGFSAPALV
ALFRQWHTTVHAAPGALAPVFAFLGPGFEVRGGPVQYFAVLGFPGWPTFTVPAAAAAESARDLVRGAA
ATHAACLGAWPAVGARVVLPPRAWPAVASEAAGRLLPAFREAVARWHPTATTIQLLDPPAAVGPVWTA
RFCFSGLQAQLLAALAGLGEAGLPEARGRAGLERLDALVAAAPSEPWARAVLERLVPDACDACPALRQ
LLGGVMAAVCLQIEQTASSVKFAVCGGTGAAFWGLFNVDPGDADAAHGAIQDARRALEASVRAVLSAN
GIRPRLAPSLAPEGVYTHVVTWSQTGAWFWNSRDDTDFLQGFPLRGAAYAAAAEVMRDALRRILRRPA
AGPPEEAVCAARGVMEDACDRFVLDAFGRRLDAEYWSVLTPPGEADDPLPQTAFRGGALLDAEQYWRR
VVRVCPGGGESVGVPVDLYPRPLVLPPVDCAHHLREILREIQLVFTGVLEGVWGEGGSFVYPFDEKIR
FLFP

SEQ ID NO: 33 = predicted sequence for unknown protein encoded by UL15.5

MDGAVRRTADLFLPDSFMQEIIGGQARETGDDRPVLTKSAGERFLLYRPSTTTNSGLMAPELYVYVDP

AFTANTRASGTGIAVVGRYRDDFIIFALEHFFLRALTGSAPADIARCVVHSLAQVLALHPGAFRSVRV

AVEGNSSQDSAVAIATHVHTEMHRILASAGANGPGPELLFYHCEPPGGAVLYPFFLLNKQKTPAFEYF

IKKFNSGGVMASQELVSVTVRLQTDPVEYLSEQLNNLIETVSPNTDVRMYSGKRNGAADDLMVAVIMA

IYLAAPTGIPPAFFPITRTS

SEQ ID NO: 34 = predicted sequence for cleavage and packaging protein encoded
by UL32

MATSAPGVPSSAAVREESPGSSWKEGAFERPYVAFDPDLLALNEALCAELLAACHVVGVPPASALDED
VESDVAPAPPRPRGAAREASGGRGPGSARGPPADPTAEGLLDTGPFAAASVDTFALDRPCLVCRTIEL
YKQAYRLSPQWVADYAFLCAKCLGAPHCAASIFVAAFEFVYVMDHHFLRTKKATLVGSFARFALTIND
IHRHFFLHCCFRTDGGVPGRHAQKQPRPTPSPGAAKVQYSNYSFLAQSATRALIGTLASGGDDGAGAG
AGGGSGTQPSLTTALMNWKDCARLLDCTEGKRGGGDSCCTRAAARNGEFEAAAGALAQGGEPETWAYA
DLILLLLAGTPAVWESGPRLRAAADARRAAVSESWEAHRGARMRDAAPRFAQFAEPQPQPDLDLGPLM
ATVLKHGRGRGRTGGECLLCNLLLVRAYWLAMRRLRASVVRYSENNTSLFDCIVPVVDQLEADPEAQP
GDGGRFVSLLRAAGPEAIFKHMFCDPMCAITEMEVDPWVLFGHPRADHRDELQLHKAKLACGNEFEGR
VCIALRALIYTFKTYQVFVPKPTALATFVREAGALLRRHSISLLSLEHTLCTYV

SEQ ID NO: 35 = predicted sequence for ICP1/2 fragment enoded by UL36.4.2

MEYDSFDALLAARLESGQTLGPAGGREASLPEAPHALYRPTGQHVAVLAAATHRTPAARVTAMDLVLA

AVLLGAPVVVALRNTTAFSRESELELCLTLFDSRPGGPDAALRDVVSSDIETWAVGLLHTDLNPIENA

CLAAQLPRLSALIAERPLADGPPCLVLVDISMTPVAVLWEAPEPPGPPDVRFVGSEATEELPFVATAG

DVLAASAADADPFFARAILGRPFDASLLTGELFPGHPVYQRPLADEAGPSAPTAARDPRDLAGGDGGS

GPEDPAAPPARQADPGVLAPTLLTDATTGEPVPPRMWAWIHGLEELASDDAGGPT

SEQ ID NO: 36 = predicted sequence for ICP27 encoded by UL54

MATDIDMLIDLGLDLSDSELEEDALERDEEGRRDDPESDSSGECSSSDEDMEDPCGDGGAEAIDAAIP

KGPPARPEDAGTPEASTPRPAARRGADDPPPATTGVWSRLGTRRSASPREPHGGKVARIQPPSTKAPH

PRGGRRGRRRGRGRYGPGGADSTPKPRRRVSRNAHNQGGRHPASARTDGPGATHGEARRGGEQLDVSG

GPRPRGTRQAPPPLMALSLTPPHADGRAPVPERKAPSADTIDPAVRAVLRSISERAAVERISESFGRS

ALVMQDPFGGMPFPAANSPWAPVLATQAGGFDAETRRVSWETLVAHGPSLYRTFAANPRAASTAKAMR

DCVLRQENLIEALASADETLAWCKMCIHHNLPLRPQDPIIGTAAAVLENLATRLRPFLQCYLKARGLC

GLDDLCSRRRLSDIKDIASFVLVILARLANRVERGVSEIDYTTVGVGAGETMHFYIPGACMAGLIEIL

DTHRQECSSRVCELTASHTIAPLYVHGKYFYCNSLF

SEQ ID NO: 37 = virion protein encoded by UL49.5

MTGKPARLGRWVVLLFVALVAGVPGEPPNAAGARGVIGDAQCRGDSAGVVSVPGVLVP

FYLGMTSMGVCMIAHVYQICQRALAAGSA

SEQ ID NO: 38 = gG2 encoded by US4

NRWGSGVPGPINPPNSDVVFPGGSPVAQYCYAYPRLDDPGPLGSADA
GRQDLPRRVVRHEPLGRSFLTGGLVLLAPPVRGFGAPNATYAARVTYYRLTRACRQPILLRQYGGCRG
GEPPSPKTCGSYTYTYQGGGPPTRYALVNASLLVPIWDRAAETFEYQIELGGELHVGLLWVEVGGEGP
GPTAPPQAARAEGGPCVPPVPAGRPWRSVPPVWYSAPNPGFRGLRFRERCLPPQTPAAPSDLPRVAFA
PQSLLVGITGRTFIRMARPTEDVGVLPPHWAPGALDDGPYAPFPPRPRFRR

SEQ ID NO: 39 = nucleotide sequence for RS1 (ICP4), full-length
ATGTCGTACTACCATCACCATCACCATCACAGTGCCGAACAGCGTAAAAGAAAAAACCACCACCAC
GACCCAAGGACGTGGAGCTGAAGTTGCTATGGCGGATGAGGATGGAGGCCGCTTGAGAGCTGCTGCTG
AGACTACTGGAGGACCTGGATCACCGGACCCTGCCGATGGACCCCCCCTACACCAAACCCCGATCGT
AGACCGGCTGCTAGACCTGGATTCGGATGGCATGGAGGACCCGAGGAAAACGAGGACGAGGCGGACGA
CGCCGCTGCCGACGCCGACGCCGATGAGGCTGCCCCTGCTTCTGGAGAGGCGGTAGACGAACCTGCTG
CCGATGGAGTTGTTAGCCCTAGGCAATTGGCTTTGTTGGCGAGCATGGTAGACGAGGCTGTGAGAACA
ATCCCTTCCCCTCCCCCTGAACGTGATGGAGCACAAGAGGAGGCGGCTAGGAGTCCCTCACCACCCCG
TACACCTTCTATGAGAGCGGATTACGGCGAGGAAAACGACGACGACGATGATGATGACGACGATG
ATCGTGATGCCGGACGCTGGGTTAGGGGACCTGAAACCACTTCTGCTGTCCGTGGAGCATACCCCGAT
CCTATGGCGAGTTTGAGCCCTAGACCACCTGCCCCGAGGAGACACCACCACCACCATCATAGGCG
TAGACGTGCTCCTAGACGTCGTTCTGCCGCTAGTGACTCTTCCAAATCTGGCTCTTCTTCATCTGCCT
CTTCCGCTTCATCTTCGGCCTCATCGTCCTCTTCGGCATCCGCTTCGAGTAGTGATGATGATGATGAC
GACGACGCTGCTAGAGCCCCCGCTTCTGCTGCCGACCACGCTGCTGGCGGAACTTTGGGAGCCGACGA
CGAGGAGGCGGGAGTTCCTGCTCGTGCCCCGGGAGCTGCTCCGAGGCCTTCTCCACCCCGTGCTGAAC
CTGCTCCGGCTAGAACACCGGCCGCTACTGCTGGTAGACTGGAGCGTAGACGTGCCCGTGCTGCTGTG
GCTGGTAGAGATGCTACTGGCCGCTTCACTGCTGGCCGTCCTAGACGTGTTGAACTGGACGCCGATGC
TGCTTCTGGTGCTTTCTACGCCCGTTACCGTGATGGTTACGTGTCTGGTGAACCTTGGCCTGGCGCTG
GTCCACCTCCGCCCGGACGTGTACTCTACGGTGGATTGGGCGATTCTCGCCCTGGTCTGTGGGGCGCT
CCGGAGGCTGAGGAGGCTAGAGCCCGTTTCGAGGCTTCTGGTGCCCCTGCTCCTGTTTGGGCTCCTGA
ATTGGGCGACGCTGCTCAACAATACGCCCTCATCACACGCTTGCTGTACACTCCCGACGCCGAGGCTA
TGGGATGGCTCCAAAACCCTAGAGTTGCCCCTGGTGATGTTGCTCTGGATCAGGCTTGTTTCCGTATC
TCCGGCGCTGCTCGTAACTCTTCTTCGTTCATCTCCGGTTCTGTGGCTAGAGCTGTGCCTCACTTGGG
ATACGCCATGGCCGCTGGACGTTTCGGCTGGGGACTGGCTCATGTTGCTGCCGCTGTAGCAATGTCTA
GACGCTACGACCGTGCTCAAAAAGGATTCTTGCTCACGTCACTGAGGCGTGCTTACGCCCCTTTGTTG
GCCCGTGAAAACGCTGCCCTCACTGGCGCCCGTACCCCCGATGACGGTGGCGACGCCAACCGCCACGA
TGGTGATGATGCTAGAGGCAAACCCGCTGCCGCTGCTGCTCCTTTGCCCTCTGCCGCCGCTTCCCCTG
CCGATGAACGTGCTGTTCCTGCCGGTTACGGTGCCGCTGGTGTGTTGGCTGCTTTGGGACGCTTGAGT
GCTGCCCCGGCTAGTGCCCCCGCTGGTGCCGATGACGATGACGATGACGATGGTGCTGGCGGAGGCGG
TGGCGGTAGACGTGCTGAGGCTGGACGTGTTGCTGTTGAATGCCTGGCTGCCTGTAGAGGAATCTTGG
AGGCTCTGGCCGAGGGATTCGACGGAGACTTGGCGGCTGTACCGGGACTGGCGGGAGCGAGGCCTGCC
GCTCCACCTCGCCCCGGTCCTGCTGGTGCTGCCGCTCCTCCTCATGCCGACGCTCCTAGACTCCGTGC

TTGGCTCCGTGAACTCCGTTTCGTTCGTGACGCTTTGGTTCTGATGAGACTGAGAGGCGACTTGAGAG
TGGCTGGAGGATCCGAGGCTGCTGTTGCTGCTGTCCGTGCTGTTTCTTTGGTTGCTGGTGCTTTGGGC
CCTGCTTTGCCGAGATCTCCCCGTTTGTTGTCGAGTGCCGCCGCTGCTGCCGCCGATTTGTTGTTCCA
AAACCAATCCCTCCGCCCTCTGCTCGCCGACACTGTTGCCGCTGCCGATTCTCTGGCTGCTCCGGCTT
CTGCCCCACGTGAAGCTCGTAAACGTAAATCACCCGCTCCGGCTCGTGCTCCCCCTGGTGGCGCCCCT
AGACCCCCTAAAAAATCCCGTGCCGATGCCCCTAGACCTGCTGCTGCTCCCCCCGCTGGTGCTGCTCC
CCCCGCTCCCCCTACTCCCCCCCCACGCCCACCTCGTCCCGCTGCCCTCACACGCCGTCCTGCTGAGG
GACCCGATCCACAAGGCGGCTGGCGTAGACAACCTCCTGGCCCATCCCATACACCGGCACCATCTGCC
GCTGCTTTGGAGGCTTACTGTGCTCCTCGTGCTGTGGCTGAACTCACCGATCATCCGCTGTTCCCTGC
TCCCTGGCGTCCCGCCCTCATGTTCGATCCTAGAGCTTTGGCTTCCTTGGCCGCTCGTTGTGCTGCCC
CTCCCCCTGGCGGTGCTCCGGCTGCTTTCGGTCCTCTCCGTGCCTCTGGTCCACTCCGCCGTGCCGCT
GCCTGGATGAGACAAGTTCCCGACCCTGAGGATGTTAGAGTTGTGATCTTGTACTCGCCCTTGCCTGG
CGAGGATTTGGCCGCTGGTAGAGCTGGCGGTGGCCCCCCTCCTGAATGGTCTGCTGAACGTGGTGGTT
TGTCTTGCTTGTTGGCCGCCCTGGGAAACCGTCTGTGTGGTCCTGCTACTGCTGCTTGGGCTGGAAAC
TGGACTGGCGCTCCCGATGTTTCTGCTCTCGGTGCTCAAGGAGTTTTGCTGCTCTCTACTCGTGACTT
GGCATTCGCTGGAGCTGTTGAATTCCTGGGACTCTTGGCTGGCGCTTGTGATAGGAGACTCATCGTCG
TAAACGCTGTGAGAGCTGCCGATTGGCCTGCCGATGGTCCTGTTGTGTCTCGTCAACACGCTTACTTG
GCTTGTGAAGTGTTGCCCGCTGTCCAATGTGCTGTTCGCTGGCCTGCTGCTCGTGATCTGAGGCGTAC
TGTTCTGGCTAGTGGTCGTGTTTTCGGACCTGGTGTTTTCGCTCGTGTCGAAGCTGCTCACGCTAGAC
TGTACCCCGATGCCCCACCCCTCCGTTTGTGTCGTGGAGCAAACGTTCGCTACCGTGTCCGTACTCGT
TTCGGACCCGATACTCTGGTTCCAATGTCCCCTCGTGAATACCGTCGTGCTGTTCTGCCTGCCCTCGA
TGGACGTGCTGCCGCTTCTGGCGCTGGTGACGCTATGGCTCCTGGCGCTCCGGACTTCTGTGAGGATG
AGGCTCACTCACATCGTGCCTGTGCCCGCTGGGGACTGGGCGCTCCATTGAGGCCTGTATACGTGGCA
CTGGGCCGTGATGCTGTTAGAGGCGGACCCGCTGAATTGAGAGGCCCTCGTCGTGAATTCTGTGCTAG
GGCTCTGCTCGAACCCGATGGAGATGCTCCTCCTTTGGTACTCCGTGACGACGCCGATGCTGGTCCTC
CCCCACAAATTCGCTGGGCTAGTGCTGCTGGACGTGCTGGTACTGTATTGGCTGCTGCTGGCGGTGGC
GTTGAAGTTGTTGGTACTGCCGCTGGACTCGCTACACCTCCCCGCCGTGAACCTGTAGACATGGATGC
TGAACTCGAGGATGATGACGACGGATTGTTCGGAGAGTAATAG

SEQ ID NO: 40 = US6ΔTMR

ATGAAGTTCCTCGTGAACGTGGCCCTGGTGTTCATGGTGGTGTACATCAGCTACATCTACGCCAACCG

TTGGAAGTACGCTCTGGCTGACCCATCCCTGAAGATGGCTGACCCCAACCGTTTCCGTGGCAAGAACC

TGCCCGTGCTGGACCAGCTGACCGACCCCCCTGGCGTGAAGCGTGTGTACCACATCCAGCCATCCCTC

GAAGACCCCTTCCAGCCCCCCTCCATCCCCATCACCGTGTACTACGCTGTGCTGGAACGCGCTTGCCG

TTCCGTGCTGCTGCACGCTCCTTCCGAGGCTCCCCAGATCGTGCGTGGTGCTTCCGACGAGGCTCGCA

AGCACACCTACAACCTGACTATCGCTTGGTACAGGATGGGTGACAACTGCGCTATCCCTATCACCGTC

ATGGAATACACCGAGTGCCCCTACAACAAGTCCCTGGGCGTGTGCCCTATCCGTACCCAGCCCCGTTG

GTCCTACTACGACTCCTTCAGCGCTGTGTCCGAGGACAACCTGGGTTTCCTGATGCACGCTCCCGCTT

TCGAGACTGCTGGCACCTACCTGCGTCTGGTCAAGATCAACGACTGGACCGAGATCACCCAGTTCATC
CTGGAACACCGTGCTCGTGCTTCGTGCAAGTACGCCCTGCCCCTGCGTATCCCTCCTGCTGCTTGCCT
GACCTCCAAGGCTTACCAGCAGGGCGTGACCGTGGACTCCATCGGCATGCTGCCCCGTTTCATCCCCG
AGAACCAGCGTACCGTGGCTCTGTACTCTCTGAAGATCGCTGGCTGGCACGGTCCTAAGCCCCCCTAC
ACCTCCACTCTGCTGCCCCCTGAGCTGTCCGACACCACCAACGCTACTCAGCCCGAGTTGGTGCCTGA
GGACCCCGAGGACTCCGCTCTGTTGGAGGACCCCGCTGGAACCGTGTCCTCCCAGATCCCCCCCAACT
GGCACATCCCTTCCATCCAGGACGTGGCCCCTCACCACGCTCCAGCTGCTCCCTCCAACCCCCGTCGT
CGTGCTCAGATGGCTCCCAAGCGTCTGCGTCTGCCCCACATCCGTGACGACGACGCTCCTCCATCCCA
CCAGCCCCTGTTCTACCACCACCACCATCACCACTAATAA

SEQ ID NO: 41 = nucleotide sequence for RL1 (ICP34.5)
ATGTCTCGTCGTCGTGGTCCTCGTCGTCGTGGTCCTCGTCGTCGTCCGCGTCCGGGTGCGCCGGCGGT
ACCACGCCCGGGTGCGCCGGCAGTGCCGCGTCCAGGCGCACTGCCTACCGCGGACTCTCAAATGGTGC
CGGCGTATGATTCTGGTACTGCCGTCGAATCTGCTCCGGCAGCGAGCTCCCTGCTGCGTCGTTGGCTG
CTGGTCCCTCAGGCGGACGATTCCGATGACGCAGACTACGCGGGCAACGACGACGCGGAGTGGGCTAA
CAGCCCGCCAAGCGAGGGTGGTGGCAAAGCGCCGGAGGCTCCGCACGCAGCGCCTGCCGCAGCGTGCC
CGCCTCCGCCTCCTCGTAAAGAACGTGGCCCTCAACGTCCTCTGCCGCCGCACCTGGCTCTGCGTCTG
CGTACTACCACTGAGTACCTGGCGCGTCTGTCTCTGCGTCGTCGCCGTCCGCCGGCTAGCCCGCCGGC
CGATGCACCGCGTGGCAAAGTGTGCTTCTCTCCACGTGTTCAAGTTCGTCACCTGGTGGCTTGGGAAA
CGGCTGCCCGTCTGGCTCGCCGTGGCAGCTGGGCACGTGAGCGCGCAGACCGTGACCGCTTCCGTCGC
CGTGTGGCGGCTGCTGAAGCCGTTATCGGCCCGTGCCTGGAACCTGAGGCTCGCGCTCGCGCGCGTGC
GCGCGCTCGTGCCCACGAAGATGGCGGTCCAGCAGAGGAAGAAGAGGCAGCTGCAGCAGCGCGCGGTA
GCTCCGCGGCTGCGGGTCCAGGTCGTCGTGCCGTA

SEQ ID NO: 42 = nucleotide seqeunce for RL2 (ICP0)

ATGTCGTACTACCATCACCATCACCATCACATGGAGCCACGTCCTGGTACTTCTTCTCGCGCTGATCC

TGGTCCTGAACGTCCGCCACGCCAGACTCCGGGCACCCAGCCGGCCGCCCCTCACGCTTGGGGCATGC

TGAACGATATGCAGTGGCTGGCGTCCTCTGATTCCGAAGAGGAGACTGAGGTTGGTATCAGCGATGAT

GATCTGCACCGCGACTCTACCAGCGAAGCAGGTTCCACTGACACCGAAATGTTTGAAGCGGGCCTGAT

GGATGCCGCGACCCCGCCGGCTCGTCCGCCGGCTGAACGTCAGGGTAGCCCTACGCCTGCGGATGCGC

AAGGCTCTTGTGGTGGTGGTCCAGTAGGCGAAGAGGAGGCTGAGGCCGGTGGCGGCGGTGATGTGTGT

GCGGTTTGTACCGATGAAATCGCACCGCCGCTGCGTTGTCAGTCTTTCCCGTGCCTGCACCCGTTTTG

CATTCCGTGCATGAAAACCTGGATCCCGCTGCGCAACACTTGCCCGCTGTGCAACACTCCGGTTGCTT

ATCTGATCGTTGGTGTAACCGCATCTGGTTCCTTTTCTACCATCCCGATTGTCAACGACCCACGTACG

CGTGTTGAGGCGGAGGCGGCTGTACGTGCGGGCACCGCGGTGGACTTTATCTGGACCGGTAACCCGCG

CACCGCGCCACGCTCCCTGTCTCTGGGTGGCCATACCGTTCGTGCTCTGAGCCCGACCCCACCTTGGC

CAGGCACCGATGACGAAGACGACGATCTGGCTGACGTTGACTATGTTCCGCCGGCACCGCGTCGCGCA

CCACGCCGTGGTGGCGGTGGCGCCGGTGCGACGCGCGGTACCTCCCAGCCGGCAGCAACTCGCCCAGC
ACCGCCGGGTGCCCCGCGTTCTAGCAGCTCCGGTGGCGCACCGCTGCGTGCTGGCGTGGGTTCTGGTT
CCGGTGGTGGTCCGGCCGTGGCGGCTGTCGTCCCGCGTGTGGCTTCTCTGCCACCGGCAGCTGGTGGC
GGTCGTGCTCAAGCTCGTCGTGTCGGCGAGGACGCAGCGGCTGCTGAGGGCCGTACTCCACCGGCCCG
TCAACCGCGCGCAGCACAGGAACCGCCGATCGTGATCTCCGATTCCCCGCCACCGAGCCCGCGTCGCC
CGGCGGGTCCGGGTCCGCTGTCTTTTGTATCCTCCAGCTCTGCTCAGGTAAGCAGCGGTCCTGGCGGT
GGCGGCCTGCCACAGTCCTCTGGTCGTGCTGCTCGTCCTCGTGCGGCGGTTGCTCCTCGTGTACGTTC
TCCGCCACGCGCTGCTGCCGCGCCGGTCGTTTCTGCCTCTGCTGACGCGGCAGGTCCGGCTCCGCCTG
CAGTTCCGGTTGATGCACACCGTGCACCGCGCTCTCGTATGACCCAGGCGCAGACTGATACCCAGGCA
CAATCCCTGGGTCGCGCGGGTGCGACTGACGCTCGTGGTAGCGGTGGTCCGGGCGCTGAAGGTGGCCC
GGGTGTTCCACGCGGTACTAACACTCCGGGCGCTGCGCCACACGCGGCTGAAGGTGCGGCTGCACGTC
CGCGTAAACGTCGTGGTTCCGACAGCGGTCCGGCTGCAAGCAGCAGCGCGAGCTCTTCCGCTGCGCCT
CGCAGCCCGCTGGCGCCGCAGGGTGTTGGCGCCAAGCGTGCTGCTCCGCGTCGTGCACCGGACTCCGA
TTCTGGCGACCGCGGTCACGGCCCGCTGGCCCCTGCTAGCGCAGGCGCTGCGCCGCCATCCGCCAGCC
CGTCTTCTCAGGCAGCTGTGGCTGCGGCGTCCTCTTCTTCCGCTAGCAGCTCTTCCGCCTCTTCTAGC
AGCGCGTCCTCTAGCAGCGCATCTTCCTCTTCTGCTTCTTCTTCTAGCGCTTCTAGCTCTTCCGCGTC
CTCTTCCGCTGGCGGTGCAGGCGGCTCTGTTGCTTCCGCCAGCGGCGCAGGTGAGCGTCGTGAAACGA
GCCTGGGCCCACGTGCTGCTGCACCGCGTGGCCCGCGTAAGTGTGCGCGCAAGACCCGCCACGCTGAA
GGCGGTCCGGAGCCGGGTGCGCGTGATCCGGCTCCGGGTCTGACCCGTTACCTGCCGATTGCGGGTGT
GTCCTCCGTTGTGGCACTGGCGCCGTATGTGAACAAAACTGTCACGGGCGATTGCCTGCCTGTTCTGG
ACATGGAAACCGGTCATATCGGCGCTTACGTCGTTCTGGTTGACCAAACCGGCAACGTGGCGGATCTG
CTGCGTGCGGCCGCTCCGGCTTGGTCCCGTCGTACCCTGCTGCCGGAACATGCTCGCAACTGTGTACG
CCCACCGGATTACCCAACCCCGCCGGCCTCCGAGTGGAACTCCCTGTGGATGACCCCGGTTGGTAACA
TGCTGTTCGACCAGGGCACGCTGGTTGGTGCTCTGGACTTTCACGGCCTGCGCTCCCGTCACCCGTGG
TCCCGTGAGCAAGGCGCTCCGGCCCCTGCGGGCGATGCCCCGGCTGGCCACGGCGAGAGTACTAGAGG
ATCATAA

SEQ ID NO: 43 = nucleotide sequence for UL36.3.4.1

ATGTCGTACTACCATCACCATCACCATCACGCCGCTCAACGTGCTAGGGGATCCTCTGAACGCTGGGC

TGCTGGTGTCGAGGCTGCTTTGGATAGAGTGGAGAACCGTGCCGAATTCGATGTTGTCGAGCTGAGGA

GACTCCAAGCTTTGGCTGGTACTCACGGCTACAACCCTCGTGATTTCCGTAAACGTGCCGAACAGGCT

TTGGCGGCAAACGCTGAGGCCGTAACATTGGCTCTGGACACTGCCTTCGCTTTCAACCCATACACGCC

CGAAAACCAACGTCATCCTATGCTCCCACCTCTCGCTGCTATTCACCGCCTGGGATGGAGCGCTGCTT

TCCATGCTGCTGCTGAAACTTACGCCGACATGTTCCGTGTCGATGCCGAACCACTGGCTAGACTGCTC

CGTATCGCTGAGGGACTGCTGGAGATGGCTCAAGCTGGCGACGGATTCATCGATTACCATGAGGCTGT

CGGTAGACTGGCCGATGATATGACTTCTGTGCCCGGATTGAGGCGCTACGTTCCTTTCTTCCAACATG

GCTACGCCGATTACGTGGAACTGAGAGATCGCCTGGATGCTATTAGGGCCGACGTCCATAGAGCACTC

GGTGGTGTTCCGCTGGATTTGGCGGCTGCTGCCGAACAAATTTCCGCTGCTCGTAACGATCCTGAGGC

TACTGCTGAATTGGTCCGTACTGGTGTAACATTGCCTTGCCCTAGTGAGGACGCTCTCGTGGCTTGTG
CTGCTGCCCTGGAGAGAGTCGATCAATCTCCCGTGAAAAACACGGCTTACGCCGAATACGTTGCCTTC
GTGACCCGTCAAGACACTGCTGAGACTAAAGACGCTGTGGTCCGTGCTAAACAACAACGTGCTGAGGC
CACTGAACGTGTTATGGCTGGCCTGAGAGAGGCTCTGGCTGCTAGAGAACGTCGTGCTCAAATTGAGG
CTGAGGGATTGGCAAACCTGAAAACCATGCTCAAAGTCGTGGCTGTACCCGCTACTGTTGCTAAAACT
CTCGACCAGGCTCGTAGTGTTGCCGAAATTGCCGATCAAGTCGAAGTGTTGCTGGATCAAACCGAAAA
AACTCGTGAACTGGATGTGCCTGCTGTGATCTGGCTCGAACACGCCCAAAGAACATTCGAGACACACC
CTTTGTCTGCCGCTCGTGGTGATGGTCCTGGACCCTTGGCTCGTCATGCTGGCCGCCTCGGTGCCCTC
TTCGATACTCGTCGTAGAGTAGACGCCTTGAGGAGATCCCTGGAGGAGGCTGAGGCTGAATGGACGA
AGTTTGGGGACGCTTCGGTAGAGTGAGGGGCGGAGCGTGGAAATCTCCGGAGGGATTCCGTGCAATGC
ATGAGCAACTGAGGGCCCTCCAAGACACAACAAACACCGTGTCTGGCCTGAGGGCTCAACCTGCTTAC
GAACGCTTGTCTGCTCGCTACCAAGGAGTACTCGGAGCGAAAGGCGCTGAGAGAGCTGAGGCTGTTGA
GGAACTCGGTGCTCGTGTCACTAAACACACCGCTCTGTGTGCTAGGCTGAGAGATGAGGTCGTCCGTA
GAGTGCCTTGGGAAATGAACTTCGATGCTCTGGGAGGATTGTTGGCTGAGTTCGATGCCGCTGCTGCC
GATTTGGCACCTTGGGCTGTAGAGGAATTCCGTGGTGCTAGAGAACTCATTCAATACCGTATGGGCCT
GTACTCTGCCTACGCTAGAGCTGGAGGACAAACTGGTGCTGGAGCTGAATCTGCTCCTGCTCCTTTGC
TCGTGGATCTGAGGGCTTTGGATGCTCGTGCTCGTGCTTCTTCTTCCCCTGAGGGACATGAAGTGGAC
CCACAACTGCTGAGGAGGCGTGGAGAGGCTTACTTGAGAGCTGGCGGCGACCCTGGACCTCTCGTGCT
CCGTGAAGCTGTTTCTGCTTTGGACCTGCCATTCGCCACATCTTTCTTGGCCCCCGATGGAACTCCCC
TCCAATACGCTTTGTGCTTCCCTGCCGTAACGGACAAACTCGGAGCTTTGCTCATGAGGCCCGAGGCC
GCTTGTGTTAGACCTCCTTTGCCTACCGATGTGCTGGAATCTGCCCCAACTGTGACTGCCATGTACGT
ACTCACTGTGGTCAACCGCCTCCAACTGGCATTGAGTGATGCTCAAGCGGCAAACTTCCAACTGTTCG
GTCGTTTCGTTCGTCATAGGCAGGCAACCTGGGGAGCGTCAATGGATGCCGCCGCTGAATTGTACGTT
GCCCTGGTGGCTACAACTCTCACACGTGAATTCGGTTGTCGCTGGGCACAATTGGGATGGGCTAGTGG
AGCTGCTGCTCCTAGACCCCCACCTGGACCCCGTGGCTCACAACGTCACTGTGTGGCATTCAACGAGA
ACGATGTCCTCGTCGCTTTGGTTGCCGGTGTTCCCGAACACATCTACAACTTCTGGCGCCTGGACTTG
GTCCGTCAACACGAGTACATGCACCTCACACTGGAGCGTGCCTTCGAGGATGCTGCCGAGTCTATGCT
CTTCGTTCAACGCCTCACTCCACATCCCGACGCTCGTATTAGAGTTCTGCCGACCTTCTTGGATGGTG
GTCCTCCTACACGTGGTCTGTTGTTCGGAACCCGCTTGGCGGACTGGCGTCGTGGTAAACTGTCTGAA
ACCGACCCATTGGCCCCATGGAGATCTGCTTTGGAACTCGGAACCCAACGTCGTGACGTGCCTGCTTT
GGGAAAACTGTCCCCTGCTCAAGCTTTGGCCGCTGTGTCGGTACTGGGCCGTATGTGCTTGCCCTCGG
CTGCCTTGGCTGCTTTGTGGACCTGTATGTTCCCCGACGACTACACTGAATACGACTCATTCGACGCC
CTCTTGGCGGCTCGCCTGGAATCGGGACAAACATTGGGACCTGCTGGCGGTAGAGAGGCTTCATTGTA
ATAG

SEQ ID NO: 44 = nucleotide sequence for UL36.4.2.5

ATGTCGTACTACCATCACCATCACCATCACGAATACGACTCCTTCGACGCTTTGTTGGCTGCTAGACT

GGAATCTGGTCAAACCTTGGGACCCGCTGGCGGTAGAGAGGCTTCTTTGCCCGAGGCTCCTCATGCTT

TGTACCGTCCAACCGGACAACATGTTGCTGTGTTGGCGGCTGCTACTCATAGAACCCCTGCTGCTCGT
GTTACTGCTATGGACCTGGTCTTGGCGGCCGTTTTGCTGGGCGCTCCTGTGGTGGTCGCTCTGAGAAA
CACTACTGCCTTCTCCCGTGAATCCGAATTGGAACTGTGCCTCACCCTGTTCGATTCTCGTCCCGGCG
GACCGGATGCTGCCCTGAGAGATGTGGTATCCTCCGACATTGAAACCTGGGCTGTGGGCTTGCTCCAC
ACCGATTTGAACCCTATTGAGAACGCTTGCTTGGCGGCTCAACTGCCACGCTTGTCTGCCCTCATTGC
TGAACGTCCTTTGGCCGATGGACCCCCTTGTTTGGTGTTGGTGGACATTTCGATGACACCTGTCGCTG
TTTTGTGGGAGGCCCCTGAACCACCTGGCCCTCCCGATGTTCGTTTCGTCGGTAGCGAGGCCACTGAG
GAATTGCCTTTCGTGGCTACTGCTGGTGATGTTTTGGCGGCGAGTGCTGCCGATGCCGATCCTTTCTT
CGCTCGTGCTATCCTGGGCCGTCCTTTCGATGCTTCTCTGCTCACTGGTGAACTGTTCCCTGGTCACC
CCGTTTACCAACGTCCCCTGGCGGATGAGGCTGGTCCTTCTGCTCCTACTGCCGCTCGTGATCCTAGA
GATCTGGCTGGAGGCGACGGTGGATCCGGACCTGAGGATCCCGCTGCTCCACCTGCTAGACAGGCCGA
TCCTGGTGTTTTGGCTCCTACTCTGCTCACCGATGCTACTACTGGCGAACCTGTGCCACCCCGTATGT
GGGCTTGGATTCATGGACTGGAGGAACTGGCTTCCGATGATGCCGGCGGTCCTACCCCAAACCCTGCC
CCGGCTTTGCTGCCCCCTCCTGCTACGGATCAATCTGTCCCCACTTCCCAATACGCCCCTAGACCAAT
TGGCCCGGCTGCCACTGCTAGAGAAACTCGTCCTTCCGTTCCCCCTCAACAAAACACTGGTCGTGTCC
CTGTGGCTCCACGTGATGACCCTAGACCTTCCCCCCCTACTCCTTCCCCCCCTGCCGATGCTGCTTTG
CCACCTCCTGCCTTCTCTGGTTCTGCTGCTGCTTTCTCCGCTGCTGTTCCACGTGTTCGTCGTTCTAG
GCGTACTCGTGCCAAATCCCGTGCCCCTCGTGCTTCTGCCCCACCCGAGGGATGGCGTCCCCCCGCTT
TGCCTGCCCCTGTTGCTCCTGTGGCGGCTTCTGCTCGTCCCCCCGATCAACCTCCTACTCCCGAATCT
GCTCCCCCGGCTTGGGTTTCCGCTCTGCCATTGCCACCCGGACCTGCTAGTGCTCGTGGTGCTTTCCC
TGCTCCAACCTTGGCCCCTATTCCCCCACCCCCCGCTGAGGGAGCTGTTGTTCCCGGTGGTGATCGTA
GACGTGGTCGCCGTCAAACAACTGCTGGACCATCCCCTACACCGCCACGTGGCCCGGCTGCTGGTCCT
CCTCGTCGCCTCACTAGGCCTGCTGTTGCTAGTCTGTCCGCTTCTTTGAACTCTCTGCCTTCCCCCCG
TGATCCTGCCGATCATGCTGCTGCCGTTTCTGCTGCCGCCGCTGCCGTACCACCTTCACCTGGACTGG
CTCCCCCAACTTCTGCTGTCCAAACCTCTCCTCCTCCCTTGGCGCCTGGTCCTGTTGCCCCATCTGAA
CCTTTGTGTGGCTGGGTTGTGCCTGGAGGCCCTGTTGCTAGACGTCCCCCACCCCAATCTCCGGCTAC
TAAACCGGCTGCTCGTACCCGTATTAGGGCTCGTTCTGTGCCCCAACCACCCTTGCCCCAACCTCCAC
TGCCTCAACCCCCCTTGCCTCAACCCCCTCTCCCCCAACCACCTCTGCCTCAACCTCCGCTGCCCCAA
CCTCCTTTGCCCCAACCTCCTTTGCCCCAACCTCCTTTGCCCCAACCTCCGCTGCCCCAACCTCCGCT
GCCACCTGTTACTCGTACACTCACTCCCCAATCTCGTGACTCTGTGCCTACACCTGAGTCTCCAACTC
ACACAAACACCCACTTGCCCGTTAGTGCTGTGACTTCTTGGGCTTCGTCCCTGGCTCTCCATGTGGAT
TCTGCCCCTCCCCCTGCTTCATTGCTCCAAACTCTCCACATTTCCTCCGATGATGAACACTCCGACGC
CGACTCACTCCGCTTCTCCGATTCCGATGACACTGAGGCTCTCGATCCTTTGCCTCCTGAACCTCACT
TGCCACCTGCCGATGAACCCCCCGGACCTCTGGCTGCCGACCATCTCCAATCACCTCACTCACAATTC
GGTCCTTTGCCCGTTCAAGCGAACGCTGTTCTGTCTCGTCGTTACGTGAGATCAACTGGCCGTTCTGC
CTTGGCTGTGCTCATTAGAGCTTGTCGCCGTATCCAACAACAACTCCAGCGTACTAGGAGAGCACTCT
TCCAACGCTCAAACGCCGTGCTCACATCACTCCACCATGTCCGTATGCTCTTGGGATAATAG

SEQ ID NO: 45 = nucleotide sequence for US12 (ICP47)

ATGTCTTGGGCTCTGAAAACCACCGACATGTTCCTGGACTCTTCTCGTTGCACCCACCGTACCTACGG
TGACGTTTGCGCTGAAATCCACAAACGTGAACGTGAAGACCGTGAAGCTGCTCGTACCGCTGTTACCG
ACCCGGAACTGCCGCTGCTGTGCCCGCCGGACGTTCGTTCTGACCCGGCTTCTCGTAACCCGACCCAG
CAGACCCGTGGTTGCGCTCGTTCTAACGAACGTCAGGACCGTGTTCTGGCTCCGTGA

SEQ ID NO: 46 = nucleotide sequence for US4

ATGAAGTTCCTCGTGAACGTGGCCCTGGTGTTCATGGTGGTGTACATCAGCTACATCTACGCTAACCG

TTGGGGTTCCGGCGTGCCCGGTCCCATCAACCCCCCCAACTCCGACGTGGTGTTCCCCGGTGGTTCCC

CCGTGGCTCAGTACTGCTACGCTTACCCCCGTCTGGACGACCCTGGTCCCCTGGGTTCTGCTGACGCT

GGTCGTCAGGACCTGCCCCGTCGTGTCGTGCGTCACGAGCCCCTGGGTCGTAGCTTCCTGACCGGTGG

CCTGGTGCTGTTGGCTCCCCCTGTGCGCGGTTTCGGTGCTCCCAACGCTACCTACGCTGCTCGTGTGA

CCTACTACCGTCTGACCCGTGCTTGCCGTCAGCCCATCCTGCTGCGTCAGTACGGTGGTTGCCGTGGT

GGAGAGCCCCCATCCCCCAAGACCTGCGGTTCTTACACCTACACCTACCAGGGTGGTGGTCCCCCTAC

CCGTTACGCTCTGGTCAACGCTTCCCTGCTGGTGCCCATCTGGGACCGTGCTGCTGAGACTTTCGAGT

ACCAGATCGAGCTGGGTGGCGAGCTGCACGTGGGTCTGCTGTGGGTGGAAGTGGGTGGAGAGGGTCCC

GGTCCTACCGCTCCTCCTCAGGCTGCTCGTGCTGAGGGTGGTCCTTGCGTGCCACCCGTGCCTGCTGG

TCGTCCTTGGCGTTCCGTGCCCCCCGTGTGGTACTCCGCTCCCAACCCCGGTTTCCGCGGTCTGCGTT

TCCGTGAGCGTTGCCTGCCTCCCCAGACCCCTGCTGCTCCTTCCGACCTGCCTCGTGTGGCTTTCGCT

CCCCAGTCCCTGCTCGTGGGTATCACCGGTCGTACCTTCATCCGTATGGCTCGTCCCACCGAGGACGT

GGGTGTCCTGCCTCCTCACTGGGCTCCAGGTGCTCTGGACGACGGTCCCTACGCTCCCTTCCCCCCTC

GTCCCCGTTTCCGTCGTCACCACCACCATCACCACTAATAA

SEQ ID NO: 117 = RS1.2

ATGTCGTACTACCATCACCATCACCATCACATGGTGCTGTACGGCGGGCTGGGCGACAGCCGCCCCGG

CCTCTGGGGGGCGCCCGAGGCGGAGGCGCGGCCCGGTTCGAGGCCTCGGGCGCCCCGGCGCCCG

TGTGGGCGCCCGAGCTGGGCGACGCGGCGCAGCAGTACGCCCTGATCACGCGGCTGCTGTACACGCCG

GACGCGGAGGCGATGGGGTGGCTCCAGAACCCGCGCGTGGCGCCCGGGGACGTGGCGCTGGACCAGGC

CTGCTTCCGGATCTCGGGCGCGGCGCGCAACAGCAGCTCCTTCATCTCCGGCAGCGTGGCGCGGGCCG

TGCCCCACCTGGGGTACGCCATGGCGGCGGGCCGCTTCGGCTGGGGCCTGGCGCACGTGGCGGCCGCC

GTGGCCATGAGCCGCCGCTACGACCGCGCGCAGAAGGGCTTCCTGCTGACCAGCCTGCGCCGCGCCTA

CGCGCCCCTGCTGGCGCGCGAGAACGCGGCGCTGACCGGGGCGCGGACCCCCGACGACGGCGGCGACG

CCAACCGCCGCGACGGCGACGACGCCCGCGGGAAGCCCGCCGCCGCCGCCGCCCCGTTGCCGTCGGCG

GCGGCGTCGCCGGCCGACGAGCGCGCGGTGCCCGCCGGCTACGGCGCCGCGGGGGTGCTCGCCGCCCT

GGGGCGCCTGAGCGCCGCGCCCGCCTCCGCGCCGGCCGGGGCCGACGACGACGACGACGACGACGACG

GCGCCGGCGGTGGTGGCGGTGGTGGCGGTGGTGGCGGCGGCCGGCGCGCGGAGGCGGGCCGCGTGGCC

GTGGAGTGCCTGGCCGCCTGCCGCGGGATCCTGGAGGCGCTGGCGGAGGGCTTCGACGGCGACCTGGC

GGCCGTGCCGGGGCTGGCCGGAGCCCGGCCCGCCGCGCCCCCGCGCCCGGGGCCCGCGGGCGCGGCCG

CCCCGCCGCACGCCGACGCGCCCCGCCTGCGCCTGGCTGCGCGAGCTGCGGTTCGTGCGCGACGCG
CTGGTGCTGATGCGCCTGCGCGGGACCTGCGCGTGGCCGGCGGCAGCGAGGCCGCCGTGGCCGCCGT
GCGCGCCGTGAGCCTGGTCGCCGGGGCCCTGGGCCCGGCGCTGCCGCGGAGCCCGCGCCTGCTGAGCT
CCGCCGCCGCCGCCGCCGCGGACCTGCTCTTCCAGAACCAGAGCCTGAGTACTAGAGGATCATAA

SEQ ID NO: 118 = UL1(cytoplasmic),gL full length

ATGTCGTACTACCATCACCATCACCATCACATGGGGTTCGTCTGTCTGTTTGGGCTTGTCGTTATGGG

AGCCTGGGGGGCGTGGGGTGGGTCACAGGCAACCGAATATGTTCTTCGTAGTGTTATTGCCAAAGAGG

TGGGGGACATACTAAGAGTGCCTTGCATGCGGACCCCCGCGGACGATGTTTCTTGGCGCTACGAGGCC

CCGTCCGTTATTGACTATGCCCGCATAGACGGAATATTTCTTCGCTATCACTGCCCGGGGTTGGACAC

GTTTTTGTGGGATAGGCACGCCCAGAGGGCGTATCTTGTTAACCCCTTTCTCTTTGCGGCGGGATTTT

TGGAGGACTTGAGTCACTCTGTGTTTCCGGCCGACACCCAGGAAACAACGACGCGCCGGGCCCTTTAT

AAAGAGATACGCGATGCGTTGGGCAGTCGAAAACAGGCCGTCAGCCACGCACCCGTCAGGGCCGGGTG

TGTAAACTTTGACTACTCACGCACTCGCCGCTGCGTCGGGCGACGCGATTTACGGCCTGCCAACACCA

CGTCAACGTGGGAACCGCCTGTGTCGTCGGACGATGAAGCGAGCTCGCAGTCGAAGCCCCTCGCCACC

CAGCCGCCCGTCCTCGCCCTTTCGAACGCCCCCCCACGGCGGGTCTCCCCGACGCGAGGTCGGCGCCG

GCATACTCGCCTCCGACGCAACTGA

SEQ ID NO: 119 = UL1(Secreted),gL full length (preferred Ag)

ATGAAGTTCCTCGTGAACGTGGCCCTGGTGTTCATGGTGGTGTACATCAGCTACATCTACGCCAACCG

TTGGGGGTTCGTCTGTCTGTTTGGGCTTGTCGTTATGGGAGCCTGGGGGGCGTGGGGTGGGTCACAGG

CAACCGAATATGTTCTTCGTAGTGTTATTGCCAAAGAGGTGGGGGACATACTAAGAGTGCCTTGCATG

CGGACCCCCGCGGACGATGTTTCTTGGCGCTACGAGGCCCCGTCCGTTATTGACTATGCCCGCATAGA

CGGAATATTTCTTCGCTATCACTGCCCGGGGTTGGACACGTTTTTGTGGATAGGCACGCCCAGAGGG

CGTATCTTGTTAACCCCTTTCTCTTTGCGGCGGATTTTTGGAGGACTTGAGTCACTCTGTGTTTCCG

GCCGACACCCAGGAAACAACGACGCGCCGGGCCCTTTATAAAGAGATACGCGATGCGTTGGGCAGTCG

AAAACAGGCCGTCAGCCACGCACCCGTCAGGGCCGGGTGTGTAAACTTTGACTACTCACGCACTCGCC

GCTGCGTCGGGCGACGCGATTTACGGCCTGCCAACACCACGTCAACGTGGGAACCGCCTGTGTCGTCG

GACGATGAAGCGAGCTCGCAGTCGAAGCCCCTCGCCACCCAGCCGCCCGTCCTCGCCCTTTCGAACGC

CCCCCCACGGCGGGTCTCCCCGACGCGAGGTCGGCGCCGGCATACTCGCCTCCGACGCAACCATCACC

ATCACCATCACTGA

SEQ ID NO: 120 UL19 delta TEV VP5 full length

ATGTCGTACTACCATCACCATCACCATCACATGGCCGCTCCTGCCCGCGACCCCCGGGTTACCGGTA

CGCCGCGGCCATGGTGCCCACCGGCTCCATCCTGAGTACGATCGAGGTGGCGTCCCACCGCAGACTCT

TTGATTTTTTCGCCCGCGTGCGCTCCGACGAAAACAGCCTGTATGACGTAGAGTTTGACGCCCTGCTG

GGGTCCTACTGCAACACCCTGTCGCTCGTGCGCTTTCTGGAGCTCGGCCTGTCCGTGGCGTGCGTGTG

CACCAAGTTCCCGGAGCTGGCTTACATGAACGAAGGGCGTGTGCAGTTCGAGGTCCACCAGCCCCTCA
TCGCCCGCGACGGCCCGCACCCCGTCGAGCAGCCCGTGCATAATTACATGACGAAGGTCATCGACCGC
CGGGCCCTGAACGCCGCCTTCAGCCTGGCCACCGAGGCCATTGCCCTGCTCACGGGGGAGGCCCTGGA
CGGGACGGGCATTAGCCTGCATCGCCAGCTGCGCGCCATCCAGCAGCTCGCGCGCAACGTCCAGGCCG
TCCTGGGGGCGTTTGAGCGCGGCACGGCCGACCAGATGCTGCACGTGCTGTTGGAGAAGGCGCCTCCC
CTGGCCCTGCTGTTGCCCATGCAACGATATCTCGACAACGGGCGCCTGGCGACCAGGGTTGCCCGGGC
GACCCTGGTCGCCGAGCTGAAGCGGAGCTTTTGCGACACGAGCTTCTTCCTGGGCAAGGCGGGCCATC
GCCGCGAGGCCATCGAGGCCTGGCTCGTGGACCTGACCACGGCGACGCAGCCGTCCGTGGCCGTGCCC
CGCCTGACGCACGCCGACACGCGCGGGCGGCCGGTCGACGGGGTGCTGGTCACCACCGCCGCCATCAA
ACAGCGCCTCCTGCAGTCCTTCCTGAAGGTGGAGGACACCGAGGCCGACGTGCCGGTGACCTACGGCG
AGATGGTCTTGAACGGGGCCAACCTCGTCACGGCGCTGGTGATGGGCAAGGCCGTGCGGAGCCTGGAC
GACGTGGGCCGCCACCTGCTGGAGATGCAGGAGGAGCAACTCGAGGCGAACCGGGAGACGCTGGATGA
ACTCGAAAGCGCCCCCCAGACAACGCGCGTGCGCGCGGATCTGGTGGCCATAGGCGACAGGCTGGTCT
TCCTGGAGGCCCTGGAGAAGCGCATCTACGCCGCCACCAACGTGCCCTACCCCCTGGTGGGCGCCATG
GACCTGACGTTCGTCCTGCCCCTGGGGCTGTTCAACCCGGCCATGGAGCGCTTCGCCGCGCACGCCGG
GGACCTGGTGCCCGCCCCCGGCCACCCGGAGCCCCGCGCGTTCCCTCCCCGGCAGCTGTTTTTTGGG
GAAAGGACCACCAGGTTCTGCGGCTGTCCATGGAGAACGCGGTCGGGACCGTGTGTCATCCTTCGCTC
ATGAACATCGACGCGGCCGTCGGGGGCGTGAACCACGACCCCGTCGAGGCCGCGAATCCGTACGGGGC
GTACGTCGCGGCCCCGGCCGGCCCCGGCGCGGACATGCAGCAGCGTTTTCTGAACGCCTGGCGGCAGC
GCCTCGCCCACGGCCGGGTCCGGTGGGTCGCCGAGTGCCAGATGACCGCGGAGCAGTTCATGCAGCCC
GACAACGCCAACCTGGCTCTGGAGCTGCACCCCGCGTTCGACTTCTTCGCGGGCGTGGCCGACGTCGA
GCTTCCCGGCGGCGAAGTCCCCCCGGCCGGTCCGGGGGCGATCCAGGCCACCTGGCGCGTGGTCAACG
GCAACCTGCCCCTGGCGCTGTGTCCGGTGGCGTTTCGTGACGCCCGGGGCCTGGAGCTCGGCGTTGGC
CGCCACGCCATGGCGCCGGCTACCATAGCCGCCGTCCGCGGGGCGTTCGAGGACCGCAGCTACCCGGC
GGTGTTCTACCTGCTGCAAGCCGCGATTCACGGCAGCGAGCACGTGTTCTGCGCCCTGGCGCGGCTCG
TGACTCAGTGCATCACCAGCTACTGGAACAACACGCGATGCGCGGCGTTCGTGAACGACTACTCGCTG
GTCTCGTACATCGTGACCTACCTCGGGGGCGACCTCCCCGAGGAGTGCATGGCCGTGTATCGGGACCT
GGTGGCCCACGTCGAGGCCCTGGCCCAGCTGGTGGACGACTTTACCCTGCCGGGCCCGGAGCTGGGCG
GGCAGGCTCAGGCCGAGCTGAATCACCTGATGCGCGACCCGGCGCTGCTGCCGCCCCTCGTGTGGGAC
TGCGACGGCCTTATGCGACACGCGGCCCTGGACCGCCACCGAGACTGCCGGATTGACGCGGGGGAGCA
CGAGCCCGTCTACGCGGCGGCGTGCAACGTGGCGACGGCCGACTTTAACCGCAACGACGGCCGGCTGC
TGCACAACACCCAGGCCCGCGCGGCCGACGCCGCCGACGACCGGCCGCACCGGCCGGCCGACTGGACC
GTCCACCACAAAATCTACTATTACGTGCTGGTGCCGGCCTTCTCGCGGGGGCGCTGCTGCACCGCGGG
GGTCCGCTTCGACCGCGTGTACGCCACGCTGCAGAACATGGTGGTCCCGGAGATCGCCCCCGGCGAGG
AGTGCCCGAGCGATCCCGTGACCGACCCCGCCCACCCGCTGCATCCCGCCAATCTGGTGGCCAACACG
GTCAACGCCATGTTCCACAACGGGCGCGTCGTCGTCGACGGCCCGCCATGCTCACGCTGCAGGTGCT
GGCGCACAACATGGCCGAGCGCACGACGGCGCTGCTGTGCTCCGCGGCGCCCGACGCGGGCGCCAACA
CCGCGTCGACGGCCAACATGCGCATCTTCGACGGGGCGCTGCACGCCGGCGTGCTGCTCATGGCCCCC
CAGCACCTGGACCACACCATCCAAAATGGCGAATACTTCTACGTCCTGCCCGTCCACGCGCTGTTTGC

GGGCGCCGACCACGTGGCCAACGCGCCCAACTTCCCCCGGCCCTGCGCGACCTGGCGCGCCACGTCC
CCCTGGTCCCCCGGCCCTGGGGCCAACTACTTCTCCTCCATCCGCCAGCCCGTGGTGCAGCACGCC
CGCGAGAGCGCGGCGGGGAGAACGCGCTGACCTACGCGCTCATGGCGGGTACTTCAAGATGAGCCC
CGTGGCCCTGTATCACCAGCTCAAGACGGGCCTCCACCCCGGGTTCGGGTTCACCGTCGTGCGGCAGG
ACCGCTTCGTGACCGAGAACGTGCTGTTTTCCGAGCGCGCGTCGGAGGCGTACTTTCTGGGCCAGCTC
CAGGTGGCCCGCCACGAAACGGGCGGGGGGTCAGCTTCACGCTCACCCAGCCGCGCGGAAACGTGGA
CCTGGGTGTGGGCTACACCGCCGTCGCGGCCACGGCCACCGTCCGCAACCCCGTTACGGACATGGGCA
ACCTCCCCCAAAACTTTTACCTCGGCCGCGGGGCCCCCCGCTGCTAGACAACGCGGCCGCCGTGTAC
CTGCGCAACGCGGTCGTGGCGGGAAACCGGCTGGGGCCGGCCCAGCCCCTCCCGGTCTTTGGCTGCGC
CCAGGTGCCGCGGCGCGCCGGCATGGACCACGGGCAGGATGCCGTGTGTGAGTTCATCGCCACCCCCG
TGGCCACGGACATCAACTACTTTCGCCGGCCCTGCAACCCGCGGGACGCGCGGCCGGCGGCGTGTAC
GCGGGGGACAAGGAGGGGGACGTCATAGCCCTCATGTACGACCACGGCCAGAGCGACCCGGCGCGGCC
CTTCGCGGCCACGGCCAACCCGTGGGCGTCGCAGCGGTTCTCGTACGGGGACCTGCTGTACAACGGGG
CCTATCACCTCAACGGGGCCTCGCCCGTCCTCAGCCCCTGCTTCAAGTTCTTCACCGCGGCCGACATC
ACGGCCAAACATCGCTGCCTGGAGCGTCTTATCGTGGAAACGGGATCGGCGGTATCCACGGCCACCGC
TGCCAGCGACGTGCAGTTTAAGCGCCCGCCGGGGTGCCGCGAGCTCGTGGAAGACCCGTGCGGCCTGT
TTCAGGAAGCCTACCCGATCACCTGCGCCAGCGACCCCGCCCTGCTACGCAGCGCCCGCGATGGGGAG
GCCCACGCGCGAGAGACCCACTTTACGCAGTATCTCATCTACGACGCCTCCCCGCTAAAGGCCTGTC
TCTGTAA

SEQ ID NO: 121 = RS1.1

Atgagtgccgaacagcgtaaaaagaaaaaaccaccaccacgacccaaggacgtggagctgaagttgc
tatggcggatgaggatggaggccgcttgagagctgctgctgagactactggaggacctggatcaccgg
accctgccgatggaccccccctacaccaaaccccgatcgtagaccggctgctagacctggattcgga
tggcatggaggacccgaggaaaacgaggacgaggcggacgacgccgctgccgacgccgacgccgatga
ggctgcccctgcttctggagaggcggtagacgaacctgctgccgatggagttgttagccctaggcaat
tggctttgttggcgagcatggtagacgaggctgtgagaacaatcccttcccctcccctgaacgtgat
ggagcacaagaggaggcggctaggagtccctcaccaccccgtacaccttctatgagagcggattacgg
cgaggaaaacgacgacgacgacgatgatgatgacgacgatgatcgtgatgccggacgctgggttaggg
gacctgaaaccacttctgctgtccgtggagcataccccgatcctatggcgagtttgagccctagacca
cctgccccgaggagacaccaccaccaccaccatcataggcgtagacgtgctcctagacgtcgttctgc
cgctagtgactcttccaaatctggctcttcttcatctgcctcttccgcttcatcttcggcctcatcgt
cctcttcggcatccgcttcgagtagtgatgatgatgatgacgacgacgctgctagagcccccgcttct
gctgccgaccacgctgctggcggaactttgggagccgacgacgaggaggcgggagttcctgctcgtgc
cccgggagctgctccgaggccttctccaccccgtgctgaacctgctccggctagaacaccggccgcta
ctgctggtagactggagcgtagacgtgcccgtgctgctgtggctggtagagatgctactggccgcttc
actgctggccgtcctagacgtgttgaactggacgccgatgctgcttctggtgctttctacgcccgtta

ccgtgatggttacgtgtctggtgaaccttggcctggcgctggtccacctccgcccggacgtgtactct
acggtggattgggcgattctcgccctggtctgtggggcgctccg

SEQ ID NO: 122 = RS1.3.1

tcgagtgccgccgctgctgccgccgatttgttgttccaaaaccaatccctccgccctctgctcgccga

cactgttgccgctgccgattctctggctgctccggcttctgccccacgtgaagctcgtaaacgtaaat

cacccgctccggctcgtgctccccctggtggcgcccctagacccccctaaaaatcccgtgccgatgcc

cctagacctgctgctgctccccccgctggtgctgctccccccgctcccctactccccccccacgccc

acctcgtcccgctgccctcacacgccgtcctgctgagggacccgatccacaaggcggctggcgtagac

aacctcctggcccatcccatacaccggcaccatctgccgctgctttggaggcttactgtgctcctcgt

gctgtggctgaactcaccgatcatccgctgttccctgctccctggcgtcccgccctcatgttcgatcc

tagagctttggcttccttggccgctcgttgtgctgcccctccccctggcggtgctccggctgctttcg

gtcctctccgtgcctctggtccactccgccgtgccgctgcctggatgagacaagttcccgaccctgag

gatgttagagttgtgatcttgtactcgcccttgcctggcgaggatttggccgctggtagagctggcgg

tggccccccctcctgaatggtctgctgaacgtggtggtttgtcttgcttgttggccgccctgggaaacc

gtctgtgtggtcctgctactgctgcttgggctggaaactggactggcgctcccgatgtttctgctctc

ggtgctcaa

SEQ ID NO: 123 = RS1.3.2

Tgggctggaaactggactggcgctcccgatgtttctgctctcggtgctcaaggagttttgctgctctc

tactcgtgacttggcattcgctggagctgttgaattcctgggactcttggctggcgcttgtgatagga

gactcatcgtcgtaaacgctgtgagagctgccgattggcctgccgatggtcctgttgtgtctcgtcaa

cacgcttacttggcttgtgaagtgttgcccgctgtccaatgtgctgttcgctggcctgctgctcgtga

tctgaggcgtactgttctggctagtggtcgtgttttcggacctggtgttttcgctcgtgtcgaagctg

ctcacgctagactgtaccccgatgccccacccctccgtttgtgtcgtggagcaaacgttcgctaccgt

gtccgtactcgtttcggacccgatactctggttccaatgtcccctcgtgaataccgtcgtgctgttct

gcctgccctcgatggacgtgctgccgcttctggcgctggtgacgctatggctcctggcgctccggact

tctgtgaggatgaggctcactcacatcgtgcctgtgcccgctggggactgggcgctccattgaggcct

gtatacgtggcactgggccgtgatgctgttagaggcggacccgctgaattgagaggccctcgtcgtga

attctgtgctagggctctgctcgaacccgatggagatgctcctcctttggtactccgtgacgacgccg

atgctggtcctcccccacaaattcgctgggctagtgctgctggacgtgctggtactgtattggctgct

gctggcggtggcgttgaagttgttggtactgccgctggactcgctacacctccccgccgtgaacctgt

agacatggatgctgaactcgaggatgatgacgacggattgttcggagag

SEQ ID NO: 124 = RS1.3

tcgagtgccgccgctgctgccgccgatttgttgttccaaaaccaatccctccgccctctgctcgccga

cactgttgccgctgccgattctctggctgctccggcttctgccccacgtgaagctcgtaaacgtaaat

cacccgctccggctcgtgctccccctggtggcgcccctagacccccctaaaaatcccgtgccgatgcc

cctagacctgctgctgctcccccgctggtgctgctcccccgctcccctactcccccccacgccc
acctcgtcccgctgccctcacacgccgtcctgctgagggacccgatccacaaggcggctggcgtagac
aacctcctggcccatcccatacaccggcaccatctgccgctgctttggaggcttactgtgctcctcgt
gctgtggctgaactcaccgatcatccgctgttccctgctccctggcgtcccgccctcatgttcgatcc
tagagctttggcttccttggccgctcgttgtgctgcccctcccctggcggtgctccggctgctttcg
gtcctctccgtgcctctggtccactccgccgtgccgctgcctggatgagacaagttcccgaccctgag
gatgttagagttgtgatcttgtactcgcccttgcctggcgaggatttggccgctggtagagctggcgg
tggccccctcctgaatggtctgctgaacgtggtggtttgtcttgcttgttggccgccctgggaaacc
gtctgtgtggtcctgctactgctgcttgggctggaaactggactggcgctcccgatgtttctgctctc
ggtgctcaaggagttttgctgctctctactcgtgacttggcattcgctggagctgttgaattcctggg
actcttggctggcgcttgtgataggagactcatcgtcgtaaacgctgtgagagctgccgattggcctg
ccgatggtcctgttgtgtctcgtcaacacgcttacttggcttgtgaagtgttgcccgctgtccaatgt
gctgttcgctggcctgctgctcgtgatctgaggcgtactgttctggctagtggtcgtgttttcggacc
tggtgttttcgctcgtgtcgaagctgctcacgctagactgtaccccgatgccccacccctccgtttgt
gtcgtggagcaaacgttcgctaccgtgtccgtactcgtttcggacccgatactctggttccaatgtcc
cctcgtgaataccgtcgtgctgttctgcctgccctcgatggacgtgctgccgcttctggcgctggtga
cgctatggctcctggcgctccggacttctgtgaggatgaggctcactcacatcgtgcctgtgcccgct
ggggactgggcgctccattgaggcctgtatacgtggcactgggccgtgatgctgttagaggcggaccc
gctgaattgagaggccctcgtcgtgaattctgtgctagggctctgctcgaacccgatggagatgctcc
tcctttggtactccgtgacgacgccgatgctggtcctcccccacaaattcgctgggctagtgctgctg
gacgtgctggtactgtattggctgctgctggcggtggcgttgaagttgttggtactgccgctggactc
gctacacctccccgccgtgaacctgtagacatggatgctgaactcgaggatgatgacgacggattgtt
cggagag

SEQ ID NO: 125 = RS1.4

actgctggccgtcctagacgtgttgaactggacgccgatgctgcttctggtgctttctacgcccgtta

ccgtgatggttacgtgtctggtgaaccttggcctggcgctggtccacctccgcccggacgtgtactct

acggtggattgggcgattctcgccctggtctgtggggcgctccggaggctgaggaggctagagcccgt

ttcgaggcttctggtgcccctgctcctgtttgggctcctgaattgggcgacgctgctcaacaatacgc

cctcatcacacgcttgctgtacactcccgacgccgaggctatgggatggctccaaaaccctagagttg

cccctggtgatgttgctctggatcaggcttgtttccgtatctccggcgctgctcgtaactcttcttcg

ttcatctccggttctgtggctagagctgtgcctcacttgggatacgccatggccgctggacgtttcgg

ctggggactggctcatgttgctgccgctgtagcaatgtctagacgctacgaccgtgctcaaaaaggat

tcttgctcacgtcactgaggcgtgcttacgcccctttgttggcccgtgaaaacgctgccctcactggc

gcccgtacccccgatgacggtggcgacgccaaccgccacgatggtgatgatgctagaggcaaacccgc

tgccgctgctgctcctttgccctctgccgccgcttcccctgccgatgaacgtgctgttcctgccggtt

acggtgccgctggtgtgttggctgctttgggacgcttgagtgctgccccggctagtgcccccgctggt

gccgatgacgatgacgatgacgatggtgctggcggaggcggtggcggtagacgtgctgaggctggacg

tgttgctgttgaatgcctggctgcctgtagaggaatcttggaggctctggccgagggattcgacggag
acttggcggctgtaccggactggcgggagcgaggcctgccgctccacctcgccccggtcctgctggt
gctgccgctcctcctcatgccgacgctcctagactccgtgcttggctccgtgaactccgtttcgttcg
tgacgctttggttctgatgagactgagaggcgacttgagagtggctggaggatccgaggctgctgttg
ctgctgtccgtgctgtttctttggttgctggtgctttgggccctgctttgccgagatctccccgtttg
ttgtcgagtgccgccgctgctgccgccgatttgttgttccaaaaccaatccctccgccctctgctcgc
cgacactgttgccgctgccgattctctggctgctccggcttctgccccacgtgaagctcgtaaacgta
aatcacccgctccggctcgtgctccccctggtggcgcccctagacccccctaaaaatcccgtgccgat
gcccctagacctgctgctgctcccccgctggtgctgctcccccgctcccccctactcccccccccacg
cccacctcgtcccgctgccctcacacgccgtcctgctgagggacccgatccacaaggcggctggcgta
gacaacctcctggcccatcccatacaccggcaccatctgccgctgctttggaggcttactgtgct

SEQ ID NO: 126 = RS1.5

gccgctgccgattctctggctgctccggcttctgccccacgtgaagctcgtaaacgtaaatcacccgc

tccggctcgtgctccccctggtggcgcccctagacccccctaaaaatcccgtgccgatgcccctagac

ctgctgctgctcccccgctggtgctgctcccccgctcccctactcccccccacgcccacctcgt

cccgctgccctcacacgccgtcctgctgagggacccgatccacaaggcggctggcgtagacaacctcc

tggcccatcccatacaccggcaccatctgccgctgctttggaggcttactgtgctcctcgtgctgtgg

ctgaactcaccgatcatccgctgttccctgctccctggcgtcccgccctcatgttcgatcctagagct

ttggcttccttggccgctcgttgtgctgcccctcccctggcggtgctccggctgctttcggtcctct

ccgtgcctctggtccactccgccgtgccgctgcctggatgagacaagttcccgaccctgaggatgtta

gagttgtgatcttgtactcgcccttgcctggcgaggatttggccgctggtagagctggcggtggcccc

cctcctgaatggtctgctgaacgtggtggtttgtcttgcttgttggccgccctgggaaaccgtctgtg

tggtcctgctactgctgcttgggctggaaactggactggcgctcccgatgtttctgctctcggtgctc

aaggagttttgctgctctctactcgtgacttggcattcgctggagctgttgaattcctgggactcttg

gctggcgcttgtgataggagactcatcgtcgtaaacgctgtgagagctgccgattggcctgccgatgg

tcctgttgtgtctcgtcaacacgcttacttggcttgtgaagtgttgcccgctgtccaatgtgctgttc

gctggcctgctgctcgtgatctgaggcgtactgttctggctagtggtcgtgttttcggacctggtgtt

ttcgctcgtgtcgaagctgctcacgctagactgtaccccgatgccccacccctccgtttgtgtcgtgg

agcaaacgttcgctaccgtgtccgtactcgtttcggacccgatactctggttccaatgtcccctcgtg

aataccgtcgtgctgttctgcctgccctcgatggacgtgctgccgcttctggcgctggtgacgctatg

gctcctggcgctccggacttctgtgaggatgaggctcactcacatcgtgcctgtgcccgctggggact

gggcgctccattgaggcctgtatacgtggcactgggccgtgatgctgttagaggcggacccgctgaat

tgagaggccctcgtcgtgaattctgtgctagggctctgctcgaacccgatggagatgctcctcctttg

gtactccgtgacgacgccgatgctggtcctcccccacaaattcgctgggctagtgctgctggacgtgc

tggtactgtattggctgctgctggcggtggcgttgaagttgttggtactgccgctggactcgctacac

ctccccgccgtgaacctgtagacatggatgctgaactcgaggatgatgacgacggattgttcggagag

SEQ ID NO: 127 = RS1.6

caccaccaccaccaccatcataggcgtagacgtgctcctagacgtcgttctgccgctagtgactcttc

caaatctggctcttcttcatctgcctcttccgcttcatcttcggcctcatcgtcctcttcggcatccg

cttcgagtagtgatgatgatgatgacgacgacgctgctagagcccccgcttctgctgccgaccacgct

gctggcggaactttgggagccgacgacgaggaggcgggagttcctgctcgtgccccgggagctgctcc

gaggccttctccaccccgtgctgaacctgctccggctagaacaccggccgctactgctggtagactgg

agcgtagacgtgcccgtgctgctgtggctggtagagatgctactggccgcttcactgctggccgtcct

agacgtgttgaactggacgccgatgctgcttctggtgctttctacgcccgttaccgtgatggttacgt

gtctggtgaaccttggcctggcgctggtccacctccgcccggacgtgtactctacggtggattgggcg

attctcgccctggtctgtggggcgctccggaggctgaggaggctagagcccgtttcgaggcttctggt

gcccctgctcctgtttgggctcctgaattgggcgacgctgctcaacaatacgccctcatcacacgctt

gctgtacactcccgacgccgaggctatgggatggctccaaaaccctagagttgcccctggtgatgttg

ctctggatcaggcttgtttccgtatctccggcgctgctcgtaactcttcttcgttcatctccggttct

gtggctagagctgtgcctcacttgggatacgccatggccgctggacgtttcggctggggactggctca

tgttgctgccgctgtagcaatgtctagacgctacgaccgtgctcaaaaaggattcttgctcacgtcac

tgaggcgtgcttacgcccctttgttggcccgtgaaaacgctgccctcactggcgcccgtacccccgat

gacggtggcgacgccaaccgccacgatggtgatgatgctagaggcaaacccgctgccgctgctgctcc

tttgccctctgccgccgcttccccctgccgatgaacgtgctgttcctgccggttacggtgccgctggtg

tgttggctgctttgggacgcttgagtgctgccccggctagtgcccccgctggtgccgatgacgatgac

gatgacgatggtgctggcggaggcggtggcggtagacgtgctgaggctggacgtgttgctgttgaatg

cctggctgcctgtagaggaatcttggaggctctggccgagggattcgacggagacttggcggctgtac

cgggactggcgggagcgaggcctgccgctccacctcgccccggtcctgctggtgctgccgctcctcct

catgccgacgctcctagactccgtgcttggctccgtgaactccgtttcgttcgtgacgctttggttct

gatgagactgagaggcgacttgagagtggctggaggatccgaggctgctgttgctgctgtccgtgctg

tttctttggttgctggtgctttgggccctgctttgccgagatctccccgtttgttgtcgagtgccgcc

gctgctgccgccgatttgttgttccaaaaccaatccctccgccctctgctcgccgacactgttgccgc

tgccgattctctggctgctccggcttctgccccacgtgaagctcgtaaacgtaaatcacccgctccgg

ctcgtgctccccctggtggcgcccctagacccccctaaaaatcccgtgccgatgcccctagacctgct

gctgctcccccgctggtgctgctcccccgctcccccctactcccccccacgcccacctcgtcccgc

tgccctcacacgccgtcctgctgagggacccgatccacaaggcggctggcgtagacaacctcctggcc

catcccatacaccggcaccatctgccgctgctttggaggcttactgtgctcctcgtgctgtggctgaa

ctcaccgatcatccgctgttccctgctccctggcgtcccgccctcatgttcgatcctagagctttggc

ttccttggccgctcgttgtgctgcccctccccctggcggtgctccggctgctttcggtcctctccgtg

cctctggtccactccgccgtgccgctgcctggatgagacaagttcccgaccctgaggatgttagagtt

gtgatcttgtactcgcccttgcctggcgaggatttggccgctggtagagctggcggtggccccctcc

tgaatggtctgctgaacgtggtggtttgtcttgcttgttggccgccctgggaaaccgtctgtgtggtc

ctgctactgctgcttgggctggaaactggactggcgctcccgatgtttctgctctcggtgctcaagga

gttttgctgctctctactcgtgacttggcattcgctggagctgttgaattcctgggactcttggctgg
cgcttgtgataggagactcatcgtcgtaaacgctgtgagagctgccgattggcctgccgatggtcctg
ttgtgtctcgtcaacacgcttacttggcttgtgaagtgttgcccgctgtccaatgtgctgttcgctgg
cctgctgctcgtgatctgaggcgtactgttctggctagtggtcgtgttttcggacctggtgttttcgc
tcgtgtcgaagctgctcacgctagactgtaccccgatgccccacccctccgtttgtgtcgtggagcaa
acgttcgctaccgtgtccgtactcgtttcggacccgatactctggttccaatgtcccctcgtgaatac
cgtcgtgctgttctgcctgccctcgatggacgtgctgccgcttctggcgctggtgacgctatggctcc
tggcgctccggacttctgtgaggatgaggctcactcacatcgtgcctgtgcccgctggggactgggcg
ctccattgaggcctgtatacgtggcactgggccgtgatgctgttagaggcggacccgctgaattgaga
ggccctcgtcgtgaattctgtgctagggctctgctcgaacccgatggagatgctcctcctttggtact
ccgtgacgacgccgatgctggtcctcccccacaaattcgctggctagtgctgctggacgtgctggta
ctgtattggctgctgctggcggtggcgttgaagttgttggtactgccgctggactcgctacacctccc
cgccgtgaacctgtagacatggatgctgaactcgaggatgatgacgacggattgttcggagagtaa

SEQ ID NO: 128 = RS1.7

atgagtgccgaacagcgtaaaaagaaaaaaccaccaccacgacccaaggacgtggagctgaagttgc

tatggcggatgaggatggaggccgcttgagagctgctgctgagactactggaggacctggatcaccgg

accctgccgatggacccccccctacaccaaaccccgatcgtagaccggctgctagacctggattcgga

tggcatggaggacccgaggaaaacgaggacgaggcggacgacgccgctgccgacgccgacgccgatga

ggctgcccctgcttctggagaggcggtagacgaacctgctgccgatggagttgttagccctaggcaat

tggctttgttggcgagcatggtagacgaggctgtgagaacaatcccttcccctcccccctgaacgtgat

ggagcacaagaggaggcggctaggagtccctcaccaccccgtacaccttctatgagagcggattacgg

cgaggaaaacgacgacgacgacgatgatgatgacgacgatgatcgtgatgccggacgctgggttaggg

gacctgaaaccacttctgctgtccgtggagcataccccgatcctatggcgagtttgagccctagacca

cctgccccgaggagacaccaccaccaccatcataggcgtagacgtgctcctagacgtcgttctgc

cgctagtgactcttccaaatctggctcttcttcatctgcctcttccgcttcatcttcggcctcatcgt

cctcttcggcatccgcttcgagtagtgatgatgatgatgacgacgacgctgctagagcccccgcttct

gctgccgaccacgctgctggcggaactttgggagccgacgacgaggaggcgggagttcctgctcgtgc

cccgggagctgctccgaggccttctccaccccgtgctgaacctgctccggctagaacaccggccgcta

ctgctggtagactggagcgtagacgtgcccgtgctgctgtggctggtagagatgctactggccgcttc

actgctggccgtcctagacgtgttgaactggacgccgatgctgcttctggtgctttctacgcccgtta

ccgtgatggttacgtgtctggtgaaccttggcctggcgctggtccacctccgcccggacgtgtactct

acggtggattgggcgcccgtacccccgatgacggtggcgacgccaaccgccacgatggtgatgatgct

agaggcaaacccgctgccgctgctgctcctttgccctctgccgccgcttcccctgccgatgaacgtgc

tgttcctgccggttacggtgccgctggtgtgttggctgctttgggacgcttgagtgctgccccggcta

gtgcccccgctggtgccgatgacgatgacgatgacgatggtgctggcggaggcggtggcggtagacgt

gctgaggctggacgtgttgctgttgaatgcctggctgcctgtagaggaatcttggaggctctggccga

gggattcgacggagacttggcggctgtaccggactggcgggagcgaggcctgccgctccacctcgcc

ccggtcctgctggtgctgccgctcctcctcatgccgacgctcctagactccgtgcttggctccgtgaa

ctccgtttcgttcgtgacgctttggttctgatgagactgagaggcgacttgagagtggctggaggatc
cgaggctgctgttgctgctgtccgtgctgtttctttggttgctggtgctttgggccctgctttgccga
gatctccccgtttgttgtcgagtgccgccgctgctgccgccgatttgttgttccaaaaccaatccctc
cgccctctgctcgccgacactgttgccgctgccgattctctggctgctccggcttctgccccacgtga
agctcgtaaacgtaaatcacccgctccggctcgtgctccccctggtggcgcccctagacccccctaaaa
aatcccgtgccgatgcccctagacctgctgctgctcccccgctggtgctgctcccccgctcccccct
actcccccccacgcccacctcgtcccgctgccctcacacgccgtcctgctgagggacccgatccaca
aggcggctggcgtagacaacctcctggcccatcccatacaccggcaccatctgccgctgctttggagg
cttactgtgctcctcgtgctgtggctgaactcaccgatcatccgctgttccctgctccctggcgtccc
gccctcatgttcgatcctagagctttggcttccttggccgctcgttgtgctgcccctcccccctggcgg
tgctccggctgctttcggtcctctccgtgcctctggtccactccgccgtgccgctgcctggatgagac
aagttcccgaccctgaggatgttagagttgtgatcttgtactcgcccttgcctggcgaggatttggcc
gctggtagagctggcggtggccccctcctgaatggtctgctgaacgtggtggtttgtcttgcttgtt
ggccgccctgggaaaccgtctgtgtggtcctgctactgctgcttgggctggaaactggactggcgctc
ccgatgtttctgctctcggtgctcaaggagttttgctgctctctactcgtgacttggcattcgctgga
gctgttgaattcctggactcttggctggcgcttgtgataggagactcatcgtcgtaaacgctgtgag
agctgccgattggcctgccgatggtcctgttgtgtctcgtcaacacgcttacttggcttgtgaagtgt
tgcccgctgtccaatgtgctgttcgctggcctgctgctcgtgatctgaggcgtactgttctggctagt
ggtcgtgttttcggacctggtgttttcgctcgtgtcgaagctgctcacgctagactgtaccccgatgc
cccacccctccgtttgtgtcgtggagcaaacgttcgctaccgtgtccgtactcgtttcggacccgata
ctctggttccaatgtcccctcgtgaataccgtcgtgctgttctgcctgccctcgatggacgtgctgcc
gcttctggcgctggtgacgctatggctcctggcgctccggacttctgtgaggatgaggctcactcaca
tcgtgcctgtgcccgctggggactgggcgctccattgaggcctgtatacgtggcactgggccgtgatg
ctgttagaggcggacccgctgaattgagaggccctcgtcgtgaattctgtgctagggctctgctcgaa
cccgatggagatgctcctcctttggtactccgtgacgacgccgatgctggtcctcccccacaaattcg
ctgggctagtgctgctggacgtgctggtactgtattggctgctgctggcggtggcgttgaagttgttg
gtactgccgctggactcgctacacctccccgccgtgaacctgtagacatggatgctgaactcgaggat
gatgacgacggattgttcggagag

SEQ ID NO: 129 = RS1.8

atgagtgccgaacagcgtaaaaagaaaaaaccaccaccacgacccaaggacgtggagctgaagttgc

tatggcggatgaggatggaggccgcttgagagctgctgctgagactactggaggacctggatcaccgg

accctgccgatggacccccccctacaccaaaccccgatcgtagaccggctgctagacctggattcgga

tggcatggaggacccgaggaaaacgaggacgaggcggacgacgccgctgccgacgccgacgccgatga

ggctgcccctgcttctggagaggcggtagacgaacctgctgccgatggagttgttagccctaggcaat

tggctttgttggcgagcatggtagacgaggctgtgagaacaatcccttcccctcccccctgaacgtgat

ggagcacaagaggaggcggctaggagtccctcaccaccccgtacaccttctatgagagcggattacgg

cgaggaaaacgacgacgacgacgatgatgatgacgacgatgatcgtgatgccggacgctgggttaggg

gacctgaaaccacttctgctgtccgtggagcataccccgatcctatggcgagtttgagccctagacca
cctgccccgaggagacaccaccaccaccatcataggcgtagacgtgctcctagacgtcgttctgc
cgctagtgactcttccaaatctggctcttcttcatctgcctcttccgcttcatcttcggcctcatcgt
cctcttcggcatccgcttcgagtagtgatgatgatgatgacgacgacgctgctagagcccccgcttct
gctgccgaccacgctgctggcggaactttgggagccgacgacgaggaggcgggagttcctgctcgtgc
cccgggagctgctccgaggccttctccaccccgtgctgaacctgctccggctagaacaccggccgcta
ctgctggtagactggagcgtagacgtgcccgtgctgctgtggctggtagagatgctactggccgcttc
actgctggccgtcctagacgtgttgaactggacgccgatgctgcttctggtgctttctacgcccgtta
ccgtgatggttacgtgtctggtgaaccttggcctggcgctggtccacctccgcccggacgtgtactct
acggtggattgggcgattctcgccctggtctgtggggcgctccggaggctgaggaggctagagcccgt
ttcgaggcttctggtgcccctgctcctgtttgggctcctgaattgggcgacgctgctcaacaatacgc
cctcatcacacgcttgctgtacactcccgacgccgaggctatgggatggctccaaaaccctagagttg
cccctggtgatgttgctctggatcaggcttgtttccgtatctccggcgctgctcgtaactcttcttcg
ttcatctccggttctgtggctagagctgtgcctcacttgggatacgccatggccgctggacgtttcgg
ctggggactggctcatgttgctgccgctgtagcaatgtctagacgctacgaccgtgctcaaaaaggat
tcttgctcacgtcactgaggcgtgcttacgcccctttgttggcccgtgaaaacgctgccctcactggc
gcccgtacccccgatgacggtggcgacgccaaccgccacgatggtgatgatgctagaggcaaacccgc
tgccgctgctgctcctttgccctctgccgccgcttcccctgccgatgaacgtgctgttcctgccggtt
acggtgccgctggtgtgttggctgctttgggacgcttgagtgctgccccggctagtgcccccgctggt
gccgatgacgatgacgatgacgatggtgctggcggaggcggtggcggtagacgtgctgaggctggacg
tgttgctgttgaatgcctggctgcctgtagaggaatcttggaggctctggccgagggattcgacggag
acttggcggctgtaccgggactggcgggagcgaggcctgccgctccacctcgccccggtcctgctggt
gctgccgctcctcctcatgccgacgctcctagactccgtgcttggctccgtgaactccgtttcgttcg
tgacgctttggttctgatgagactgagaggcgacttgagagtggctggaggatccgaggctgctgttg
ctgctgtccgtgctgtttctttggttgctggtgctttgggccctgctttgccgagatctccccgtttg
ttgtcgagtgccgccgctgctgccgccgatttgttgttccaaaaccaatccctccgccctctgctcgc
cgacactgttgccgctgccgattctctggctgctccggcttctacaccggcaccatctgccgctgctt
tggaggcttactgtgctcctcgtgctgtggctgaactcaccgatcatccgctgttccctgctccctgg
cgtcccgccctcatgttcgatcctagagctttggcttccttggccgctcgttgtgctgcccctccccc
tggcggtgctccggctgctttcggtcctctccgtgcctctggtccactccgccgtgccgctgcctgga
tgagacaagttcccgaccctgaggatgttagagttgtgatcttgtactcgcccttgcctggcgaggat
ttggccgctggtagagctggcggtggccccctcctgaatggtctgctgaacgtggtggtttgtcttg
cttgttggccgccctgggaaaccgtctgtgtggtcctgctactgctgcttgggctggaaactggactg
gcgctcccgatgtttctgctctcggtgctcaaggagttttgctgctctctactcgtgacttggcattc
gctggagctgttgaattcctgggactcttggctggcgcttgtgataggagactcatcgtcgtaaacgc
tgtgagagctgccgattggcctgccgatggtcctgttgtgtctcgtcaacacgcttacttggcttgtg
aagtgttgcccgctgtccaatgtgctgttcgctggcctgctgctcgtgatctgaggcgtactgttctg
gctagtggtcgtgttttcggacctggtgttttcgctcgtgtcgaagctgctcacgctagactgtaccc
cgatgccccacccctccgtttgtgtcgtggagcaaacgttcgctaccgtgtccgtactcgtttcggac

ccgatactctggttccaatgtcccctcgtgaataccgtcgtgctgttctgcctgccctcgatggacgt
gctgccgcttctggcgctggtgacgctatggctcctggcgctccggacttctgtgaggatgaggctca
ctcacatcgtgcctgtgcccgctggggactgggcgctccattgaggcctgtatacgtggcactgggcc
gtgatgctgttagaggcggacccgctgaattgagaggccctcgtcgtgaattctgtgctagggctctg
ctcgaacccgatggagatgctcctcctttggtactccgtgacgacgccgatgctggtcctcccccaca
aattcgctgggctagtgctgctggacgtgctggtactgtattggctgctgctggcggtggcgttgaag
ttgttggtactgccgctggactcgctacacctccccgccgtgaacctgtagacatggatgctgaactc
gaggatgatgacgacggattgttcggagag

SEQ ID NO: 130 = His tag
   HHHHHH
SEQ ID NO: 131 = Tag
   MSYYHHHHHH
SEQ ID NO: 132 = Secretion Signal
   MKFLVNVALVFMVVYISYIYA

SEQ ID NO: 133 = UL49.5

ATGTCGTACTACCATCACCATCACCATCACATGACGGGGAAACCCGCAAGACTGGGCCGCTGGGTGGT

GCTGTTGTTCGTCGCGCTCGTCGCGGGCGTGCCCGGGGAGCCGCCGAACGCGGCAGGCGCACGCGGCG

TTATCGGGGACGCGCAATGCCGGGGCGACAGCGCCGGTGTGGTGTCCGTCCCGGGGGTCCTGGTGCCC

TTTTATCTAGGCATGACCTCGATGGGCGTATGTATGATCGCGCACGTGTATCAGATATGCCAGCGGGC

ACTGGCCGCCGGGTCAGCCTGA

SEQ ID NO: 134 = UL10
ATGGGACGCCGGGCCCCCAGGGGATCCCCCGAGGCCGCGCCGGGCGCCGACGTCGCGCCCGGGGCGCG
GGCGGCGTGGTGGGTCTGGTGTGTGCAGGTGGCGACGTTCATCGTCTCGGCCATCTGCGTCGTGGGGC
TCCTGGTGCTGGCCTCTGTGTTCCGGGACAGGTTTCCCTGCCTTTACGCCCCCGCGACCTCTTATGCG
AAGGCGAACGCCACGGTCGAGGTGCGCGGGGGTGTAGCCGTCCCCCTCCGGTTGGACACGCAGAGCCT
GCTGGCCACGTACGCAATTACGTCTACGCTGTTGCTGGCGGCGGCCGTGTACGCCGCGGTGGGCGCGG
TGACCTCGCGCTACGAGCGCGCGCTGGATGCGGCCCGTCGCCTGGCGGCGGCCCGTATGGCGATGCCA
CACGCCACGCTAATCGCCGGAAACGTCTGCGCGTGGCTGTTGCAGATCACAGTCCTGCTGCTGGCCCA

CCGCATCAGCCAGCTGGCCCACCTTATCTACGTCCTGCACTTTGCGTGCCTCGTGTATCTCGCGGCCC
ATTTTTGCACCAGGGGGTCCTGAGCGGACGTACCTGCGTCAGGTTCACGGCCTGATTGACCCGGCG
CCGACGCACCATCGTATCGTCGGTCCGGTGCGGGCAGTAATGACAAACGCCTTATTACTGGGCACCCT
CCTGTGCACGGCCGCCGCCGCGGTCTCGTTGAACACGATCGCCGCCCTGAACTTCAACTTTTCCGCCC
CGAGCATGCTCATCTGCCTGACGACGCTGTTCGCCCTGCTTGTCGTGTCGCTGTTGTTGGTGGTCGAG
GGGGTGCTGTGTCACTACGTGCGCGTGTTGGTGGGCCCCCACCTCGGGGCCATCGCCGCCACCGGCAT
CGTCGGCCTGGCCTGCGAGCACTACCACACCGGTGGTTACTACGTGGTGGAGCAGCAGTGGCCGGGGG
CCCAGACGGGAGTCCGCGTCGCCCTGGCGCTCGTCGCCGCCTTTGCCCTCGCCATGGCCGTGCTTCGG
TGCACGCGCGCCTACCTGTATCACCGGCGACACCACACTAAATTTTTCGTGCGCATGCGCGACACCCG
GCACCGCGCCCATTCGGCGCTTCGACGCGTACGCAGCTCCATGCGCGGTTCTAGGCGTGGCGGGCCGC
CCGGAGACCCGGGCTACGCGGAAACCCCCTACGCGAGCGTGTCCCACCACGCCGAGATCGACCGGTAT
GGGGATTCCGACGGGGACCCGATCTACGACGAAGTGGCCCCCGACCACGAGGCCGAGCTCTACGCCCG
AGTGCAACGCCCCGGGCCTGTGCCCGACGCCGAGCCCATTTACGACACCGTGGAGGGGTATGCGCCAA
GGTCCGCGGGGGAGCCGGTGTACAGCACCGTTCGGCGATGGTAG

Certain embodiments of the invention are set out in the following numbered paragraphs:
1. A vaccine formulation comprising a pharmaceutical-acceptable carrier and at least one polypeptide consisting of SEQ ID NOS: 2, 3, 4 and 5 or an immunogenic fragment thereof, and optionally further comprising SEQ ID NO:1 or an immunogenic fragment thereof.
2. The vaccine formulation of paragraph 1, comprising a first polypeptide consisting of SEQ ID NO: 1 and a second polypeptide consisting of SEQ ID NO:3 or SEQ ID NO:4.
3. The vaccine formulation of paragraph 1, comprising a first polypeptide consisting of SEQ ID NO:2 and a second polypeptide consisting of SEQ ID NO:3 or SEQ ID NO:4.
4. The vaccine formulation of paragraph 1, comprising a first polypeptide consisting of SEQ ID NO:3 and a second polypeptide consisting of SEQ ID NO: 4.
5. The vaccine formulation of paragraph 1, comprising a first polypeptide consisting of SEQ ID NO: 1, a second polypeptide consisting of SEQ ID NO:3, and a third polypeptide consisting of SEQ ID NO:4.
6. The vaccine formulation of paragraph 1, comprising a first polypeptide consisting of SEQ ID NO:2, a second polypeptide consisting of SEQ ID NO:3, and a third polypeptide consisting of SEQ ID NO:4.
7. A vaccine formulation comprising a pharmaceutically acceptable carrier, an adjuvant comprising one or more purified fractions of quillaja saponins, and at least one polypeptide comprising any of SEQ ID NOS: 2, 3, 4 and 5 or an immunogenic fragment thereof, and optionally further comprising SEQ ID NO:1 or an immunogenic fragment thereof.
8. A vaccine formulation comprising a pharmaceutically-acceptable carrier and a polypeptide consisting of SEQ ID NO: 2 or an immunogenic fragment thereof.
9. The vaccine formulation of paragraph 8, wherein the fragment is a truncated fragment of SEQ ID NO:2 wherein from 1-20 amino acid residues are removed from the N-terminus, C-terminus, or both.
10. The vaccine formulation of paragraph 9, wherein the fragment is a truncated fragment of SEQ ID NO:2 wherein from 1-10 amino acid residues are removed from the N-terminus, C-terminus, or both.
11. A vaccine formulation comprising a pharmaceutically-acceptable carrier and a polypeptide comprising SEQ ID NO:5, wherein the polypeptide lacks all or at least 8 contiguous amino acid residues of the transmembrane domain spanning residues 341-363.
12. A vaccine formulation comprising a pharmaceutically-acceptable carrier and a polypeptide comprising SEQ ID NO:4.
13. A vaccine formulation comprising a pharmaceutically-acceptable carrier, a polypeptide comprising SEQ ID NO:5.
14. The vaccine formulation of paragraph 13, wherein the polypeptide is unglycosylated
15. A vaccine formulation comprising a pharmaceutically-acceptable carrier, a polypeptide comprising SEQ ID NO:3.
16. The vaccine formulation of any of paragraph 1, 8, 11, 13, or 15, wherein the at least one polypeptide is conjugated to an immunogenic carrier.
17. The vaccine formulation of any of paragraph 1, 8, 11, 13, or 15, further comprising an adjuvant.
18. The vaccine formulation of paragraph 17, wherein the adjuvant is one or more purified fractions of quillaja saponins.
19. The vaccine formulation of paragraph 18, wherein the adjuvant comprises saponin fraction A and saponin fraction C.
20. The vaccine formulation of paragraph 19, wherein the adjuvant comprises cholesterol, phosphatidyl choline, saponin fraction A and saponin fraction C.
21. The vaccine formulation of paragraph 20, wherein the adjuvant is in the form of particles.
22. The vaccine formulation of paragraph 21, wherein particles comprising saponin fraction A are substantially free of saponin fraction C and particles comprising saponin fraction C are substantially free of saponin fraction A.
23. The vaccine formulation of any of paragraph 1, 8, 11, 13, or 15, wherein the vaccine formulation comprises 5-200 µg of the at least one polypeptide and 5-200 µg of the adjuvant.
24. The vaccine of formulation of any of paragraph 1, 7, 8, 11, 13 or 15, wherein the polypeptide is a fusion protein further comprising a tag.
25. The vaccine formulation of any of paragraph 1-24, wherein the vaccine formulation inhibits HSV-2 symptoms.
26. The vaccine formulation of paragraph 25, wherein the vaccine formulation reduces the number of herpetic lesions.
27. The vaccine formulation of paragraph 25, wherein the vaccine formulation reduces the number of days a subject experiences herpetic lesions.
28. The vaccine formulation of any of paragraph 1-24, wherein the vaccine formulation inhibits infection by HSV-2 in an uninfected subject.
29. The vaccine formulation of paragraph 28, wherein the vaccine formulation increases the IgG titer to one or more HSV-2 antigens.
30. The vaccine formulation of paragraph 28, wherein the vaccine formulation increases the T cell response to one or more HSV-2 antigens.
31. The vaccine formulation of paragraph 28, wherein the vaccine formulation reduces the number of herpetic lesions at the onset of HSV-2 infection.
32. The vaccine formulation of any of paragraph 1-24, wherein the vaccine formulation inhibits HSV-2 symptoms or inhibits infection by HSV-2 in three or fewer doses.
33. A method for treating a subject suffering from or susceptible to HSV-2 infection, comprising administering an effective amount of a vaccine formulation according to paragraph 1-24.
34. The method of paragraph 33, wherein the method inhibits HSV-2 symptoms.
35. The method of paragraph 34, wherein the method reduces the number of herpetic lesions.
36. The method of paragraph 35, wherein the method reduces the number of days a subject experiences herpetic lesions.
37. The method of paragraph 33, wherein the method inhibits infection by HSV-2 in an uninfected subject.
38. The method of paragraph 37, wherein the method increases the IgG titer to one or more HSV-2 antigens.
39. The method of paragraph 37, wherein the method increases the T cell response to one or more HSV-2 antigens.
40. The method of paragraph 37, wherein the method reduces the number of herpetic lesions at the onset of HSV-2 infection.
41. The method of paragraph 33, wherein the method treats a subject within a three dose regimen.
42. The method of paragraph 33, wherein the subject is a human.

## Claims

1. A vaccine formulation comprising a pharmaceutically-acceptable carrier and a polypeptide comprising the amino acid sequence of SEQ ID NO: 5 having an internal deletion of 8-24 contiguous amino acid residues selected from amino acid residues 340-363 of SEQ ID NO: 5.

2. The vaccine formulation of claim 1, wherein the polypeptide consists of the amino acid sequence of SEQ ID NO: 5 having an internal deletion of 8-24 contiguous amino acid residues selected from amino acid residues 340-363 of SEQ ID NO: 5.

3. A vaccine formulation comprising a pharmaceutically-acceptable carrier and (a) a polypeptide comprising, or consisting of, the amino acid sequence of SEQ ID NO: 4; or (b) a polypeptide comprising the amino acid sequence of SEQ ID NO: 4 having a deletion of 1, 2, or 3 amino acid residues at the N-terminus, C-terminus, or both.

4. The vaccine formulation of any one of the preceding claims, further comprising a second polypeptide which:
(a) comprises an amino acid sequence having at least 90% identity to SEQ ID NO: 1; or
(b) consists of an amino acid sequence having at least 90% identity to SEQ ID NO: 1.

5. The vaccine formulation of claim 1 or claim 3, further comprising a second polypeptide which:
(a) comprises, or consists of, an amino acid sequence having at least 90% identity to SEQ ID NO: 2; or
(b) comprises, or consists of, at least 8 contiguous amino acids of SEQ ID NO: 2.

6. A vaccine formulation comprising a pharmaceutically-acceptable carrier and a polypeptide comprising the amino acid sequence of SEQ ID NO: 4 having a deletion of 1-20 amino acid residues at the N-terminus, C-terminus, or both, of SEQ ID NO: 4.

7. The vaccine formulation of any one of the preceding claims, further comprising a third polypeptide comprising SEQ ID NO: 3 or an immunogenic fragment comprising at least 8 contiguous amino acids of SEQ ID NO: 3.

8. A vaccine formulation comprising a pharmaceutically-acceptable carrier, an adjuvant comprising one or more purified fractions of quillaja saponins, a first polypeptide comprising the amino acid sequence of SEQ ID NO: 4 or comprising the amino acid sequence of SEQ ID NO: 4 having a deletion of 1, 2, or 3 amino acid residues at the N-terminus, C-terminus, or both, and a second polypeptide comprising the amino acid sequence having at least 90% identity to SEQ ID NO: 1.

9. A vaccine formulation comprising a pharmaceutically-acceptable carrier, a first polypeptide comprising the amino acid sequence of SEQ ID NO: 4 or comprising the amino acid sequence of SEQ ID NO:4 having a deletion of 1, 2, or 3 amino acid residues at the N-terminus, C-terminus, or both, and a second polypeptide comprising the amino acid sequence having at least 90% identity to SEQ ID NO: 2.

10. The vaccine formulation of claim 9, wherein the first polypeptide consists of the amino acid sequence of SEQ ID NO: 4 or consists of the amino acid sequence of SEQ ID NO: 4 having a deletion of 1, 2, or 3 amino acid residues at the N-terminus, C-terminus, or both, and the second polypeptide consists of the amino acid sequence having at least 90% identity to SEQ ID NO: 2.

11. The vaccine formulation of claim 9 or 10, further comprising an adjuvant.

12. A vaccine formulation comprising a pharmaceutically-acceptable carrier and an HSV-2 gD2 polypeptide, wherein the gD2 polypeptide has an internal deletion of all or part of a transmembrane domain.

13. The vaccine formulation of claim 6, wherein the polypeptide comprises the amino acid sequence of SEQ ID NO: 4 having a deletion of 1, 2, or 3 amino acid residues at the N-terminus, C-terminus, or both.


SEQUENCE LISTING

<110> LONG, DEBORAH
      FLECHTNER, JESSICA
      SKOBERNE, MOJCA

<120> VACCINES AGAINST HERPES SIMPLEX VIRUS TYPE 2: COMPOSITIONS AND
      METHODS FOR ELICITING AN IMMUNE RESPONSE

<130> 105592-0005-101

<140> 12/786,425
<141> 2010-05-24

<150> 61/305,918
<151> 2010-02-18

<150> 61/240,626
<151> 2009-09-08

<150> 61/240,587
<151> 2009-09-08

<150> 61/235,628
<151> 2009-08-20

<150> 61/180,784
<151> 2009-05-22

<160> 137

<170> PatentIn version 3.5

<210> 1
<211> 1317
<212> PRT
<213> Human herpesvirus 2

<400> 1
Ser Ala Glu Gln Arg Lys Lys Lys Lys Thr Thr Thr Thr Thr Gln Gly
1               5                   10                  15

Arg Gly Ala Glu Val Ala Met Ala Asp Glu Asp Gly Gly Arg Leu Arg
            20                  25                  30

Ala Ala Ala Glu Thr Thr Gly Gly Pro Gly Ser Pro Asp Pro Ala Asp
        35                  40                  45

Gly Pro Pro Pro Thr Pro Asn Pro Asp Arg Arg Pro Ala Ala Arg Pro
    50                  55                  60

Gly Phe Gly Trp His Gly Gly Pro Glu Glu Asn Glu Asp Glu Ala Asp
65                  70                  75                  80

Asp Ala Ala Ala Asp Ala Asp Ala Asp Glu Ala Ala Pro Ala Ser Gly
                85                  90                  95

Glu Ala Val Asp Glu Pro Ala Ala Asp Gly Val Val Ser Pro Arg Gln
            100                 105                 110

Leu Ala Leu Leu Ala Ser Met Val Asp Glu Ala Val Arg Thr Ile Pro
        115                 120                 125

Ser Pro Pro Pro Glu Arg Asp Gly Ala Gln Glu Glu Ala Ala Arg Ser
    130                 135                 140

Pro Ser Pro Pro Arg Thr Pro Ser Met Arg Ala Asp Tyr Gly Glu Glu
145                 150                 155                 160

Asn Asp Asp Asp Asp Asp Asp Asp Asp Asp Asp Asp Arg Asp Ala Gly
                165                 170                 175

Arg Trp Val Arg Gly Pro Glu Thr Thr Ser Ala Val Arg Gly Ala Tyr
            180                 185                 190

Pro Asp Pro Met Ala Ser Leu Ser Pro Arg Pro Pro Ala Pro Arg Arg
        195                 200                 205

His His His His His His His Arg Arg Arg Arg Ala Pro Arg Arg Arg
    210                 215                 220

Ser Ala Ala Ser Asp Ser Ser Lys Ser Gly Ser Ser Ser Ser Ala Ser
225                 230                 235                 240

Ser Ala Ser Ser Ser Ala Ser Ser Ser Ser Ser Ala Ser Ala Ser Ser
                245                 250                 255

Ser Asp Asp Asp Asp Asp Asp Asp Ala Ala Arg Ala Pro Ala Ser Ala
            260                 265                 270

Ala Asp His Ala Ala Gly Gly Thr Leu Gly Ala Asp Asp Glu Glu Ala
        275                 280                 285

Gly Val Pro Ala Arg Ala Pro Gly Ala Ala Pro Arg Pro Ser Pro Pro
    290                 295                 300

Arg Ala Glu Pro Ala Pro Ala Arg Thr Pro Ala Ala Thr Ala Gly Arg
305                 310                 315                 320

Leu Glu Arg Arg Arg Ala Arg Ala Ala Val Ala Gly Arg Asp Ala Thr
                325                 330                 335

Gly Arg Phe Thr Ala Gly Arg Pro Arg Arg Val Glu Leu Asp Ala Asp
            340                 345                 350

Ala Ala Ser Gly Ala Phe Tyr Ala Arg Tyr Arg Asp Gly Tyr Val Ser
        355                 360                 365

Gly Glu Pro Trp Pro Gly Ala Gly Pro Pro Pro Pro Gly Arg Val Leu
    370                 375                 380

Tyr Gly Gly Leu Gly Asp Ser Arg Pro Gly Leu Trp Gly Ala Pro Glu
385                 390                 395                 400

Ala Glu Glu Ala Arg Ala Arg Phe Glu Ala Ser Gly Ala Pro Ala Pro
                405                 410                 415

Val Trp Ala Pro Glu Leu Gly Asp Ala Ala Gln Gln Tyr Ala Leu Ile
            420                 425                 430

Thr Arg Leu Leu Tyr Thr Pro Asp Ala Glu Ala Met Gly Trp Leu Gln
        435                 440                 445

Asn Pro Arg Val Ala Pro Gly Asp Val Ala Leu Asp Gln Ala Cys Phe
    450                 455                 460

Arg Ile Ser Gly Ala Ala Arg Asn Ser Ser Ser Phe Ile Ser Gly Ser
465                 470                 475                 480

Val Ala Arg Ala Val Pro His Leu Gly Tyr Ala Met Ala Ala Gly Arg
                485                 490                 495

Phe Gly Trp Gly Leu Ala His Val Ala Ala Ala Val Ala Met Ser Arg
            500                 505                 510

Arg Tyr Asp Arg Ala Gln Lys Gly Phe Leu Leu Thr Ser Leu Arg Arg
        515                 520                 525

Ala Tyr Ala Pro Leu Leu Ala Arg Glu Asn Ala Ala Leu Thr Gly Ala
    530                 535                 540

Arg Thr Pro Asp Asp Gly Gly Asp Ala Asn Arg His Asp Gly Asp Asp
545                 550                 555                 560

Ala Arg Gly Lys Pro Ala Ala Ala Ala Ala Pro Leu Pro Ser Ala Ala
                565                 570                 575

Ala Ser Pro Ala Asp Glu Arg Ala Val Pro Ala Gly Tyr Gly Ala Ala
            580                 585                 590

Gly Val Leu Ala Ala Leu Gly Arg Leu Ser Ala Ala Pro Ala Ser Ala
        595                 600                 605

Pro Ala Gly Ala Asp Asp Asp Asp Asp Asp Asp Gly Ala Gly Gly Gly
    610                 615                 620

Gly Gly Gly Arg Arg Ala Glu Ala Gly Arg Val Ala Val Glu Cys Leu
625                 630                 635                 640

Ala Ala Cys Arg Gly Ile Leu Glu Ala Leu Ala Glu Gly Phe Asp Gly
                645                 650                 655

Asp Leu Ala Ala Val Pro Gly Leu Ala Gly Ala Arg Pro Ala Ala Pro
            660                 665                 670

Pro Arg Pro Gly Pro Ala Gly Ala Ala Ala Pro Pro His Ala Asp Ala
        675                 680                 685

Pro Arg Leu Arg Ala Trp Leu Arg Glu Leu Arg Phe Val Arg Asp Ala
    690                 695                 700

Leu Val Leu Met Arg Leu Arg Gly Asp Leu Arg Val Ala Gly Gly Ser
705                 710                 715                 720

Glu Ala Ala Val Ala Ala Val Arg Ala Val Ser Leu Val Ala Gly Ala
                725                 730                 735

Leu Gly Pro Ala Leu Pro Arg Ser Pro Arg Leu Leu Ser Ser Ala Ala
            740                 745                 750

Ala Ala Ala Ala Asp Leu Leu Phe Gln Asn Gln Ser Leu Arg Pro Leu
        755                 760                 765

Leu Ala Asp Thr Val Ala Ala Ala Asp Ser Leu Ala Ala Pro Ala Ser
    770                 775                 780

Ala Pro Arg Glu Ala Arg Lys Arg Lys Ser Pro Ala Pro Ala Arg Ala
785                 790                 795                 800

Pro Pro Gly Gly Ala Pro Arg Pro Pro Lys Lys Ser Arg Ala Asp Ala
                805                 810                 815

Pro Arg Pro Ala Ala Ala Pro Pro Ala Gly Ala Ala Pro Pro Ala Pro
            820                 825                 830

Pro Thr Pro Pro Pro Arg Pro Pro Arg Pro Ala Ala Leu Thr Arg Arg
        835                 840                 845

Pro Ala Glu Gly Pro Asp Pro Gln Gly Gly Trp Arg Arg Gln Pro Pro

850                 855                 860

Gly Pro Ser His Thr Pro Ala Pro Ser Ala Ala Ala Leu Glu Ala Tyr
865                 870                 875                 880

Cys Ala Pro Arg Ala Val Ala Glu Leu Thr Asp His Pro Leu Phe Pro
                885                 890                 895

Ala Pro Trp Arg Pro Ala Leu Met Phe Asp Pro Arg Ala Leu Ala Ser
            900                 905                 910

Leu Ala Ala Arg Cys Ala Ala Pro Pro Pro Gly Gly Ala Pro Ala Ala
        915                 920                 925

Phe Gly Pro Leu Arg Ala Ser Gly Pro Leu Arg Arg Ala Ala Ala Trp
    930                 935                 940

Met Arg Gln Val Pro Asp Pro Glu Asp Val Arg Val Val Ile Leu Tyr
945                 950                 955                 960

Ser Pro Leu Pro Gly Glu Asp Leu Ala Ala Gly Arg Ala Gly Gly Gly
                965                 970                 975

Pro Pro Pro Glu Trp Ser Ala Glu Arg Gly Gly Leu Ser Cys Leu Leu
            980                 985                 990

Ala Ala Leu Gly Asn Arg Leu Cys  Gly Pro Ala Thr Ala  Ala Trp Ala
        995                 1000                1005

Gly Asn  Trp Thr Gly Ala Pro  Asp Val Ser Ala Leu  Gly Ala Gln
    1010                1015                1020

Gly Val  Leu Leu Leu Ser Thr  Arg Asp Leu Ala Phe  Ala Gly Ala
    1025                1030                1035

Val Glu  Phe Leu Gly Leu Leu  Ala Gly Ala Cys Asp  Arg Arg Leu
    1040                1045                1050

Ile Val  Val Asn Ala Val Arg  Ala Ala Asp Trp Pro  Ala Asp Gly
    1055                1060                1065

Pro Val  Val Ser Arg Gln His  Ala Tyr Leu Ala Cys  Glu Val Leu
    1070                1075                1080

Pro Ala  Val Gln Cys Ala Val  Arg Trp Pro Ala Ala  Arg Asp Leu
    1085                1090                1095

Arg Arg Thr Val Leu Ala Ser Gly Arg Val Phe Gly Pro Gly Val
    1100                1105                1110

Phe Ala Arg Val Glu Ala Ala His Ala Arg Leu Tyr Pro Asp Ala
    1115                1120                1125

Pro Pro Leu Arg Leu Cys Arg Gly Ala Asn Val Arg Tyr Arg Val
    1130                1135                1140

Arg Thr Arg Phe Gly Pro Asp Thr Leu Val Pro Met Ser Pro Arg
    1145                1150                1155

Glu Tyr Arg Arg Ala Val Leu Pro Ala Leu Asp Gly Arg Ala Ala
    1160                1165                1170

Ala Ser Gly Ala Gly Asp Ala Met Ala Pro Gly Ala Pro Asp Phe
    1175                1180                1185

Cys Glu Asp Glu Ala His Ser His Arg Ala Cys Ala Arg Trp Gly
    1190                1195                1200

Leu Gly Ala Pro Leu Arg Pro Val Tyr Val Ala Leu Gly Arg Asp
    1205                1210                1215

Ala Val Arg Gly Gly Pro Ala Glu Leu Arg Gly Pro Arg Arg Glu
    1220                1225                1230

Phe Cys Ala Arg Ala Leu Leu Glu Pro Asp Gly Asp Ala Pro Pro
    1235                1240                1245

Leu Val Leu Arg Asp Asp Ala Asp Ala Gly Pro Pro Pro Gln Ile
    1250                1255                1260

Arg Trp Ala Ser Ala Ala Gly Arg Ala Gly Thr Val Leu Ala Ala
    1265                1270                1275

Ala Gly Gly Gly Val Glu Val Val Gly Thr Ala Ala Gly Leu Ala
    1280                1285                1290

Thr Pro Pro Arg Arg Glu Pro Val Asp Met Asp Ala Glu Leu Glu
    1295                1300                1305

Asp Asp Asp Asp Gly Leu Phe Gly Glu
    1310                1315

<210> 2
<211> 391
<212> PRT

<213> Artificial Sequence

<220>
<223> Description of Artificial Sequence: Synthetic
      polypeptide

<400> 2
Met Val Leu Tyr Gly Gly Leu Gly Asp Ser Arg Pro Gly Leu Trp Gly
1               5                   10                  15

Ala Pro Glu Ala Glu Glu Ala Arg Ala Arg Phe Glu Ala Ser Gly Ala
            20                  25                  30

Pro Ala Pro Val Trp Ala Pro Glu Leu Gly Asp Ala Ala Gln Gln Tyr
        35                  40                  45

Ala Leu Ile Thr Arg Leu Leu Tyr Thr Pro Asp Ala Glu Ala Met Gly
    50                  55                  60

Trp Leu Gln Asn Pro Arg Val Ala Pro Gly Asp Val Ala Leu Asp Gln
65                  70                  75                  80

Ala Cys Phe Arg Ile Ser Gly Ala Ala Arg Asn Ser Ser Ser Phe Ile
                85                  90                  95

Ser Gly Ser Val Ala Arg Ala Val Pro His Leu Gly Tyr Ala Met Ala
            100                 105                 110

Ala Gly Arg Phe Gly Trp Gly Leu Ala His Val Ala Ala Ala Val Ala
        115                 120                 125

Met Ser Arg Arg Tyr Asp Arg Ala Gln Lys Gly Phe Leu Leu Thr Ser
    130                 135                 140

Leu Arg Arg Ala Tyr Ala Pro Leu Leu Ala Arg Glu Asn Ala Ala Leu
145                 150                 155                 160

Thr Gly Ala Arg Thr Pro Asp Asp Gly Gly Asp Ala Asn Arg Arg Asp
                165                 170                 175

Gly Asp Asp Ala Arg Gly Lys Pro Ala Ala Ala Ala Ala Pro Leu Pro
            180                 185                 190

Ser Ala Ala Ala Ser Pro Ala Asp Glu Arg Ala Val Pro Ala Gly Tyr
        195                 200                 205

Gly Ala Ala Gly Val Leu Ala Ala Leu Gly Arg Leu Ser Ala Ala Pro
    210                 215                 220

Ala Ser Ala Pro Ala Gly Ala Asp Asp Asp Asp Asp Asp Asp Asp Gly
225                 230                 235                 240

Ala Gly Gly Gly Gly Gly Gly Gly Gly Gly Gly Gly Gly Arg Arg Ala
                245                 250                 255

Glu Ala Gly Arg Val Ala Val Glu Cys Leu Ala Ala Cys Arg Gly Ile
            260                 265                 270

Leu Glu Ala Leu Ala Glu Gly Phe Asp Gly Asp Leu Ala Ala Val Pro
        275                 280                 285

Gly Leu Ala Gly Ala Arg Pro Ala Ala Pro Pro Arg Pro Gly Pro Ala
    290                 295                 300

Gly Ala Ala Ala Pro Pro His Ala Asp Ala Pro Arg Leu Arg Ala Trp
305                 310                 315                 320

Leu Arg Glu Leu Arg Phe Val Arg Asp Ala Leu Val Leu Met Arg Leu
                325                 330                 335

Arg Gly Asp Leu Arg Val Ala Gly Gly Ser Glu Ala Ala Val Ala Ala
            340                 345                 350

Val Arg Ala Val Ser Leu Val Ala Gly Ala Leu Gly Pro Ala Leu Pro
        355                 360                 365

Arg Ser Pro Arg Leu Leu Ser Ser Ala Ala Ala Ala Ala Ala Asp Leu
    370                 375                 380

Leu Phe Gln Asn Gln Ser Leu
385                 390

<210> 3
<211> 224
<212> PRT
<213> Human herpesvirus 2

<400> 3
Met Gly Phe Val Cys Leu Phe Gly Leu Val Val Met Gly Ala Trp Gly
1               5                   10                  15

Ala Trp Gly Gly Ser Gln Ala Thr Glu Tyr Val Leu Arg Ser Val Ile
            20                  25                  30

Ala Lys Glu Val Gly Asp Ile Leu Arg Val Pro Cys Met Arg Thr Pro
        35                  40                  45

Ala Asp Asp Val Ser Trp Arg Tyr Glu Ala Pro Ser Val Ile Asp Tyr

50                  55                  60

Ala Arg Ile Asp Gly Ile Phe Leu Arg Tyr His Cys Pro Gly Leu Asp
65                  70                  75                  80

Thr Phe Leu Trp Asp Arg His Ala Gln Arg Ala Tyr Leu Val Asn Pro
                85                  90                  95

Phe Leu Phe Ala Ala Gly Phe Leu Glu Asp Leu Ser His Ser Val Phe
            100                 105                 110

Pro Ala Asp Thr Gln Glu Thr Thr Thr Arg Arg Ala Leu Tyr Lys Glu
        115                 120                 125

Ile Arg Asp Ala Leu Gly Ser Arg Lys Gln Ala Val Ser His Ala Pro
    130                 135                 140

Val Arg Ala Gly Cys Val Asn Phe Asp Tyr Ser Arg Thr Arg Arg Cys
145                 150                 155                 160

Val Gly Arg Arg Asp Leu Arg Pro Ala Asn Thr Thr Ser Thr Trp Glu
                165                 170                 175

Pro Pro Val Ser Ser Asp Asp Glu Ala Ser Ser Gln Ser Lys Pro Leu
            180                 185                 190

Ala Thr Gln Pro Pro Val Leu Ala Leu Ser Asn Ala Pro Pro Arg Arg
        195                 200                 205

Val Ser Pro Thr Arg Gly Arg Arg Arg His Thr Arg Leu Arg Arg Asn
    210                 215                 220

<210> 4
<211> 347
<212> PRT
<213> Artificial Sequence

<220>
<223> Description of Artificial Sequence: Synthetic
      polypeptide

<400> 4
Asn Arg Trp Lys Tyr Ala Leu Ala Asp Pro Ser Leu Lys Met Ala Asp
1               5                   10                  15

Pro Asn Arg Phe Arg Gly Lys Asn Leu Pro Val Leu Asp Gln Leu Thr
            20                  25                  30

Asp Pro Pro Gly Val Lys Arg Val Tyr His Ile Gln Pro Ser Leu Glu
        35                  40                  45

Asp Pro Phe Gln Pro Pro Ser Ile Pro Ile Thr Val Tyr Tyr Ala Val
    50                  55                  60

Leu Glu Arg Ala Cys Arg Ser Val Leu Leu His Ala Pro Ser Glu Ala
65                  70                  75                  80

Pro Gln Ile Val Arg Gly Ala Ser Asp Glu Ala Arg Lys His Thr Tyr
                85                  90                  95

Asn Leu Thr Ile Ala Trp Tyr Arg Met Gly Asp Asn Cys Ala Ile Pro
            100                 105                 110

Ile Thr Val Met Glu Tyr Thr Glu Cys Pro Tyr Asn Lys Ser Leu Gly
        115                 120                 125

Val Cys Pro Ile Arg Thr Gln Pro Arg Trp Ser Tyr Tyr Asp Ser Phe
    130                 135                 140

Ser Ala Val Ser Glu Asp Asn Leu Gly Phe Leu Met His Ala Pro Ala
145                 150                 155                 160

Phe Glu Thr Ala Gly Thr Tyr Leu Arg Leu Val Lys Ile Asn Asp Trp
                165                 170                 175

Thr Glu Ile Thr Gln Phe Ile Leu Glu His Arg Ala Arg Ala Ser Cys
            180                 185                 190

Lys Tyr Ala Leu Pro Leu Arg Ile Pro Pro Ala Ala Cys Leu Thr Ser
        195                 200                 205

Lys Ala Tyr Gln Gln Gly Val Thr Val Asp Ser Ile Gly Met Leu Pro
    210                 215                 220

Arg Phe Ile Pro Glu Asn Gln Arg Thr Val Ala Leu Tyr Ser Leu Lys
225                 230                 235                 240

Ile Ala Gly Trp His Gly Pro Lys Pro Pro Tyr Thr Ser Thr Leu Leu
                245                 250                 255

Pro Pro Glu Leu Ser Asp Thr Thr Asn Ala Thr Gln Pro Glu Leu Val
            260                 265                 270

Pro Glu Asp Pro Glu Asp Ser Ala Leu Leu Glu Asp Pro Ala Gly Thr
        275                 280                 285

Val Ser Ser Gln Ile Pro Pro Asn Trp His Ile Pro Ser Ile Gln Asp

290                 295                 300

Val Ala Pro His His Ala Pro Ala Ala Pro Ser Asn Pro Arg Arg Arg
305                 310                 315                 320

Ala Gln Met Ala Pro Lys Arg Leu Arg Leu Pro His Ile Arg Asp Asp
                325                 330                 335

Asp Ala Pro Pro Ser His Gln Pro Leu Phe Tyr
            340                 345

<210> 5
<211> 393
<212> PRT
<213> Artificial Sequence

<220>
<223> Description of Artificial Sequence: Synthetic
      polypeptide

<400> 5
Met Gly Arg Leu Thr Ser Gly Val Gly Thr Ala Ala Leu Leu Val Val
1               5                   10                  15

Ala Val Gly Leu Arg Val Val Cys Ala Lys Tyr Ala Leu Ala Asp Pro
            20                  25                  30

Ser Leu Lys Met Ala Asp Pro Asn Arg Phe Arg Gly Lys Asn Leu Pro
        35                  40                  45

Val Leu Asp Gln Leu Thr Asp Pro Pro Gly Val Lys Arg Val Tyr His
    50                  55                  60

Ile Gln Pro Ser Leu Glu Asp Pro Phe Gln Pro Pro Ser Ile Pro Ile
65                  70                  75                  80

Thr Val Tyr Tyr Ala Val Leu Glu Arg Ala Cys Arg Ser Val Leu Leu
                85                  90                  95

His Ala Pro Ser Glu Ala Pro Gln Ile Val Arg Gly Ala Ser Asp Glu
            100                 105                 110

Ala Arg Lys His Thr Tyr Asn Leu Thr Ile Ala Trp Tyr Arg Met Gly
        115                 120                 125

Asp Asn Cys Ala Ile Pro Ile Thr Val Met Glu Tyr Thr Glu Cys Pro
    130                 135                 140

Tyr Asn Lys Ser Leu Gly Val Cys Pro Ile Arg Thr Gln Pro Arg Trp
145                 150                 155                 160

Ser Tyr Tyr Asp Ser Phe Ser Ala Val Ser Glu Asp Asn Leu Gly Phe
                165                 170                 175

Leu Met His Ala Pro Ala Phe Glu Thr Ala Gly Thr Tyr Leu Arg Leu
            180                 185                 190

Val Lys Ile Asn Asp Trp Thr Glu Ile Thr Gln Phe Ile Leu Glu His
        195                 200                 205

Arg Ala Arg Ala Ser Cys Lys Tyr Ala Leu Pro Leu Arg Ile Pro Pro
    210                 215                 220

Ala Ala Cys Leu Thr Ser Lys Ala Tyr Gln Gln Gly Val Thr Val Asp
225                 230                 235                 240

Ser Ile Gly Met Leu Pro Arg Phe Ile Pro Glu Asn Gln Arg Thr Val
                245                 250                 255

Ala Leu Tyr Ser Leu Lys Ile Ala Gly Trp His Gly Pro Lys Pro Pro
            260                 265                 270

Tyr Thr Ser Thr Leu Leu Pro Pro Glu Leu Ser Asp Thr Thr Asn Ala
        275                 280                 285

Thr Gln Pro Glu Leu Val Pro Glu Asp Pro Glu Asp Ser Ala Leu Leu
    290                 295                 300

Glu Asp Pro Ala Gly Thr Val Ser Ser Gln Ile Pro Pro Asn Trp His
305                 310                 315                 320

Ile Pro Ser Ile Gln Asp Val Ala Pro His His Ala Pro Ala Ala Pro
                325                 330                 335

Ser Asn Pro Gly Leu Ile Ile Gly Ala Leu Ala Gly Ser Thr Leu Ala
            340                 345                 350

Val Leu Val Ile Gly Gly Ile Ala Phe Trp Val Arg Arg Arg Ala Gln
        355                 360                 365

Met Ala Pro Lys Arg Leu Arg Leu Pro His Ile Arg Asp Asp Asp Ala
    370                 375                 380

Pro Pro Ser His Gln Pro Leu Phe Tyr
385                 390

<210> 6

<211> 261
<212> PRT
<213> Human herpesvirus 2

<400> 6
Met Ser Arg Arg Arg Gly Pro Arg Arg Arg Gly Pro Arg Arg Arg Pro
1               5                   10                  15

Arg Pro Gly Ala Pro Ala Val Pro Arg Pro Gly Ala Pro Ala Val Pro
            20                  25                  30

Arg Pro Gly Ala Leu Pro Thr Ala Asp Ser Gln Met Val Pro Ala Tyr
        35                  40                  45

Asp Ser Gly Thr Ala Val Glu Ser Ala Pro Ala Ala Ser Ser Leu Leu
    50                  55                  60

Arg Arg Trp Leu Leu Val Pro Gln Ala Asp Asp Ser Asp Asp Ala Asp
65                  70                  75                  80

Tyr Ala Gly Asn Asp Asp Ala Glu Trp Ala Asn Ser Pro Pro Ser Glu
                85                  90                  95

Gly Gly Gly Lys Ala Pro Glu Ala Pro His Ala Ala Pro Ala Ala Ala
            100                 105                 110

Cys Pro Pro Pro Pro Pro Arg Lys Glu Arg Gly Pro Gln Arg Pro Leu
        115                 120                 125

Pro Pro His Leu Ala Leu Arg Leu Arg Thr Thr Thr Glu Tyr Leu Ala
    130                 135                 140

Arg Leu Ser Leu Arg Arg Arg Arg Pro Pro Ala Ser Pro Pro Ala Asp
145                 150                 155                 160

Ala Pro Arg Gly Lys Val Cys Phe Ser Pro Arg Val Gln Val Arg His
                165                 170                 175

Leu Val Ala Trp Glu Thr Ala Ala Arg Leu Ala Arg Arg Gly Ser Trp
            180                 185                 190

Ala Arg Glu Arg Ala Asp Arg Asp Arg Phe Arg Arg Arg Val Ala Ala
        195                 200                 205

Ala Glu Ala Val Ile Gly Pro Cys Leu Glu Pro Glu Ala Arg Ala Arg
    210                 215                 220

Ala Arg Ala Arg Ala Arg Ala His Glu Asp Gly Gly Pro Ala Glu Glu
225                 230                 235                 240

Glu Glu Ala Ala Ala Ala Ala Arg Gly Ser Ser Ala Ala Ala Gly Pro
                245                 250                 255

Gly Arg Arg Ala Val
            260

<210> 7
<211> 825
<212> PRT
<213> Human herpesvirus 2

<400> 7
Met Glu Pro Arg Pro Gly Thr Ser Ser Arg Ala Asp Pro Gly Pro Glu
1               5                   10                  15

Arg Pro Pro Arg Gln Thr Pro Gly Thr Gln Pro Ala Ala Pro His Ala
            20                  25                  30

Trp Gly Met Leu Asn Asp Met Gln Trp Leu Ala Ser Ser Asp Ser Glu
        35                  40                  45

Glu Glu Thr Glu Val Gly Ile Ser Asp Asp Asp Leu His Arg Asp Ser
    50                  55                  60

Thr Ser Glu Ala Gly Ser Thr Asp Thr Glu Met Phe Glu Ala Gly Leu
65                  70                  75                  80

Met Asp Ala Ala Thr Pro Pro Ala Arg Pro Pro Ala Glu Arg Gln Gly
                85                  90                  95

Ser Pro Thr Pro Ala Asp Ala Gln Gly Ser Cys Gly Gly Gly Pro Val
            100                 105                 110

Gly Glu Glu Glu Ala Glu Ala Gly Gly Gly Gly Asp Val Cys Ala Val
        115                 120                 125

Cys Thr Asp Glu Ile Ala Pro Pro Leu Arg Cys Gln Ser Phe Pro Cys
    130                 135                 140

Leu His Pro Phe Cys Ile Pro Cys Met Lys Thr Trp Ile Pro Leu Arg
145                 150                 155                 160

Asn Thr Cys Pro Leu Cys Asn Thr Pro Val Ala Tyr Leu Ile Val Gly
                165                 170                 175

Val Thr Ala Ser Gly Ser Phe Ser Thr Ile Pro Ile Val Asn Asp Pro
            180                 185                 190

Arg Thr Arg Val Glu Ala Glu Ala Ala Val Arg Ala Gly Thr Ala Val
        195                 200                 205

Asp Phe Ile Trp Thr Gly Asn Pro Arg Thr Ala Pro Arg Ser Leu Ser
    210                 215                 220

Leu Gly Gly His Thr Val Arg Ala Leu Ser Pro Thr Pro Pro Trp Pro
225                 230                 235                 240

Gly Thr Asp Asp Glu Asp Asp Asp Leu Ala Asp Val Asp Tyr Val Pro
                245                 250                 255

Pro Ala Pro Arg Arg Ala Pro Arg Arg Gly Gly Gly Gly Ala Gly Ala
            260                 265                 270

Thr Arg Gly Thr Ser Gln Pro Ala Ala Thr Arg Pro Ala Pro Pro Gly
        275                 280                 285

Ala Pro Arg Ser Ser Ser Ser Gly Gly Ala Pro Leu Arg Ala Gly Val
    290                 295                 300

Gly Ser Gly Ser Gly Gly Gly Pro Ala Val Ala Ala Val Val Pro Arg
305                 310                 315                 320

Val Ala Ser Leu Pro Pro Ala Ala Gly Gly Gly Arg Ala Gln Ala Arg
                325                 330                 335

Arg Val Gly Glu Asp Ala Ala Ala Ala Glu Gly Arg Thr Pro Pro Ala
            340                 345                 350

Arg Gln Pro Arg Ala Ala Gln Glu Pro Pro Ile Val Ile Ser Asp Ser
        355                 360                 365

Pro Pro Pro Ser Pro Arg Arg Pro Ala Gly Pro Gly Pro Leu Ser Phe
    370                 375                 380

Val Ser Ser Ser Ser Ala Gln Val Ser Ser Gly Pro Gly Gly Gly Gly
385                 390                 395                 400

Leu Pro Gln Ser Ser Gly Arg Ala Ala Arg Pro Arg Ala Ala Val Ala
                405                 410                 415

Pro Arg Val Arg Ser Pro Pro Arg Ala Ala Ala Ala Pro Val Val Ser
            420                 425                 430

Ala Ser Ala Asp Ala Ala Gly Pro Ala Pro Pro Ala Val Pro Val Asp
        435                 440                 445

Ala His Arg Ala Pro Arg Ser Arg Met Thr Gln Ala Gln Thr Asp Thr
    450                 455                 460

Gln Ala Gln Ser Leu Gly Arg Ala Gly Ala Thr Asp Ala Arg Gly Ser
465                 470                 475                 480

Gly Gly Pro Gly Ala Glu Gly Gly Pro Gly Val Pro Arg Gly Thr Asn
                485                 490                 495

Thr Pro Gly Ala Ala Pro His Ala Ala Glu Gly Ala Ala Ala Arg Pro
            500                 505                 510

Arg Lys Arg Arg Gly Ser Asp Ser Gly Pro Ala Ala Ser Ser Ser Ala
        515                 520                 525

Ser Ser Ser Ala Ala Pro Arg Ser Pro Leu Ala Pro Gln Gly Val Gly
    530                 535                 540

Ala Lys Arg Ala Ala Pro Arg Arg Ala Pro Asp Ser Asp Ser Gly Asp
545                 550                 555                 560

Arg Gly His Gly Pro Leu Ala Pro Ala Ser Ala Gly Ala Ala Pro Pro
                565                 570                 575

Ser Ala Ser Pro Ser Ser Gln Ala Ala Val Ala Ala Ala Ser Ser Ser
            580                 585                 590

Ser Ala Ser Ser Ser Ser Ala Ser Ser Ser Ser Ala Ser Ser Ser Ser
        595                 600                 605

Ala Ser Ser Ser Ser Ala Ser Ser Ser Ser Ala Ser Ser Ser Ser Ala
    610                 615                 620

Ser Ser Ser Ala Gly Gly Ala Gly Gly Ser Val Ala Ser Ala Ser Gly
625                 630                 635                 640

Ala Gly Glu Arg Arg Glu Thr Ser Leu Gly Pro Arg Ala Ala Ala Pro
                645                 650                 655

Arg Gly Pro Arg Lys Cys Ala Arg Lys Thr Arg His Ala Glu Gly Gly
            660                 665                 670

Pro Glu Pro Gly Ala Arg Asp Pro Ala Pro Gly Leu Thr Arg Tyr Leu
        675                 680                 685

Pro Ile Ala Gly Val Ser Ser Val Val Ala Leu Ala Pro Tyr Val Asn

690                 695                 700

Lys Thr Val Thr Gly Asp Cys Leu Pro Val Leu Asp Met Glu Thr Gly
705                 710                 715                 720

His Ile Gly Ala Tyr Val Val Leu Val Asp Gln Thr Gly Asn Val Ala
                725                 730                 735

Asp Leu Leu Arg Ala Ala Ala Pro Ala Trp Ser Arg Arg Thr Leu Leu
            740                 745                 750

Pro Glu His Ala Arg Asn Cys Val Arg Pro Pro Asp Tyr Pro Thr Pro
        755                 760                 765

Pro Ala Ser Glu Trp Asn Ser Leu Trp Met Thr Pro Val Gly Asn Met
    770                 775                 780

Leu Phe Asp Gln Gly Thr Leu Val Gly Ala Leu Asp Phe His Gly Leu
785                 790                 795                 800

Arg Ser Arg His Pro Trp Ser Arg Glu Gln Gly Ala Pro Ala Pro Ala
                805                 810                 815

Gly Asp Ala Pro Ala Gly His Gly Glu
            820                 825

<210> 8
<211> 400
<212> PRT
<213> Artificial Sequence

<220>
<223> Description of Artificial Sequence: Synthetic
      polypeptide

<400> 8
Met Ser Ala Glu Gln Arg Lys Lys Lys Lys Thr Thr Thr Thr Thr Gln
1               5                   10                  15

Gly Arg Gly Ala Glu Val Ala Met Ala Asp Glu Asp Gly Gly Arg Leu
            20                  25                  30

Arg Ala Ala Ala Glu Thr Thr Gly Gly Pro Gly Ser Pro Asp Pro Ala
        35                  40                  45

Asp Gly Pro Pro Pro Thr Pro Asn Pro Asp Arg Arg Pro Ala Ala Arg
    50                  55                  60

Pro Gly Phe Gly Trp His Gly Gly Pro Glu Glu Asn Glu Asp Glu Ala
65                  70                  75                  80

Asp Asp Ala Ala Ala Asp Ala Asp Ala Asp Glu Ala Ala Pro Ala Ser
                85                  90                  95

Gly Glu Ala Val Asp Glu Pro Ala Ala Asp Gly Val Val Ser Pro Arg
            100                 105                 110

Gln Leu Ala Leu Leu Ala Ser Met Val Asp Glu Ala Val Arg Thr Ile
        115                 120                 125

Pro Ser Pro Pro Pro Glu Arg Asp Gly Ala Gln Glu Glu Ala Ala Arg
    130                 135                 140

Ser Pro Ser Pro Pro Arg Thr Pro Ser Met Arg Ala Asp Tyr Gly Glu
145                 150                 155                 160

Glu Asn Asp Asp Asp Asp Asp Asp Asp Asp Asp Asp Asp Arg Asp Ala
                165                 170                 175

Gly Arg Trp Val Arg Gly Pro Glu Thr Thr Ser Ala Val Arg Gly Ala
            180                 185                 190

Tyr Pro Asp Pro Met Ala Ser Leu Ser Pro Arg Pro Pro Ala Pro Arg
        195                 200                 205

Arg His His His His His His His Arg Arg Arg Arg Ala Pro Arg Arg
    210                 215                 220

Arg Ser Ala Ala Ser Asp Ser Ser Lys Ser Gly Ser Ser Ser Ser Ala
225                 230                 235                 240

Ser Ser Ala Ser Ser Ser Ala Ser Ser Ser Ser Ser Ala Ser Ala Ser
                245                 250                 255

Ser Ser Asp Asp Asp Asp Asp Asp Asp Ala Ala Arg Ala Pro Ala Ser
            260                 265                 270

Ala Ala Asp His Ala Ala Gly Gly Thr Leu Gly Ala Asp Asp Glu Glu
        275                 280                 285

Ala Gly Val Pro Ala Arg Ala Pro Gly Ala Ala Pro Arg Pro Ser Pro
    290                 295                 300

Pro Arg Ala Glu Pro Ala Pro Ala Arg Thr Pro Ala Ala Thr Ala Gly
305                 310                 315                 320

Arg Leu Glu Arg Arg Arg Ala Arg Ala Ala Val Ala Gly Arg Asp Ala

325                 330                 335

Thr Gly Arg Phe Thr Ala Gly Arg Pro Arg Arg Val Glu Leu Asp Ala
            340                 345                 350

Asp Ala Ala Ser Gly Ala Phe Tyr Ala Arg Tyr Arg Asp Gly Tyr Val
        355                 360                 365

Ser Gly Glu Pro Trp Pro Gly Ala Gly Pro Pro Pro Pro Gly Arg Val
    370                 375                 380

Leu Tyr Gly Gly Leu Gly Asp Ser Arg Pro Gly Leu Trp Gly Ala Pro
385                 390                 395                 400

<210> 9
<211> 275
<212> PRT
<213> Artificial Sequence

<220>
<223> Description of Artificial Sequence: Synthetic
      polypeptide

<400> 9
Ser Ser Ala Ala Ala Ala Ala Ala Asp Leu Leu Phe Gln Asn Gln Ser
1               5                   10                  15

Leu Arg Pro Leu Leu Ala Asp Thr Val Ala Ala Ala Asp Ser Leu Ala
            20                  25                  30

Ala Pro Ala Ser Ala Pro Arg Glu Ala Arg Lys Arg Lys Ser Pro Ala
        35                  40                  45

Pro Ala Arg Ala Pro Pro Gly Gly Ala Pro Arg Pro Pro Lys Lys Ser
    50                  55                  60

Arg Ala Asp Ala Pro Arg Pro Ala Ala Ala Pro Pro Ala Gly Ala Ala
65                  70                  75                  80

Pro Pro Ala Pro Pro Thr Pro Pro Pro Arg Pro Pro Arg Pro Ala Ala
                85                  90                  95

Leu Thr Arg Arg Pro Ala Glu Gly Pro Asp Pro Gln Gly Gly Trp Arg
            100                 105                 110

Arg Gln Pro Pro Gly Pro Ser His Thr Pro Ala Pro Ser Ala Ala Ala
        115                 120                 125

Leu Glu Ala Tyr Cys Ala Pro Arg Ala Val Ala Glu Leu Thr Asp His
    130                 135                 140

Pro Leu Phe Pro Ala Pro Trp Arg Pro Ala Leu Met Phe Asp Pro Arg
145                 150                 155                 160

Ala Leu Ala Ser Leu Ala Ala Arg Cys Ala Ala Pro Pro Pro Gly Gly
                165                 170                 175

Ala Pro Ala Ala Phe Gly Pro Leu Arg Ala Ser Gly Pro Leu Arg Arg
            180                 185                 190

Ala Ala Ala Trp Met Arg Gln Val Pro Asp Pro Glu Asp Val Arg Val
        195                 200                 205

Val Ile Leu Tyr Ser Pro Leu Pro Gly Glu Asp Leu Ala Ala Gly Arg
    210                 215                 220

Ala Gly Gly Gly Pro Pro Pro Glu Trp Ser Ala Glu Arg Gly Gly Leu
225                 230                 235                 240

Ser Cys Leu Leu Ala Ala Leu Gly Asn Arg Leu Cys Gly Pro Ala Thr
                245                 250                 255

Ala Ala Trp Ala Gly Asn Trp Thr Gly Ala Pro Asp Val Ser Ala Leu
            260                 265                 270

Gly Ala Gln
        275

<210> 10
<211> 311
<212> PRT
<213> Artificial Sequence

<220>
<223> Description of Artificial Sequence: Synthetic
      polypeptide

<400> 10
Trp Ala Gly Asn Trp Thr Gly Ala Pro Asp Val Ser Ala Leu Gly Ala
1               5                   10                  15

Gln Gly Val Leu Leu Leu Ser Thr Arg Asp Leu Ala Phe Ala Gly Ala
            20                  25                  30

Val Glu Phe Leu Gly Leu Leu Ala Gly Ala Cys Asp Arg Arg Leu Ile
        35                  40                  45

Val Val Asn Ala Val Arg Ala Ala Asp Trp Pro Ala Asp Gly Pro Val
    50                  55                  60

Val Ser Arg Gln His Ala Tyr Leu Ala Cys Glu Val Leu Pro Ala Val
65                  70                  75                  80

Gln Cys Ala Val Arg Trp Pro Ala Ala Arg Asp Leu Arg Arg Thr Val
                85                  90                  95

Leu Ala Ser Gly Arg Val Phe Gly Pro Gly Val Phe Ala Arg Val Glu
            100                 105                 110

Ala Ala His Ala Arg Leu Tyr Pro Asp Ala Pro Pro Leu Arg Leu Cys
        115                 120                 125

Arg Gly Ala Asn Val Arg Tyr Arg Val Arg Thr Arg Phe Gly Pro Asp
    130                 135                 140

Thr Leu Val Pro Met Ser Pro Arg Glu Tyr Arg Arg Ala Val Leu Pro
145                 150                 155                 160

Ala Leu Asp Gly Arg Ala Ala Ala Ser Gly Ala Gly Asp Ala Met Ala
                165                 170                 175

Pro Gly Ala Pro Asp Phe Cys Glu Asp Glu Ala His Ser His Arg Ala
            180                 185                 190

Cys Ala Arg Trp Gly Leu Gly Ala Pro Leu Arg Pro Val Tyr Val Ala
        195                 200                 205

Leu Gly Arg Asp Ala Val Arg Gly Gly Pro Ala Glu Leu Arg Gly Pro
    210                 215                 220

Arg Arg Glu Phe Cys Ala Arg Ala Leu Leu Glu Pro Asp Gly Asp Ala
225                 230                 235                 240

Pro Pro Leu Val Leu Arg Asp Asp Ala Asp Ala Gly Pro Pro Pro Gln
                245                 250                 255

Ile Arg Trp Ala Ser Ala Ala Gly Arg Ala Gly Thr Val Leu Ala Ala
            260                 265                 270

Ala Gly Gly Gly Val Glu Val Val Gly Thr Ala Ala Gly Leu Ala Thr
        275                 280                 285

Pro Pro Arg Arg Glu Pro Val Asp Met Asp Ala Glu Leu Glu Asp Asp
    290                 295                 300

Asp Asp Gly Leu Phe Gly Glu
305                 310

<210> 11
<211> 569
<212> PRT
<213> Artificial Sequence

<220>
<223> Description of Artificial Sequence: Synthetic
      polypeptide

<400> 11
Ser Ser Ala Ala Ala Ala Ala Ala Asp Leu Leu Phe Gln Asn Gln Ser
1               5                   10                  15

Leu Arg Pro Leu Leu Ala Asp Thr Val Ala Ala Ala Asp Ser Leu Ala
            20                  25                  30

Ala Pro Ala Ser Ala Pro Arg Glu Ala Arg Lys Arg Lys Ser Pro Ala
        35                  40                  45

Pro Ala Arg Ala Pro Pro Gly Gly Ala Pro Arg Pro Pro Lys Lys Ser
    50                  55                  60

Arg Ala Asp Ala Pro Arg Pro Ala Ala Ala Pro Pro Ala Gly Ala Ala
65                  70                  75                  80

Pro Pro Ala Pro Pro Thr Pro Pro Pro Arg Pro Pro Arg Pro Ala Ala
                85                  90                  95

Leu Thr Arg Arg Pro Ala Glu Gly Pro Asp Pro Gln Gly Gly Trp Arg
            100                 105                 110

Arg Gln Pro Pro Gly Pro Ser His Thr Pro Ala Pro Ser Ala Ala Ala
        115                 120                 125

Leu Glu Ala Tyr Cys Ala Pro Arg Ala Val Ala Glu Leu Thr Asp His
    130                 135                 140

Pro Leu Phe Pro Ala Pro Trp Arg Pro Ala Leu Met Phe Asp Pro Arg
145                 150                 155                 160

Ala Leu Ala Ser Leu Ala Ala Arg Cys Ala Ala Pro Pro Pro Gly Gly
                165                 170                 175

Ala Pro Ala Ala Phe Gly Pro Leu Arg Ala Ser Gly Pro Leu Arg Arg
            180                 185                 190

Ala Ala Ala Trp Met Arg Gln Val Pro Asp Pro Glu Asp Val Arg Val
        195                 200                 205

Val Ile Leu Tyr Ser Pro Leu Pro Gly Glu Asp Leu Ala Ala Gly Arg
    210                 215                 220

Ala Gly Gly Gly Pro Pro Pro Glu Trp Ser Ala Glu Arg Gly Gly Leu
225                 230                 235                 240

Ser Cys Leu Leu Ala Ala Leu Gly Asn Arg Leu Cys Gly Pro Ala Thr
                245                 250                 255

Ala Ala Trp Ala Gly Asn Trp Thr Gly Ala Pro Asp Val Ser Ala Leu
            260                 265                 270

Gly Ala Gln Gly Val Leu Leu Leu Ser Thr Arg Asp Leu Ala Phe Ala
        275                 280                 285

Gly Ala Val Glu Phe Leu Gly Leu Leu Ala Gly Ala Cys Asp Arg Arg
    290                 295                 300

Leu Ile Val Val Asn Ala Val Arg Ala Ala Asp Trp Pro Ala Asp Gly
305                 310                 315                 320

Pro Val Val Ser Arg Gln His Ala Tyr Leu Ala Cys Glu Val Leu Pro
                325                 330                 335

Ala Val Gln Cys Ala Val Arg Trp Pro Ala Ala Arg Asp Leu Arg Arg
            340                 345                 350

Thr Val Leu Ala Ser Gly Arg Val Phe Gly Pro Gly Val Phe Ala Arg
        355                 360                 365

Val Glu Ala Ala His Ala Arg Leu Tyr Pro Asp Ala Pro Pro Leu Arg
    370                 375                 380

Leu Cys Arg Gly Ala Asn Val Arg Tyr Arg Val Arg Thr Arg Phe Gly
385                 390                 395                 400

Pro Asp Thr Leu Val Pro Met Ser Pro Arg Glu Tyr Arg Arg Ala Val
                405                 410                 415

Leu Pro Ala Leu Asp Gly Arg Ala Ala Ala Ser Gly Ala Gly Asp Ala
            420                 425                 430

Met Ala Pro Gly Ala Pro Asp Phe Cys Glu Asp Glu Ala His Ser His
        435                 440                 445

Arg Ala Cys Ala Arg Trp Gly Leu Gly Ala Pro Leu Arg Pro Val Tyr
    450                 455                 460

Val Ala Leu Gly Arg Asp Ala Val Arg Gly Gly Pro Ala Glu Leu Arg
465                 470                 475                 480

Gly Pro Arg Arg Glu Phe Cys Ala Arg Ala Leu Leu Glu Pro Asp Gly
                485                 490                 495

Asp Ala Pro Pro Leu Val Leu Arg Asp Asp Ala Asp Ala Gly Pro Pro
            500                 505                 510

Pro Gln Ile Arg Trp Ala Ser Ala Ala Gly Arg Ala Gly Thr Val Leu
        515                 520                 525

Ala Ala Ala Gly Gly Gly Val Glu Val Val Gly Thr Ala Ala Gly Leu
    530                 535                 540

Ala Thr Pro Pro Arg Arg Glu Pro Val Asp Met Asp Ala Glu Leu Glu
545                 550                 555                 560

Asp Asp Asp Asp Gly Leu Phe Gly Glu
                565

<210> 12
<211> 543
<212> PRT
<213> Artificial Sequence

<220>
<223> Description of Artificial Sequence: Synthetic
      polypeptide

<400> 12
Thr Ala Gly Arg Pro Arg Arg Val Glu Leu Asp Ala Asp Ala Ala Ser
1               5                   10                  15

Gly Ala Phe Tyr Ala Arg Tyr Arg Asp Gly Tyr Val Ser Gly Glu Pro
            20                  25                  30

Trp Pro Gly Ala Gly Pro Pro Pro Pro Gly Arg Val Leu Tyr Gly Gly
        35                  40                  45

Leu Gly Asp Ser Arg Pro Gly Leu Trp Gly Ala Pro Glu Ala Glu Glu
    50                  55                  60

Ala Arg Ala Arg Phe Glu Ala Ser Gly Ala Pro Ala Pro Val Trp Ala
65                  70                  75                  80

Pro Glu Leu Gly Asp Ala Ala Gln Gln Tyr Ala Leu Ile Thr Arg Leu
                85                  90                  95

Leu Tyr Thr Pro Asp Ala Glu Ala Met Gly Trp Leu Gln Asn Pro Arg
            100                 105                 110

Val Ala Pro Gly Asp Val Ala Leu Asp Gln Ala Cys Phe Arg Ile Ser
        115                 120                 125

Gly Ala Ala Arg Asn Ser Ser Ser Phe Ile Ser Gly Ser Val Ala Arg
    130                 135                 140

Ala Val Pro His Leu Gly Tyr Ala Met Ala Ala Gly Arg Phe Gly Trp
145                 150                 155                 160

Gly Leu Ala His Val Ala Ala Ala Val Ala Met Ser Arg Arg Tyr Asp
                165                 170                 175

Arg Ala Gln Lys Gly Phe Leu Leu Thr Ser Leu Arg Arg Ala Tyr Ala
            180                 185                 190

Pro Leu Leu Ala Arg Glu Asn Ala Ala Leu Thr Gly Ala Arg Thr Pro
        195                 200                 205

Asp Asp Gly Gly Asp Ala Asn Arg His Asp Gly Asp Asp Ala Arg Gly
    210                 215                 220

Lys Pro Ala Ala Ala Ala Ala Pro Leu Pro Ser Ala Ala Ala Ser Pro
225                 230                 235                 240

Ala Asp Glu Arg Ala Val Pro Ala Gly Tyr Gly Ala Ala Gly Val Leu
                245                 250                 255

Ala Ala Leu Gly Arg Leu Ser Ala Ala Pro Ala Ser Ala Pro Ala Gly
            260                 265                 270

Ala Asp Asp Asp Asp Asp Asp Asp Gly Ala Gly Gly Gly Gly Gly Gly
        275                 280                 285

Arg Arg Ala Glu Ala Gly Arg Val Ala Val Glu Cys Leu Ala Ala Cys
    290                 295                 300

Arg Gly Ile Leu Glu Ala Leu Ala Glu Gly Phe Asp Gly Asp Leu Ala
305                 310                 315                 320

Ala Val Pro Gly Leu Ala Gly Ala Arg Pro Ala Ala Pro Pro Arg Pro
                325                 330                 335

Gly Pro Ala Gly Ala Ala Ala Pro Pro His Ala Asp Ala Pro Arg Leu
            340                 345                 350

Arg Ala Trp Leu Arg Glu Leu Arg Phe Val Arg Asp Ala Leu Val Leu
        355                 360                 365

Met Arg Leu Arg Gly Asp Leu Arg Val Ala Gly Gly Ser Glu Ala Ala
    370                 375                 380

Val Ala Ala Val Arg Ala Val Ser Leu Val Ala Gly Ala Leu Gly Pro
385                 390                 395                 400

Ala Leu Pro Arg Ser Pro Arg Leu Leu Ser Ser Ala Ala Ala Ala Ala
                405                 410                 415

Ala Asp Leu Leu Phe Gln Asn Gln Ser Leu Arg Pro Leu Leu Ala Asp
            420                 425                 430

Thr Val Ala Ala Ala Asp Ser Leu Ala Ala Pro Ala Ser Ala Pro Arg
        435                 440                 445

Glu Ala Arg Lys Arg Lys Ser Pro Ala Pro Ala Arg Ala Pro Pro Gly
    450                 455                 460

Gly Ala Pro Arg Pro Pro Lys Lys Ser Arg Ala Asp Ala Pro Arg Pro
465                 470                 475                 480

Ala Ala Ala Pro Pro Ala Gly Ala Ala Pro Pro Ala Pro Pro Thr Pro
                485                 490                 495

Pro Pro Arg Pro Pro Arg Pro Ala Ala Leu Thr Arg Arg Pro Ala Glu
            500                 505                 510

Gly Pro Asp Pro Gln Gly Gly Trp Arg Arg Gln Pro Pro Gly Pro Ser
        515                 520                 525

His Thr Pro Ala Pro Ser Ala Ala Ala Leu Glu Ala Tyr Cys Ala
    530                 535                 540

<210> 13
<211> 544
<212> PRT
<213> Artificial Sequence

<220>
<223> Description of Artificial Sequence: Synthetic
      polypeptide

<400> 13
Ala Ala Ala Asp Ser Leu Ala Ala Pro Ala Ser Ala Pro Arg Glu Ala
1               5                   10                  15

Arg Lys Arg Lys Ser Pro Ala Pro Ala Arg Ala Pro Pro Gly Gly Ala
            20                  25                  30

Pro Arg Pro Pro Lys Lys Ser Arg Ala Asp Ala Pro Arg Pro Ala Ala
        35                  40                  45

Ala Pro Pro Ala Gly Ala Ala Pro Pro Ala Pro Pro Thr Pro Pro Pro
    50                  55                  60

Arg Pro Pro Arg Pro Ala Ala Leu Thr Arg Arg Pro Ala Glu Gly Pro
65                  70                  75                  80

Asp Pro Gln Gly Gly Trp Arg Arg Gln Pro Pro Gly Pro Ser His Thr
                85                  90                  95

Pro Ala Pro Ser Ala Ala Ala Leu Glu Ala Tyr Cys Ala Pro Arg Ala
            100                 105                 110

Val Ala Glu Leu Thr Asp His Pro Leu Phe Pro Ala Pro Trp Arg Pro
        115                 120                 125

Ala Leu Met Phe Asp Pro Arg Ala Leu Ala Ser Leu Ala Ala Arg Cys
    130                 135                 140

Ala Ala Pro Pro Pro Gly Gly Ala Pro Ala Ala Phe Gly Pro Leu Arg
145                 150                 155                 160

Ala Ser Gly Pro Leu Arg Arg Ala Ala Ala Trp Met Arg Gln Val Pro
                165                 170                 175

Asp Pro Glu Asp Val Arg Val Val Ile Leu Tyr Ser Pro Leu Pro Gly
            180                 185                 190

Glu Asp Leu Ala Ala Gly Arg Ala Gly Gly Gly Pro Pro Pro Glu Trp
        195                 200                 205

Ser Ala Glu Arg Gly Gly Leu Ser Cys Leu Leu Ala Ala Leu Gly Asn
    210                 215                 220

Arg Leu Cys Gly Pro Ala Thr Ala Ala Trp Ala Gly Asn Trp Thr Gly
225                 230                 235                 240

Ala Pro Asp Val Ser Ala Leu Gly Ala Gln Gly Val Leu Leu Leu Ser
                245                 250                 255

Thr Arg Asp Leu Ala Phe Ala Gly Ala Val Glu Phe Leu Gly Leu Leu
            260                 265                 270

Ala Gly Ala Cys Asp Arg Arg Leu Ile Val Val Asn Ala Val Arg Ala
        275                 280                 285

Ala Asp Trp Pro Ala Asp Gly Pro Val Val Ser Arg Gln His Ala Tyr
    290                 295                 300

Leu Ala Cys Glu Val Leu Pro Ala Val Gln Cys Ala Val Arg Trp Pro
305                 310                 315                 320

Ala Ala Arg Asp Leu Arg Arg Thr Val Leu Ala Ser Gly Arg Val Phe
                325                 330                 335

Gly Pro Gly Val Phe Ala Arg Val Glu Ala Ala His Ala Arg Leu Tyr
            340                 345                 350

Pro Asp Ala Pro Pro Leu Arg Leu Cys Arg Gly Ala Asn Val Arg Tyr
        355                 360                 365

Arg Val Arg Thr Arg Phe Gly Pro Asp Thr Leu Val Pro Met Ser Pro
    370                 375                 380

Arg Glu Tyr Arg Arg Ala Val Leu Pro Ala Leu Asp Gly Arg Ala Ala
385                 390                 395                 400

Ala Ser Gly Ala Gly Asp Ala Met Ala Pro Gly Ala Pro Asp Phe Cys
                405                 410                 415

Glu Asp Glu Ala His Ser His Arg Ala Cys Ala Arg Trp Gly Leu Gly
            420                 425                 430

Ala Pro Leu Arg Pro Val Tyr Val Ala Leu Gly Arg Asp Ala Val Arg
        435                 440                 445

Gly Gly Pro Ala Glu Leu Arg Gly Pro Arg Arg Glu Phe Cys Ala Arg
    450                 455                 460

Ala Leu Leu Glu Pro Asp Gly Asp Ala Pro Pro Leu Val Leu Arg Asp
465                 470                 475                 480

Asp Ala Asp Ala Gly Pro Pro Pro Gln Ile Arg Trp Ala Ser Ala Ala
                485                 490                 495

Gly Arg Ala Gly Thr Val Leu Ala Ala Ala Gly Gly Gly Val Glu Val
            500                 505                 510

Val Gly Thr Ala Ala Gly Leu Ala Thr Pro Pro Arg Arg Glu Pro Val

515                 520                 525

Asp Met Asp Ala Glu Leu Glu Asp Asp Asp Asp Gly Leu Phe Gly Glu
    530                 535                 540

<210> 14
<211> 1109
<212> PRT
<213> Artificial Sequence

<220>
<223> Description of Artificial Sequence: Synthetic
      polypeptide

<400> 14
His His His His His His His Arg Arg Arg Arg Ala Pro Arg Arg Arg
1               5                   10                  15

Ser Ala Ala Ser Asp Ser Ser Lys Ser Gly Ser Ser Ser Ser Ala Ser
            20                  25                  30

Ser Ala Ser Ser Ser Ala Ser Ser Ser Ser Ser Ala Ser Ala Ser Ser
        35                  40                  45

Ser Asp Asp Asp Asp Asp Asp Asp Ala Ala Arg Ala Pro Ala Ser Ala
    50                  55                  60

Ala Asp His Ala Ala Gly Gly Thr Leu Gly Ala Asp Asp Glu Glu Ala
65                  70                  75                  80

Gly Val Pro Ala Arg Ala Pro Gly Ala Ala Pro Arg Pro Ser Pro Pro
                85                  90                  95

Arg Ala Glu Pro Ala Pro Ala Arg Thr Pro Ala Ala Thr Ala Gly Arg
            100                 105                 110

Leu Glu Arg Arg Arg Ala Arg Ala Ala Val Ala Gly Arg Asp Ala Thr
        115                 120                 125

Gly Arg Phe Thr Ala Gly Arg Pro Arg Arg Val Glu Leu Asp Ala Asp
    130                 135                 140

Ala Ala Ser Gly Ala Phe Tyr Ala Arg Tyr Arg Asp Gly Tyr Val Ser
145                 150                 155                 160

Gly Glu Pro Trp Pro Gly Ala Gly Pro Pro Pro Pro Gly Arg Val Leu
                165                 170                 175

Tyr Gly Gly Leu Gly Asp Ser Arg Pro Gly Leu Trp Gly Ala Pro Glu
            180                 185                 190

Ala Glu Glu Ala Arg Ala Arg Phe Glu Ala Ser Gly Ala Pro Ala Pro
        195                 200                 205

Val Trp Ala Pro Glu Leu Gly Asp Ala Ala Gln Gln Tyr Ala Leu Ile
    210                 215                 220

Thr Arg Leu Leu Tyr Thr Pro Asp Ala Glu Ala Met Gly Trp Leu Gln
225                 230                 235                 240

Asn Pro Arg Val Ala Pro Gly Asp Val Ala Leu Asp Gln Ala Cys Phe
                245                 250                 255

Arg Ile Ser Gly Ala Ala Arg Asn Ser Ser Ser Phe Ile Ser Gly Ser
            260                 265                 270

Val Ala Arg Ala Val Pro His Leu Gly Tyr Ala Met Ala Ala Gly Arg
        275                 280                 285

Phe Gly Trp Gly Leu Ala His Val Ala Ala Ala Val Ala Met Ser Arg
    290                 295                 300

Arg Tyr Asp Arg Ala Gln Lys Gly Phe Leu Leu Thr Ser Leu Arg Arg
305                 310                 315                 320

Ala Tyr Ala Pro Leu Leu Ala Arg Glu Asn Ala Ala Leu Thr Gly Ala
                325                 330                 335

Arg Thr Pro Asp Asp Gly Gly Asp Ala Asn Arg His Asp Gly Asp Asp
            340                 345                 350

Ala Arg Gly Lys Pro Ala Ala Ala Ala Ala Pro Leu Pro Ser Ala Ala
        355                 360                 365

Ala Ser Pro Ala Asp Glu Arg Ala Val Pro Ala Gly Tyr Gly Ala Ala
    370                 375                 380

Gly Val Leu Ala Ala Leu Gly Arg Leu Ser Ala Ala Pro Ala Ser Ala
385                 390                 395                 400

Pro Ala Gly Ala Asp Asp Asp Asp Asp Asp Asp Gly Ala Gly Gly Gly
                405                 410                 415

Gly Gly Gly Arg Arg Ala Glu Ala Gly Arg Val Ala Val Glu Cys Leu
            420                 425                 430

Ala Ala Cys Arg Gly Ile Leu Glu Ala Leu Ala Glu Gly Phe Asp Gly

435                 440                 445

Asp Leu Ala Ala Val Pro Gly Leu Ala Gly Ala Arg Pro Ala Ala Pro
    450                 455                 460

Pro Arg Pro Gly Pro Ala Gly Ala Ala Ala Pro Pro His Ala Asp Ala
465                 470                 475                 480

Pro Arg Leu Arg Ala Trp Leu Arg Glu Leu Arg Phe Val Arg Asp Ala
                485                 490                 495

Leu Val Leu Met Arg Leu Arg Gly Asp Leu Arg Val Ala Gly Gly Ser
            500                 505                 510

Glu Ala Ala Val Ala Ala Val Arg Ala Val Ser Leu Val Ala Gly Ala
        515                 520                 525

Leu Gly Pro Ala Leu Pro Arg Ser Pro Arg Leu Leu Ser Ser Ala Ala
    530                 535                 540

Ala Ala Ala Ala Asp Leu Leu Phe Gln Asn Gln Ser Leu Arg Pro Leu
545                 550                 555                 560

Leu Ala Asp Thr Val Ala Ala Ala Asp Ser Leu Ala Ala Pro Ala Ser
                565                 570                 575

Ala Pro Arg Glu Ala Arg Lys Arg Lys Ser Pro Ala Pro Ala Arg Ala
            580                 585                 590

Pro Pro Gly Gly Ala Pro Arg Pro Pro Lys Lys Ser Arg Ala Asp Ala
        595                 600                 605

Pro Arg Pro Ala Ala Ala Pro Pro Ala Gly Ala Ala Pro Pro Ala Pro
    610                 615                 620

Pro Thr Pro Pro Pro Arg Pro Pro Arg Pro Ala Ala Leu Thr Arg Arg
625                 630                 635                 640

Pro Ala Glu Gly Pro Asp Pro Gln Gly Gly Trp Arg Arg Gln Pro Pro
                645                 650                 655

Gly Pro Ser His Thr Pro Ala Pro Ser Ala Ala Ala Leu Glu Ala Tyr
            660                 665                 670

Cys Ala Pro Arg Ala Val Ala Glu Leu Thr Asp His Pro Leu Phe Pro
        675                 680                 685

Ala Pro Trp Arg Pro Ala Leu Met Phe Asp Pro Arg Ala Leu Ala Ser
    690                 695                 700

Leu Ala Ala Arg Cys Ala Ala Pro Pro Pro Gly Gly Ala Pro Ala Ala
705                 710                 715                 720

Phe Gly Pro Leu Arg Ala Ser Gly Pro Leu Arg Arg Ala Ala Ala Trp
                725                 730                 735

Met Arg Gln Val Pro Asp Pro Glu Asp Val Arg Val Val Ile Leu Tyr
            740                 745                 750

Ser Pro Leu Pro Gly Glu Asp Leu Ala Ala Gly Arg Ala Gly Gly Gly
        755                 760                 765

Pro Pro Pro Glu Trp Ser Ala Glu Arg Gly Gly Leu Ser Cys Leu Leu
    770                 775                 780

Ala Ala Leu Gly Asn Arg Leu Cys Gly Pro Ala Thr Ala Ala Trp Ala
785                 790                 795                 800

Gly Asn Trp Thr Gly Ala Pro Asp Val Ser Ala Leu Gly Ala Gln Gly
                805                 810                 815

Val Leu Leu Leu Ser Thr Arg Asp Leu Ala Phe Ala Gly Ala Val Glu
            820                 825                 830

Phe Leu Gly Leu Leu Ala Gly Ala Cys Asp Arg Arg Leu Ile Val Val
        835                 840                 845

Asn Ala Val Arg Ala Ala Asp Trp Pro Ala Asp Gly Pro Val Val Ser
    850                 855                 860

Arg Gln His Ala Tyr Leu Ala Cys Glu Val Leu Pro Ala Val Gln Cys
865                 870                 875                 880

Ala Val Arg Trp Pro Ala Ala Arg Asp Leu Arg Arg Thr Val Leu Ala
                885                 890                 895

Ser Gly Arg Val Phe Gly Pro Gly Val Phe Ala Arg Val Glu Ala Ala
            900                 905                 910

His Ala Arg Leu Tyr Pro Asp Ala Pro Pro Leu Arg Leu Cys Arg Gly
        915                 920                 925

Ala Asn Val Arg Tyr Arg Val Arg Thr Arg Phe Gly Pro Asp Thr Leu
    930                 935                 940

Val Pro Met Ser Pro Arg Glu Tyr Arg Arg Ala Val Leu Pro Ala Leu
945                 950                 955                 960

Asp Gly Arg Ala Ala Ala Ser Gly Ala Gly Asp Ala Met Ala Pro Gly
                965                 970                 975

Ala Pro Asp Phe Cys Glu Asp Glu Ala His Ser His Arg Ala Cys Ala
            980                 985                 990

Arg Trp Gly Leu Gly Ala Pro Leu  Arg Pro Val Tyr Val  Ala Leu Gly
        995                 1000                1005

Arg Asp  Ala Val Arg Gly Gly  Pro Ala Glu Leu Arg  Gly Pro Arg
    1010                1015                1020

Arg Glu  Phe Cys Ala Arg Ala  Leu Leu Glu Pro Asp  Gly Asp Ala
    1025                1030                1035

Pro Pro  Leu Val Leu Arg Asp  Asp Ala Asp Ala Gly  Pro Pro Pro
    1040                1045                1050

Gln Ile  Arg Trp Ala Ser Ala  Ala Gly Arg Ala Gly  Thr Val Leu
    1055                1060                1065

Ala Ala  Ala Gly Gly Gly Val  Glu Val Val Gly Thr  Ala Ala Gly
    1070                1075                1080

Leu Ala  Thr Pro Pro Arg Arg  Glu Pro Val Asp Met  Asp Ala Glu
    1085                1090                1095

Leu Glu  Asp Asp Asp Asp Gly  Leu Phe Gly Glu
    1100                1105

<210> 15
<211> 1164
<212> PRT
<213> Artificial Sequence

<220>
<223> Description of Artificial Sequence: Synthetic
      polypeptide

<400> 15
Met Ser Ala Glu Gln Arg Lys Lys Lys Lys Thr Thr Thr Thr Thr Gln
1               5                   10                  15

Gly Arg Gly Ala Glu Val Ala Met Ala Asp Glu Asp Gly Gly Arg Leu
            20                  25                  30

Arg Ala Ala Ala Glu Thr Thr Gly Gly Pro Gly Ser Pro Asp Pro Ala
        35                  40                  45

Asp Gly Pro Pro Pro Thr Pro Asn Pro Asp Arg Arg Pro Ala Ala Arg
    50                  55                  60

Pro Gly Phe Gly Trp His Gly Gly Pro Glu Glu Asn Glu Asp Glu Ala
65                  70                  75                  80

Asp Asp Ala Ala Ala Asp Ala Asp Ala Asp Glu Ala Ala Pro Ala Ser
                85                  90                  95

Gly Glu Ala Val Asp Glu Pro Ala Ala Asp Gly Val Val Ser Pro Arg
            100                 105                 110

Gln Leu Ala Leu Leu Ala Ser Met Val Asp Glu Ala Val Arg Thr Ile
        115                 120                 125

Pro Ser Pro Pro Pro Glu Arg Asp Gly Ala Gln Glu Glu Ala Ala Arg
    130                 135                 140

Ser Pro Ser Pro Pro Arg Thr Pro Ser Met Arg Ala Asp Tyr Gly Glu
145                 150                 155                 160

Glu Asn Asp Asp Asp Asp Asp Asp Asp Asp Asp Asp Asp Arg Asp Ala
                165                 170                 175

Gly Arg Trp Val Arg Gly Pro Glu Thr Thr Ser Ala Val Arg Gly Ala
            180                 185                 190

Tyr Pro Asp Pro Met Ala Ser Leu Ser Pro Arg Pro Pro Ala Pro Arg
        195                 200                 205

Arg His His His His His His His Arg Arg Arg Arg Ala Pro Arg Arg
    210                 215                 220

Arg Ser Ala Ala Ser Asp Ser Ser Lys Ser Gly Ser Ser Ser Ser Ala
225                 230                 235                 240

Ser Ser Ala Ser Ser Ser Ala Ser Ser Ser Ser Ser Ala Ser Ala Ser
                245                 250                 255

Ser Ser Asp Asp Asp Asp Asp Asp Asp Ala Ala Arg Ala Pro Ala Ser
            260                 265                 270

Ala Ala Asp His Ala Ala Gly Gly Thr Leu Gly Ala Asp Asp Glu Glu
        275                 280                 285

Ala Gly Val Pro Ala Arg Ala Pro Gly Ala Ala Pro Arg Pro Ser Pro
    290                 295                 300

Pro Arg Ala Glu Pro Ala Pro Ala Arg Thr Pro Ala Ala Thr Ala Gly
305                 310                 315                 320

Arg Leu Glu Arg Arg Arg Ala Arg Ala Ala Val Ala Gly Arg Asp Ala
                325                 330                 335

Thr Gly Arg Phe Thr Ala Gly Arg Pro Arg Arg Val Glu Leu Asp Ala
            340                 345                 350

Asp Ala Ala Ser Gly Ala Phe Tyr Ala Arg Tyr Arg Asp Gly Tyr Val
        355                 360                 365

Ser Gly Glu Pro Trp Pro Gly Ala Gly Pro Pro Pro Pro Gly Arg Val
    370                 375                 380

Leu Tyr Gly Gly Leu Gly Ala Arg Thr Pro Asp Asp Gly Gly Asp Ala
385                 390                 395                 400

Asn Arg His Asp Gly Asp Asp Ala Arg Gly Lys Pro Ala Ala Ala Ala
                405                 410                 415

Ala Pro Leu Pro Ser Ala Ala Ala Ser Pro Ala Asp Glu Arg Ala Val
            420                 425                 430

Pro Ala Gly Tyr Gly Ala Ala Gly Val Leu Ala Ala Leu Gly Arg Leu
        435                 440                 445

Ser Ala Ala Pro Ala Ser Ala Pro Ala Gly Ala Asp Asp Asp Asp Asp
    450                 455                 460

Asp Asp Gly Ala Gly Gly Gly Gly Gly Gly Arg Arg Ala Glu Ala Gly
465                 470                 475                 480

Arg Val Ala Val Glu Cys Leu Ala Ala Cys Arg Gly Ile Leu Glu Ala
                485                 490                 495

Leu Ala Glu Gly Phe Asp Gly Asp Leu Ala Ala Val Pro Gly Leu Ala
            500                 505                 510

Gly Ala Arg Pro Ala Ala Pro Pro Arg Pro Gly Pro Ala Gly Ala Ala
        515                 520                 525

Ala Pro Pro His Ala Asp Ala Pro Arg Leu Arg Ala Trp Leu Arg Glu
    530                 535                 540

Leu Arg Phe Val Arg Asp Ala Leu Val Leu Met Arg Leu Arg Gly Asp
545                 550                 555                 560

Leu Arg Val Ala Gly Gly Ser Glu Ala Ala Val Ala Ala Val Arg Ala
                565                 570                 575

Val Ser Leu Val Ala Gly Ala Leu Gly Pro Ala Leu Pro Arg Ser Pro
            580                 585                 590

Arg Leu Leu Ser Ser Ala Ala Ala Ala Ala Ala Asp Leu Leu Phe Gln
        595                 600                 605

Asn Gln Ser Leu Arg Pro Leu Leu Ala Asp Thr Val Ala Ala Ala Asp
    610                 615                 620

Ser Leu Ala Ala Pro Ala Ser Ala Pro Arg Glu Ala Arg Lys Arg Lys
625                 630                 635                 640

Ser Pro Ala Pro Ala Arg Ala Pro Pro Gly Gly Ala Pro Arg Pro Pro
                645                 650                 655

Lys Lys Ser Arg Ala Asp Ala Pro Arg Pro Ala Ala Ala Pro Pro Ala
            660                 665                 670

Gly Ala Ala Pro Pro Ala Pro Pro Thr Pro Pro Pro Arg Pro Pro Arg
        675                 680                 685

Pro Ala Ala Leu Thr Arg Arg Pro Ala Glu Gly Pro Asp Pro Gln Gly
    690                 695                 700

Gly Trp Arg Arg Gln Pro Pro Gly Pro Ser His Thr Pro Ala Pro Ser
705                 710                 715                 720

Ala Ala Ala Leu Glu Ala Tyr Cys Ala Pro Arg Ala Val Ala Glu Leu
                725                 730                 735

Thr Asp His Pro Leu Phe Pro Ala Pro Trp Arg Pro Ala Leu Met Phe
            740                 745                 750

Asp Pro Arg Ala Leu Ala Ser Leu Ala Ala Arg Cys Ala Ala Pro Pro
        755                 760                 765

Pro Gly Gly Ala Pro Ala Ala Phe Gly Pro Leu Arg Ala Ser Gly Pro
    770                 775                 780

Leu Arg Arg Ala Ala Ala Trp Met Arg Gln Val Pro Asp Pro Glu Asp

785                 790                 795                 800

Val Arg Val Val Ile Leu Tyr Ser Pro Leu Pro Gly Glu Asp Leu Ala
                805                 810                 815

Ala Gly Arg Ala Gly Gly Gly Pro Pro Pro Glu Trp Ser Ala Glu Arg
            820                 825                 830

Gly Gly Leu Ser Cys Leu Leu Ala Ala Leu Gly Asn Arg Leu Cys Gly
        835                 840                 845

Pro Ala Thr Ala Ala Trp Ala Gly Asn Trp Thr Gly Ala Pro Asp Val
    850                 855                 860

Ser Ala Leu Gly Ala Gln Gly Val Leu Leu Leu Ser Thr Arg Asp Leu
865                 870                 875                 880

Ala Phe Ala Gly Ala Val Glu Phe Leu Gly Leu Leu Ala Gly Ala Cys
                885                 890                 895

Asp Arg Arg Leu Ile Val Val Asn Ala Val Arg Ala Ala Asp Trp Pro
            900                 905                 910

Ala Asp Gly Pro Val Val Ser Arg Gln His Ala Tyr Leu Ala Cys Glu
        915                 920                 925

Val Leu Pro Ala Val Gln Cys Ala Val Arg Trp Pro Ala Ala Arg Asp
    930                 935                 940

Leu Arg Arg Thr Val Leu Ala Ser Gly Arg Val Phe Gly Pro Gly Val
945                 950                 955                 960

Phe Ala Arg Val Glu Ala Ala His Ala Arg Leu Tyr Pro Asp Ala Pro
                965                 970                 975

Pro Leu Arg Leu Cys Arg Gly Ala Asn Val Arg Tyr Arg Val Arg Thr
            980                 985                 990

Arg Phe Gly Pro Asp Thr Leu Val  Pro Met Ser Pro Arg  Glu Tyr Arg
        995                 1000                1005

Arg Ala  Val Leu Pro Ala Leu  Asp Gly Arg Ala Ala  Ala Ser Gly
    1010                1015                1020

Ala Gly  Asp Ala Met Ala Pro  Gly Ala Pro Asp Phe  Cys Glu Asp
    1025                1030                1035

Glu Ala His Ser His Arg Ala Cys Ala Arg Trp Gly Leu Gly Ala
    1040                1045                1050

Pro Leu Arg Pro Val Tyr Val Ala Leu Gly Arg Asp Ala Val Arg
    1055                1060                1065

Gly Gly Pro Ala Glu Leu Arg Gly Pro Arg Arg Glu Phe Cys Ala
    1070                1075                1080

Arg Ala Leu Leu Glu Pro Asp Gly Asp Ala Pro Pro Leu Val Leu
    1085                1090                1095

Arg Asp Asp Ala Asp Ala Gly Pro Pro Pro Gln Ile Arg Trp Ala
    1100                1105                1110

Ser Ala Ala Gly Arg Ala Gly Thr Val Leu Ala Ala Ala Gly Gly
    1115                1120                1125

Gly Val Glu Val Val Gly Thr Ala Ala Gly Leu Ala Thr Pro Pro
    1130                1135                1140

Arg Arg Glu Pro Val Asp Met Asp Ala Glu Leu Glu Asp Asp Asp
    1145                1150                1155

Asp Gly Leu Phe Gly Glu
    1160

<210> 16
<211> 1234
<212> PRT
<213> Artificial Sequence

<220>
<223> Description of Artificial Sequence: Synthetic
      polypeptide

<400> 16
Met Ser Ala Glu Gln Arg Lys Lys Lys Lys Thr Thr Thr Thr Thr Gln
1               5                   10                  15

Gly Arg Gly Ala Glu Val Ala Met Ala Asp Glu Asp Gly Gly Arg Leu
            20                  25                  30

Arg Ala Ala Ala Glu Thr Thr Gly Gly Pro Gly Ser Pro Asp Pro Ala
        35                  40                  45

Asp Gly Pro Pro Pro Thr Pro Asn Pro Asp Arg Arg Pro Ala Ala Arg
    50                  55                  60

Pro Gly Phe Gly Trp His Gly Gly Pro Glu Glu Asn Glu Asp Glu Ala

65                  70                  75                  80

Asp Asp Ala Ala Ala Asp Ala Asp Ala Asp Glu Ala Ala Pro Ala Ser
                85                  90                  95

Gly Glu Ala Val Asp Glu Pro Ala Ala Asp Gly Val Val Ser Pro Arg
            100                 105                 110

Gln Leu Ala Leu Leu Ala Ser Met Val Asp Glu Ala Val Arg Thr Ile
        115                 120                 125

Pro Ser Pro Pro Pro Glu Arg Asp Gly Ala Gln Glu Glu Ala Ala Arg
    130                 135                 140

Ser Pro Ser Pro Pro Arg Thr Pro Ser Met Arg Ala Asp Tyr Gly Glu
145                 150                 155                 160

Glu Asn Asp Asp Asp Asp Asp Asp Asp Asp Asp Asp Asp Arg Asp Ala
                165                 170                 175

Gly Arg Trp Val Arg Gly Pro Glu Thr Thr Ser Ala Val Arg Gly Ala
            180                 185                 190

Tyr Pro Asp Pro Met Ala Ser Leu Ser Pro Arg Pro Pro Ala Pro Arg
        195                 200                 205

Arg His His His His His His His Arg Arg Arg Arg Ala Pro Arg Arg
    210                 215                 220

Arg Ser Ala Ala Ser Asp Ser Ser Lys Ser Gly Ser Ser Ser Ser Ala
225                 230                 235                 240

Ser Ser Ala Ser Ser Ser Ala Ser Ser Ser Ser Ser Ala Ser Ala Ser
                245                 250                 255

Ser Ser Asp Asp Asp Asp Asp Asp Asp Ala Ala Arg Ala Pro Ala Ser
            260                 265                 270

Ala Ala Asp His Ala Ala Gly Gly Thr Leu Gly Ala Asp Asp Glu Glu
        275                 280                 285

Ala Gly Val Pro Ala Arg Ala Pro Gly Ala Ala Pro Arg Pro Ser Pro
    290                 295                 300

Pro Arg Ala Glu Pro Ala Pro Ala Arg Thr Pro Ala Ala Thr Ala Gly
305                 310                 315                 320

Arg Leu Glu Arg Arg Arg Ala Arg Ala Ala Val Ala Gly Arg Asp Ala
                325                 330                 335

Thr Gly Arg Phe Thr Ala Gly Arg Pro Arg Arg Val Glu Leu Asp Ala
            340                 345                 350

Asp Ala Ala Ser Gly Ala Phe Tyr Ala Arg Tyr Arg Asp Gly Tyr Val
        355                 360                 365

Ser Gly Glu Pro Trp Pro Gly Ala Gly Pro Pro Pro Pro Gly Arg Val
    370                 375                 380

Leu Tyr Gly Gly Leu Gly Asp Ser Arg Pro Gly Leu Trp Gly Ala Pro
385                 390                 395                 400

Glu Ala Glu Glu Ala Arg Ala Arg Phe Glu Ala Ser Gly Ala Pro Ala
                405                 410                 415

Pro Val Trp Ala Pro Glu Leu Gly Asp Ala Ala Gln Gln Tyr Ala Leu
            420                 425                 430

Ile Thr Arg Leu Leu Tyr Thr Pro Asp Ala Glu Ala Met Gly Trp Leu
        435                 440                 445

Gln Asn Pro Arg Val Ala Pro Gly Asp Val Ala Leu Asp Gln Ala Cys
    450                 455                 460

Phe Arg Ile Ser Gly Ala Ala Arg Asn Ser Ser Ser Phe Ile Ser Gly
465                 470                 475                 480

Ser Val Ala Arg Ala Val Pro His Leu Gly Tyr Ala Met Ala Ala Gly
                485                 490                 495

Arg Phe Gly Trp Gly Leu Ala His Val Ala Ala Ala Val Ala Met Ser
            500                 505                 510

Arg Arg Tyr Asp Arg Ala Gln Lys Gly Phe Leu Leu Thr Ser Leu Arg
        515                 520                 525

Arg Ala Tyr Ala Pro Leu Leu Ala Arg Glu Asn Ala Ala Leu Thr Gly
    530                 535                 540

Ala Arg Thr Pro Asp Asp Gly Gly Asp Ala Asn Arg His Asp Gly Asp
545                 550                 555                 560

Asp Ala Arg Gly Lys Pro Ala Ala Ala Ala Ala Pro Leu Pro Ser Ala
                565                 570                 575

Ala Ala Ser Pro Ala Asp Glu Arg Ala Val Pro Ala Gly Tyr Gly Ala
            580                 585                 590

Ala Gly Val Leu Ala Ala Leu Gly Arg Leu Ser Ala Ala Pro Ala Ser
        595                 600                 605

Ala Pro Ala Gly Ala Asp Asp Asp Asp Asp Asp Asp Gly Ala Gly Gly
    610                 615                 620

Gly Gly Gly Gly Arg Arg Ala Glu Ala Gly Arg Val Ala Val Glu Cys
625                 630                 635                 640

Leu Ala Ala Cys Arg Gly Ile Leu Glu Ala Leu Ala Glu Gly Phe Asp
                645                 650                 655

Gly Asp Leu Ala Ala Val Pro Gly Leu Ala Gly Ala Arg Pro Ala Ala
            660                 665                 670

Pro Pro Arg Pro Gly Pro Ala Gly Ala Ala Ala Pro Pro His Ala Asp
        675                 680                 685

Ala Pro Arg Leu Arg Ala Trp Leu Arg Glu Leu Arg Phe Val Arg Asp
    690                 695                 700

Ala Leu Val Leu Met Arg Leu Arg Gly Asp Leu Arg Val Ala Gly Gly
705                 710                 715                 720

Ser Glu Ala Ala Val Ala Ala Val Arg Ala Val Ser Leu Val Ala Gly
                725                 730                 735

Ala Leu Gly Pro Ala Leu Pro Arg Ser Pro Arg Leu Leu Ser Ser Ala
            740                 745                 750

Ala Ala Ala Ala Ala Asp Leu Leu Phe Gln Asn Gln Ser Leu Arg Pro
        755                 760                 765

Leu Leu Ala Asp Thr Val Ala Ala Ala Asp Ser Leu Ala Ala Pro Ala
    770                 775                 780

Ser Thr Pro Ala Pro Ser Ala Ala Ala Leu Glu Ala Tyr Cys Ala Pro
785                 790                 795                 800

Arg Ala Val Ala Glu Leu Thr Asp His Pro Leu Phe Pro Ala Pro Trp
                805                 810                 815

Arg Pro Ala Leu Met Phe Asp Pro Arg Ala Leu Ala Ser Leu Ala Ala
            820                 825                 830

Arg Cys Ala Ala Pro Pro Pro Gly Gly Ala Pro Ala Ala Phe Gly Pro
        835                 840                 845

Leu Arg Ala Ser Gly Pro Leu Arg Arg Ala Ala Ala Trp Met Arg Gln
    850                 855                 860

Val Pro Asp Pro Glu Asp Val Arg Val Val Ile Leu Tyr Ser Pro Leu
865                 870                 875                 880

Pro Gly Glu Asp Leu Ala Ala Gly Arg Ala Gly Gly Gly Pro Pro Pro
                885                 890                 895

Glu Trp Ser Ala Glu Arg Gly Gly Leu Ser Cys Leu Leu Ala Ala Leu
            900                 905                 910

Gly Asn Arg Leu Cys Gly Pro Ala Thr Ala Ala Trp Ala Gly Asn Trp
        915                 920                 925

Thr Gly Ala Pro Asp Val Ser Ala Leu Gly Ala Gln Gly Val Leu Leu
    930                 935                 940

Leu Ser Thr Arg Asp Leu Ala Phe Ala Gly Ala Val Glu Phe Leu Gly
945                 950                 955                 960

Leu Leu Ala Gly Ala Cys Asp Arg Arg Leu Ile Val Val Asn Ala Val
                965                 970                 975

Arg Ala Ala Asp Trp Pro Ala Asp Gly Pro Val Val Ser Arg Gln His
            980                 985                 990

Ala Tyr Leu Ala Cys Glu Val Leu Pro Ala Val Gln Cys Ala Val Arg
        995                 1000                1005

Trp Pro Ala Ala Arg Asp Leu Arg Arg Thr Val Leu Ala Ser Gly
    1010                1015                1020

Arg Val Phe Gly Pro Gly Val Phe Ala Arg Val Glu Ala Ala His
    1025                1030                1035

Ala Arg Leu Tyr Pro Asp Ala Pro Pro Leu Arg Leu Cys Arg Gly
    1040                1045                1050

Ala Asn Val Arg Tyr Arg Val Arg Thr Arg Phe Gly Pro Asp Thr
    1055                1060                1065

Leu Val Pro Met Ser Pro Arg Glu Tyr Arg Arg Ala Val Leu Pro

1070                1075                1080

Ala Leu Asp Gly Arg Ala Ala Ala Ser Gly Ala Gly Asp Ala Met
    1085                1090                1095

Ala Pro Gly Ala Pro Asp Phe Cys Glu Asp Glu Ala His Ser His
    1100                1105                1110

Arg Ala Cys Ala Arg Trp Gly Leu Gly Ala Pro Leu Arg Pro Val
    1115                1120                1125

Tyr Val Ala Leu Gly Arg Asp Ala Val Arg Gly Gly Pro Ala Glu
    1130                1135                1140

Leu Arg Gly Pro Arg Arg Glu Phe Cys Ala Arg Ala Leu Leu Glu
    1145                1150                1155

Pro Asp Gly Asp Ala Pro Pro Leu Val Leu Arg Asp Asp Ala Asp
    1160                1165                1170

Ala Gly Pro Pro Pro Gln Ile Arg Trp Ala Ser Ala Ala Gly Arg
    1175                1180                1185

Ala Gly Thr Val Leu Ala Ala Ala Gly Gly Gly Val Glu Val Val
    1190                1195                1200

Gly Thr Ala Ala Gly Leu Ala Thr Pro Pro Arg Arg Glu Pro Val
    1205                1210                1215

Asp Met Asp Ala Glu Leu Glu Asp Asp Asp Asp Gly Leu Phe Gly
    1220                1225                1230

Glu

<210> 17
<211> 255
<212> PRT
<213> Artificial Sequence

<220>
<223> Description of Artificial Sequence: Synthetic
      polypeptide

<400> 17
Met Phe Ser Ala Ser Thr Thr Pro Glu Gln Pro Leu Gly Leu Ser Gly
1               5                   10                  15

Asp Ala Thr Pro Pro Leu Pro Thr Ser Val Pro Leu Asp Trp Ala Ala
            20                  25                  30

Phe Arg Arg Ala Phe Leu Ile Asp Asp Ala Trp Arg Pro Leu Leu Glu
        35                  40                  45

Pro Glu Leu Ala Asn Pro Leu Thr Ala Arg Leu Leu Ala Glu Tyr Asp
    50                  55                  60

Arg Arg Cys Gln Thr Glu Glu Val Leu Pro Pro Arg Glu Asp Val Phe
65                  70                  75                  80

Ser Trp Thr Arg Tyr Cys Thr Pro Asp Asp Val Arg Val Val Ile Ile
                85                  90                  95

Gly Gln Asp Pro Tyr His His Pro Gly Gln Ala His Gly Leu Ala Phe
            100                 105                 110

Ser Val Arg Ala Asp Val Pro Val Pro Pro Ser Leu Arg Asn Val Leu
        115                 120                 125

Ala Ala Val Lys Asn Cys Tyr Pro Asp Ala Arg Met Ser Gly Arg Gly
    130                 135                 140

Cys Leu Glu Lys Trp Ala Arg Asp Gly Val Leu Leu Leu Asn Thr Thr
145                 150                 155                 160

Leu Thr Val Lys Arg Gly Ala Ala Ala Ser His Ser Lys Leu Gly Trp
                165                 170                 175

Asp Arg Phe Val Gly Gly Val Val Gln Arg Leu Ala Ala Arg Arg Pro
            180                 185                 190

Gly Leu Val Phe Met Leu Trp Gly Ala His Ala Gln Asn Ala Ile Arg
        195                 200                 205

Pro Asp Pro Arg Gln His Tyr Val Leu Lys Phe Ser His Pro Ser Pro
    210                 215                 220

Leu Ser Lys Val Pro Phe Gly Thr Cys Gln His Phe Leu Ala Ala Asn
225                 230                 235                 240

Arg Tyr Leu Glu Thr Arg Asp Ile Met Pro Ile Asp Trp Ser Val
                245                 250                 255

<210> 18
<211> 96
<212> PRT
<213> Artificial Sequence

<220>
<223> Description of Artificial Sequence: Synthetic
      polypeptide

<400> 18
Met Gly Leu Ala Phe Ser Gly Ala Arg Pro Cys Cys Cys Arg His Asn
1               5                   10                  15

Val Ile Thr Thr Asp Gly Gly Glu Val Val Ser Leu Thr Ala His Glu
            20                  25                  30

Phe Asp Val Val Asp Ile Glu Ser Glu Glu Glu Gly Asn Phe Tyr Val
        35                  40                  45

Pro Pro Asp Val Arg Val Val Thr Arg Ala Pro Gly Pro Gln Tyr Arg
    50                  55                  60

Arg Ala Ser Asp Pro Pro Ser Arg His Thr Arg Arg Arg Asp Pro Asp
65                  70                  75                  80

Val Ala Arg Pro Pro Ala Thr Leu Thr Pro Pro Leu Ser Asp Ser Glu
                85                  90                  95

<210> 19
<211> 226
<212> PRT
<213> Human herpesvirus 2

<400> 19
Asn Arg Trp Gly Phe Val Cys Leu Phe Gly Leu Val Val Met Gly Ala
1               5                   10                  15

Trp Gly Ala Trp Gly Gly Ser Gln Ala Thr Glu Tyr Val Leu Arg Ser
            20                  25                  30

Val Ile Ala Lys Glu Val Gly Asp Ile Leu Arg Val Pro Cys Met Arg
        35                  40                  45

Thr Pro Ala Asp Asp Val Ser Trp Arg Tyr Glu Ala Pro Ser Val Ile
    50                  55                  60

Asp Tyr Ala Arg Ile Asp Gly Ile Phe Leu Arg Tyr His Cys Pro Gly
65                  70                  75                  80

Leu Asp Thr Phe Leu Trp Asp Arg His Ala Gln Arg Ala Tyr Leu Val
                85                  90                  95

Asn Pro Phe Leu Phe Ala Ala Gly Phe Leu Glu Asp Leu Ser His Ser
            100                 105                 110

Val Phe Pro Ala Asp Thr Gln Glu Thr Thr Thr Arg Arg Ala Leu Tyr
        115                 120                 125

Lys Glu Ile Arg Asp Ala Leu Gly Ser Arg Lys Gln Ala Val Ser His
    130                 135                 140

Ala Pro Val Arg Ala Gly Cys Val Asn Phe Asp Tyr Ser Arg Thr Arg
145                 150                 155                 160

Arg Cys Val Gly Arg Arg Asp Leu Arg Pro Ala Asn Thr Thr Ser Thr
                165                 170                 175

Trp Glu Pro Pro Val Ser Ser Asp Asp Glu Ala Ser Ser Gln Ser Lys
            180                 185                 190

Pro Leu Ala Thr Gln Pro Pro Val Leu Ala Leu Ser Asn Ala Pro Pro
        195                 200                 205

Arg Arg Val Ser Pro Thr Arg Gly Arg Arg Arg His Thr Arg Leu Arg
    210                 215                 220

Arg Asn
225

<210> 20
<211> 1388
<212> PRT
<213> Artificial Sequence

<220>
<223> Description of Artificial Sequence: Synthetic
      polypeptide

<400> 20
Asp Tyr Asp Ile Pro Thr Thr Glu Asn Leu Tyr Phe Gln Gly Met Ala
1               5                   10                  15

Ala Pro Ala Arg Asp Pro Pro Gly Tyr Arg Tyr Ala Ala Ala Met Val
            20                  25                  30

Pro Thr Gly Ser Ile Leu Ser Thr Ile Glu Val Ala Ser His Arg Arg
        35                  40                  45

Leu Phe Asp Phe Phe Ala Arg Val Arg Ser Asp Glu Asn Ser Leu Tyr
    50                  55                  60

Asp Val Glu Phe Asp Ala Leu Leu Gly Ser Tyr Cys Asn Thr Leu Ser
65                  70                  75                  80

Leu Val Arg Phe Leu Glu Leu Gly Leu Ser Val Ala Cys Val Cys Thr

85                  90                  95

Lys Phe Pro Glu Leu Ala Tyr Met Asn Glu Gly Arg Val Gln Phe Glu
            100                 105                 110

Val His Gln Pro Leu Ile Ala Arg Asp Gly Pro His Pro Val Glu Gln
        115                 120                 125

Pro Val His Asn Tyr Met Thr Lys Val Ile Asp Arg Arg Ala Leu Asn
    130                 135                 140

Ala Ala Phe Ser Leu Ala Thr Glu Ala Ile Ala Leu Leu Thr Gly Glu
145                 150                 155                 160

Ala Leu Asp Gly Thr Gly Ile Ser Leu His Arg Gln Leu Arg Ala Ile
                165                 170                 175

Gln Gln Leu Ala Arg Asn Val Gln Ala Val Leu Gly Ala Phe Glu Arg
            180                 185                 190

Gly Thr Ala Asp Gln Met Leu His Val Leu Leu Glu Lys Ala Pro Pro
        195                 200                 205

Leu Ala Leu Leu Leu Pro Met Gln Arg Tyr Leu Asp Asn Gly Arg Leu
    210                 215                 220

Ala Thr Arg Val Ala Arg Ala Thr Leu Val Ala Glu Leu Lys Arg Ser
225                 230                 235                 240

Phe Cys Asp Thr Ser Phe Phe Leu Gly Lys Ala Gly His Arg Arg Glu
                245                 250                 255

Ala Ile Glu Ala Trp Leu Val Asp Leu Thr Thr Ala Thr Gln Pro Ser
            260                 265                 270

Val Ala Val Pro Arg Leu Thr His Ala Asp Thr Arg Gly Arg Pro Val
        275                 280                 285

Asp Gly Val Leu Val Thr Thr Ala Ala Ile Lys Gln Arg Leu Leu Gln
    290                 295                 300

Ser Phe Leu Lys Val Glu Asp Thr Glu Ala Asp Val Pro Val Thr Tyr
305                 310                 315                 320

Gly Glu Met Val Leu Asn Gly Ala Asn Leu Val Thr Ala Leu Val Met
                325                 330                 335

Gly Lys Ala Val Arg Ser Leu Asp Asp Val Gly Arg His Leu Leu Glu
        340                 345                 350

Met Gln Glu Glu Gln Leu Glu Ala Asn Arg Glu Thr Leu Asp Glu Leu
    355                 360                 365

Glu Ser Ala Pro Gln Thr Thr Arg Val Arg Ala Asp Leu Val Ala Ile
    370                 375                 380

Gly Asp Arg Leu Val Phe Leu Glu Ala Leu Glu Lys Arg Ile Tyr Ala
385                 390                 395                 400

Ala Thr Asn Val Pro Tyr Pro Leu Val Gly Ala Met Asp Leu Thr Phe
                405                 410                 415

Val Leu Pro Leu Gly Leu Phe Asn Pro Ala Met Glu Arg Phe Ala Ala
        420                 425                 430

His Ala Gly Asp Leu Val Pro Ala Pro Gly His Pro Glu Pro Arg Ala
    435                 440                 445

Phe Pro Pro Arg Gln Leu Phe Phe Trp Gly Lys Asp His Gln Val Leu
    450                 455                 460

Arg Leu Ser Met Glu Asn Ala Val Gly Thr Val Cys His Pro Ser Leu
465                 470                 475                 480

Met Asn Ile Asp Ala Ala Val Gly Gly Val Asn His Asp Pro Val Glu
                485                 490                 495

Ala Ala Asn Pro Tyr Gly Ala Tyr Val Ala Ala Pro Ala Gly Pro Gly
        500                 505                 510

Ala Asp Met Gln Gln Arg Phe Leu Asn Ala Trp Arg Gln Arg Leu Ala
    515                 520                 525

His Gly Arg Val Arg Trp Val Ala Glu Cys Gln Met Thr Ala Glu Gln
    530                 535                 540

Phe Met Gln Pro Asp Asn Ala Asn Leu Ala Leu Glu Leu His Pro Ala
545                 550                 555                 560

Phe Asp Phe Phe Ala Gly Val Ala Asp Val Glu Leu Pro Gly Gly Glu
                565                 570                 575

Val Pro Pro Ala Gly Pro Gly Ala Ile Gln Ala Thr Trp Arg Val Val
        580                 585                 590

Asn Gly Asn Leu Pro Leu Ala Leu Cys Pro Val Ala Phe Arg Asp Ala
        595                 600                 605

Arg Gly Leu Glu Leu Gly Val Gly Arg His Ala Met Ala Pro Ala Thr
    610                 615                 620

Ile Ala Ala Val Arg Gly Ala Phe Glu Asp Arg Ser Tyr Pro Ala Val
625                 630                 635                 640

Phe Tyr Leu Leu Gln Ala Ala Ile His Gly Ser Glu His Val Phe Cys
                645                 650                 655

Ala Leu Ala Arg Leu Val Thr Gln Cys Ile Thr Ser Tyr Trp Asn Asn
            660                 665                 670

Thr Arg Cys Ala Ala Phe Val Asn Asp Tyr Ser Leu Val Ser Tyr Ile
        675                 680                 685

Val Thr Tyr Leu Gly Gly Asp Leu Pro Glu Glu Cys Met Ala Val Tyr
    690                 695                 700

Arg Asp Leu Val Ala His Val Glu Ala Leu Ala Gln Leu Val Asp Asp
705                 710                 715                 720

Phe Thr Leu Pro Gly Pro Glu Leu Gly Gly Gln Ala Gln Ala Glu Leu
                725                 730                 735

Asn His Leu Met Arg Asp Pro Ala Leu Leu Pro Pro Leu Val Trp Asp
            740                 745                 750

Cys Asp Gly Leu Met Arg His Ala Ala Leu Asp Arg His Arg Asp Cys
        755                 760                 765

Arg Ile Asp Ala Gly Glu His Glu Pro Val Tyr Ala Ala Ala Cys Asn
    770                 775                 780

Val Ala Thr Ala Asp Phe Asn Arg Asn Asp Gly Arg Leu Leu His Asn
785                 790                 795                 800

Thr Gln Ala Arg Ala Ala Asp Ala Ala Asp Asp Arg Pro His Arg Pro
                805                 810                 815

Ala Asp Trp Thr Val His His Lys Ile Tyr Tyr Tyr Val Leu Val Pro
            820                 825                 830

Ala Phe Ser Arg Gly Arg Cys Cys Thr Ala Gly Val Arg Phe Asp Arg
        835                 840                 845

Val Tyr Ala Thr Leu Gln Asn Met Val Val Pro Glu Ile Ala Pro Gly
    850                 855                 860

Glu Glu Cys Pro Ser Asp Pro Val Thr Asp Pro Ala His Pro Leu His
865                 870                 875                 880

Pro Ala Asn Leu Val Ala Asn Thr Val Asn Ala Met Phe His Asn Gly
                885                 890                 895

Arg Val Val Val Asp Gly Pro Ala Met Leu Thr Leu Gln Val Leu Ala
            900                 905                 910

His Asn Met Ala Glu Arg Thr Thr Ala Leu Leu Cys Ser Ala Ala Pro
        915                 920                 925

Asp Ala Gly Ala Asn Thr Ala Ser Thr Ala Asn Met Arg Ile Phe Asp
    930                 935                 940

Gly Ala Leu His Ala Gly Val Leu Leu Met Ala Pro Gln His Leu Asp
945                 950                 955                 960

His Thr Ile Gln Asn Gly Glu Tyr Phe Tyr Val Leu Pro Val His Ala
                965                 970                 975

Leu Phe Ala Gly Ala Asp His Val Ala Asn Ala Pro Asn Phe Pro Pro
            980                 985                 990

Ala Leu Arg Asp Leu Ala Arg His Val Pro Leu Val Pro Pro Ala Leu
        995                 1000                1005

Gly Ala Asn Tyr Phe Ser Ser Ile Arg Gln Pro Val Val Gln His
    1010                1015                1020

Ala Arg Glu Ser Ala Ala Gly Glu Asn Ala Leu Thr Tyr Ala Leu
    1025                1030                1035

Met Ala Gly Tyr Phe Lys Met Ser Pro Val Ala Leu Tyr His Gln
    1040                1045                1050

Leu Lys Thr Gly Leu His Pro Gly Phe Gly Phe Thr Val Val Arg
    1055                1060                1065

Gln Asp Arg Phe Val Thr Glu Asn Val Leu Phe Ser Glu Arg Ala
    1070                1075                1080

Ser Glu Ala Tyr Phe Leu Gly Gln Leu Gln Val Ala Arg His Glu

1085                1090                1095

Thr Gly Gly Gly Val Ser Phe Thr Leu Thr Gln Pro Arg Gly Asn
    1100                1105                1110

Val Asp Leu Gly Val Gly Tyr Thr Ala Val Ala Ala Thr Ala Thr
    1115                1120                1125

Val Arg Asn Pro Val Thr Asp Met Gly Asn Leu Pro Gln Asn Phe
    1130                1135                1140

Tyr Leu Gly Arg Gly Ala Pro Pro Leu Leu Asp Asn Ala Ala Ala
    1145                1150                1155

Val Tyr Leu Arg Asn Ala Val Val Ala Gly Asn Arg Leu Gly Pro
    1160                1165                1170

Ala Gln Pro Leu Pro Val Phe Gly Cys Ala Gln Val Pro Arg Arg
    1175                1180                1185

Ala Gly Met Asp His Gly Gln Asp Ala Val Cys Glu Phe Ile Ala
    1190                1195                1200

Thr Pro Val Ala Thr Asp Ile Asn Tyr Phe Arg Arg Pro Cys Asn
    1205                1210                1215

Pro Arg Gly Arg Ala Ala Gly Gly Val Tyr Ala Gly Asp Lys Glu
    1220                1225                1230

Gly Asp Val Ile Ala Leu Met Tyr Asp His Gly Gln Ser Asp Pro
    1235                1240                1245

Ala Arg Pro Phe Ala Ala Thr Ala Asn Pro Trp Ala Ser Gln Arg
    1250                1255                1260

Phe Ser Tyr Gly Asp Leu Leu Tyr Asn Gly Ala Tyr His Leu Asn
    1265                1270                1275

Gly Ala Ser Pro Val Leu Ser Pro Cys Phe Lys Phe Phe Thr Ala
    1280                1285                1290

Ala Asp Ile Thr Ala Lys His Arg Cys Leu Glu Arg Leu Ile Val
    1295                1300                1305

Glu Thr Gly Ser Ala Val Ser Thr Ala Thr Ala Ala Ser Asp Val
    1310                1315                1320

Gln Phe Lys Arg Pro Pro Gly Cys Arg Glu Leu Val Glu Asp Pro
    1325                1330                1335

Cys Gly Leu Phe Gln Glu Ala Tyr Pro Ile Thr Cys Ala Ser Asp
    1340                1345                1350

Pro Ala Leu Leu Arg Ser Ala Arg Asp Gly Glu Ala His Ala Arg
    1355                1360                1365

Glu Thr His Phe Thr Gln Tyr Leu Ile Tyr Asp Ala Ser Pro Leu
    1370                1375                1380

Lys Gly Leu Ser Leu
    1385

<210> 21
<211> 1374
<212> PRT
<213> Artificial Sequence

<220>
<223> Description of Artificial Sequence: Synthetic
polypeptide

<400> 21
Met Ala Ala Pro Ala Arg Asp Pro Pro Gly Tyr Arg Tyr Ala Ala Ala
1               5                   10                  15

Met Val Pro Thr Gly Ser Ile Leu Ser Thr Ile Glu Val Ala Ser His
            20                  25                  30

Arg Arg Leu Phe Asp Phe Phe Ala Arg Val Arg Ser Asp Glu Asn Ser
        35                  40                  45

Leu Tyr Asp Val Glu Phe Asp Ala Leu Leu Gly Ser Tyr Cys Asn Thr
    50                  55                  60

Leu Ser Leu Val Arg Phe Leu Glu Leu Gly Leu Ser Val Ala Cys Val
65                  70                  75                  80

Cys Thr Lys Phe Pro Glu Leu Ala Tyr Met Asn Glu Gly Arg Val Gln
                85                  90                  95

Phe Glu Val His Gln Pro Leu Ile Ala Arg Asp Gly Pro His Pro Val
            100                 105                 110

Glu Gln Pro Val His Asn Tyr Met Thr Lys Val Ile Asp Arg Arg Ala
        115                 120                 125

Leu Asn Ala Ala Phe Ser Leu Ala Thr Glu Ala Ile Ala Leu Leu Thr

130                 135                 140

Gly Glu Ala Leu Asp Gly Thr Gly Ile Ser Leu His Arg Gln Leu Arg
145                 150                 155                 160

Ala Ile Gln Gln Leu Ala Arg Asn Val Gln Ala Val Leu Gly Ala Phe
                165                 170                 175

Glu Arg Gly Thr Ala Asp Gln Met Leu His Val Leu Leu Glu Lys Ala
            180                 185                 190

Pro Pro Leu Ala Leu Leu Leu Pro Met Gln Arg Tyr Leu Asp Asn Gly
        195                 200                 205

Arg Leu Ala Thr Arg Val Ala Arg Ala Thr Leu Val Ala Glu Leu Lys
    210                 215                 220

Arg Ser Phe Cys Asp Thr Ser Phe Phe Leu Gly Lys Ala Gly His Arg
225                 230                 235                 240

Arg Glu Ala Ile Glu Ala Trp Leu Val Asp Leu Thr Thr Ala Thr Gln
                245                 250                 255

Pro Ser Val Ala Val Pro Arg Leu Thr His Ala Asp Thr Arg Gly Arg
            260                 265                 270

Pro Val Asp Gly Val Leu Val Thr Thr Ala Ala Ile Lys Gln Arg Leu
        275                 280                 285

Leu Gln Ser Phe Leu Lys Val Glu Asp Thr Glu Ala Asp Val Pro Val
    290                 295                 300

Thr Tyr Gly Glu Met Val Leu Asn Gly Ala Asn Leu Val Thr Ala Leu
305                 310                 315                 320

Val Met Gly Lys Ala Val Arg Ser Leu Asp Asp Val Gly Arg His Leu
                325                 330                 335

Leu Glu Met Gln Glu Glu Gln Leu Glu Ala Asn Arg Glu Thr Leu Asp
            340                 345                 350

Glu Leu Glu Ser Ala Pro Gln Thr Thr Arg Val Arg Ala Asp Leu Val
        355                 360                 365

Ala Ile Gly Asp Arg Leu Val Phe Leu Glu Ala Leu Glu Lys Arg Ile
    370                 375                 380

Tyr Ala Ala Thr Asn Val Pro Tyr Pro Leu Val Gly Ala Met Asp Leu
385                 390                 395                 400

Thr Phe Val Leu Pro Leu Gly Leu Phe Asn Pro Ala Met Glu Arg Phe
                405                 410                 415

Ala Ala His Ala Gly Asp Leu Val Pro Ala Pro Gly His Pro Glu Pro
            420                 425                 430

Arg Ala Phe Pro Pro Arg Gln Leu Phe Phe Trp Gly Lys Asp His Gln
        435                 440                 445

Val Leu Arg Leu Ser Met Glu Asn Ala Val Gly Thr Val Cys His Pro
    450                 455                 460

Ser Leu Met Asn Ile Asp Ala Ala Val Gly Gly Val Asn His Asp Pro
465                 470                 475                 480

Val Glu Ala Ala Asn Pro Tyr Gly Ala Tyr Val Ala Ala Pro Ala Gly
                485                 490                 495

Pro Gly Ala Asp Met Gln Gln Arg Phe Leu Asn Ala Trp Arg Gln Arg
            500                 505                 510

Leu Ala His Gly Arg Val Arg Trp Val Ala Glu Cys Gln Met Thr Ala
        515                 520                 525

Glu Gln Phe Met Gln Pro Asp Asn Ala Asn Leu Ala Leu Glu Leu His
    530                 535                 540

Pro Ala Phe Asp Phe Phe Ala Gly Val Ala Asp Val Glu Leu Pro Gly
545                 550                 555                 560

Gly Glu Val Pro Pro Ala Gly Pro Gly Ala Ile Gln Ala Thr Trp Arg
                565                 570                 575

Val Val Asn Gly Asn Leu Pro Leu Ala Leu Cys Pro Val Ala Phe Arg
            580                 585                 590

Asp Ala Arg Gly Leu Glu Leu Gly Val Gly Arg His Ala Met Ala Pro
        595                 600                 605

Ala Thr Ile Ala Ala Val Arg Gly Ala Phe Glu Asp Arg Ser Tyr Pro
    610                 615                 620

Ala Val Phe Tyr Leu Leu Gln Ala Ala Ile His Gly Ser Glu His Val
625                 630                 635                 640

Phe Cys Ala Leu Ala Arg Leu Val Thr Gln Cys Ile Thr Ser Tyr Trp
                645                 650                 655

Asn Asn Thr Arg Cys Ala Ala Phe Val Asn Asp Tyr Ser Leu Val Ser
            660                 665                 670

Tyr Ile Val Thr Tyr Leu Gly Gly Asp Leu Pro Glu Glu Cys Met Ala
        675                 680                 685

Val Tyr Arg Asp Leu Val Ala His Val Glu Ala Leu Ala Gln Leu Val
    690                 695                 700

Asp Asp Phe Thr Leu Pro Gly Pro Glu Leu Gly Gly Gln Ala Gln Ala
705                 710                 715                 720

Glu Leu Asn His Leu Met Arg Asp Pro Ala Leu Leu Pro Pro Leu Val
                725                 730                 735

Trp Asp Cys Asp Gly Leu Met Arg His Ala Ala Leu Asp Arg His Arg
            740                 745                 750

Asp Cys Arg Ile Asp Ala Gly Glu His Glu Pro Val Tyr Ala Ala Ala
        755                 760                 765

Cys Asn Val Ala Thr Ala Asp Phe Asn Arg Asn Asp Gly Arg Leu Leu
    770                 775                 780

His Asn Thr Gln Ala Arg Ala Ala Asp Ala Ala Asp Asp Arg Pro His
785                 790                 795                 800

Arg Pro Ala Asp Trp Thr Val His His Lys Ile Tyr Tyr Tyr Val Leu
                805                 810                 815

Val Pro Ala Phe Ser Arg Gly Arg Cys Cys Thr Ala Gly Val Arg Phe
            820                 825                 830

Asp Arg Val Tyr Ala Thr Leu Gln Asn Met Val Val Pro Glu Ile Ala
        835                 840                 845

Pro Gly Glu Glu Cys Pro Ser Asp Pro Val Thr Asp Pro Ala His Pro
    850                 855                 860

Leu His Pro Ala Asn Leu Val Ala Asn Thr Val Asn Ala Met Phe His
865                 870                 875                 880

Asn Gly Arg Val Val Val Asp Gly Pro Ala Met Leu Thr Leu Gln Val
                885                 890                 895

Leu Ala His Asn Met Ala Glu Arg Thr Thr Ala Leu Leu Cys Ser Ala
            900                 905                 910

Ala Pro Asp Ala Gly Ala Asn Thr Ala Ser Thr Ala Asn Met Arg Ile
        915                 920                 925

Phe Asp Gly Ala Leu His Ala Gly Val Leu Leu Met Ala Pro Gln His
    930                 935                 940

Leu Asp His Thr Ile Gln Asn Gly Glu Tyr Phe Tyr Val Leu Pro Val
945                 950                 955                 960

His Ala Leu Phe Ala Gly Ala Asp His Val Ala Asn Ala Pro Asn Phe
                965                 970                 975

Pro Pro Ala Leu Arg Asp Leu Ala Arg His Val Pro Leu Val Pro Pro
            980                 985                 990

Ala Leu Gly Ala Asn Tyr Phe Ser Ser Ile Arg Gln Pro Val Val Gln
        995                 1000                1005

His Ala Arg Glu Ser Ala Ala Gly Glu Asn Ala Leu Thr Tyr Ala
    1010                1015                1020

Leu Met Ala Gly Tyr Phe Lys Met Ser Pro Val Ala Leu Tyr His
    1025                1030                1035

Gln Leu Lys Thr Gly Leu His Pro Gly Phe Gly Phe Thr Val Val
    1040                1045                1050

Arg Gln Asp Arg Phe Val Thr Glu Asn Val Leu Phe Ser Glu Arg
    1055                1060                1065

Ala Ser Glu Ala Tyr Phe Leu Gly Gln Leu Gln Val Ala Arg His
    1070                1075                1080

Glu Thr Gly Gly Gly Val Ser Phe Thr Leu Thr Gln Pro Arg Gly
    1085                1090                1095

Asn Val Asp Leu Gly Val Gly Tyr Thr Ala Val Ala Ala Thr Ala
    1100                1105                1110

Thr Val Arg Asn Pro Val Thr Asp Met Gly Asn Leu Pro Gln Asn
    1115                1120                1125

Phe Tyr Leu Gly Arg Gly Ala Pro Pro Leu Leu Asp Asn Ala Ala

1130                1135                1140

Ala Val Tyr Leu Arg Asn Ala Val Val Ala Gly Asn Arg Leu Gly
    1145                1150                1155

Pro Ala Gln Pro Leu Pro Val Phe Gly Cys Ala Gln Val Pro Arg
    1160                1165                1170

Arg Ala Gly Met Asp His Gly Gln Asp Ala Val Cys Glu Phe Ile
    1175                1180                1185

Ala Thr Pro Val Ala Thr Asp Ile Asn Tyr Phe Arg Arg Pro Cys
    1190                1195                1200

Asn Pro Arg Gly Arg Ala Ala Gly Gly Val Tyr Ala Gly Asp Lys
    1205                1210                1215

Glu Gly Asp Val Ile Ala Leu Met Tyr Asp His Gly Gln Ser Asp
    1220                1225                1230

Pro Ala Arg Pro Phe Ala Ala Thr Ala Asn Pro Trp Ala Ser Gln
    1235                1240                1245

Arg Phe Ser Tyr Gly Asp Leu Leu Tyr Asn Gly Ala Tyr His Leu
    1250                1255                1260

Asn Gly Ala Ser Pro Val Leu Ser Pro Cys Phe Lys Phe Phe Thr
    1265                1270                1275

Ala Ala Asp Ile Thr Ala Lys His Arg Cys Leu Glu Arg Leu Ile
    1280                1285                1290

Val Glu Thr Gly Ser Ala Val Ser Thr Ala Thr Ala Ala Ser Asp
    1295                1300                1305

Val Gln Phe Lys Arg Pro Pro Gly Cys Arg Glu Leu Val Glu Asp
    1310                1315                1320

Pro Cys Gly Leu Phe Gln Glu Ala Tyr Pro Ile Thr Cys Ala Ser
    1325                1330                1335

Asp Pro Ala Leu Leu Arg Ser Ala Arg Asp Gly Glu Ala His Ala
    1340                1345                1350

Arg Glu Thr His Phe Thr Gln Tyr Leu Ile Tyr Asp Ala Ser Pro
    1355                1360                1365

Leu Lys  Gly Leu Ser Leu
    1370

<210> 22
<211> 3122
<212> PRT
<213> Artificial Sequence

<220>
<223> Description of Artificial Sequence: Synthetic
      polypeptide

<400> 22
Met Ile Pro Ala Ala Leu Pro His Pro Thr Met Lys Arg Gln Gly Asp
1               5                   10                  15

Arg Asp Ile Val Val Thr Gly Val Arg Asn Gln Phe Ala Thr Asp Leu
            20                  25                  30

Glu Pro Gly Gly Ser Val Ser Cys Met Arg Ser Ser Leu Ser Phe Leu
        35                  40                  45

Ser Leu Leu Phe Asp Val Gly Pro Arg Asp Val Leu Ser Ala Glu Ala
    50                  55                  60

Ile Glu Gly Cys Leu Val Glu Gly Gly Glu Trp Thr Arg Ala Ala Ala
65                  70                  75                  80

Gly Ser Gly Pro Pro Arg Met Cys Ser Ile Ile Glu Leu Pro Asn Phe
                85                  90                  95

Leu Glu Tyr Pro Ala Ala Arg Gly Gly Leu Arg Cys Val Phe Ser Arg
            100                 105                 110

Val Tyr Gly Glu Val Gly Phe Phe Gly Glu Pro Thr Ala Gly Leu Leu
        115                 120                 125

Glu Thr Gln Cys Pro Ala His Thr Phe Phe Ala Gly Pro Trp Ala Met
    130                 135                 140

Arg Pro Leu Ser Tyr Thr Leu Leu Thr Ile Gly Pro Leu Gly Met Gly
145                 150                 155                 160

Leu Tyr Arg Asp Gly Asp Thr Ala Tyr Leu Phe Asp Pro His Gly Leu
                165                 170                 175

Pro Ala Gly Thr Pro Ala Phe Ile Ala Lys Val Arg Ala Gly Asp Val
            180                 185                 190

Tyr Pro Tyr Leu Thr Tyr Tyr Ala His Asp Arg Pro Lys Val Arg Trp

195                 200                 205

Ala Gly Ala Met Val Phe Phe Val Pro Ser Gly Pro Gly Ala Val Ala
    210                 215                 220

Pro Ala Asp Leu Thr Ala Ala Ala Leu His Leu Tyr Gly Ala Ser Glu
225                 230                 235                 240

Thr Tyr Leu Gln Asp Glu Pro Phe Val Glu Arg Arg Val Ala Ile Thr
                245                 250                 255

His Pro Leu Arg Gly Glu Ile Gly Gly Leu Gly Ala Leu Phe Val Gly
            260                 265                 270

Val Val Pro Arg Gly Asp Gly Glu Gly Ser Gly Pro Val Val Pro Ala
        275                 280                 285

Leu Pro Ala Pro Thr His Val Gln Thr Pro Gly Ala Asp Arg Pro Pro
    290                 295                 300

Glu Ala Pro Arg Gly Ala Ser Gly Pro Pro Asp Thr Pro Gln Ala Gly
305                 310                 315                 320

His Pro Asn Arg Pro Pro Asp Asp Val Trp Ala Ala Ala Leu Glu Gly
                325                 330                 335

Thr Pro Pro Ala Lys Pro Ser Ala Pro Asp Ala Ala Ala Ser Gly Pro
            340                 345                 350

Pro His Ala Ala Pro Pro Pro Gln Thr Pro Ala Gly Asp Ala Ala Glu
        355                 360                 365

Glu Ala Glu Asp Leu Arg Val Leu Glu Val Gly Ala Val Pro Val Gly
    370                 375                 380

Arg His Arg Ala Arg Tyr Ser Thr Gly Leu Pro Lys Arg Arg Arg Pro
385                 390                 395                 400

Thr Trp Thr Pro Pro Ser Ser Val Glu Asp Leu Thr Ser Gly Glu Arg
                405                 410                 415

Pro Ala Pro Lys Ala Pro Pro Ala Lys Ala Lys Lys Lys Ser Ala Pro
            420                 425                 430

Lys Lys Lys Ala Pro Val Ala Ala Glu Val Pro Ala Ser Ser Pro Thr
        435                 440                 445

Pro Ile Ala Ala Thr Val Pro Pro Ala Pro Asp Thr Pro Pro Gln Ser
    450                 455                 460

Gly Gln Gly Gly Gly Asp Asp Gly Pro Ala Ser Pro Ser Ser Pro Ser
465                 470                 475                 480

Val Leu Glu Thr Leu Gly Ala Arg Arg Pro Pro Glu Pro Pro Gly Ala
                485                 490                 495

Asp Leu Ala Gln Leu Phe Glu Val His Pro Asn Val Ala Ala Thr Ala
            500                 505                 510

Val Arg Leu Ala Ala Arg Asp Ala Ala Leu Ala Arg Glu Val Ala Ala
        515                 520                 525

Cys Ser Gln Leu Thr Ile Asn Ala Leu Arg Ser Pro Tyr Pro Ala His
    530                 535                 540

Pro Gly Leu Leu Glu Leu Cys Val Ile Phe Phe Phe Glu Arg Val Leu
545                 550                 555                 560

Ala Phe Leu Ile Glu Asn Gly Ala Arg Thr His Thr Gln Ala Gly Val
                565                 570                 575

Ala Gly Pro Ala Ala Ala Leu Leu Asp Phe Thr Leu Arg Met Leu Pro
            580                 585                 590

Arg Lys Thr Ala Val Gly Asp Phe Leu Ala Ser Thr Arg Met Ser Leu
        595                 600                 605

Ala Asp Val Ala Ala His Arg Pro Leu Ile Gln His Val Leu Asp Glu
    610                 615                 620

Asn Ser Gln Ile Gly Arg Leu Ala Leu Ala Lys Leu Val Leu Val Ala
625                 630                 635                 640

Arg Asp Val Ile Arg Glu Thr Asp Ala Phe Tyr Gly Asp Leu Ala Asp
                645                 650                 655

Leu Asp Leu Gln Leu Arg Ala Ala Pro Pro Ala Asn Leu Tyr Ala Arg
            660                 665                 670

Leu Gly Glu Trp Leu Leu Glu Arg Ser Arg Ala His Pro Asn Thr Leu
        675                 680                 685

Phe Ala Pro Ala Thr Pro Thr His Pro Glu Pro Leu Leu His Arg Ile
    690                 695                 700

Gln Ala Leu Ala Gln Phe Ala Arg Gly Glu Glu Met Arg Val Glu Ala
705                 710                 715                 720

Glu Ala Arg Glu Met Arg Glu Ala Leu Asp Ala Leu Ala Arg Gly Val
                725                 730                 735

Asp Ser Val Ser Gln Arg Ala Gly Pro Leu Thr Val Met Pro Val Pro
            740                 745                 750

Ala Ala Pro Gly Ala Gly Gly Arg Ala Pro Cys Pro Pro Ala Leu Gly
        755                 760                 765

Pro Glu Ala Ile Gln Ala Arg Leu Glu Asp Val Arg Ile Gln Ala Arg
    770                 775                 780

Arg Ala Ile Glu Ser Ala Val Lys Glu Tyr Phe His Arg Gly Ala Val
785                 790                 795                 800

Tyr Ser Ala Lys Ala Leu Gln Ala Ser Asp Ser His Asp Cys Arg Phe
                805                 810                 815

His Val Ala Ser Ala Ala Val Val Pro Met Val Gln Leu Leu Glu Ser
            820                 825                 830

Leu Pro Ala Phe Asp Gln His Thr Arg Asp Val Ala Gln Arg Ala Ala
        835                 840                 845

Leu Pro Pro Pro Pro Pro Leu Ala Thr Ser Pro Gln Ala Ile Leu Leu
    850                 855                 860

Arg Asp Leu Leu Gln Arg Gly Gln Pro Leu Asp Ala Pro Glu Asp Leu
865                 870                 875                 880

Ala Ala Trp Leu Ser Val Leu Thr Asp Ala Ala Thr Gln Gly Leu Ile
                885                 890                 895

Glu Arg Lys Pro Leu Glu Glu Leu Ala Arg Ser Ile His Gly Ile Asn
            900                 905                 910

Asp Gln Gln Ala Arg Arg Ser Ser Gly Leu Ala Glu Leu Gln Arg Phe
        915                 920                 925

Asp Ala Leu Asp Ala Ala Leu Ala Gln Gln Leu Asp Ser Asp Ala Ala
    930                 935                 940

Phe Val Pro Ala Thr Gly Pro Ala Pro Tyr Val Asp Gly Gly Gly Leu
945                 950                 955                 960

Ser Pro Glu Ala Thr Arg Met Ala Glu Asp Ala Leu Arg Gln Ala Arg
                965                 970                 975

Ala Met Glu Ala Ala Lys Met Thr Ala Glu Leu Ala Pro Glu Ala Arg
            980                 985                 990

Ser Arg Leu Arg Glu Arg Ala His  Ala Leu Glu Ala Met  Leu Asn Asp
        995                 1000                 1005

Ala Arg  Glu Arg Ala Lys Val  Ala His Asp Ala Arg  Glu Lys Phe
    1010                 1015                 1020

Leu His  Lys Leu Gln Gly Val  Leu Arg Pro Leu Pro  Asp Phe Val
    1025                 1030                 1035

Gly Leu  Lys Ala Cys Pro Ala  Val Leu Ala Thr Leu  Arg Ala Ser
    1040                 1045                 1050

Leu Pro  Ala Gly Trp Thr Asp  Leu Ala Asp Ala Val  Arg Gly Pro
    1055                 1060                 1065

Pro Pro  Glu Val Thr Ala Ala  Leu Arg Ala Asp Leu  Trp Gly Leu
    1070                 1075                 1080

Leu Gly  Gln Tyr Arg Glu Ala  Leu Glu His Pro Thr  Pro Asp Thr
    1085                 1090                 1095

Ala Thr  Ala Leu Ala Gly Leu  His Pro Ala Phe Val  Val Val Leu
    1100                 1105                 1110

Lys Thr  Leu Phe Ala Asp Ala  Pro Glu Thr Pro Val  Leu Val Gln
    1115                 1120                 1125

Phe Phe  Ser Asp His Ala Pro  Thr Ile Ala Lys Ala  Val Ser Asn
    1130                 1135                 1140

Ala Ile  Asn Ala Gly Ser Ala  Ala Val Ala Thr Ala  Ser Pro Ala
    1145                 1150                 1155

Ala Thr  Val Asp Ala Ala Val  Arg Ala His Gly Ala  Leu Ala Asp
    1160                 1165                 1170

Ala Val  Ser Ala Leu Gly Ala  Ala Ala Arg Asp Pro  Ala Ser Pro
    1175                 1180                 1185

Leu Ser  Phe Leu Ala Val Leu  Ala Asp Ser Ala Ala  Gly Tyr Val

1190                1195                1200

Lys Ala Thr Arg Leu Ala Leu Glu Ala Arg Gly Ala Ile Asp Glu
    1205                1210                1215

Leu Thr Thr Leu Gly Ser Ala Ala Ala Asp Leu Val Val Gln Ala
    1220                1225                1230

Arg Arg Ala Cys Ala Gln Pro Glu Gly Asp His Ala Ala Leu Ile
    1235                1240                1245

Asp Ala Ala Ala Arg Ala Thr Thr Ala Ala Arg Glu Ser Leu Ala
    1250                1255                1260

Gly His Glu Ala Gly Phe Gly Gly Leu Leu His Ala Glu Gly Thr
    1265                1270                1275

Ala Gly Asp His Ser Pro Ser Gly Arg Ala Leu Gln Glu Leu Gly
    1280                1285                1290

Lys Val Ile Gly Ala Thr Arg Arg Arg Ala Asp Glu Leu Glu Ala
    1295                1300                1305

Ala Val Ala Asp Leu Thr Ala Lys Met Ala Ala Gln Arg Ala Arg
    1310                1315                1320

Gly Ser Ser Glu Arg Trp Ala Ala Gly Val Glu Ala Ala Leu Asp
    1325                1330                1335

Arg Val Glu Asn Arg Ala Glu Phe Asp Val Val Glu Leu Arg Arg
    1340                1345                1350

Leu Gln Ala Leu Ala Gly Thr His Gly Tyr Asn Pro Arg Asp Phe
    1355                1360                1365

Arg Lys Arg Ala Glu Gln Ala Leu Ala Ala Asn Ala Glu Ala Val
    1370                1375                1380

Thr Leu Ala Leu Asp Thr Ala Phe Ala Phe Asn Pro Tyr Thr Pro
    1385                1390                1395

Glu Asn Gln Arg His Pro Met Leu Pro Pro Leu Ala Ala Ile His
    1400                1405                1410

Arg Leu Gly Trp Ser Ala Ala Phe His Ala Ala Ala Glu Thr Tyr
    1415                1420                1425

Ala Asp Met Phe Arg Val Asp Ala Glu Pro Leu Ala Arg Leu Leu
    1430                1435                1440

Arg Ile Ala Glu Gly Leu Leu Glu Met Ala Gln Ala Gly Asp Gly
    1445                1450                1455

Phe Ile Asp Tyr His Glu Ala Val Gly Arg Leu Ala Asp Asp Met
    1460                1465                1470

Thr Ser Val Pro Gly Leu Arg Arg Tyr Val Pro Phe Phe Gln His
    1475                1480                1485

Gly Tyr Ala Asp Tyr Val Glu Leu Arg Asp Arg Leu Asp Ala Ile
    1490                1495                1500

Arg Ala Asp Val His Arg Ala Leu Gly Gly Val Pro Leu Asp Leu
    1505                1510                1515

Ala Ala Ala Ala Glu Gln Ile Ser Ala Ala Arg Asn Asp Pro Glu
    1520                1525                1530

Ala Thr Ala Glu Leu Val Arg Thr Gly Val Thr Leu Pro Cys Pro
    1535                1540                1545

Ser Glu Asp Ala Leu Val Ala Cys Ala Ala Ala Leu Glu Arg Val
    1550                1555                1560

Asp Gln Ser Pro Val Lys Asn Thr Ala Tyr Ala Glu Tyr Val Ala
    1565                1570                1575

Phe Val Thr Arg Gln Asp Thr Ala Glu Thr Lys Asp Ala Val Val
    1580                1585                1590

Arg Ala Lys Gln Gln Arg Ala Glu Ala Thr Glu Arg Val Met Ala
    1595                1600                1605

Gly Leu Arg Glu Ala Leu Ala Ala Arg Glu Arg Arg Ala Gln Ile
    1610                1615                1620

Glu Ala Glu Gly Leu Ala Asn Leu Lys Thr Met Leu Lys Val Val
    1625                1630                1635

Ala Val Pro Ala Thr Val Ala Lys Thr Leu Asp Gln Ala Arg Ser
    1640                1645                1650

Val Ala Glu Ile Ala Asp Gln Val Glu Val Leu Leu Asp Gln Thr
    1655                1660                1665

Glu Lys Thr Arg Glu Leu Asp Val Pro Ala Val Ile Trp Leu Glu
    1670                1675                1680

His Ala Gln Arg Thr Phe Glu Thr His Pro Leu Ser Ala Ala Arg
    1685                1690                1695

Gly Asp Gly Pro Gly Pro Leu Ala Arg His Ala Gly Arg Leu Gly
    1700                1705                1710

Ala Leu Phe Asp Thr Arg Arg Arg Val Asp Ala Leu Arg Arg Ser
    1715                1720                1725

Leu Glu Glu Ala Glu Ala Glu Trp Asp Glu Val Trp Gly Arg Phe
    1730                1735                1740

Gly Arg Val Arg Gly Gly Ala Trp Lys Ser Pro Glu Gly Phe Arg
    1745                1750                1755

Ala Met His Glu Gln Leu Arg Ala Leu Gln Asp Thr Thr Asn Thr
    1760                1765                1770

Val Ser Gly Leu Arg Ala Gln Pro Ala Tyr Glu Arg Leu Ser Ala
    1775                1780                1785

Arg Tyr Gln Gly Val Leu Gly Ala Lys Gly Ala Glu Arg Ala Glu
    1790                1795                1800

Ala Val Glu Glu Leu Gly Ala Arg Val Thr Lys His Thr Ala Leu
    1805                1810                1815

Cys Ala Arg Leu Arg Asp Glu Val Val Arg Arg Val Pro Trp Glu
    1820                1825                1830

Met Asn Phe Asp Ala Leu Gly Gly Leu Leu Ala Glu Phe Asp Ala
    1835                1840                1845

Ala Ala Ala Asp Leu Ala Pro Trp Ala Val Glu Glu Phe Arg Gly
    1850                1855                1860

Ala Arg Glu Leu Ile Gln Tyr Arg Met Gly Leu Tyr Ser Ala Tyr
    1865                1870                1875

Ala Arg Ala Gly Gly Gln Thr Gly Ala Gly Ala Glu Ser Ala Pro
    1880                1885                1890

Ala Pro Leu Leu Val Asp Leu Arg Ala Leu Asp Ala Arg Ala Arg
    1895                1900                1905

Ala Ser Ser Ser Pro Glu Gly His Glu Val Asp Pro Gln Leu Leu
    1910                1915                1920

Arg Arg Arg Gly Glu Ala Tyr Leu Arg Ala Gly Gly Asp Pro Gly
    1925                1930                1935

Pro Leu Val Leu Arg Glu Ala Val Ser Ala Leu Asp Leu Pro Phe
    1940                1945                1950

Ala Thr Ser Phe Leu Ala Pro Asp Gly Thr Pro Leu Gln Tyr Ala
    1955                1960                1965

Leu Cys Phe Pro Ala Val Thr Asp Lys Leu Gly Ala Leu Leu Met
    1970                1975                1980

Arg Pro Glu Ala Ala Cys Val Arg Pro Pro Leu Pro Thr Asp Val
    1985                1990                1995

Leu Glu Ser Ala Pro Thr Val Thr Ala Met Tyr Val Leu Thr Val
    2000                2005                2010

Val Asn Arg Leu Gln Leu Ala Leu Ser Asp Ala Gln Ala Ala Asn
    2015                2020                2025

Phe Gln Leu Phe Gly Arg Phe Val Arg His Arg Gln Ala Thr Trp
    2030                2035                2040

Gly Ala Ser Met Asp Ala Ala Ala Glu Leu Tyr Val Ala Leu Val
    2045                2050                2055

Ala Thr Thr Leu Thr Arg Glu Phe Gly Cys Arg Trp Ala Gln Leu
    2060                2065                2070

Gly Trp Ala Ser Gly Ala Ala Ala Pro Arg Pro Pro Pro Gly Pro
    2075                2080                2085

Arg Gly Ser Gln Arg His Cys Val Ala Phe Asn Glu Asn Asp Val
    2090                2095                2100

Leu Val Ala Leu Val Ala Gly Val Pro Glu His Ile Tyr Asn Phe
    2105                2110                2115

Trp Arg Leu Asp Leu Val Arg Gln His Glu Tyr Met His Leu Thr
    2120                2125                2130

Leu Glu Arg Ala Phe Glu Asp Ala Ala Glu Ser Met Leu Phe Val

2135                2140                2145

Gln Arg Leu Thr Pro His Pro Asp Ala Arg Ile Arg Val Leu Pro
    2150                2155                2160

Thr Phe Leu Asp Gly Gly Pro Pro Thr Arg Gly Leu Leu Phe Gly
    2165                2170                2175

Thr Arg Leu Ala Asp Trp Arg Arg Gly Lys Leu Ser Glu Thr Asp
    2180                2185                2190

Pro Leu Ala Pro Trp Arg Ser Ala Leu Glu Leu Gly Thr Gln Arg
    2195                2200                2205

Arg Asp Val Pro Ala Leu Gly Lys Leu Ser Pro Ala Gln Ala Leu
    2210                2215                2220

Ala Ala Val Ser Val Leu Gly Arg Met Cys Leu Pro Ser Ala Ala
    2225                2230                2235

Leu Ala Ala Leu Trp Thr Cys Met Phe Pro Asp Asp Tyr Thr Glu
    2240                2245                2250

Tyr Asp Ser Phe Asp Ala Leu Leu Ala Ala Arg Leu Glu Ser Gly
    2255                2260                2265

Gln Thr Leu Gly Pro Ala Gly Gly Arg Glu Ala Ser Leu Pro Glu
    2270                2275                2280

Ala Pro His Ala Leu Tyr Arg Pro Thr Gly Gln His Val Ala Val
    2285                2290                2295

Leu Ala Ala Ala Thr His Arg Thr Pro Ala Ala Arg Val Thr Ala
    2300                2305                2310

Met Asp Leu Val Leu Ala Ala Val Leu Leu Gly Ala Pro Val Val
    2315                2320                2325

Val Ala Leu Arg Asn Thr Thr Ala Phe Ser Arg Glu Ser Glu Leu
    2330                2335                2340

Glu Leu Cys Leu Thr Leu Phe Asp Ser Arg Pro Gly Gly Pro Asp
    2345                2350                2355

Ala Ala Leu Arg Asp Val Val Ser Ser Asp Ile Glu Thr Trp Ala
    2360                2365                2370

Val Gly Leu Leu His Thr Asp Leu Asn Pro Ile Glu Asn Ala Cys
    2375                2380                2385

Leu Ala Ala Gln Leu Pro Arg Leu Ser Ala Leu Ile Ala Glu Arg
    2390                2395                2400

Pro Leu Ala Asp Gly Pro Pro Cys Leu Val Leu Val Asp Ile Ser
    2405                2410                2415

Met Thr Pro Val Ala Val Leu Trp Glu Ala Pro Glu Pro Pro Gly
    2420                2425                2430

Pro Pro Asp Val Arg Phe Val Gly Ser Glu Ala Thr Glu Glu Leu
    2435                2440                2445

Pro Phe Val Ala Thr Ala Gly Asp Val Leu Ala Ala Ser Ala Ala
    2450                2455                2460

Asp Ala Asp Pro Phe Phe Ala Arg Ala Ile Leu Gly Arg Pro Phe
    2465                2470                2475

Asp Ala Ser Leu Leu Thr Gly Glu Leu Phe Pro Gly His Pro Val
    2480                2485                2490

Tyr Gln Arg Pro Leu Ala Asp Glu Ala Gly Pro Ser Ala Pro Thr
    2495                2500                2505

Ala Ala Arg Asp Pro Arg Asp Leu Ala Gly Gly Asp Gly Gly Ser
    2510                2515                2520

Gly Pro Glu Asp Pro Ala Ala Pro Pro Ala Arg Gln Ala Asp Pro
    2525                2530                2535

Gly Val Leu Ala Pro Thr Leu Leu Thr Asp Ala Thr Thr Gly Glu
    2540                2545                2550

Pro Val Pro Pro Arg Met Trp Ala Trp Ile His Gly Leu Glu Glu
    2555                2560                2565

Leu Ala Ser Asp Asp Ala Gly Gly Pro Thr Pro Asn Pro Ala Pro
    2570                2575                2580

Ala Leu Leu Pro Pro Pro Ala Thr Asp Gln Ser Val Pro Thr Ser
    2585                2590                2595

Gln Tyr Ala Pro Arg Pro Ile Gly Pro Ala Ala Thr Ala Arg Glu
    2600                2605                2610

Thr Arg Pro Ser Val Pro Pro Gln Gln Asn Thr Gly Arg Val Pro
    2615                2620                2625

Val Ala Pro Arg Asp Asp Pro Arg Pro Ser Pro Pro Thr Pro Ser
    2630                2635                2640

Pro Pro Ala Asp Ala Ala Leu Pro Pro Pro Ala Phe Ser Gly Ser
    2645                2650                2655

Ala Ala Ala Phe Ser Ala Ala Val Pro Arg Val Arg Arg Ser Arg
    2660                2665                2670

Arg Thr Arg Ala Lys Ser Arg Ala Pro Arg Ala Ser Ala Pro Pro
    2675                2680                2685

Glu Gly Trp Arg Pro Pro Ala Leu Pro Ala Pro Val Ala Pro Val
    2690                2695                2700

Ala Ala Ser Ala Arg Pro Pro Asp Gln Pro Pro Thr Pro Glu Ser
    2705                2710                2715

Ala Pro Pro Ala Trp Val Ser Ala Leu Pro Leu Pro Pro Gly Pro
    2720                2725                2730

Ala Ser Ala Arg Gly Ala Phe Pro Ala Pro Thr Leu Ala Pro Ile
    2735                2740                2745

Pro Pro Pro Pro Ala Glu Gly Ala Val Val Pro Gly Gly Asp Arg
    2750                2755                2760

Arg Arg Gly Arg Arg Gln Thr Thr Ala Gly Pro Ser Pro Thr Pro
    2765                2770                2775

Pro Arg Gly Pro Ala Ala Gly Pro Pro Arg Arg Leu Thr Arg Pro
    2780                2785                2790

Ala Val Ala Ser Leu Ser Ala Ser Leu Asn Ser Leu Pro Ser Pro
    2795                2800                2805

Arg Asp Pro Ala Asp His Ala Ala Ala Val Ser Ala Ala Ala Ala
    2810                2815                2820

Ala Val Pro Pro Ser Pro Gly Leu Ala Pro Pro Thr Ser Ala Val
    2825                2830                2835

Gln Thr Ser Pro Pro Pro Leu Ala Pro Gly Pro Val Ala Pro Ser
    2840                2845                2850

Glu Pro Leu Cys Gly Trp Val Val Pro Gly Gly Pro Val Ala Arg
    2855                2860                2865

Arg Pro Pro Pro Gln Ser Pro Ala Thr Lys Pro Ala Ala Arg Thr
    2870                2875                2880

Arg Ile Arg Ala Arg Ser Val Pro Gln Pro Pro Leu Pro Gln Pro
    2885                2890                2895

Pro Leu Pro Gln Pro Pro Leu Pro Gln Pro Pro Leu Pro Gln Pro
    2900                2905                2910

Pro Leu Pro Gln Pro Pro Leu Pro Gln Pro Pro Leu Pro Gln Pro
    2915                2920                2925

Pro Leu Pro Gln Pro Pro Leu Pro Gln Pro Pro Leu Pro Gln Pro
    2930                2935                2940

Pro Leu Pro Pro Val Thr Arg Thr Leu Thr Pro Gln Ser Arg Asp
    2945                2950                2955

Ser Val Pro Thr Pro Glu Ser Pro Thr His Thr Asn Thr His Leu
    2960                2965                2970

Pro Val Ser Ala Val Thr Ser Trp Ala Ser Ser Leu Ala Leu His
    2975                2980                2985

Val Asp Ser Ala Pro Pro Pro Ala Ser Leu Leu Gln Thr Leu His
    2990                2995                3000

Ile Ser Ser Asp Asp Glu His Ser Asp Ala Asp Ser Leu Arg Phe
    3005                3010                3015

Ser Asp Ser Asp Asp Thr Glu Ala Leu Asp Pro Leu Pro Pro Glu
    3020                3025                3030

Pro His Leu Pro Pro Ala Asp Glu Pro Pro Gly Pro Leu Ala Ala
    3035                3040                3045

Asp His Leu Gln Ser Pro His Ser Gln Phe Gly Pro Leu Pro Val
    3050                3055                3060

Gln Ala Asn Ala Val Leu Ser Arg Arg Tyr Val Arg Ser Thr Gly
    3065                3070                3075

Arg Ser Ala Leu Ala Val Leu Ile Arg Ala Cys Arg Arg Ile Gln

3080                 3085                 3090

Gln Gln  Leu Gln Arg Thr Arg  Arg Ala Leu Phe Gln  Arg Ser Asn
    3095                 3100                 3105

Ala Val  Leu Thr Ser Leu His  His Val Arg Met Leu  Leu Gly
    3110                 3115                 3120

<210> 23
<211> 964
<212> PRT
<213> Artificial Sequence

<220>
<223> Description of Artificial Sequence: Synthetic
      polypeptide

<400> 23
Ala Ala Gln Arg Ala Arg Gly Ser Ser Glu Arg Trp Ala Ala Gly Val
1               5                   10                  15

Glu Ala Ala Leu Asp Arg Val Glu Asn Arg Ala Glu Phe Asp Val Val
            20                  25                  30

Glu Leu Arg Arg Leu Gln Ala Leu Ala Gly Thr His Gly Tyr Asn Pro
        35                  40                  45

Arg Asp Phe Arg Lys Arg Ala Glu Gln Ala Leu Ala Ala Asn Ala Glu
    50                  55                  60

Ala Val Thr Leu Ala Leu Asp Thr Ala Phe Ala Phe Asn Pro Tyr Thr
65                  70                  75                  80

Pro Glu Asn Gln Arg His Pro Met Leu Pro Pro Leu Ala Ala Ile His
                85                  90                  95

Arg Leu Gly Trp Ser Ala Ala Phe His Ala Ala Ala Glu Thr Tyr Ala
            100                 105                 110

Asp Met Phe Arg Val Asp Ala Glu Pro Leu Ala Arg Leu Leu Arg Ile
        115                 120                 125

Ala Glu Gly Leu Leu Glu Met Ala Gln Ala Gly Asp Gly Phe Ile Asp
    130                 135                 140

Tyr His Glu Ala Val Gly Arg Leu Ala Asp Asp Met Thr Ser Val Pro
145                 150                 155                 160

Gly Leu Arg Arg Tyr Val Pro Phe Phe Gln His Gly Tyr Ala Asp Tyr
                165                 170                 175

Val Glu Leu Arg Asp Arg Leu Asp Ala Ile Arg Ala Asp Val His Arg
            180                 185                 190

Ala Leu Gly Gly Val Pro Leu Asp Leu Ala Ala Ala Ala Glu Gln Ile
        195                 200                 205

Ser Ala Ala Arg Asn Asp Pro Glu Ala Thr Ala Glu Leu Val Arg Thr
    210                 215                 220

Gly Val Thr Leu Pro Cys Pro Ser Glu Asp Ala Leu Val Ala Cys Ala
225                 230                 235                 240

Ala Ala Leu Glu Arg Val Asp Gln Ser Pro Val Lys Asn Thr Ala Tyr
                245                 250                 255

Ala Glu Tyr Val Ala Phe Val Thr Arg Gln Asp Thr Ala Glu Thr Lys
            260                 265                 270

Asp Ala Val Val Arg Ala Lys Gln Gln Arg Ala Glu Ala Thr Glu Arg
        275                 280                 285

Val Met Ala Gly Leu Arg Glu Ala Leu Ala Ala Arg Glu Arg Arg Ala
    290                 295                 300

Gln Ile Glu Ala Glu Gly Leu Ala Asn Leu Lys Thr Met Leu Lys Val
305                 310                 315                 320

Val Ala Val Pro Ala Thr Val Ala Lys Thr Leu Asp Gln Ala Arg Ser
                325                 330                 335

Val Ala Glu Ile Ala Asp Gln Val Glu Val Leu Leu Asp Gln Thr Glu
            340                 345                 350

Lys Thr Arg Glu Leu Asp Val Pro Ala Val Ile Trp Leu Glu His Ala
        355                 360                 365

Gln Arg Thr Phe Glu Thr His Pro Leu Ser Ala Ala Arg Gly Asp Gly
    370                 375                 380

Pro Gly Pro Leu Ala Arg His Ala Gly Arg Leu Gly Ala Leu Phe Asp
385                 390                 395                 400

Thr Arg Arg Arg Val Asp Ala Leu Arg Arg Ser Leu Glu Glu Ala Glu
                405                 410                 415

Ala Glu Trp Asp Glu Val Trp Gly Arg Phe Gly Arg Val Arg Gly Gly

420                 425                 430

Ala Trp Lys Ser Pro Glu Gly Phe Arg Ala Met His Glu Gln Leu Arg
        435                 440                 445

Ala Leu Gln Asp Thr Thr Asn Thr Val Ser Gly Leu Arg Ala Gln Pro
    450                 455                 460

Ala Tyr Glu Arg Leu Ser Ala Arg Tyr Gln Gly Val Leu Gly Ala Lys
465                 470                 475                 480

Gly Ala Glu Arg Ala Glu Ala Val Glu Glu Leu Gly Ala Arg Val Thr
                485                 490                 495

Lys His Thr Ala Leu Cys Ala Arg Leu Arg Asp Glu Val Val Arg Arg
            500                 505                 510

Val Pro Trp Glu Met Asn Phe Asp Ala Leu Gly Gly Leu Leu Ala Glu
        515                 520                 525

Phe Asp Ala Ala Ala Ala Asp Leu Ala Pro Trp Ala Val Glu Glu Phe
    530                 535                 540

Arg Gly Ala Arg Glu Leu Ile Gln Tyr Arg Met Gly Leu Tyr Ser Ala
545                 550                 555                 560

Tyr Ala Arg Ala Gly Gly Gln Thr Gly Ala Gly Ala Glu Ser Ala Pro
                565                 570                 575

Ala Pro Leu Leu Val Asp Leu Arg Ala Leu Asp Ala Arg Ala Arg Ala
            580                 585                 590

Ser Ser Ser Pro Glu Gly His Glu Val Asp Pro Gln Leu Leu Arg Arg
        595                 600                 605

Arg Gly Glu Ala Tyr Leu Arg Ala Gly Gly Asp Pro Gly Pro Leu Val
    610                 615                 620

Leu Arg Glu Ala Val Ser Ala Leu Asp Leu Pro Phe Ala Thr Ser Phe
625                 630                 635                 640

Leu Ala Pro Asp Gly Thr Pro Leu Gln Tyr Ala Leu Cys Phe Pro Ala
                645                 650                 655

Val Thr Asp Lys Leu Gly Ala Leu Leu Met Arg Pro Glu Ala Ala Cys
            660                 665                 670

Val Arg Pro Pro Leu Pro Thr Asp Val Leu Glu Ser Ala Pro Thr Val
        675                 680                 685

Thr Ala Met Tyr Val Leu Thr Val Val Asn Arg Leu Gln Leu Ala Leu
    690                 695                 700

Ser Asp Ala Gln Ala Ala Asn Phe Gln Leu Phe Gly Arg Phe Val Arg
705                 710                 715                 720

His Arg Gln Ala Thr Trp Gly Ala Ser Met Asp Ala Ala Ala Glu Leu
                725                 730                 735

Tyr Val Ala Leu Val Ala Thr Thr Leu Thr Arg Glu Phe Gly Cys Arg
            740                 745                 750

Trp Ala Gln Leu Gly Trp Ala Ser Gly Ala Ala Ala Pro Arg Pro Pro
        755                 760                 765

Pro Gly Pro Arg Gly Ser Gln Arg His Cys Val Ala Phe Asn Glu Asn
    770                 775                 780

Asp Val Leu Val Ala Leu Val Ala Gly Val Pro Glu His Ile Tyr Asn
785                 790                 795                 800

Phe Trp Arg Leu Asp Leu Val Arg Gln His Glu Tyr Met His Leu Thr
                805                 810                 815

Leu Glu Arg Ala Phe Glu Asp Ala Ala Glu Ser Met Leu Phe Val Gln
            820                 825                 830

Arg Leu Thr Pro His Pro Asp Ala Arg Ile Arg Val Leu Pro Thr Phe
        835                 840                 845

Leu Asp Gly Gly Pro Pro Thr Arg Gly Leu Leu Phe Gly Thr Arg Leu
    850                 855                 860

Ala Asp Trp Arg Arg Gly Lys Leu Ser Glu Thr Asp Pro Leu Ala Pro
865                 870                 875                 880

Trp Arg Ser Ala Leu Glu Leu Gly Thr Gln Arg Arg Asp Val Pro Ala
                885                 890                 895

Leu Gly Lys Leu Ser Pro Ala Gln Ala Leu Ala Ala Val Ser Val Leu
            900                 905                 910

Gly Arg Met Cys Leu Pro Ser Ala Ala Leu Ala Ala Leu Trp Thr Cys
        915                 920                 925

Met Phe Pro Asp Asp Tyr Thr Glu Tyr Asp Ser Phe Asp Ala Leu Leu
    930                 935                 940

Ala Ala Arg Leu Glu Ser Gly Gln Thr Leu Gly Pro Ala Gly Gly Arg
945                 950                 955                 960

Glu Ala Ser Leu

<210> 24
<211> 870
<212> PRT
<213> Artificial Sequence

<220>
<223> Description of Artificial Sequence: Synthetic
      polypeptide

<400> 24
Glu Tyr Asp Ser Phe Asp Ala Leu Leu Ala Ala Arg Leu Glu Ser Gly
1               5                   10                  15

Gln Thr Leu Gly Pro Ala Gly Gly Arg Glu Ala Ser Leu Pro Glu Ala
            20                  25                  30

Pro His Ala Leu Tyr Arg Pro Thr Gly Gln His Val Ala Val Leu Ala
        35                  40                  45

Ala Ala Thr His Arg Thr Pro Ala Ala Arg Val Thr Ala Met Asp Leu
    50                  55                  60

Val Leu Ala Ala Val Leu Leu Gly Ala Pro Val Val Val Ala Leu Arg
65                  70                  75                  80

Asn Thr Thr Ala Phe Ser Arg Glu Ser Glu Leu Glu Leu Cys Leu Thr
                85                  90                  95

Leu Phe Asp Ser Arg Pro Gly Gly Pro Asp Ala Ala Leu Arg Asp Val
            100                 105                 110

Val Ser Ser Asp Ile Glu Thr Trp Ala Val Gly Leu Leu His Thr Asp
        115                 120                 125

Leu Asn Pro Ile Glu Asn Ala Cys Leu Ala Ala Gln Leu Pro Arg Leu
    130                 135                 140

Ser Ala Leu Ile Ala Glu Arg Pro Leu Ala Asp Gly Pro Pro Cys Leu
145                 150                 155                 160

Val Leu Val Asp Ile Ser Met Thr Pro Val Ala Val Leu Trp Glu Ala
                165                 170                 175

Pro Glu Pro Pro Gly Pro Pro Asp Val Arg Phe Val Gly Ser Glu Ala
            180                 185                 190

Thr Glu Glu Leu Pro Phe Val Ala Thr Ala Gly Asp Val Leu Ala Ala
        195                 200                 205

Ser Ala Ala Asp Ala Asp Pro Phe Phe Ala Arg Ala Ile Leu Gly Arg
    210                 215                 220

Pro Phe Asp Ala Ser Leu Leu Thr Gly Glu Leu Phe Pro Gly His Pro
225                 230                 235                 240

Val Tyr Gln Arg Pro Leu Ala Asp Glu Ala Gly Pro Ser Ala Pro Thr
                245                 250                 255

Ala Ala Arg Asp Pro Arg Asp Leu Ala Gly Gly Asp Gly Gly Ser Gly
            260                 265                 270

Pro Glu Asp Pro Ala Ala Pro Pro Ala Arg Gln Ala Asp Pro Gly Val
        275                 280                 285

Leu Ala Pro Thr Leu Leu Thr Asp Ala Thr Thr Gly Glu Pro Val Pro
    290                 295                 300

Pro Arg Met Trp Ala Trp Ile His Gly Leu Glu Glu Leu Ala Ser Asp
305                 310                 315                 320

Asp Ala Gly Gly Pro Thr Pro Asn Pro Ala Pro Ala Leu Leu Pro Pro
                325                 330                 335

Pro Ala Thr Asp Gln Ser Val Pro Thr Ser Gln Tyr Ala Pro Arg Pro
            340                 345                 350

Ile Gly Pro Ala Ala Thr Ala Arg Glu Thr Arg Pro Ser Val Pro Pro
        355                 360                 365

Gln Gln Asn Thr Gly Arg Val Pro Val Ala Pro Arg Asp Asp Pro Arg
    370                 375                 380

Pro Ser Pro Pro Thr Pro Ser Pro Pro Ala Asp Ala Ala Leu Pro Pro
385                 390                 395                 400

Pro Ala Phe Ser Gly Ser Ala Ala Ala Phe Ser Ala Ala Val Pro Arg
                405                 410                 415

Val Arg Arg Ser Arg Arg Thr Arg Ala Lys Ser Arg Ala Pro Arg Ala
            420                 425                 430

Ser Ala Pro Pro Glu Gly Trp Arg Pro Pro Ala Leu Pro Ala Pro Val
        435                 440                 445

Ala Pro Val Ala Ala Ser Ala Arg Pro Pro Asp Gln Pro Pro Thr Pro
    450                 455                 460

Glu Ser Ala Pro Pro Ala Trp Val Ser Ala Leu Pro Leu Pro Pro Gly
465                 470                 475                 480

Pro Ala Ser Ala Arg Gly Ala Phe Pro Ala Pro Thr Leu Ala Pro Ile
                485                 490                 495

Pro Pro Pro Pro Ala Glu Gly Ala Val Val Pro Gly Gly Asp Arg Arg
            500                 505                 510

Arg Gly Arg Arg Gln Thr Thr Ala Gly Pro Ser Pro Thr Pro Pro Arg
        515                 520                 525

Gly Pro Ala Ala Gly Pro Pro Arg Arg Leu Thr Arg Pro Ala Val Ala
    530                 535                 540

Ser Leu Ser Ala Ser Leu Asn Ser Leu Pro Ser Pro Arg Asp Pro Ala
545                 550                 555                 560

Asp His Ala Ala Ala Val Ser Ala Ala Ala Ala Ala Val Pro Pro Ser
                565                 570                 575

Pro Gly Leu Ala Pro Pro Thr Ser Ala Val Gln Thr Ser Pro Pro Pro
            580                 585                 590

Leu Ala Pro Gly Pro Val Ala Pro Ser Glu Pro Leu Cys Gly Trp Val
        595                 600                 605

Val Pro Gly Gly Pro Val Ala Arg Arg Pro Pro Pro Gln Ser Pro Ala
    610                 615                 620

Thr Lys Pro Ala Ala Arg Thr Arg Ile Arg Ala Arg Ser Val Pro Gln
625                 630                 635                 640

Pro Pro Leu Pro Gln Pro Pro Leu Pro Gln Pro Pro Leu Pro Gln Pro
                645                 650                 655

Pro Leu Pro Gln Pro Pro Leu Pro Gln Pro Pro Leu Pro Gln Pro Pro
            660                 665                 670

Leu Pro Gln Pro Pro Leu Pro Gln Pro Pro Leu Pro Gln Pro Pro Leu
        675                 680                 685

Pro Gln Pro Pro Leu Pro Pro Val Thr Arg Thr Leu Thr Pro Gln Ser
    690                 695                 700

Arg Asp Ser Val Pro Thr Pro Glu Ser Pro Thr His Thr Asn Thr His
705                 710                 715                 720

Leu Pro Val Ser Ala Val Thr Ser Trp Ala Ser Ser Leu Ala Leu His
                725                 730                 735

Val Asp Ser Ala Pro Pro Pro Ala Ser Leu Leu Gln Thr Leu His Ile
            740                 745                 750

Ser Ser Asp Asp Glu His Ser Asp Ala Asp Ser Leu Arg Phe Ser Asp
        755                 760                 765

Ser Asp Asp Thr Glu Ala Leu Asp Pro Leu Pro Pro Glu Pro His Leu
    770                 775                 780

Pro Pro Ala Asp Glu Pro Pro Gly Pro Leu Ala Ala Asp His Leu Gln
785                 790                 795                 800

Ser Pro His Ser Gln Phe Gly Pro Leu Pro Val Gln Ala Asn Ala Val
                805                 810                 815

Leu Ser Arg Arg Tyr Val Arg Ser Thr Gly Arg Ser Ala Leu Ala Val
            820                 825                 830

Leu Ile Arg Ala Cys Arg Arg Ile Gln Gln Gln Leu Gln Arg Thr Arg
        835                 840                 845

Arg Ala Leu Phe Gln Arg Ser Asn Ala Val Leu Thr Ser Leu His His
    850                 855                 860

Val Arg Met Leu Leu Gly
865                 870

<210> 25
<211> 337
<212> PRT
<213> Artificial Sequence

<220>
<223> Description of Artificial Sequence: Synthetic
      polypeptide

<400> 25
Met Asp Pro Ala Val Ser Pro Ala Ser Thr Asp Pro Leu Asp Thr His
1               5                   10                  15

Ala Ser Gly Ala Gly Ala Ala Pro Ile Pro Val Cys Pro Thr Pro Glu
            20                  25                  30

Arg Tyr Phe Tyr Thr Ser Gln Cys Pro Asp Ile Asn His Leu Arg Ser
        35                  40                  45

Leu Ser Ile Leu Asn Arg Trp Leu Glu Thr Glu Leu Val Phe Val Gly
    50                  55                  60

Asp Glu Glu Asp Val Ser Lys Leu Ser Glu Gly Glu Leu Gly Phe Tyr
65                  70                  75                  80

Arg Phe Leu Phe Ala Phe Leu Ser Ala Ala Asp Asp Leu Val Thr Glu
                85                  90                  95

Asn Leu Gly Gly Leu Ser Gly Leu Phe Glu Gln Lys Asp Ile Leu His
            100                 105                 110

Tyr Tyr Val Glu Gln Glu Cys Ile Glu Val Val His Ser Arg Val Tyr
        115                 120                 125

Asn Ile Ile Gln Leu Val Leu Phe His Asn Asn Asp Gln Ala Arg Arg
    130                 135                 140

Ala Tyr Val Ala Arg Thr Ile Asn His Pro Ala Ile Arg Val Lys Val
145                 150                 155                 160

Asp Trp Leu Glu Ala Arg Val Arg Glu Cys Asp Ser Ile Pro Glu Lys
                165                 170                 175

Phe Ile Leu Met Ile Leu Ile Glu Gly Val Phe Phe Ala Ala Ser Phe
            180                 185                 190

Ala Ala Ile Ala Tyr Leu Arg Thr Asn Asn Leu Leu Arg Val Thr Cys
        195                 200                 205

Gln Ser Asn Asp Leu Ile Ser Arg Asp Glu Ala Val His Thr Thr Ala
    210                 215                 220

Ser Cys Tyr Ile Tyr Asn Asn Tyr Leu Gly Gly His Ala Lys Pro Glu
225                 230                 235                 240

Ala Ala Arg Val Tyr Arg Leu Phe Arg Glu Ala Val Asp Ile Glu Ile
                245                 250                 255

Gly Phe Ile Arg Ser Gln Ala Pro Thr Asp Ser Ser Ile Leu Ser Pro
            260                 265                 270

Gly Ala Leu Ala Ala Ile Glu Asn Tyr Val Arg Phe Ser Ala Asp Arg
        275                 280                 285

Leu Leu Gly Leu Ile His Met Gln Pro Leu Tyr Ser Ala Pro Ala Pro
    290                 295                 300

Asp Ala Ser Phe Pro Leu Ser Leu Met Ser Thr Asp Lys His Thr Asn
305                 310                 315                 320

Phe Phe Glu Cys Arg Ser Thr Ser Tyr Ala Gly Ala Val Val Asn Asp
                325                 330                 335

Leu

<210> 26
<211> 86
<212> PRT
<213> Human herpesvirus 2

<400> 26
Met Ser Trp Ala Leu Lys Thr Thr Asp Met Phe Leu Asp Ser Ser Arg
1               5                   10                  15

Cys Thr His Arg Thr Tyr Gly Asp Val Cys Ala Glu Ile His Lys Arg
            20                  25                  30

Glu Arg Glu Asp Arg Glu Ala Ala Arg Thr Ala Val Thr Asp Pro Glu
        35                  40                  45

Leu Pro Leu Leu Cys Pro Pro Asp Val Arg Ser Asp Pro Ala Ser Arg
    50                  55                  60

Asn Pro Thr Gln Gln Thr Arg Gly Cys Ala Arg Ser Asn Glu Arg Gln
65                  70                  75                  80

Asp Arg Val Leu Ala Pro
                85

<210> 27
<211> 467
<212> PRT
<213> Human herpesvirus 2

<400> 27
Met Gly Arg Arg Ala Pro Arg Gly Ser Pro Glu Ala Ala Pro Gly Ala

1               5                   10                  15

Asp Val Ala Pro Gly Ala Arg Ala Ala Trp Trp Val Trp Cys Val Gln
            20                  25                  30

Val Ala Thr Phe Ile Val Ser Ala Ile Cys Val Val Gly Leu Leu Val
        35                  40                  45

Leu Ala Ser Val Phe Arg Asp Arg Phe Pro Cys Leu Tyr Ala Pro Ala
    50                  55                  60

Thr Ser Tyr Ala Lys Ala Asn Ala Thr Val Glu Val Arg Gly Gly Val
65                  70                  75                  80

Ala Val Pro Leu Arg Leu Asp Thr Gln Ser Leu Leu Ala Thr Tyr Ala
                85                  90                  95

Ile Thr Ser Thr Leu Leu Leu Ala Ala Ala Val Tyr Ala Ala Val Gly
            100                 105                 110

Ala Val Thr Ser Arg Tyr Glu Arg Ala Leu Asp Ala Ala Arg Arg Leu
        115                 120                 125

Ala Ala Ala Arg Met Ala Met Pro His Ala Thr Leu Ile Ala Gly Asn
    130                 135                 140

Val Cys Ala Trp Leu Leu Gln Ile Thr Val Leu Leu Leu Ala His Arg
145                 150                 155                 160

Ile Ser Gln Leu Ala His Leu Ile Tyr Val Leu His Phe Ala Cys Leu
                165                 170                 175

Val Tyr Leu Ala Ala His Phe Cys Thr Arg Gly Val Leu Ser Gly Thr
            180                 185                 190

Tyr Leu Arg Gln Val His Gly Leu Ile Asp Pro Ala Pro Thr His His
        195                 200                 205

Arg Ile Val Gly Pro Val Arg Ala Val Met Thr Asn Ala Leu Leu Leu
    210                 215                 220

Gly Thr Leu Leu Cys Thr Ala Ala Ala Ala Val Ser Leu Asn Thr Ile
225                 230                 235                 240

Ala Ala Leu Asn Phe Asn Phe Ser Ala Pro Ser Met Leu Ile Cys Leu
                245                 250                 255

Thr Thr Leu Phe Ala Leu Leu Val Val Ser Leu Leu Leu Val Val Glu
            260                 265                 270

Gly Val Leu Cys His Tyr Val Arg Val Leu Val Gly Pro His Leu Gly
        275                 280                 285

Ala Ile Ala Ala Thr Gly Ile Val Gly Leu Ala Cys Glu His Tyr His
    290                 295                 300

Thr Gly Gly Tyr Tyr Val Val Glu Gln Gln Trp Pro Gly Ala Gln Thr
305                 310                 315                 320

Gly Val Arg Val Ala Leu Ala Leu Val Ala Ala Phe Ala Leu Ala Met
                325                 330                 335

Ala Val Leu Arg Cys Thr Arg Ala Tyr Leu Tyr His Arg Arg His His
            340                 345                 350

Thr Lys Phe Phe Val Arg Met Arg Asp Thr Arg His Arg Ala His Ser
        355                 360                 365

Ala Leu Arg Arg Val Arg Ser Ser Met Arg Gly Ser Arg Arg Gly Gly
    370                 375                 380

Pro Pro Gly Asp Pro Gly Tyr Ala Glu Thr Pro Tyr Ala Ser Val Ser
385                 390                 395                 400

His His Ala Glu Ile Asp Arg Tyr Gly Asp Ser Asp Gly Asp Pro Ile
                405                 410                 415

Tyr Asp Glu Val Ala Pro Asp His Glu Ala Glu Leu Tyr Ala Arg Val
            420                 425                 430

Gln Arg Pro Gly Pro Val Pro Asp Ala Glu Pro Ile Tyr Asp Thr Val
        435                 440                 445

Glu Gly Tyr Ala Pro Arg Ser Ala Gly Glu Pro Val Tyr Ser Thr Val
    450                 455                 460

Arg Arg Trp
465

<210> 28
<211> 734
<212> PRT
<213> Artificial Sequence

<220>
<223> Description of Artificial Sequence: Synthetic

polypeptide

<400> 28
Met Phe Gly Gln Gln Leu Ala Ser Asp Val Gln Gln Tyr Leu Glu Arg
1               5                   10                  15

Leu Glu Lys Gln Arg Gln Gln Lys Val Gly Val Asp Glu Ala Ser Ala
            20                  25                  30

Gly Leu Thr Leu Gly Gly Asp Ala Leu Arg Val Pro Phe Leu Asp Phe
        35                  40                  45

Ala Thr Ala Thr Pro Lys Arg His Gln Thr Val Val Pro Gly Val Gly
    50                  55                  60

Thr Leu His Asp Cys Cys Glu His Ser Pro Leu Phe Ser Ala Val Ala
65                  70                  75                  80

Arg Arg Leu Leu Phe Asn Ser Leu Val Pro Ala Gln Leu Arg Gly Arg
                85                  90                  95

Asp Phe Gly Gly Asp His Thr Ala Lys Leu Glu Phe Leu Ala Pro Glu
            100                 105                 110

Leu Val Arg Ala Val Ala Arg Leu Arg Phe Arg Glu Cys Ala Pro Glu
        115                 120                 125

Asp Ala Val Pro Gln Arg Asn Ala Tyr Tyr Ser Val Leu Asn Thr Phe
    130                 135                 140

Gln Ala Leu His Arg Ser Glu Ala Phe Arg Gln Leu Val His Phe Val
145                 150                 155                 160

Arg Asp Phe Ala Gln Leu Leu Lys Thr Ser Phe Arg Ala Ser Ser Leu
                165                 170                 175

Ala Glu Thr Thr Gly Pro Pro Lys Lys Arg Ala Lys Val Asp Val Ala
            180                 185                 190

Thr His Gly Gln Thr Tyr Gly Thr Leu Glu Leu Phe Gln Lys Met Ile
        195                 200                 205

Leu Met His Ala Thr Tyr Phe Leu Ala Ala Val Leu Leu Gly Asp His
    210                 215                 220

Ala Glu Gln Val Asn Thr Phe Leu Arg Leu Val Phe Glu Ile Pro Leu
225                 230                 235                 240

Phe Ser Asp Thr Ala Val Arg His Phe Arg Gln Arg Ala Thr Val Phe
                245                 250                 255

Leu Val Pro Arg Arg His Gly Lys Thr Trp Phe Leu Val Pro Leu Ile
            260                 265                 270

Ala Leu Ser Leu Ala Ser Phe Arg Gly Ile Lys Ile Gly Tyr Thr Ala
        275                 280                 285

His Ile Arg Lys Ala Thr Glu Pro Val Phe Asp Glu Ile Asp Ala Cys
    290                 295                 300

Leu Arg Gly Trp Phe Gly Ser Ser Arg Val Asp His Val Lys Gly Glu
305                 310                 315                 320

Thr Ile Ser Phe Ser Phe Pro Asp Gly Ser Arg Ser Thr Ile Val Phe
                325                 330                 335

Ala Ser Ser His Asn Thr Asn Gly Ile Arg Gly Gln Asp Phe Asn Leu
            340                 345                 350

Leu Phe Val Asp Glu Ala Asn Phe Ile Arg Pro Asp Ala Val Gln Thr
        355                 360                 365

Ile Met Gly Phe Leu Asn Gln Ala Asn Cys Lys Ile Ile Phe Val Ser
    370                 375                 380

Ser Thr Asn Thr Gly Lys Ala Ser Thr Ser Phe Leu Tyr Asn Leu Arg
385                 390                 395                 400

Gly Ala Ala Asp Glu Leu Leu Asn Val Val Thr Tyr Ile Cys Asp Asp
                405                 410                 415

His Met Pro Arg Val Val Thr His Thr Asn Ala Thr Ala Cys Ser Cys
            420                 425                 430

Tyr Ile Leu Asn Lys Pro Val Phe Ile Thr Met Asp Gly Ala Val Arg
        435                 440                 445

Arg Thr Ala Asp Leu Phe Leu Pro Asp Ser Phe Met Gln Glu Ile Ile
    450                 455                 460

Gly Gly Gln Ala Arg Glu Thr Gly Asp Asp Arg Pro Val Leu Thr Lys
465                 470                 475                 480

Ser Ala Gly Glu Arg Phe Leu Leu Tyr Arg Pro Ser Thr Thr Thr Asn
                485                 490                 495

Ser Gly Leu Met Ala Pro Glu Leu Tyr Val Tyr Val Asp Pro Ala Phe
            500                 505                 510

Thr Ala Asn Thr Arg Ala Ser Gly Thr Gly Ile Ala Val Val Gly Arg
        515                 520                 525

Tyr Arg Asp Asp Phe Ile Ile Phe Ala Leu Glu His Phe Phe Leu Arg
    530                 535                 540

Ala Leu Thr Gly Ser Ala Pro Ala Asp Ile Ala Arg Cys Val Val His
545                 550                 555                 560

Ser Leu Ala Gln Val Leu Ala Leu His Pro Gly Ala Phe Arg Ser Val
                565                 570                 575

Arg Val Ala Val Glu Gly Asn Ser Ser Gln Asp Ser Ala Val Ala Ile
            580                 585                 590

Ala Thr His Val His Thr Glu Met His Arg Ile Leu Ala Ser Ala Gly
        595                 600                 605

Ala Asn Gly Pro Gly Pro Glu Leu Leu Phe Tyr His Cys Glu Pro Pro
    610                 615                 620

Gly Gly Ala Val Leu Tyr Pro Phe Phe Leu Leu Asn Lys Gln Lys Thr
625                 630                 635                 640

Pro Ala Phe Glu Tyr Phe Ile Lys Lys Phe Asn Ser Gly Gly Val Met
                645                 650                 655

Ala Ser Gln Glu Leu Val Ser Val Thr Val Arg Leu Gln Thr Asp Pro
            660                 665                 670

Val Glu Tyr Leu Ser Glu Gln Leu Asn Asn Leu Ile Glu Thr Val Ser
        675                 680                 685

Pro Asn Thr Asp Val Arg Met Tyr Ser Gly Lys Arg Asn Gly Ala Ala
    690                 695                 700

Asp Asp Leu Met Val Ala Val Ile Met Ala Ile Tyr Leu Ala Ala Pro
705                 710                 715                 720

Thr Gly Ile Pro Pro Ala Phe Phe Pro Ile Thr Arg Thr Ser
                725                 730

<210> 29
<211> 329

<212> PRT
<213> Artificial Sequence

<220>
<223> Description of Artificial Sequence: Synthetic
      polypeptide

<400> 29
Met Asn Pro Val Ser Ala Ser Gly Ala Pro Ala Pro Pro Pro Pro Gly
1               5                   10                  15

Asp Gly Ser Tyr Leu Trp Ile Pro Ala Ser His Tyr Asn Gln Leu Val
            20                  25                  30

Thr Gly Gln Ser Ala Pro Arg His Pro Pro Leu Thr Ala Cys Gly Leu
        35                  40                  45

Pro Ala Ala Gly Thr Val Ala Tyr Gly His Pro Gly Ala Gly Pro Ser
    50                  55                  60

Pro His Tyr Pro Pro Pro Pro Ala His Pro Tyr Pro Gly Met Leu Phe
65                  70                  75                  80

Ala Gly Pro Ser Pro Leu Glu Ala Gln Ile Ala Ala Leu Val Gly Ala
                85                  90                  95

Ile Ala Ala Asp Arg Gln Ala Gly Gly Leu Pro Ala Ala Ala Gly Asp
            100                 105                 110

His Gly Ile Arg Gly Ser Ala Lys Arg Arg Arg His Glu Val Glu Gln
        115                 120                 125

Pro Glu Tyr Asp Cys Gly Arg Asp Glu Pro Asp Arg Asp Phe Pro Tyr
    130                 135                 140

Tyr Pro Gly Glu Ala Arg Pro Glu Pro Arg Pro Val Asp Ser Arg Arg
145                 150                 155                 160

Ala Ala Arg Gln Ala Ser Gly Pro His Glu Thr Ile Thr Ala Leu Val
                165                 170                 175

Gly Ala Val Thr Ser Leu Gln Gln Glu Leu Ala His Met Arg Ala Arg
            180                 185                 190

Thr His Ala Pro Tyr Gly Pro Tyr Pro Pro Val Gly Pro Tyr His His
        195                 200                 205

Pro His Ala Asp Thr Glu Thr Pro Ala Gln Pro Pro Arg Tyr Pro Ala
    210                 215                 220

Lys Ala Val Tyr Leu Pro Pro Pro His Ile Ala Pro Pro Gly Pro Pro
225                 230                 235                 240

Leu Ser Gly Ala Val Pro Pro Pro Ser Tyr Pro Pro Val Ala Val Thr
                245                 250                 255

Pro Gly Pro Ala Pro Pro Leu His Gln Pro Ser Pro Ala His Ala His
            260                 265                 270

Pro Pro Pro Pro Pro Pro Gly Pro Thr Pro Pro Pro Ala Ala Ser Leu
        275                 280                 285

Pro Gln Pro Glu Ala Pro Gly Ala Glu Ala Gly Ala Leu Val Asn Ala
    290                 295                 300

Ser Ser Ala Ala His Val Asn Val Asp Thr Ala Arg Ala Ala Asp Leu
305                 310                 315                 320

Phe Val Ser Gln Met Met Gly Ser Arg
                325

<210> 30
<211> 1240
<212> PRT
<213> Artificial Sequence

<220>
<223> Description of Artificial Sequence: Synthetic
      polypeptide

<400> 30
Met Phe Cys Ala Ala Gly Gly Pro Ala Ser Pro Gly Gly Lys Pro Ala
1               5                   10                  15

Ala Arg Ala Ala Ser Gly Phe Phe Ala Pro His Asn Pro Arg Gly Ala
            20                  25                  30

Thr Gln Thr Ala Pro Pro Pro Cys Arg Arg Gln Asn Phe Tyr Asn Pro
        35                  40                  45

His Leu Ala Gln Thr Gly Thr Gln Pro Lys Ala Leu Gly Pro Ala Gln
    50                  55                  60

Arg His Thr Tyr Tyr Ser Glu Cys Asp Glu Phe Arg Phe Ile Ala Pro
65                  70                  75                  80

Arg Ser Leu Asp Glu Asp Ala Pro Ala Glu Gln Arg Thr Gly Val His
                85                  90                  95

Asp Gly Arg Leu Arg Arg Ala Pro Lys Val Tyr Cys Gly Gly Asp Glu
            100                 105                 110

Arg Asp Val Leu Arg Val Gly Pro Glu Gly Phe Trp Pro Arg Arg Leu
        115                 120                 125

Arg Leu Trp Gly Gly Ala Asp His Ala Pro Glu Gly Phe Asp Pro Thr
    130                 135                 140

Val Thr Val Phe His Val Tyr Asp Ile Leu Glu His Val Glu His Ala
145                 150                 155                 160

Tyr Ser Met Arg Ala Ala Gln Leu His Glu Arg Phe Met Asp Ala Ile
                165                 170                 175

Thr Pro Ala Gly Thr Val Ile Thr Leu Leu Gly Leu Thr Pro Glu Gly
            180                 185                 190

His Arg Val Ala Val His Val Tyr Gly Thr Arg Gln Tyr Phe Tyr Met
        195                 200                 205

Asn Lys Ala Glu Val Asp Arg His Leu Gln Cys Arg Ala Pro Arg Asp
    210                 215                 220

Leu Cys Glu Arg Leu Ala Ala Ala Leu Arg Glu Ser Pro Gly Ala Ser
225                 230                 235                 240

Phe Arg Gly Ile Ser Ala Asp His Phe Glu Ala Glu Val Val Glu Arg
                245                 250                 255

Ala Asp Val Tyr Tyr Tyr Glu Thr Arg Pro Thr Leu Tyr Tyr Arg Val
            260                 265                 270

Phe Val Arg Ser Gly Arg Ala Leu Ala Tyr Leu Cys Asp Asn Phe Cys
        275                 280                 285

Pro Ala Ile Arg Lys Tyr Glu Gly Gly Val Asp Ala Thr Thr Arg Phe
    290                 295                 300

Ile Leu Asp Asn Pro Gly Phe Val Thr Phe Gly Trp Tyr Arg Leu Lys
305                 310                 315                 320

Pro Gly Arg Gly Asn Ala Pro Ala Gln Pro Arg Pro Pro Thr Ala Phe
                325                 330                 335

Gly Thr Ser Ser Asp Val Glu Phe Asn Cys Thr Ala Asp Asn Leu Ala
            340                 345                 350

Val Glu Gly Ala Met Cys Asp Leu Pro Ala Tyr Lys Leu Met Cys Phe
        355                 360                 365

Asp Ile Glu Cys Lys Ala Gly Gly Glu Asp Glu Leu Ala Phe Pro Val
    370                 375                 380

Ala Glu Arg Pro Glu Asp Leu Val Ile Gln Ile Ser Cys Leu Leu Tyr
385                 390                 395                 400

Asp Leu Ser Thr Thr Ala Leu Glu His Ile Leu Leu Phe Ser Leu Gly
                405                 410                 415

Ser Cys Asp Leu Pro Glu Ser His Leu Ser Asp Leu Ala Ser Arg Gly
            420                 425                 430

Leu Pro Ala Pro Val Val Leu Glu Phe Asp Ser Glu Phe Glu Met Leu
        435                 440                 445

Leu Ala Phe Met Thr Phe Val Lys Gln Tyr Gly Pro Glu Phe Val Thr
    450                 455                 460

Gly Tyr Asn Ile Ile Asn Phe Asp Trp Pro Phe Val Leu Thr Lys Leu
465                 470                 475                 480

Thr Glu Ile Tyr Lys Val Pro Leu Asp Gly Tyr Gly Arg Met Asn Gly
                485                 490                 495

Arg Gly Val Phe Arg Val Trp Asp Ile Gly Gln Ser His Phe Gln Lys
            500                 505                 510

Arg Ser Lys Ile Lys Val Asn Gly Met Val Asn Ile Asp Met Tyr Gly
        515                 520                 525

Ile Ile Thr Asp Lys Val Lys Leu Ser Ser Tyr Lys Leu Asn Ala Val
    530                 535                 540

Ala Glu Ala Val Leu Lys Asp Lys Lys Lys Asp Leu Ser Tyr Arg Asp
545                 550                 555                 560

Ile Pro Ala Tyr Tyr Ala Ser Gly Pro Ala Gln Arg Gly Val Ile Gly
                565                 570                 575

Glu Tyr Cys Val Gln Asp Ser Leu Leu Val Gly Gln Leu Phe Phe Lys
            580                 585                 590

Phe Leu Pro His Leu Glu Leu Ser Ala Val Ala Arg Leu Ala Gly Ile
        595                 600                 605

Asn Ile Thr Arg Thr Ile Tyr Asp Gly Gln Gln Ile Arg Val Phe Thr
    610                 615                 620

Cys Leu Leu Arg Leu Ala Gly Gln Lys Gly Phe Ile Leu Pro Asp Thr
625                 630                 635                 640

Gln Gly Arg Phe Arg Gly Leu Asp Lys Glu Ala Pro Lys Arg Pro Ala
                645                 650                 655

Val Pro Arg Gly Glu Gly Glu Arg Pro Gly Asp Gly Asn Gly Asp Glu
            660                 665                 670

Asp Lys Asp Asp Asp Glu Asp Gly Asp Glu Asp Gly Asp Glu Arg Glu
        675                 680                 685

Glu Val Ala Arg Glu Thr Gly Gly Arg His Val Gly Tyr Gln Gly Ala
    690                 695                 700

Arg Val Leu Asp Pro Thr Ser Gly Phe His Val Asp Pro Val Val Val
705                 710                 715                 720

Phe Asp Phe Ala Ser Leu Tyr Pro Ser Ile Ile Gln Ala His Asn Leu
                725                 730                 735

Cys Phe Ser Thr Leu Ser Leu Arg Pro Glu Ala Val Ala His Leu Glu
            740                 745                 750

Ala Asp Arg Asp Tyr Leu Glu Ile Glu Val Gly Gly Arg Arg Leu Phe
        755                 760                 765

Phe Val Lys Ala His Val Arg Glu Ser Leu Leu Ser Ile Leu Leu Arg
    770                 775                 780

Asp Trp Leu Ala Met Arg Lys Gln Ile Arg Ser Arg Ile Pro Gln Ser
785                 790                 795                 800

Thr Pro Glu Glu Ala Val Leu Leu Asp Lys Gln Gln Ala Ala Ile Lys
                805                 810                 815

Val Val Cys Asn Ser Val Tyr Gly Phe Thr Gly Val Gln His Gly Leu
            820                 825                 830

Leu Pro Cys Leu His Val Ala Ala Thr Val Thr Thr Ile Gly Arg Glu
        835                 840                 845

Met Leu Leu Ala Thr Arg Ala Tyr Val His Ala Arg Trp Ala Glu Phe

850                 855                 860

Asp Gln Leu Leu Ala Asp Phe Pro Glu Ala Ala Gly Met Arg Ala Pro
865                 870                 875                 880

Gly Pro Tyr Ser Met Arg Ile Ile Tyr Gly Asp Thr Asp Ser Ile Phe
                885                 890                 895

Val Leu Cys Arg Gly Leu Thr Ala Ala Gly Leu Val Ala Met Gly Asp
            900                 905                 910

Lys Met Ala Ser His Ile Ser Arg Ala Leu Phe Leu Pro Pro Ile Lys
        915                 920                 925

Leu Glu Cys Glu Lys Thr Phe Thr Lys Leu Leu Leu Ile Ala Lys Lys
    930                 935                 940

Lys Tyr Ile Gly Val Ile Cys Gly Gly Lys Met Leu Ile Lys Gly Val
945                 950                 955                 960

Asp Leu Val Arg Lys Asn Asn Cys Ala Phe Ile Asn Arg Thr Ser Arg
                965                 970                 975

Ala Leu Val Asp Leu Leu Phe Tyr Asp Asp Thr Val Ser Gly Ala Ala
            980                 985                 990

Ala Ala Leu Ala Glu Arg Pro Ala  Glu Glu Trp Leu Ala  Arg Pro Leu
        995                 1000                1005

Pro Glu  Gly Leu Gln Ala Phe  Gly Ala Val Leu Val  Asp Ala His
    1010                1015                1020

Arg Arg  Ile Thr Asp Pro Glu  Arg Asp Ile Gln Asp  Phe Val Leu
    1025                1030                1035

Thr Ala  Glu Leu Ser Arg His  Pro Arg Ala Tyr Thr  Asn Lys Arg
    1040                1045                1050

Leu Ala  His Leu Thr Val Tyr  Tyr Lys Leu Met Ala  Arg Arg Ala
    1055                1060                1065

Gln Val  Pro Ser Ile Lys Asp  Arg Ile Pro Tyr Val  Ile Val Ala
    1070                1075                1080

Gln Thr  Arg Glu Val Glu Glu  Thr Val Ala Arg Leu  Ala Ala Leu
    1085                1090                1095

Arg Glu Leu Asp Ala Ala Ala Pro Gly Asp Glu Pro Ala Pro Pro
    1100                1105                1110

Ala Ala Leu Pro Ser Pro Ala Lys Arg Pro Arg Glu Thr Pro Ser
    1115                1120                1125

His Ala Asp Pro Pro Gly Gly Ala Ser Lys Pro Arg Lys Leu Leu
    1130                1135                1140

Val Ser Glu Leu Ala Glu Asp Pro Gly Tyr Ala Ile Ala Arg Gly
    1145                1150                1155

Val Pro Leu Asn Thr Asp Tyr Tyr Phe Ser His Leu Leu Gly Ala
    1160                1165                1170

Ala Cys Val Thr Phe Lys Ala Leu Phe Gly Asn Asn Ala Lys Ile
    1175                1180                1185

Thr Glu Ser Leu Leu Lys Arg Phe Ile Pro Glu Thr Trp His Pro
    1190                1195                1200

Pro Asp Asp Val Ala Ala Arg Leu Arg Ala Ala Gly Phe Gly Pro
    1205                1210                1215

Ala Gly Ala Gly Ala Thr Ala Glu Glu Thr Arg Arg Met Leu His
    1220                1225                1230

Arg Ala Phe Asp Thr Leu Ala
    1235                1240

<210> 31
<211> 881
<212> PRT
<213> Artificial Sequence

<220>
<223> Description of Artificial Sequence: Synthetic
      polypeptide

<400> 31
Met Ala Ala Ser Gly Gly Glu Gly Ser Arg Asp Val Arg Ala Pro Gly
1               5                   10                  15

Pro Pro Pro Gln Gln Pro Gly Ala Arg Pro Ala Val Arg Phe Arg Asp
            20                  25                  30

Glu Ala Phe Leu Asn Phe Thr Ser Met His Gly Val Gln Pro Ile Ile
        35                  40                  45

Ala Arg Ile Arg Glu Leu Ser Gln Gln Gln Leu Asp Val Thr Gln Val

50                  55                  60

Pro Arg Leu Gln Trp Phe Arg Asp Val Ala Ala Leu Glu Val Pro Thr
65                  70                  75                  80

Gly Leu Pro Leu Arg Glu Phe Pro Phe Ala Ala Tyr Leu Ile Thr Gly
                85                  90                  95

Asn Ala Gly Ser Gly Lys Ser Thr Cys Val Gln Thr Leu Asn Glu Val
            100                 105                 110

Leu Asp Cys Val Val Thr Gly Ala Thr Arg Ile Ala Ala Gln Asn Met
        115                 120                 125

Tyr Val Lys Leu Ser Gly Ala Phe Leu Ser Arg Pro Ile Asn Thr Ile
    130                 135                 140

Phe His Glu Phe Gly Phe Arg Gly Asn His Val Gln Ala Gln Leu Gly
145                 150                 155                 160

Gln His Pro Tyr Thr Leu Ala Ser Ser Pro Ala Ser Leu Glu Asp Leu
                165                 170                 175

Gln Arg Arg Asp Leu Thr Tyr Tyr Trp Glu Val Ile Leu Asp Ile Thr
            180                 185                 190

Lys Arg Ala Leu Ala Ala His Gly Gly Glu Asp Ala Arg Asn Glu Phe
        195                 200                 205

His Ala Leu Thr Ala Leu Glu Gln Thr Leu Gly Leu Gly Gln Gly Ala
    210                 215                 220

Leu Thr Arg Leu Ala Ser Val Thr His Gly Ala Leu Pro Ala Phe Thr
225                 230                 235                 240

Arg Ser Asn Ile Ile Val Ile Asp Glu Ala Gly Leu Leu Gly Arg His
                245                 250                 255

Leu Leu Thr Thr Val Val Tyr Cys Trp Trp Met Ile Asn Ala Leu Tyr
            260                 265                 270

His Thr Pro Gln Tyr Ala Gly Arg Leu Arg Pro Val Leu Val Cys Val
        275                 280                 285

Gly Ser Pro Thr Gln Thr Ala Ser Leu Glu Ser Thr Phe Glu His Gln
    290                 295                 300

Lys Leu Arg Cys Ser Val Arg Gln Ser Glu Asn Val Leu Thr Tyr Leu
305                 310                 315                 320

Ile Cys Asn Arg Thr Leu Arg Glu Tyr Thr Arg Leu Ser His Ser Trp
                325                 330                 335

Ala Ile Phe Ile Asn Asn Lys Arg Cys Val Glu His Glu Phe Gly Asn
            340                 345                 350

Leu Met Lys Val Leu Glu Tyr Gly Leu Pro Ile Thr Glu Glu His Met
        355                 360                 365

Gln Phe Val Asp Arg Phe Val Val Pro Glu Ser Tyr Ile Thr Asn Pro
    370                 375                 380

Ala Asn Leu Pro Gly Trp Thr Arg Leu Phe Ser Ser His Lys Glu Val
385                 390                 395                 400

Ser Ala Tyr Met Ala Lys Leu His Ala Tyr Leu Lys Val Thr Arg Glu
                405                 410                 415

Gly Glu Phe Val Val Phe Thr Leu Pro Val Leu Thr Phe Val Ser Val
            420                 425                 430

Lys Glu Phe Asp Glu Tyr Arg Arg Leu Thr Gln Gln Pro Thr Leu Thr
        435                 440                 445

Met Glu Lys Trp Ile Thr Ala Asn Ala Ser Arg Ile Thr Asn Tyr Ser
    450                 455                 460

Gln Ser Gln Asp Gln Asp Ala Gly His Val Arg Cys Glu Val His Ser
465                 470                 475                 480

Lys Gln Gln Leu Val Val Ala Arg Asn Asp Ile Thr Tyr Val Leu Asn
                485                 490                 495

Ser Gln Val Ala Val Thr Ala Arg Leu Arg Lys Met Val Phe Gly Phe
            500                 505                 510

Asp Gly Thr Phe Arg Thr Phe Glu Ala Val Leu Arg Asp Asp Ser Phe
        515                 520                 525

Val Lys Thr Gln Gly Glu Thr Ser Val Glu Phe Ala Tyr Arg Phe Leu
    530                 535                 540

Ser Arg Leu Met Phe Gly Gly Leu Ile His Phe Tyr Asn Phe Leu Gln
545                 550                 555                 560

Arg Pro Gly Leu Asp Ala Thr Gln Arg Thr Leu Ala Tyr Gly Arg Leu
                565                 570                 575

Gly Glu Leu Thr Ala Glu Leu Leu Ser Leu Arg Arg Asp Ala Ala Gly
            580                 585                 590

Ala Ser Ala Thr Arg Ala Ala Asp Thr Ser Asp Arg Ser Pro Gly Glu
        595                 600                 605

Arg Ala Phe Asn Phe Lys His Leu Gly Pro Arg Asp Gly Gly Pro Asp
    610                 615                 620

Asp Phe Pro Asp Asp Asp Leu Asp Val Ile Phe Ala Gly Leu Asp Glu
625                 630                 635                 640

Gln Gln Leu Asp Val Phe Tyr Cys His Tyr Ala Leu Glu Glu Pro Glu
                645                 650                 655

Thr Thr Ala Ala Val His Ala Gln Phe Gly Leu Leu Lys Arg Ala Phe
            660                 665                 670

Leu Gly Arg Tyr Leu Ile Leu Arg Glu Leu Phe Gly Glu Val Phe Glu
        675                 680                 685

Ser Ala Pro Phe Ser Thr Tyr Val Asp Asn Val Ile Phe Arg Gly Cys
    690                 695                 700

Glu Leu Leu Thr Gly Ser Pro Arg Gly Gly Leu Met Ser Val Ala Leu
705                 710                 715                 720

Gln Thr Asp Asn Tyr Thr Leu Met Gly Tyr Thr Tyr Thr Arg Val Phe
                725                 730                 735

Ala Phe Ala Glu Glu Leu Arg Arg Arg His Ala Thr Ala Gly Val Ala
            740                 745                 750

Glu Phe Leu Glu Glu Ser Pro Leu Pro Tyr Ile Val Leu Arg Asp Gln
        755                 760                 765

His Gly Phe Met Ser Val Val Asn Thr Asn Ile Ser Glu Phe Val Glu
    770                 775                 780

Ser Ile Asp Ser Thr Glu Leu Ala Met Ala Ile Asn Ala Asp Tyr Gly
785                 790                 795                 800

Ile Ser Ser Lys Leu Ala Met Thr Ile Thr Arg Ser Gln Gly Leu Ser
                805                 810                 815

Leu Asp Lys Val Ala Ile Cys Phe Thr Pro Gly Asn Leu Arg Leu Asn
            820                 825                 830

Ser Ala Tyr Val Ala Met Ser Arg Thr Thr Ser Ser Glu Phe Leu His
        835                 840                 845

Met Asn Leu Asn Pro Leu Arg Glu Arg His Glu Arg Asp Asp Val Ile
    850                 855                 860

Ser Glu His Ile Leu Ser Ala Leu Arg Asp Pro Asn Val Val Ile Val
865                 870                 875                 880

Tyr

<210> 32
<211> 752
<212> PRT
<213> Artificial Sequence

<220>
<223> Description of Artificial Sequence: Synthetic
      polypeptide

<400> 32
Met Glu Ala Pro Gly Ile Val Trp Val Glu Glu Ser Val Ser Ala Ile
1               5                   10                  15

Thr Leu Tyr Ala Val Trp Leu Pro Pro Arg Thr Arg Asp Cys Leu His
            20                  25                  30

Ala Leu Leu Tyr Leu Val Cys Arg Asp Ala Ala Gly Glu Ala Arg Ala
        35                  40                  45

Arg Phe Ala Glu Val Ser Val Gly Ser Ser Asp Leu Gln Asp Phe Tyr
    50                  55                  60

Gly Ser Pro Asp Val Ser Ala Pro Gly Ala Val Ala Ala Ala Arg Ala
65                  70                  75                  80

Ala Thr Ala Pro Ala Ala Ser Pro Leu Glu Pro Leu Gly Asp Pro Thr
                85                  90                  95

Leu Trp Arg Ala Leu Tyr Ala Cys Val Leu Ala Ala Leu Glu Arg Gln
            100                 105                 110

Thr Gly Arg Trp Ala Leu Phe Val Pro Leu Arg Leu Gly Trp Asp Pro
        115                 120                 125

Gln Thr Gly Leu Val Val Arg Val Glu Arg Ala Ser Trp Gly Pro Pro
        130                 135                 140

Ala Ala Pro Arg Ala Ala Leu Leu Asp Val Glu Ala Lys Val Asp Val
145                 150                 155                 160

Asp Pro Leu Ala Leu Ser Ala Arg Val Ala Glu His Pro Gly Ala Arg
                165                 170                 175

Leu Ala Trp Ala Arg Leu Ala Ala Ile Arg Asp Ser Pro Gln Cys Ala
            180                 185                 190

Ser Ser Ala Ser Leu Ala Val Thr Ile Thr Thr Arg Thr Ala Arg Phe
        195                 200                 205

Ala Arg Glu Tyr Thr Thr Leu Ala Phe Pro Pro Thr Arg Lys Glu Gly
    210                 215                 220

Ala Phe Ala Asp Leu Val Glu Val Cys Glu Val Gly Leu Arg Pro Arg
225                 230                 235                 240

Gly His Pro Gln Arg Val Thr Ala Arg Val Leu Leu Pro Arg Gly Tyr
                245                 250                 255

Asp Tyr Phe Val Ser Ala Gly Asp Gly Phe Ser Ala Pro Ala Leu Val
            260                 265                 270

Ala Leu Phe Arg Gln Trp His Thr Thr Val His Ala Ala Pro Gly Ala
        275                 280                 285

Leu Ala Pro Val Phe Ala Phe Leu Gly Pro Gly Phe Glu Val Arg Gly
    290                 295                 300

Gly Pro Val Gln Tyr Phe Ala Val Leu Gly Phe Pro Gly Trp Pro Thr
305                 310                 315                 320

Phe Thr Val Pro Ala Ala Ala Ala Ala Glu Ser Ala Arg Asp Leu Val
                325                 330                 335

Arg Gly Ala Ala Ala Thr His Ala Ala Cys Leu Gly Ala Trp Pro Ala
            340                 345                 350

Val Gly Ala Arg Val Val Leu Pro Pro Arg Ala Trp Pro Ala Val Ala
        355                 360                 365

Ser Glu Ala Ala Gly Arg Leu Leu Pro Ala Phe Arg Glu Ala Val Ala
    370                 375                 380

Arg Trp His Pro Thr Ala Thr Thr Ile Gln Leu Leu Asp Pro Pro Ala
385                 390                 395                 400

Ala Val Gly Pro Val Trp Thr Ala Arg Phe Cys Phe Ser Gly Leu Gln
                405                 410                 415

Ala Gln Leu Leu Ala Ala Leu Ala Gly Leu Gly Glu Ala Gly Leu Pro
            420                 425                 430

Glu Ala Arg Gly Arg Ala Gly Leu Glu Arg Leu Asp Ala Leu Val Ala
        435                 440                 445

Ala Ala Pro Ser Glu Pro Trp Ala Arg Ala Val Leu Glu Arg Leu Val
    450                 455                 460

Pro Asp Ala Cys Asp Ala Cys Pro Ala Leu Arg Gln Leu Leu Gly Gly
465                 470                 475                 480

Val Met Ala Ala Val Cys Leu Gln Ile Glu Gln Thr Ala Ser Ser Val
                485                 490                 495

Lys Phe Ala Val Cys Gly Gly Thr Gly Ala Ala Phe Trp Gly Leu Phe
            500                 505                 510

Asn Val Asp Pro Gly Asp Ala Asp Ala Ala His Gly Ala Ile Gln Asp
        515                 520                 525

Ala Arg Arg Ala Leu Glu Ala Ser Val Arg Ala Val Leu Ser Ala Asn
    530                 535                 540

Gly Ile Arg Pro Arg Leu Ala Pro Ser Leu Ala Pro Glu Gly Val Tyr
545                 550                 555                 560

Thr His Val Val Thr Trp Ser Gln Thr Gly Ala Trp Phe Trp Asn Ser
                565                 570                 575

Arg Asp Asp Thr Asp Phe Leu Gln Gly Phe Pro Leu Arg Gly Ala Ala
            580                 585                 590

Tyr Ala Ala Ala Ala Glu Val Met Arg Asp Ala Leu Arg Arg Ile Leu
        595                 600                 605

Arg Arg Pro Ala Ala Gly Pro Pro Glu Glu Ala Val Cys Ala Ala Arg
    610                 615                 620

Gly Val Met Glu Asp Ala Cys Asp Arg Phe Val Leu Asp Ala Phe Gly

625                 630                 635                 640

Arg Arg Leu Asp Ala Glu Tyr Trp Ser Val Leu Thr Pro Pro Gly Glu
                645                 650                 655

Ala Asp Asp Pro Leu Pro Gln Thr Ala Phe Arg Gly Gly Ala Leu Leu
            660                 665                 670

Asp Ala Glu Gln Tyr Trp Arg Arg Val Val Arg Val Cys Pro Gly Gly
        675                 680                 685

Gly Glu Ser Val Gly Val Pro Val Asp Leu Tyr Pro Arg Pro Leu Val
    690                 695                 700

Leu Pro Pro Val Asp Cys Ala His His Leu Arg Glu Ile Leu Arg Glu
705                 710                 715                 720

Ile Gln Leu Val Phe Thr Gly Val Leu Glu Gly Val Trp Gly Glu Gly
                725                 730                 735

Gly Ser Phe Val Tyr Pro Phe Asp Glu Lys Ile Arg Phe Leu Phe Pro
            740                 745                 750

<210> 33
<211> 292
<212> PRT
<213> Artificial Sequence

<220>
<223> Description of Artificial Sequence: Synthetic
      polypeptide

<400> 33
Met Asp Gly Ala Val Arg Arg Thr Ala Asp Leu Phe Leu Pro Asp Ser
1               5                   10                  15

Phe Met Gln Glu Ile Ile Gly Gly Gln Ala Arg Glu Thr Gly Asp Asp
            20                  25                  30

Arg Pro Val Leu Thr Lys Ser Ala Gly Glu Arg Phe Leu Leu Tyr Arg
        35                  40                  45

Pro Ser Thr Thr Thr Asn Ser Gly Leu Met Ala Pro Glu Leu Tyr Val
    50                  55                  60

Tyr Val Asp Pro Ala Phe Thr Ala Asn Thr Arg Ala Ser Gly Thr Gly
65                  70                  75                  80

Ile Ala Val Val Gly Arg Tyr Arg Asp Asp Phe Ile Ile Phe Ala Leu
                85                  90                  95

Glu His Phe Phe Leu Arg Ala Leu Thr Gly Ser Ala Pro Ala Asp Ile
            100                 105                 110

Ala Arg Cys Val Val His Ser Leu Ala Gln Val Leu Ala Leu His Pro
        115                 120                 125

Gly Ala Phe Arg Ser Val Arg Val Ala Val Glu Gly Asn Ser Ser Gln
    130                 135                 140

Asp Ser Ala Val Ala Ile Ala Thr His Val His Thr Glu Met His Arg
145                 150                 155                 160

Ile Leu Ala Ser Ala Gly Ala Asn Gly Pro Gly Pro Glu Leu Leu Phe
                165                 170                 175

Tyr His Cys Glu Pro Pro Gly Gly Ala Val Leu Tyr Pro Phe Phe Leu
            180                 185                 190

Leu Asn Lys Gln Lys Thr Pro Ala Phe Glu Tyr Phe Ile Lys Lys Phe
        195                 200                 205

Asn Ser Gly Gly Val Met Ala Ser Gln Glu Leu Val Ser Val Thr Val
    210                 215                 220

Arg Leu Gln Thr Asp Pro Val Glu Tyr Leu Ser Glu Gln Leu Asn Asn
225                 230                 235                 240

Leu Ile Glu Thr Val Ser Pro Asn Thr Asp Val Arg Met Tyr Ser Gly
                245                 250                 255

Lys Arg Asn Gly Ala Ala Asp Asp Leu Met Val Ala Val Ile Met Ala
            260                 265                 270

Ile Tyr Leu Ala Ala Pro Thr Gly Ile Pro Pro Ala Phe Phe Pro Ile
        275                 280                 285

Thr Arg Thr Ser
    290

<210> 34
<211> 598
<212> PRT
<213> Artificial Sequence

<220>
<223> Description of Artificial Sequence: Synthetic
      polypeptide

<400> 34
Met Ala Thr Ser Ala Pro Gly Val Pro Ser Ser Ala Ala Val Arg Glu
1               5                   10                  15

Glu Ser Pro Gly Ser Ser Trp Lys Glu Gly Ala Phe Glu Arg Pro Tyr
            20                  25                  30

Val Ala Phe Asp Pro Asp Leu Leu Ala Leu Asn Glu Ala Leu Cys Ala
        35                  40                  45

Glu Leu Leu Ala Ala Cys His Val Val Gly Val Pro Pro Ala Ser Ala
    50                  55                  60

Leu Asp Glu Asp Val Glu Ser Asp Val Ala Pro Ala Pro Pro Arg Pro
65                  70                  75                  80

Arg Gly Ala Ala Arg Glu Ala Ser Gly Gly Arg Gly Pro Gly Ser Ala
                85                  90                  95

Arg Gly Pro Pro Ala Asp Pro Thr Ala Glu Gly Leu Leu Asp Thr Gly
            100                 105                 110

Pro Phe Ala Ala Ala Ser Val Asp Thr Phe Ala Leu Asp Arg Pro Cys
        115                 120                 125

Leu Val Cys Arg Thr Ile Glu Leu Tyr Lys Gln Ala Tyr Arg Leu Ser
    130                 135                 140

Pro Gln Trp Val Ala Asp Tyr Ala Phe Leu Cys Ala Lys Cys Leu Gly
145                 150                 155                 160

Ala Pro His Cys Ala Ala Ser Ile Phe Val Ala Ala Phe Glu Phe Val
                165                 170                 175

Tyr Val Met Asp His His Phe Leu Arg Thr Lys Lys Ala Thr Leu Val
            180                 185                 190

Gly Ser Phe Ala Arg Phe Ala Leu Thr Ile Asn Asp Ile His Arg His
        195                 200                 205

Phe Phe Leu His Cys Cys Phe Arg Thr Asp Gly Gly Val Pro Gly Arg
    210                 215                 220

His Ala Gln Lys Gln Pro Arg Pro Thr Pro Ser Pro Gly Ala Ala Lys
225                 230                 235                 240

Val Gln Tyr Ser Asn Tyr Ser Phe Leu Ala Gln Ser Ala Thr Arg Ala
                245                 250                 255

Leu Ile Gly Thr Leu Ala Ser Gly Gly Asp Asp Gly Ala Gly Ala Gly
            260                 265                 270

Ala Gly Gly Gly Ser Gly Thr Gln Pro Ser Leu Thr Thr Ala Leu Met
        275                 280                 285

Asn Trp Lys Asp Cys Ala Arg Leu Leu Asp Cys Thr Glu Gly Lys Arg
    290                 295                 300

Gly Gly Gly Asp Ser Cys Cys Thr Arg Ala Ala Ala Arg Asn Gly Glu
305                 310                 315                 320

Phe Glu Ala Ala Ala Gly Ala Leu Ala Gln Gly Gly Glu Pro Glu Thr
                325                 330                 335

Trp Ala Tyr Ala Asp Leu Ile Leu Leu Leu Leu Ala Gly Thr Pro Ala
            340                 345                 350

Val Trp Glu Ser Gly Pro Arg Leu Arg Ala Ala Ala Asp Ala Arg Arg
        355                 360                 365

Ala Ala Val Ser Glu Ser Trp Glu Ala His Arg Gly Ala Arg Met Arg
    370                 375                 380

Asp Ala Ala Pro Arg Phe Ala Gln Phe Ala Glu Pro Gln Pro Gln Pro
385                 390                 395                 400

Asp Leu Asp Leu Gly Pro Leu Met Ala Thr Val Leu Lys His Gly Arg
                405                 410                 415

Gly Arg Gly Arg Thr Gly Gly Glu Cys Leu Leu Cys Asn Leu Leu Leu
            420                 425                 430

Val Arg Ala Tyr Trp Leu Ala Met Arg Arg Leu Arg Ala Ser Val Val
        435                 440                 445

Arg Tyr Ser Glu Asn Asn Thr Ser Leu Phe Asp Cys Ile Val Pro Val
    450                 455                 460

Val Asp Gln Leu Glu Ala Asp Pro Glu Ala Gln Pro Gly Asp Gly Gly
465                 470                 475                 480

Arg Phe Val Ser Leu Leu Arg Ala Ala Gly Pro Glu Ala Ile Phe Lys
                485                 490                 495

His Met Phe Cys Asp Pro Met Cys Ala Ile Thr Glu Met Glu Val Asp

500                 505                 510

Pro Trp Val Leu Phe Gly His Pro Arg Ala Asp His Arg Asp Glu Leu
        515                 520                 525

Gln Leu His Lys Ala Lys Leu Ala Cys Gly Asn Glu Phe Glu Gly Arg
    530                 535                 540

Val Cys Ile Ala Leu Arg Ala Leu Ile Tyr Thr Phe Lys Thr Tyr Gln
545                 550                 555                 560

Val Phe Val Pro Lys Pro Thr Ala Leu Ala Thr Phe Val Arg Glu Ala
                565                 570                 575

Gly Ala Leu Leu Arg Arg His Ser Ile Ser Leu Leu Ser Leu Glu His
            580                 585                 590

Thr Leu Cys Thr Tyr Val
        595

<210> 35
<211> 327
<212> PRT
<213> Artificial Sequence

<220>
<223> Description of Artificial Sequence: Synthetic
      polypeptide

<400> 35
Met Glu Tyr Asp Ser Phe Asp Ala Leu Leu Ala Ala Arg Leu Glu Ser
1               5                   10                  15

Gly Gln Thr Leu Gly Pro Ala Gly Gly Arg Glu Ala Ser Leu Pro Glu
            20                  25                  30

Ala Pro His Ala Leu Tyr Arg Pro Thr Gly Gln His Val Ala Val Leu
        35                  40                  45

Ala Ala Ala Thr His Arg Thr Pro Ala Ala Arg Val Thr Ala Met Asp
    50                  55                  60

Leu Val Leu Ala Ala Val Leu Leu Gly Ala Pro Val Val Val Ala Leu
65                  70                  75                  80

Arg Asn Thr Thr Ala Phe Ser Arg Glu Ser Glu Leu Glu Leu Cys Leu
                85                  90                  95

Thr Leu Phe Asp Ser Arg Pro Gly Gly Pro Asp Ala Ala Leu Arg Asp
            100                 105                 110

Val Val Ser Ser Asp Ile Glu Thr Trp Ala Val Gly Leu Leu His Thr
        115                 120                 125

Asp Leu Asn Pro Ile Glu Asn Ala Cys Leu Ala Ala Gln Leu Pro Arg
    130                 135                 140

Leu Ser Ala Leu Ile Ala Glu Arg Pro Leu Ala Asp Gly Pro Pro Cys
145                 150                 155                 160

Leu Val Leu Val Asp Ile Ser Met Thr Pro Val Ala Val Leu Trp Glu
                165                 170                 175

Ala Pro Glu Pro Pro Gly Pro Pro Asp Val Arg Phe Val Gly Ser Glu
            180                 185                 190

Ala Thr Glu Glu Leu Pro Phe Val Ala Thr Ala Gly Asp Val Leu Ala
        195                 200                 205

Ala Ser Ala Ala Asp Ala Asp Pro Phe Phe Ala Arg Ala Ile Leu Gly
    210                 215                 220

Arg Pro Phe Asp Ala Ser Leu Leu Thr Gly Glu Leu Phe Pro Gly His
225                 230                 235                 240

Pro Val Tyr Gln Arg Pro Leu Ala Asp Glu Ala Gly Pro Ser Ala Pro
                245                 250                 255

Thr Ala Ala Arg Asp Pro Arg Asp Leu Ala Gly Gly Asp Gly Gly Ser
            260                 265                 270

Gly Pro Glu Asp Pro Ala Ala Pro Pro Ala Arg Gln Ala Asp Pro Gly
        275                 280                 285

Val Leu Ala Pro Thr Leu Leu Thr Asp Ala Thr Thr Gly Glu Pro Val
    290                 295                 300

Pro Pro Arg Met Trp Ala Trp Ile His Gly Leu Glu Glu Leu Ala Ser
305                 310                 315                 320

Asp Asp Ala Gly Gly Pro Thr
                325

<210> 36
<211> 512
<212> PRT
<213> Artificial Sequence

<220>
<223> Description of Artificial Sequence: Synthetic
      polypeptide

<400> 36
Met Ala Thr Asp Ile Asp Met Leu Ile Asp Leu Gly Leu Asp Leu Ser
1               5                   10                  15

Asp Ser Glu Leu Glu Glu Asp Ala Leu Glu Arg Asp Glu Glu Gly Arg
            20                  25                  30

Arg Asp Asp Pro Glu Ser Asp Ser Ser Gly Glu Cys Ser Ser Ser Asp
        35                  40                  45

Glu Asp Met Glu Asp Pro Cys Gly Asp Gly Gly Ala Glu Ala Ile Asp
    50                  55                  60

Ala Ala Ile Pro Lys Gly Pro Pro Ala Arg Pro Glu Asp Ala Gly Thr
65                  70                  75                  80

Pro Glu Ala Ser Thr Pro Arg Pro Ala Ala Arg Arg Gly Ala Asp Asp
                85                  90                  95

Pro Pro Pro Ala Thr Thr Gly Val Trp Ser Arg Leu Gly Thr Arg Arg
            100                 105                 110

Ser Ala Ser Pro Arg Glu Pro His Gly Gly Lys Val Ala Arg Ile Gln
        115                 120                 125

Pro Pro Ser Thr Lys Ala Pro His Pro Arg Gly Gly Arg Arg Gly Arg
    130                 135                 140

Arg Arg Gly Arg Gly Arg Tyr Gly Pro Gly Gly Ala Asp Ser Thr Pro
145                 150                 155                 160

Lys Pro Arg Arg Arg Val Ser Arg Asn Ala His Asn Gln Gly Gly Arg
                165                 170                 175

His Pro Ala Ser Ala Arg Thr Asp Gly Pro Gly Ala Thr His Gly Glu
            180                 185                 190

Ala Arg Arg Gly Gly Glu Gln Leu Asp Val Ser Gly Gly Pro Arg Pro
        195                 200                 205

Arg Gly Thr Arg Gln Ala Pro Pro Pro Leu Met Ala Leu Ser Leu Thr
    210                 215                 220

Pro Pro His Ala Asp Gly Arg Ala Pro Val Pro Glu Arg Lys Ala Pro
225                 230                 235                 240

Ser Ala Asp Thr Ile Asp Pro Ala Val Arg Ala Val Leu Arg Ser Ile
                245                 250                 255

Ser Glu Arg Ala Ala Val Glu Arg Ile Ser Glu Ser Phe Gly Arg Ser
            260                 265                 270

Ala Leu Val Met Gln Asp Pro Phe Gly Gly Met Pro Phe Pro Ala Ala
        275                 280                 285

Asn Ser Pro Trp Ala Pro Val Leu Ala Thr Gln Ala Gly Gly Phe Asp
    290                 295                 300

Ala Glu Thr Arg Arg Val Ser Trp Glu Thr Leu Val Ala His Gly Pro
305                 310                 315                 320

Ser Leu Tyr Arg Thr Phe Ala Ala Asn Pro Arg Ala Ala Ser Thr Ala
                325                 330                 335

Lys Ala Met Arg Asp Cys Val Leu Arg Gln Glu Asn Leu Ile Glu Ala
            340                 345                 350

Leu Ala Ser Ala Asp Glu Thr Leu Ala Trp Cys Lys Met Cys Ile His
        355                 360                 365

His Asn Leu Pro Leu Arg Pro Gln Asp Pro Ile Ile Gly Thr Ala Ala
    370                 375                 380

Ala Val Leu Glu Asn Leu Ala Thr Arg Leu Arg Pro Phe Leu Gln Cys
385                 390                 395                 400

Tyr Leu Lys Ala Arg Gly Leu Cys Gly Leu Asp Asp Leu Cys Ser Arg
                405                 410                 415

Arg Arg Leu Ser Asp Ile Lys Asp Ile Ala Ser Phe Val Leu Val Ile
            420                 425                 430

Leu Ala Arg Leu Ala Asn Arg Val Glu Arg Gly Val Ser Glu Ile Asp
        435                 440                 445

Tyr Thr Thr Val Gly Val Gly Ala Gly Glu Thr Met His Phe Tyr Ile
    450                 455                 460

Pro Gly Ala Cys Met Ala Gly Leu Ile Glu Ile Leu Asp Thr His Arg
465                 470                 475                 480

Gln Glu Cys Ser Ser Arg Val Cys Glu Leu Thr Ala Ser His Thr Ile

                485                 490                 495

Ala Pro Leu Tyr Val His Gly Lys Tyr Phe Tyr Cys Asn Ser Leu Phe
            500                 505                 510

<210> 37
<211> 87
<212> PRT
<213> Human herpesvirus 2

<400> 37
Met Thr Gly Lys Pro Ala Arg Leu Gly Arg Trp Val Val Leu Leu Phe
1               5                   10                  15

Val Ala Leu Val Ala Gly Val Pro Gly Glu Pro Pro Asn Ala Ala Gly
            20                  25                  30

Ala Arg Gly Val Ile Gly Asp Ala Gln Cys Arg Gly Asp Ser Ala Gly
        35                  40                  45

Val Val Ser Val Pro Gly Val Leu Val Pro Phe Tyr Leu Gly Met Thr
    50                  55                  60

Ser Met Gly Val Cys Met Ile Ala His Val Tyr Gln Ile Cys Gln Arg
65                  70                  75                  80

Ala Leu Ala Ala Gly Ser Ala
                85

<210> 38
<211> 302
<212> PRT
<213> Human herpesvirus 2

<400> 38
Asn Arg Trp Gly Ser Gly Val Pro Gly Pro Ile Asn Pro Pro Asn Ser
1               5                   10                  15

Asp Val Val Phe Pro Gly Gly Ser Pro Val Ala Gln Tyr Cys Tyr Ala
            20                  25                  30

Tyr Pro Arg Leu Asp Asp Pro Gly Pro Leu Gly Ser Ala Asp Ala Gly
        35                  40                  45

Arg Gln Asp Leu Pro Arg Arg Val Val Arg His Glu Pro Leu Gly Arg
    50                  55                  60

Ser Phe Leu Thr Gly Gly Leu Val Leu Leu Ala Pro Pro Val Arg Gly
65                  70                  75                  80

Phe Gly Ala Pro Asn Ala Thr Tyr Ala Ala Arg Val Thr Tyr Tyr Arg
                85                  90                  95

Leu Thr Arg Ala Cys Arg Gln Pro Ile Leu Leu Arg Gln Tyr Gly Gly
            100                 105                 110

Cys Arg Gly Gly Glu Pro Pro Ser Pro Lys Thr Cys Gly Ser Tyr Thr
        115                 120                 125

Tyr Thr Tyr Gln Gly Gly Gly Pro Pro Thr Arg Tyr Ala Leu Val Asn
    130                 135                 140

Ala Ser Leu Leu Val Pro Ile Trp Asp Arg Ala Ala Glu Thr Phe Glu
145                 150                 155                 160

Tyr Gln Ile Glu Leu Gly Gly Glu Leu His Val Gly Leu Leu Trp Val
                165                 170                 175

Glu Val Gly Gly Glu Gly Pro Gly Pro Thr Ala Pro Pro Gln Ala Ala
            180                 185                 190

Arg Ala Glu Gly Gly Pro Cys Val Pro Pro Val Pro Ala Gly Arg Pro
        195                 200                 205

Trp Arg Ser Val Pro Pro Val Trp Tyr Ser Ala Pro Asn Pro Gly Phe
    210                 215                 220

Arg Gly Leu Arg Phe Arg Glu Arg Cys Leu Pro Pro Gln Thr Pro Ala
225                 230                 235                 240

Ala Pro Ser Asp Leu Pro Arg Val Ala Phe Ala Pro Gln Ser Leu Leu
                245                 250                 255

Val Gly Ile Thr Gly Arg Thr Phe Ile Arg Met Ala Arg Pro Thr Glu
            260                 265                 270

Asp Val Gly Val Leu Pro Pro His Trp Ala Pro Gly Ala Leu Asp Asp
        275                 280                 285

Gly Pro Tyr Ala Pro Phe Pro Pro Arg Pro Arg Phe Arg Arg
    290                 295                 300

<210> 39
<211> 3987
<212> DNA
<213> Human herpesvirus 2

<400> 39
atgtcgtact accatcacca tcaccatcac agtgccgaac agcgtaaaaa gaaaaaaacc      60

accaccacga cccaaggacg tggagctgaa gttgctatgg cggatgagga tggaggccgc 120

ttgagagctg ctgctgagac tactggagga cctggatcac cggaccctgc cgatggaccc 180

ccccctacac caaaccccga tcgtagaccg gctgctagac ctggattcgg atggcatgga 240

ggacccgagg aaaacgagga cgaggcggac gacgccgctg ccgacgccga cgccgatgag 300

gctgcccctg cttctggaga ggcggtagac gaacctgctg ccgatggagt tgttagccct 360

aggcaattgg ctttgttggc gagcatggta gacgaggctg tgagaacaat cccttcccct 420

ccccctgaac gtgatggagc acaagaggag gcggctagga gtccctcacc accccgtaca 480

ccttctatga gagcggatta cggcgaggaa aacgacgacg acgacgatga tgatgacgac 540

gatgatcgtg atgccggacg ctgggttagg ggacctgaaa ccacttctgc tgtccgtgga 600

gcataccccg atcctatggc gagtttgagc cctagaccac ctgccccgag gagacaccac 660

caccaccacc atcataggcg tagacgtgct cctagacgtc gttctgccgc tagtgactct 720

tccaaatctg gctcttcttc atctgcctct tccgcttcat cttcggcctc atcgtcctct 780

tcggcatccg cttcgagtag tgatgatgat gatgacgacg acgctgctag agcccccgct 840

tctgctgccg accacgctgc tggcggaact ttgggagccg acgacgagga ggcgggagtt 900

cctgctcgtg ccccgggagc tgctccgagg ccttctccac cccgtgctga acctgctccg 960

gctagaacac cggccgctac tgctggtaga ctggagcgta gacgtgcccg tgctgctgtg 1020

gctggtagag atgctactgg ccgcttcact gctggccgtc ctagacgtgt tgaactggac 1080

gccgatgctg cttctggtgc tttctacgcc cgttaccgtg atggttacgt gtctggtgaa 1140

ccttggcctg gcgctggtcc acctccgccc ggacgtgtac tctacggtgg attgggcgat 1200

tctcgccctg gtctgtgggg cgctccggag gctgaggagg ctagagcccg tttcgaggct 1260

tctggtgccc ctgctcctgt ttgggctcct gaattgggcg acgctgctca acaatacgcc 1320

ctcatcacac gcttgctgta cactcccgac gccgaggcta tgggatggct ccaaaaccct 1380

agagttgccc ctggtgatgt tgctctggat caggcttgtt tccgtatctc cggcgctgct 1440

cgtaactctt cttcgttcat ctccggttct gtggctagag ctgtgcctca cttgggatac 1500

gccatggccg ctggacgttt cggctgggga ctggctcatg ttgctgccgc tgtagcaatg 1560

tctagacgct acgaccgtgc tcaaaaagga ttcttgctca cgtcactgag gcgtgcttac 1620

gcccctttgt tggcccgtga aaacgctgcc ctcactggcg cccgtacccc cgatgacggt 1680

ggcgacgcca accgccacga tggtgatgat gctagaggca aacccgctgc cgctgctgct 1740

cctttgccct ctgccgccgc ttcccctgcc gatgaacgtg ctgttcctgc cggttacggt 1800

gccgctggtg tgttggctgc tttgggacgc ttgagtgctg ccccggctag tgcccccgct 1860

ggtgccgatg acgatgacga tgacgatggt gctggcggag gcggtggcgg tagacgtgct 1920

gaggctggac gtgttgctgt tgaatgcctg gctgcctgta gaggaatctt ggaggctctg 1980

gccgagggat tcgacggaga cttggcggct gtaccgggac tggcgggagc gaggcctgcc 2040

gctccacctc gccccggtcc tgctggtgct gccgctcctc ctcatgccga cgctcctaga 2100

ctccgtgctt ggctccgtga actccgtttc gttcgtgacg ctttggttct gatgagactg 2160

agaggcgact tgagagtggc tggaggatcc gaggctgctg ttgctgctgt ccgtgctgtt 2220

tctttggttg ctggtgcttt gggccctgct ttgccgagat ctccccgttt gttgtcgagt 2280

gccgccgctg ctgccgccga tttgttgttc caaaaccaat ccctccgccc tctgctcgcc 2340

gacactgttg ccgctgccga ttctctggct gctccggctt ctgccccacg tgaagctcgt 2400

aaacgtaaat cacccgctcc ggctcgtgct ccccctggtg gcgcccctag accccctaaa 2460

aaatcccgtg ccgatgcccc tagacctgct gctgctcccc ccgctggtgc tgctcccccc 2520

gctcccccta ctcccccccc acgcccacct cgtcccgctg ccctcacacg ccgtcctgct 2580

gagggacccg atccacaagg cggctggcgt agacaacctc ctggcccatc ccatacaccg 2640

gcaccatctg ccgctgcttt ggaggcttac tgtgctcctc gtgctgtggc tgaactcacc 2700

gatcatccgc tgttccctgc tccctggcgt cccgccctca tgttcgatcc tagagctttg 2760

gcttccttgg ccgctcgttg tgctgcccct ccccctggcg gtgctccggc tgctttcggt 2820

cctctccgtg cctctggtcc actccgccgt gccgctgcct ggatgagaca agttcccgac 2880

cctgaggatg ttagagttgt gatcttgtac tcgcccttgc ctggcgagga tttggccgct 2940

ggtagagctg gcggtggccc ccctcctgaa tggtctgctg aacgtggtgg tttgtcttgc 3000

ttgttggccg ccctgggaaa ccgtctgtgt ggtcctgcta ctgctgcttg ggctggaaac 3060

tggactggcg ctcccgatgt ttctgctctc ggtgctcaag gagttttgct gctctctact 3120

cgtgacttgg cattcgctgg agctgttgaa ttcctgggac tcttggctgg cgcttgtgat 3180

aggagactca tcgtcgtaaa cgctgtgaga gctgccgatt ggcctgccga tggtcctgtt 3240

gtgtctcgtc aacacgctta cttggcttgt gaagtgttgc ccgctgtcca atgtgctgtt 3300

cgctggcctg ctgctcgtga tctgaggcgt actgttctgg ctagtggtcg tgttttcgga 3360

cctggtgttt tcgctcgtgt cgaagctgct cacgctagac tgtaccccga tgccccaccc 3420

ctccgtttgt gtcgtggagc aaacgttcgc taccgtgtcc gtactcgttt cggacccgat 3480

actctggttc caatgtcccc tcgtgaatac cgtcgtgctg ttctgcctgc cctcgatgga 3540

cgtgctgccg cttctggcgc tggtgacgct atggctcctg gcgctccgga cttctgtgag 3600

gatgaggctc actcacatcg tgcctgtgcc cgctggggac tgggcgctcc attgaggcct 3660

gtatacgtgg cactgggccg tgatgctgtt agaggcggac ccgctgaatt gagaggccct 3720

cgtcgtgaat tctgtgctag ggctctgctc gaacccgatg gagatgctcc tcctttggta 3780

ctccgtgacg acgccgatgc tggtcctccc ccacaaattc gctgggctag tgctgctgga 3840

cgtgctggta ctgtattggc tgctgctggc ggtggcgttg aagttgttgg tactgccgct 3900

ggactcgcta cacctccccg ccgtgaacct gtagacatgg atgctgaact cgaggatgat 3960

gacgacggat tgttcggaga gtaatag 3987

<210> 40
<211> 1128
<212> DNA
<213> Artificial Sequence

<220>
<223> Description of Artificial Sequence: Synthetic
      polynucleotide

<400> 40
atgaagttcc tcgtgaacgt ggccctggtg ttcatggtgg tgtacatcag ctacatctac 60

gccaaccgtt ggaagtacgc tctggctgac ccatccctga agatggctga ccccaaccgt 120

ttccgtggca agaacctgcc cgtgctggac cagctgaccg acccccctgg cgtgaagcgt 180

gtgtaccaca tccagccatc cctcgaagac cccttccagc ccccctccat ccccatcacc 240

gtgtactacg ctgtgctgga acgcgcttgc cgttccgtgc tgctgcacgc tccttccgag 300

gctccccaga tcgtgcgtgg tgcttccgac gaggctcgca agcacaccta caacctgact 360

atcgcttggt acaggatggg tgacaactgc gctatcccta tcaccgtcat ggaatacacc 420

gagtgcccct acaacaagtc cctgggcgtg tgccctatcc gtacccagcc ccgttggtcc 480

tactacgact ccttcagcgc tgtgtccgag gacaacctgg gtttcctgat gcacgctccc 540

gctttcgaga ctgctggcac ctacctgcgt ctggtcaaga tcaacgactg gaccgagatc 600

acccagttca tcctggaaca ccgtgctcgt gcttcgtgca agtacgccct gcccctgcgt 660

atccctcctg ctgcttgcct gacctccaag gcttaccagc agggcgtgac cgtggactcc 720

atcggcatgc tgccccgttt catccccgag aaccagcgta ccgtggctct gtactctctg 780

aagatcgctg gctggcacgg tcctaagccc ccctacacct ccactctgct gccccctgag 840

ctgtccgaca ccaccaacgc tactcagccc gagttggtgc ctgaggaccc cgaggactcc 900

gctctgttgg aggaccccgc tggaaccgtg tcctcccaga tcccccccaa ctggcacatc 960

ccttccatcc aggacgtggc ccctcaccac gctccagctg ctccctccaa cccccgtcgt 1020

cgtgctcaga tggctcccaa gcgtctgcgt ctgccccaca tccgtgacga cgacgctcct 1080

ccatcccacc agcccctgtt ctaccaccac caccatcacc actaataa 1128

<210> 41
<211> 783
<212> DNA
<213> Human herpesvirus 2

<400> 41

atgtctcgtc gtcgtggtcc tcgtcgtcgt ggtcctcgtc gtcgtccgcg tccgggtgcg 60

ccggcggtac cacgcccggg tgcgccggca gtgccgcgtc caggcgcact gcctaccgcg 120

gactctcaaa tggtgccggc gtatgattct ggtactgccg tcgaatctgc tccggcagcg 180

agctccctgc tgcgtcgttg gctgctggtc cctcaggcgg acgattccga tgacgcagac 240

tacgcgggca acgacgacgc ggagtgggct aacagcccgc caagcgaggg tggtggcaaa 300

gcgccggagg ctccgcacgc agcgcctgcc gcagcgtgcc cgcctccgcc tcctcgtaaa 360

gaacgtggcc ctcaacgtcc tctgccgccg cacctggctc tgcgtctgcg tactaccact 420

gagtacctgg cgcgtctgtc tctgcgtcgt cgccgtccgc cggctagccc gccggccgat 480

gcaccgcgtg gcaaagtgtg cttctctcca cgtgttcaag ttcgtcacct ggtggcttgg 540

gaaacggctg cccgtctggc tcgccgtggc agctgggcac gtgagcgcgc agaccgtgac 600

cgcttccgtc gccgtgtggc ggctgctgaa gccgttatcg gcccgtgcct ggaacctgag 660

gctcgcgctc gcgcgcgtgc gcgcgctcgt gcccacgaag atggcggtcc agcagaggaa 720

gaagaggcag ctgcagcagc gcgcggtagc tccgcggctg cgggtccagg tcgtcgtgcc 780

gta 783

<210> 42
<211> 2523
<212> DNA
<213> Human herpesvirus 2

<400> 42
atgtcgtact accatcacca tcaccatcac atggagccac gtcctggtac ttcttctcgc 60

gctgatcctg gtcctgaacg tccgccacgc cagactccgg gcacccagcc ggccgcccct 120

cacgcttggg gcatgctgaa cgatatgcag tggctggcgt cctctgattc cgaagaggag 180

actgaggttg gtatcagcga tgatgatctg caccgcgact ctaccagcga agcaggttcc 240

actgacaccg aaatgtttga agcgggcctg atggatgccg cgaccccgcc ggctcgtccg 300

ccggctgaac gtcagggtag ccctacgcct gcggatgcgc aaggctcttg tggtggtggt 360

ccagtaggcg aagaggaggc tgaggccggt ggcggcggtg atgtgtgtgc ggtttgtacc 420

gatgaaatcg caccgccgct gcgttgtcag tctttcccgt gcctgcaccc gttttgcatt 480

ccgtgcatga aaacctggat cccgctgcgc aacacttgcc cgctgtgcaa cactccggtt 540

gcttatctga tcgttggtgt aaccgcatct ggttcctttt ctaccatccc gattgtcaac 600

gacccacgta cgcgtgttga ggcggaggcg gctgtacgtg cgggcaccgc ggtggacttt 660

atctggaccg gtaacccgcg caccgcgcca cgctccctgt ctctgggtgg ccataccgtt 720

cgtgctctga gcccgacccc accttggcca ggcaccgatg acgaagacga cgatctggct 780

gacgttgact atgttccgcc ggcaccgcgt cgcgcaccac gccgtggtgg cggtggcgcc 840

ggtgcgacgc gcggtacctc ccagccggca gcaactcgcc cagcaccgcc gggtgccccg 900

cgttctagca gctccggtgg cgcaccgctg cgtgctggcg tgggttctgg ttccggtggt 960

ggtccggccg tggcggctgt cgtcccgcgt gtggcttctc tgccaccggc agctggtggc 1020

ggtcgtgctc aagctcgtcg tgtcggcgag gacgcagcgg ctgctgaggg ccgtactcca 1080

ccggcccgtc aaccgcgcgc agcacaggaa ccgccgatcg tgatctccga ttccccgcca 1140

ccgagcccgc gtcgcccggc gggtccgggt ccgctgtctt ttgtatcctc cagctctgct 1200

caggtaagca gcggtcctgg cggtggcggc ctgccacagt cctctggtcg tgctgctcgt 1260

cctcgtgcgg cggttgctcc tcgtgtacgt tctccgccac gcgctgctgc cgcgccggtc 1320

gtttctgcct ctgctgacgc ggcaggtccg gctccgcctg cagttccggt tgatgcacac 1380

cgtgcaccgc gctctcgtat gacccaggcg cagactgata cccaggcaca atccctgggt 1440

cgcgcgggtg cgactgacgc tcgtggtagc ggtggtccgg gcgctgaagg tggcccgggt 1500

gttccacgcg gtactaacac tccgggcgct gcgccacacg cggctgaagg tgcggctgca 1560

cgtccgcgta aacgtcgtgg ttccgacagc ggtccggctg caagcagcag cgcgagctct 1620

tccgctgcgc ctcgcagccc gctggcgccg cagggtgttg gcgccaagcg tgctgctccg 1680

cgtcgtgcac cggactccga ttctggcgac cgcggtcacg gcccgctggc ccctgctagc 1740

gcaggcgctg cgccgccatc cgccagcccg tcttctcagg cagctgtggc tgcggcgtcc 1800

tcttcttccg ctagcagctc ttccgcctct tctagcagcg cgtcctctag cagcgcatct 1860

tcctcttctg cttcttcttc tagcgcttct agctcttccg cgtcctcttc cgctggcggt 1920

gcaggcggct ctgttgcttc cgccagcggc gcaggtgagc gtcgtgaaac gagcctgggc 1980

ccacgtgctg ctgcaccgcg tggcccgcgt aagtgtgcgc gcaagacccg ccacgctgaa 2040

ggcggtccgg agccgggtgc gcgtgatccg gctccgggtc tgacccgtta cctgccgatt 2100

gcgggtgtgt cctccgttgt ggcactggcg ccgtatgtga acaaaactgt cacgggcgat 2160

tgcctgcctg ttctggacat ggaaaccggt catatcggcg cttacgtcgt tctggttgac 2220

caaaccggca acgtggcgga tctgctgcgt gcggccgctc cggcttggtc ccgtcgtacc 2280

ctgctgccgg aacatgctcg caactgtgta cgcccaccgg attacccaac cccgccggcc 2340

tccgagtgga actccctgtg gatgaccccg gttggtaaca tgctgttcga ccagggcacg 2400

ctggttggtg ctctggactt tcacggcctg cgctcccgtc acccgtggtc ccgtgagcaa 2460

ggcgctccgg cccctgcggg cgatgccccg gctggccacg gcgagagtac tagaggatca 2520

taa 2523

<210> 43
<211> 2928
<212> DNA
<213> Artificial Sequence

<220>
<223> Description of Artificial Sequence: Synthetic
      polynucleotide

<400> 43
atgtcgtact accatcacca tcaccatcac gccgctcaac gtgctagggg atcctctgaa      60

cgctgggctg ctggtgtcga ggctgctttg gatagagtgg agaaccgtgc cgaattcgat     120

gttgtcgagc tgaggagact ccaagctttg gctggtactc acggctacaa ccctcgtgat     180

ttccgtaaac gtgccgaaca ggctttggcg gcaaacgctg aggccgtaac attggctctg     240

gacactgcct tcgctttcaa cccatacacg cccgaaaacc aacgtcatcc tatgctccca     300

cctctcgctg ctattcaccg cctgggatgg agcgctgctt tccatgctgc tgctgaaact     360

tacgccgaca tgttccgtgt cgatgccgaa ccactggcta gactgctccg tatcgctgag     420

ggactgctgg agatggctca agctggcgac ggattcatcg attaccatga ggctgtcggt     480

agactggccg atgatatgac ttctgtgccc ggattgaggc gctacgttcc tttcttccaa     540

catggctacg ccgattacgt ggaactgaga gatcgcctgg atgctattag ggccgacgtc     600

catagagcac tcggtggtgt tccgctggat ttggcggctg ctgccgaaca aatttccgct     660

gctcgtaacg atcctgaggc tactgctgaa ttggtccgta ctggtgtaac attgccttgc     720

cctagtgagg acgctctcgt ggcttgtgct gctgccctgg agagagtcga tcaatctccc     780

gtgaaaaaca cggcttacgc cgaatacgtt gccttcgtga cccgtcaaga cactgctgag     840

actaaagacg ctgtggtccg tgctaaacaa caacgtgctg aggccactga acgtgttatg     900

gctggcctga gagaggctct ggctgctaga gaacgtcgtg ctcaaattga ggctgaggga     960

ttggcaaacc tgaaaaccat gctcaaagtc gtggctgtac ccgctactgt tgctaaaact    1020

ctcgaccagg ctcgtagtgt tgccgaaatt gccgatcaag tcgaagtgtt gctggatcaa    1080

accgaaaaaa ctcgtgaact ggatgtgcct gctgtgatct ggctcgaaca cgcccaaaga    1140

acattcgaga cacacccttt gtctgccgct cgtggtgatg gtcctggacc cttggctcgt    1200

catgctggcc gcctcggtgc cctcttcgat actcgtcgta gagtagacgc cttgaggaga    1260

tccctggagg aggctgaggc tgaatgggac gaagtttggg gacgcttcgg tagagtgagg    1320

ggcggagcgt ggaaatctcc ggagggattc cgtgcaatgc atgagcaact gagggccctc    1380

caagacacaa caaacaccgt gtctggcctg agggctcaac ctgcttacga acgcttgtct    1440

gctcgctacc aaggagtact cggagcgaaa ggcgctgaga gagctgaggc tgttgaggaa    1500

ctcggtgctc gtgtcactaa acacaccgct ctgtgtgcta ggctgagaga tgaggtcgtc    1560

cgtagagtgc cttgggaaat gaacttcgat gctctgggag gattgttggc tgagttcgat    1620

gccgctgctg ccgatttggc accttgggct gtagaggaat tccgtggtgc tagagaactc    1680

attcaatacc gtatgggcct gtactctgcc tacgctagag ctggaggaca aactggtgct    1740

ggagctgaat ctgctcctgc tcctttgctc gtggatctga gggctttgga tgctcgtgct 1800

cgtgcttctt cttcccctga gggacatgaa gtggacccac aactgctgag gaggcgtgga 1860

gaggcttact tgagagctgg cggcgaccct ggacctctcg tgctccgtga agctgtttct 1920

gctttggacc tgccattcgc cacatctttc ttggcccccg atggaactcc cctccaatac 1980

gctttgtgct tccctgccgt aacggacaaa ctcggagctt tgctcatgag gcccgaggcc 2040

gcttgtgtta gacctccttt gcctaccgat gtgctggaat ctgccccaac tgtgactgcc 2100

atgtacgtac tcactgtggt caaccgcctc caactggcat tgagtgatgc tcaagcggca 2160

aacttccaac tgttcggtcg tttcgttcgt cataggcagg caacctgggg agcgtcaatg 2220

gatgccgccg ctgaattgta cgttgccctg gtggctacaa ctctcacacg tgaattcggt 2280

tgtcgctggg cacaattggg atgggctagt ggagctgctg ctcctagacc cccacctgga 2340

ccccgtggct cacaacgtca ctgtgtggca ttcaacgaga acgatgtcct cgtcgctttg 2400

gttgccggtg ttcccgaaca catctacaac ttctggcgcc tggacttggt ccgtcaacac 2460

gagtacatgc acctcacact ggagcgtgcc ttcgaggatg ctgccgagtc tatgctcttc 2520

gttcaacgcc tcactccaca tcccgacgct cgtattagag ttctgccgac cttcttggat 2580

ggtggtcctc ctacacgtgg tctgttgttc ggaacccgct tggcggactg gcgtcgtggt 2640

aaactgtctg aaaccgaccc attggcccca tggagatctg ctttggaact cggaacccaa 2700

cgtcgtgacg tgcctgcttt gggaaaactg tcccctgctc aagctttggc cgctgtgtcg 2760

gtactgggcc gtatgtgctt gccctcggct gccttggctg ctttgtggac ctgtatgttc 2820

cccgacgact acactgaata cgactcattc gacgccctct tggcggctcg cctggaatcg 2880

ggacaaacat tgggacctgc tggcggtaga gaggcttcat tgtaatag 2928

<210> 44
<211> 2646
<212> DNA
<213> Artificial Sequence

<220>
<223> Description of Artificial Sequence: Synthetic
      polynucleotide

<400> 44
   atgtcgtact accatcacca tcaccatcac gaatacgact ccttcgacgc tttgttggct 60
   gctagactgg aatctggtca aaccttggga cccgctggcg gtagagaggc ttctttgccc 120
   gaggctcctc atgctttgta ccgtccaacc ggacaacatg ttgctgtgtt ggcggctgct 180
   actcatagaa cccctgctgc tcgtgttact gctatggacc tggtcttggc ggccgttttg 240
   ctgggcgctc ctgtggtggt cgctctgaga aacactactg ccttctcccg tgaatccgaa 300
   ttggaactgt gcctcaccct gttcgattct cgtcccggcg gaccggatgc tgccctgaga 360
   gatgtggtat cctccgacat tgaaacctgg gctgtgggct tgctccacac cgatttgaac 420
   cctattgaga acgcttgctt ggcggctcaa ctgccacgct tgtctgccct cattgctgaa 480
   cgtcctttgg ccgatggacc cccttgtttg gtgttggtgg acatttcgat gacacctgtc 540
   gctgttttgt gggaggcccc tgaaccacct ggccctcccg atgttcgttt cgtcggtagc 600
   gaggccactg aggaattgcc tttcgtggct actgctggtg atgttttggc ggcgagtgct 660
   gccgatgccg atcctttctt cgctcgtgct atcctgggcc gtcctttcga tgcttctctg 720
   ctcactggtg aactgttccc tggtcacccc gtttaccaac gtcccctggc ggatgaggct 780
   ggtccttctg ctcctactgc cgctcgtgat cctagagatc tggctggagg cgacggtgga 840
   tccggacctg aggatcccgc tgctccacct gctagacagg ccgatcctgg tgttttggct 900
   cctactctgc tcaccgatgc tactactggc gaacctgtgc caccccgtat gtgggcttgg 960
   attcatggac tggaggaact ggcttccgat gatgccggcg gtcctacccc aaaccctgcc 1020
   ccggctttgc tgccccctcc tgctacggat caatctgtcc ccacttccca atacgcccct 1080
   agaccaattg gcccggctgc cactgctaga gaaactcgtc cttccgttcc ccctcaacaa 1140
   aacactggtc gtgtccctgt ggctccacgt gatgacccta gaccttcccc ccctactcct 1200
   tccccccctg ccgatgctgc tttgccacct cctgccttct ctggttctgc tgctgctttc 1260
   tccgctgctg ttccacgtgt tcgtcgttct aggcgtactc gtgccaaatc ccgtgcccct 1320
   cgtgcttctg ccccacccga gggatggcgt ccccccgctt tgcctgcccc tgttgctcct 1380
   gtggcggctt ctgctcgtcc ccccgatcaa cctcctactc ccgaatctgc tcccccggct 1440
   tgggtttccg ctctgccatt gccacccgga cctgctagtg ctcgtggtgc tttccctgct 1500
   ccaaccttgg cccctattcc cccacccccc gctgagggag ctgttgttcc cggtggtgat 1560
   cgtagacgtg gtcgccgtca aacaactgct ggaccatccc ctacaccgcc acgtggcccg 1620
   gctgctggtc ctcctcgtcg cctcactagg cctgctgttg ctagtctgtc cgcttctttg 1680
   aactctctgc cttccccccg tgatcctgcc gatcatgctg ctgccgtttc tgctgccgcc 1740
   gctgccgtac caccttcacc tggactggct cccccaactt ctgctgtcca aacctctcct 1800
   cctcccttgg cgcctggtcc tgttgcccca tctgaacctt tgtgtggctg ggttgtgcct 1860
   ggaggccctg ttgctagacg tcccccaccc caatctccgg ctactaaacc ggctgctcgt 1920
   acccgtatta gggctcgttc tgtgccccaa ccacccttgc cccaacctcc actgcctcaa 1980
   ccccccttgc ctcaaccccc tctcccccaa ccacctctgc ctcaacctcc gctgccccaa 2040
   cctcctttgc cccaacctcc tttgccccaa cctcctttgc cccaacctcc gctgccccaa 2100
   cctccgctgc cacctgttac tcgtacactc actccccaat ctcgtgactc tgtgcctaca 2160
   cctgagtctc caactcacac aaacacccac ttgcccgtta gtgctgtgac ttcttgggct 2220
   tcgtccctgg ctctccatgt ggattctgcc cctccccctg cttcattgct ccaaactctc 2280
   cacatttcct ccgatgatga acactccgac gccgactcac tccgcttctc cgattccgat 2340
   gacactgagg ctctcgatcc tttgcctcct gaacctcact tgccacctgc cgatgaaccc 2400
   cccggacctc tggctgccga ccatctccaa tcacctcact cacaattcgg tcctttgccc 2460
   gttcaagcga acgctgttct gtctcgtcgt tacgtgagat caactggccg ttctgccttg 2520
   gctgtgctca ttagagcttg tcgccgtatc caacaacaac tccagcgtac taggagagca 2580
   ctcttccaac gctcaaacgc cgtgctcaca tcactccacc atgtccgtat gctcttggga 2640
   taatag 2646

<210> 45
<211> 261
<212> DNA
<213> Human herpesvirus 2

<400> 45
atgtcttggg ctctgaaaac caccgacatg ttcctggact cttctcgttg cacccaccgt 60

acctacggtg acgtttgcgc tgaaatccac aaacgtgaac gtgaagaccg tgaagctgct 120

cgtaccgctg ttaccgaccc ggaactgccg ctgctgtgcc cgccggacgt tcgttctgac 180

ccggcttctc gtaacccgac ccagcagacc cgtggttgcg ctcgttctaa cgaacgtcag 240

gaccgtgttc tggctccgtg a 261

<210> 46
<211> 993
<212> DNA
<213> Human herpesvirus 2

<400> 46
atgaagttcc tcgtgaacgt ggccctggtg ttcatggtgg tgtacatcag ctacatctac 60

gctaaccgtt ggggttccgg cgtgcccggt cccatcaacc cccccaactc cgacgtggtg 120

ttccccggtg gttcccccgt ggctcagtac tgctacgctt acccccgtct ggacgaccct 180

ggtcccctgg gttctgctga cgctggtcgt caggacctgc cccgtcgtgt cgtgcgtcac 240

gagcccctgg gtcgtagctt cctgaccggt ggcctggtgc tgttggctcc ccctgtgcgc 300

ggtttcggtg ctcccaacgc tacctacgct gctcgtgtga cctactaccg tctgacccgt 360

gcttgccgtc agcccatcct gctgcgtcag tacggtggtt gccgtggtgg agagccccca 420

tcccccaaga cctgcggttc ttacacctac acctaccagg gtggtggtcc ccctacccgt 480

tacgctctgg tcaacgcttc cctgctggtg cccatctggg accgtgctgc tgagactttc 540

gagtaccaga tcgagctggg tggcgagctg cacgtgggtc tgctgtgggt ggaagtgggt 600

ggagagggtc ccggtcctac cgctcctcct caggctgctc gtgctgaggg tggtccttgc 660

gtgccacccg tgcctgctgg tcgtccttgg cgttccgtgc cccccgtgtg gtactccgct 720

cccaaccccg gtttccgcgg tctgcgtttc cgtgagcgtt gcctgcctcc ccagacccct      780

gctgctcctt ccgacctgcc tcgtgtggct ttcgctcccc agtccctgct cgtgggtatc      840

accggtcgta ccttcatccg tatggctcgt cccaccgagg acgtgggtgt cctgcctcct      900

cactgggctc caggtgctct ggacgacggt ccctacgctc ccttcccccc tcgtccccgt      960

ttccgtcgtc accaccacca tcaccactaa taa                                   993


<210> 47
<211> 9
<212> PRT
<213> Artificial Sequence

<220>
<223> Description of Artificial Sequence: Synthetic
      peptide

<400> 47
Gly Leu Ala His Val Ala Ala Ala Val
1               5


<210> 48
<211> 9
<212> PRT
<213> Artificial Sequence

<220>
<223> Description of Artificial Sequence: Synthetic
      peptide

<400> 48
Phe Ile Ser Gly Ser Val Ala Arg Ala
1               5


<210> 49
<211> 9
<212> PRT
<213> Artificial Sequence

<220>
<223> Description of Artificial Sequence: Synthetic
      peptide

<400> 49
Gln Tyr Ala Leu Ile Thr Arg Leu Leu
1               5


<210> 50
<211> 9
<212> PRT
<213> Artificial Sequence

<220>
<223> Description of Artificial Sequence: Synthetic
      peptide

<400> 50
Arg Tyr Asp Arg Ala Gln Lys Gly Phe
1               5

<210> 51
<211> 9
<212> PRT
<213> Artificial Sequence

<220>
<223> Description of Artificial Sequence: Synthetic
      peptide

<400> 51
Gly Tyr Ala Met Ala Ala Gly Arg Phe
1               5

<210> 52
<211> 9
<212> PRT
<213> Artificial Sequence

<220>
<223> Description of Artificial Sequence: Synthetic
      peptide

<400> 52
Pro Pro His Ala Asp Ala Pro Arg Leu
1               5

<210> 53
<211> 9
<212> PRT
<213> Artificial Sequence

<220>
<223> Description of Artificial Sequence: Synthetic
      peptide

<400> 53
Lys Pro Ala Ala Ala Ala Ala Pro Leu
1               5

<210> 54
<211> 8
<212> PRT
<213> Artificial Sequence

<220>
<223> Description of Artificial Sequence: Synthetic
      peptide

<400> 54
Ser Glu Ala Ala Val Ala Ala Val
1               5

<210> 55
<211> 8

<212> PRT
<213> Artificial Sequence

<220>
<223> Description of Artificial Sequence: Synthetic
      peptide

<400> 55
Phe Gly Trp Gly Leu Ala His Val
1               5


<210> 56
<211> 9
<212> PRT
<213> Artificial Sequence

<220>
<223> Description of Artificial Sequence: Synthetic
      peptide

<400> 56
Tyr Ala Leu Ile Thr Arg Leu Leu Tyr
1               5


<210> 57
<211> 9
<212> PRT
<213> Artificial Sequence

<220>
<223> Description of Artificial Sequence: Synthetic
      peptide

<400> 57
Ala Leu Pro Arg Ser Pro Arg Leu Leu
1               5


<210> 58
<211> 9
<212> PRT
<213> Artificial Sequence

<220>
<223> Description of Artificial Sequence: Synthetic
      peptide

<400> 58
Asp Leu Leu Phe Gln Asn Gln Ser Leu
1               5


<210> 59
<211> 9
<212> PRT
<213> Artificial Sequence

<220>
<223> Description of Artificial Sequence: Synthetic
      peptide

<400> 59
Ala Asp Leu Leu Phe Gln Asn Gln Ser
1               5


<210> 60
<211> 9
<212> PRT
<213> Artificial Sequence

<220>
<223> Description of Artificial Sequence: Synthetic
      peptide

<400> 60
Ala Arg Asn Ser Ser Ser Phe Ile Ser
1               5


<210> 61
<211> 9
<212> PRT
<213> Artificial Sequence

<220>
<223> Description of Artificial Sequence: Synthetic
      peptide

<400> 61
Gln Ala Cys Phe Arg Ile Ser Gly Ala
1               5


<210> 62
<211> 9
<212> PRT
<213> Artificial Sequence

<220>
<223> Description of Artificial Sequence: Synthetic
      peptide

<400> 62
Phe Val Arg Asp Ala Leu Val Leu Met
1               5


<210> 63
<211> 9
<212> PRT
<213> Artificial Sequence

<220>
<223> Description of Artificial Sequence: Synthetic
      peptide

<400> 63
Phe Asp Gly Asp Leu Ala Ala Val Pro
1               5


<210> 64
<211> 9

<212> PRT
<213> Artificial Sequence

<220>
<223> Description of Artificial Sequence: Synthetic
      peptide

<400> 64
Gly Leu Gly Asp Ser Arg Pro Gly Leu
1               5


<210> 65
<211> 9
<212> PRT
<213> Artificial Sequence

<220>
<223> Description of Artificial Sequence: Synthetic
      peptide

<400> 65
Trp Ala Pro Glu Leu Gly Asp Ala Ala
1               5


<210> 66
<211> 9
<212> PRT
<213> Artificial Sequence

<220>
<223> Description of Artificial Sequence: Synthetic
      peptide

<400> 66
Glu Cys Leu Ala Ala Cys Arg Gly Ile
1               5


<210> 67
<211> 9
<212> PRT
<213> Artificial Sequence

<220>
<223> Description of Artificial Sequence: Synthetic
      peptide

<400> 67
Arg Ala Trp Leu Arg Glu Leu Arg Phe
1               5


<210> 68
<211> 9
<212> PRT
<213> Artificial Sequence

<220>
<223> Description of Artificial Sequence: Synthetic
      peptide

<400> 68
Ala Leu Ala Gly Ser Thr Leu Ala Val
1               5


<210> 69
<211> 9
<212> PRT
<213> Artificial Sequence

<220>
<223> Description of Artificial Sequence: Synthetic
      peptide

<400> 69
Leu Leu Glu Asp Pro Ala Gly Thr Val
1               5


<210> 70
<211> 9
<212> PRT
<213> Artificial Sequence

<220>
<223> Description of Artificial Sequence: Synthetic
      peptide

<400> 70
Val Ile Gly Gly Ile Ala Phe Trp Val
1               5


<210> 71
<211> 9
<212> PRT
<213> Artificial Sequence

<220>
<223> Description of Artificial Sequence: Synthetic
      peptide

<400> 71
Thr Val Tyr Tyr Ala Val Leu Glu Arg
1               5


<210> 72
<211> 9
<212> PRT
<213> Artificial Sequence

<220>
<223> Description of Artificial Sequence: Synthetic
      peptide

<400> 72
Lys Tyr Ala Leu Ala Asp Pro Ser Leu
1               5


<210> 73
<211> 9

<212> PRT
<213> Artificial Sequence

<220>
<223> Description of Artificial Sequence: Synthetic
      peptide

<400> 73
Ala Phe Glu Thr Ala Gly Thr Tyr Leu
1               5


<210> 74
<211> 9
<212> PRT
<213> Artificial Sequence

<220>
<223> Description of Artificial Sequence: Synthetic
      peptide

<400> 74
Ala Pro Ser Asn Pro Gly Leu Ile Ile
1               5


<210> 75
<211> 9
<212> PRT
<213> Artificial Sequence

<220>
<223> Description of Artificial Sequence: Synthetic
      peptide

<400> 75
Ile Pro Ile Thr Val Tyr Tyr Ala Val
1               5


<210> 76
<211> 9
<212> PRT
<213> Artificial Sequence

<220>
<223> Description of Artificial Sequence: Synthetic
      peptide

<400> 76
Ala Pro Pro Ser His Gln Pro Leu Phe
1               5


<210> 77
<211> 9
<212> PRT
<213> Artificial Sequence

<220>
<223> Description of Artificial Sequence: Synthetic
      peptide

<400> 77
Phe Leu Met His Ala Pro Ala Phe Glu
1               5

<210> 78
<211> 9
<212> PRT
<213> Artificial Sequence

<220>
<223> Description of Artificial Sequence: Synthetic
      peptide

<400> 78
Phe Ser Ala Val Ser Glu Asp Asn Leu
1               5

<210> 79
<211> 8
<212> PRT
<213> Artificial Sequence

<220>
<223> Description of Artificial Sequence: Synthetic
      peptide

<400> 79
Val Tyr Tyr Ala Val Leu Glu Arg
1               5

<210> 80
<211> 8
<212> PRT
<213> Artificial Sequence

<220>
<223> Description of Artificial Sequence: Synthetic
      peptide

<400> 80
Ile Gly Met Leu Pro Arg Phe Ile
1               5

<210> 81
<211> 9
<212> PRT
<213> Artificial Sequence

<220>
<223> Description of Artificial Sequence: Synthetic
      peptide

<400> 81
Tyr Thr Glu Cys Pro Tyr Asn Lys Ser
1               5

<210> 82
<211> 9

<212> PRT
<213> Artificial Sequence

<220>
<223> Description of Artificial Sequence: Synthetic
      peptide

<400> 82
Phe Leu Met His Ala Pro Ala Phe Glu
1               5


<210> 83
<211> 9
<212> PRT
<213> Artificial Sequence

<220>
<223> Description of Artificial Sequence: Synthetic
      peptide

<400> 83
Asn Leu Gly Phe Leu Met His Ala Pro
1               5


<210> 84
<211> 9
<212> PRT
<213> Artificial Sequence

<220>
<223> Description of Artificial Sequence: Synthetic
      peptide

<400> 84
Val Ile Gly Gly Ile Ala Phe Trp Val
1               5


<210> 85
<211> 9
<212> PRT
<213> Artificial Sequence

<220>
<223> Description of Artificial Sequence: Synthetic
      peptide

<400> 85
Gly Ile Ala Phe Trp Val Arg Arg Arg
1               5


<210> 86
<211> 9
<212> PRT
<213> Artificial Sequence

<220>
<223> Description of Artificial Sequence: Synthetic
      peptide

<400> 86
Ser Glu Asp Asn Leu Gly Phe Leu Met
1               5


<210> 87
<211> 9
<212> PRT
<213> Artificial Sequence

<220>
<223> Description of Artificial Sequence: Synthetic
      peptide

<400> 87
Arg Thr Gln Pro Arg Trp Ser Tyr Tyr
1               5


<210> 88
<211> 9
<212> PRT
<213> Artificial Sequence

<220>
<223> Description of Artificial Sequence: Synthetic
      peptide

<400> 88
Ile Ala Phe Trp Val Arg Arg Arg Ala
1               5


<210> 89
<211> 9
<212> PRT
<213> Artificial Sequence

<220>
<223> Description of Artificial Sequence: Synthetic
      peptide

<400> 89
Leu Val Ile Gly Gly Ile Ala Phe Trp
1               5


<210> 90
<211> 9
<212> PRT
<213> Artificial Sequence

<220>
<223> Description of Artificial Sequence: Synthetic
      peptide

<400> 90
Phe Trp Val Arg Arg Arg Ala Gln Met
1               5


<210> 91
<211> 9

<212> PRT
<213> Artificial Sequence

<220>
<223> Description of Artificial Sequence: Synthetic
      peptide

<400> 91
Pro Tyr Thr Ser Thr Leu Leu Pro Pro
1               5


<210> 92
<211> 9
<212> PRT
<213> Artificial Sequence

<220>
<223> Description of Artificial Sequence: Synthetic
      peptide

<400> 92
Val Gly Thr Ala Ala Leu Leu Val Val
1               5


<210> 93
<211> 9
<212> PRT
<213> Artificial Sequence

<220>
<223> Description of Artificial Sequence: Synthetic
      peptide

<400> 93
Thr Ala Ala Leu Leu Val Val Ala Val
1               5


<210> 94
<211> 9
<212> PRT
<213> Artificial Sequence

<220>
<223> Description of Artificial Sequence: Synthetic
      peptide

<400> 94
Thr Ser Thr Leu Leu Pro Pro Glu Leu
1               5


<210> 95
<211> 9
<212> PRT
<213> Artificial Sequence

<220>
<223> Description of Artificial Sequence: Synthetic
      peptide

<400> 95
Gly Thr Val Ser Ser Gln Ile Pro Pro
1               5

<210> 96
<211> 9
<212> PRT
<213> Artificial Sequence

<220>
<223> Description of Artificial Sequence: Synthetic
      peptide

<400> 96
Thr Ala Gly Thr Tyr Leu Arg Leu Val
1               5

<210> 97
<211> 9
<212> PRT
<213> Artificial Sequence

<220>
<223> Description of Artificial Sequence: Synthetic
      peptide

<400> 97
Gly Val Thr Val Asp Ser Ile Gly Met
1               5

<210> 98
<211> 9
<212> PRT
<213> Artificial Sequence

<220>
<223> Description of Artificial Sequence: Synthetic
      peptide

<400> 98
Ala Phe Trp Val Arg Arg Arg Ala Gln
1               5

<210> 99
<211> 9
<212> PRT
<213> Artificial Sequence

<220>
<223> Description of Artificial Sequence: Synthetic
      peptide

<400> 99
Arg Val Tyr His Ile Gln Pro Ser Leu
1               5

<210> 100
<211> 9

<212> PRT
<213> Artificial Sequence

<220>
<223> Description of Artificial Sequence: Synthetic
      peptide

<400> 100
Ala Tyr Leu Val Asn Pro Phe Leu Phe
1               5


<210> 101
<211> 9
<212> PRT
<213> Artificial Sequence

<220>
<223> Description of Artificial Sequence: Synthetic
      peptide

<400> 101
Pro Phe Leu Phe Ala Ala Gly Phe Leu
1               5


<210> 102
<211> 9
<212> PRT
<213> Artificial Sequence

<220>
<223> Description of Artificial Sequence: Synthetic
      peptide

<400> 102
Thr Glu Tyr Val Leu Arg Ser Val Ile
1               5


<210> 103
<211> 9
<212> PRT
<213> Artificial Sequence

<220>
<223> Description of Artificial Sequence: Synthetic
      peptide

<400> 103
Gly Ser Gln Ala Thr Glu Tyr Val Leu
1               5


<210> 104
<211> 9
<212> PRT
<213> Artificial Sequence

<220>
<223> Description of Artificial Sequence: Synthetic
      peptide

<400> 104
Arg Ile Asp Gly Ile Phe Leu Arg Tyr
1               5


<210> 105
<211> 9
<212> PRT
<213> Artificial Sequence

<220>
<223> Description of Artificial Sequence: Synthetic
      peptide

<400> 105
Phe Leu Glu Asp Leu Ser His Ser Val
1               5


<210> 106
<211> 9
<212> PRT
<213> Artificial Sequence

<220>
<223> Description of Artificial Sequence: Synthetic
      peptide

<400> 106
Tyr Val Leu Arg Ser Val Ile Ala Lys
1               5


<210> 107
<211> 9
<212> PRT
<213> Artificial Sequence

<220>
<223> Description of Artificial Sequence: Synthetic
      peptide

<400> 107
Tyr Val Leu Arg Ser Val Ile Ala Lys
1               5


<210> 108
<211> 9
<212> PRT
<213> Artificial Sequence

<220>
<223> Description of Artificial Sequence: Synthetic
      peptide

<400> 108
Ala Tyr Leu Val Asn Pro Phe Leu Phe
1               5


<210> 109
<211> 9

<212> PRT
<213> Artificial Sequence

<220>
<223> Description of Artificial Sequence: Synthetic
      peptide

<400> 109
Glu Thr Thr Thr Arg Arg Ala Leu Tyr
1               5


<210> 110
<211> 9
<212> PRT
<213> Artificial Sequence

<220>
<223> Description of Artificial Sequence: Synthetic
      peptide

<400> 110
Arg Ile Asp Gly Ile Phe Leu Arg Tyr
1               5


<210> 111
<211> 9
<212> PRT
<213> Artificial Sequence

<220>
<223> Description of Artificial Sequence: Synthetic
      peptide

<400> 111
Tyr Leu Val Asn Pro Phe Leu Phe Ala
1               5


<210> 112
<211> 9
<212> PRT
<213> Artificial Sequence

<220>
<223> Description of Artificial Sequence: Synthetic
      peptide

<400> 112
Phe Val Cys Leu Phe Gly Leu Val Val
1               5


<210> 113
<211> 9
<212> PRT
<213> Artificial Sequence

<220>
<223> Description of Artificial Sequence: Synthetic
      peptide

<400> 113
Leu Tyr Lys Glu Ile Arg Asp Ala Leu
1               5


<210> 114
<211> 9
<212> PRT
<213> Artificial Sequence

<220>
<223> Description of Artificial Sequence: Synthetic
      peptide

<400> 114
Gly Leu Asp Thr Phe Leu Trp Asp Arg
1               5


<210> 115
<211> 9
<212> PRT
<213> Artificial Sequence

<220>
<223> Description of Artificial Sequence: Synthetic
      peptide

<400> 115
Arg Val Ser Pro Thr Arg Gly Arg Arg
1               5


<210> 116
<211> 9
<212> PRT
<213> Artificial Sequence

<220>
<223> Description of Artificial Sequence: Synthetic
      peptide

<400> 116
Gly Leu Asp Thr Phe Leu Trp Asp Arg
1               5


<210> 117
<211> 1221
<212> DNA
<213> Artificial Sequence

<220>
<223> Description of Artificial Sequence: Synthetic
      polynucleotide

<400> 117
atgtcgtact accatcacca tcaccatcac atggtgctgt acggcgggct gggcgacagc      60

cgccccggcc tctggggggc gcccgaggcg gaggaggcgc gggcccggtt cgaggcctcg      120

ggcgccccgg cgcccgtgtg ggcgcccgag ctgggcgacg cggcgcagca gtacgccctg      180

atcacgcggc tgctgtacac gccggacgcg gaggcgatgg ggtggctcca gaacccgcgc 240

gtggcgcccg gggacgtggc gctggaccag gcctgcttcc ggatctcggg cgcggcgcgc 300

aacagcagct ccttcatctc cggcagcgtg gcgcgggccg tgccccacct ggggtacgcc 360

atggcggcgg gccgcttcgg ctggggcctg gcgcacgtgg cggccgccgt ggccatgagc 420

cgccgctacg accgcgcgca gaagggcttc ctgctgacca gcctgcgccg cgcctacgcg 480

cccctgctgg cgcgcgagaa cgcggcgctg accggggcgc ggacccccga cgacggcggc 540

gacgccaacc gccgcgacgg cgacgacgcc cgcgggaagc ccgccgccgc cgccgccccg 600

ttgccgtcgg cggcggcgtc gccggccgac gagcgcgcgg tgcccgccgg ctacggcgcc 660

gcgggggtgc tcgccgccct ggggcgcctg agcgccgcgc ccgcctccgc gccggccggg 720

gccgacgacg acgacgacga cgacgacggc gccggcggtg gtggcggtgg tggcggtggt 780

ggcggcggcc ggcgcgcgga ggcgggccgc gtggccgtgg agtgcctggc cgcctgccgc 840

gggatcctgg aggcgctggc ggagggcttc gacggcgacc tggcggccgt gccggggctg 900

gccggagccc ggcccgccgc gcccccgcgc ccggggcccg cgggcgcggc cgccccgccg 960

cacgccgacg cgccccgcct gcgcgcctgg ctgcgcgagc tgcggttcgt gcgcgacgcg 1020

ctggtgctga tgcgcctgcg cggggacctg cgcgtggccg gcggcagcga ggccgccgtg 1080

gccgccgtgc gcgccgtgag cctggtcgcc ggggccctgg gcccggcgct gccgcggagc 1140

ccgcgcctgc tgagctccgc cgccgccgcc gccgcggacc tgctcttcca gaaccagagc 1200

ctgagtacta gaggatcata a 1221

<210> 118
<211> 705
<212> DNA
<213> Human herpesvirus 2

<400> 118
atgtcgtact accatcacca tcaccatcac atggggttcg tctgtctgtt tgggcttgtc 60

gttatgggag cctggggggc gtggggtggg tcacaggcaa ccgaatatgt tcttcgtagt 120

gttattgcca aagaggtggg ggacatacta agagtgcctt gcatgcggac ccccgcggac 180

gatgtttctt ggcgctacga ggccccgtcc gttattgact atgcccgcat agacggaata 240

tttcttcgct atcactgccc ggggttggac acgtttttgt gggataggca cgcccagagg 300

gcgtatcttg ttaacccctt tctctttgcg gcgggatttt tggaggactt gagtcactct 360

gtgtttccgg ccgacaccca ggaaacaacg acgcgccggg ccctttataa agagatacgc 420

gatgcgttgg gcagtcgaaa acaggccgtc agccacgcac ccgtcagggc cgggtgtgta 480

aactttgact actcacgcac tcgccgctgc gtcgggcgac gcgatttacg gcctgccaac 540

accacgtcaa cgtgggaacc gcctgtgtcg tcggacgatg aagcgagctc gcagtcgaag 600

cccctcgcca cccagccgcc cgtcctcgcc ctttcgaacg cccccccacg gcgggtctcc 660

ccgacgcgag gtcggcgccg gcatactcgc ctccgacgca actga 705

<210> 119
<211> 762
<212> DNA
<213> Human herpesvirus 2

<400> 119
atgaagttcc tcgtgaacgt ggccctggtg ttcatggtgg tgtacatcag ctacatctac 60

gccaaccgtt gggggttcgt ctgtctgttt gggcttgtcg ttatgggagc ctggggggcg 120

tggggtgggt cacaggcaac cgaatatgtt cttcgtagtg ttattgccaa agaggtgggg 180

gacatactaa gagtgccttg catgcggacc cccgcggacg atgtttcttg gcgctacgag 240

gccccgtccg ttattgacta tgcccgcata gacggaatat ttcttcgcta tcactgcccg 300

gggttggaca cgtttttgtg ggataggcac gcccagaggg cgtatcttgt taaccccttt 360

ctctttgcgg cgggattttt ggaggacttg agtcactctg tgtttccggc cgacacccag 420

gaaacaacga cgcgccgggc cctttataaa gagatacgcg atgcgttggg cagtcgaaaa 480

caggccgtca gccacgcacc cgtcagggcc gggtgtgtaa actttgacta ctcacgcact 540

cgccgctgcg tcgggcgacg cgatttacgg cctgccaaca ccacgtcaac gtgggaaccg 600

cctgtgtcgt cggacgatga agcgagctcg cagtcgaagc ccctcgccac ccagccgccc 660

gtcctcgccc tttcgaacgc ccccccacgg cgggtctccc cgacgcgagg tcggcgccgg 720

catactcgcc tccgacgcaa ccatcaccat caccatcact ga 762

<210> 120
<211> 4155
<212> DNA
<213> Artificial Sequence

<220>
<223> Description of Artificial Sequence: Synthetic
      polynucleotide

<400> 120
atgtcgtact accatcacca tcaccatcac atggccgctc ctgcccgcga cccccgggt 60

taccggtacg ccgcggccat ggtgcccacc ggctccatcc tgagtacgat cgaggtggcg 120

tcccaccgca gactctttga ttttttcgcc cgcgtgcgct ccgacgaaaa cagcctgtat 180

gacgtagagt ttgacgccct gctggggtcc tactgcaaca ccctgtcgct cgtgcgcttt 240

ctggagctcg gcctgtccgt ggcgtgcgtg tgcaccaagt tcccggagct ggcttacatg 300

aacgaagggc gtgtgcagtt cgaggtccac cagcccctca tcgcccgcga cggcccgcac 360

cccgtcgagc agcccgtgca taattacatg acgaaggtca tcgaccgccg ggccctgaac 420

gccgccttca gcctggccac cgaggccatt gccctgctca cgggggaggc cctggacggg 480

acgggcatta gcctgcatcg ccagctgcgc gccatccagc agctcgcgcg caacgtccag 540

gccgtcctgg gggcgtttga gcgcggcacg gccgaccaga tgctgcacgt gctgttggag 600

aaggcgcctc ccctggccct gctgttgccc atgcaacgat atctcgacaa cgggcgcctg 660

gcgaccaggg ttgcccgggc gaccctggtc gccgagctga agcggagctt ttgcgacacg 720

agcttcttcc tgggcaaggc gggccatcgc cgcgaggcca tcgaggcctg gctcgtggac 780

ctgaccacgg cgacgcagcc gtccgtggcc gtgccccgcc tgacgcacgc cgacacgcgc 840

gggcggccgg tcgacggggt gctggtcacc accgccgcca tcaaacagcg cctcctgcag 900

tccttcctga aggtggagga caccgaggcc gacgtgccgg tgacctacgg cgagatggtc 960

ttgaacgggg ccaacctcgt cacggcgctg gtgatgggca aggccgtgcg gagcctggac 1020

gacgtgggcc gccacctgct ggagatgcag gaggagcaac tcgaggcgaa ccgggagacg 1080

ctggatgaac tcgaaagcgc cccccagaca acgcgcgtgc gcgcggatct ggtggccata 1140

ggcgacaggc tggtcttcct ggaggccctg gagaagcgca tctacgccgc caccaacgtg 1200

ccctaccccc tggtgggcgc catggacctg acgttcgtcc tgcccctggg gctgttcaac 1260

ccggccatgg agcgcttcgc cgcgcacgcc ggggacctgg tgcccgcccc cggccacccg 1320

gagccccgcg cgttccctcc ccggcagctg tttttttggg gaaaggacca ccaggttctg 1380

cggctgtcca tggagaacgc ggtcgggacc gtgtgtcatc cttcgctcat gaacatcgac 1440

gcggccgtcg ggggcgtgaa ccacgacccc gtcgaggccg cgaatccgta cggggcgtac 1500

gtcgcggccc cggccggccc cggcgcggac atgcagcagc gttttctgaa cgcctggcgg 1560

cagcgcctcg cccacggccg ggtccggtgg gtcgccgagt gccagatgac cgcggagcag 1620

ttcatgcagc ccgacaacgc caacctggct ctggagctgc accccgcgtt cgacttcttc 1680

gcgggcgtgg ccgacgtcga gcttcccggc ggcgaagtcc ccccggccgg tccgggggcg 1740

atccaggcca cctggcgcgt ggtcaacggc aacctgcccc tggcgctgtg tccggtggcg 1800

tttcgtgacg cccggggcct ggagctcggc gttggccgcc acgccatggc gccggctacc 1860

atagccgccg tccgcggggc gttcgaggac cgcagctacc cggcggtgtt ctacctgctg 1920

caagccgcga ttcacggcag cgagcacgtg ttctgcgccc tggcgcggct cgtgactcag 1980

tgcatcacca gctactggaa caacacgcga tgcgcggcgt tcgtgaacga ctactcgctg 2040

gtctcgtaca tcgtgaccta cctcgggggc gacctccccg aggagtgcat ggccgtgtat 2100

cgggacctgg tggcccacgt cgaggccctg gcccagctgg tggacgactt taccctgccg 2160

ggcccggagc tgggcgggca ggctcaggcc gagctgaatc acctgatgcg cgacccggcg 2220

ctgctgccgc ccctcgtgtg ggactgcgac ggccttatgc gacacgcggc cctggaccgc 2280

caccgagact gccggattga cgcgggggag cacgagcccg tctacgcggc ggcgtgcaac 2340

gtggcgacgg ccgactttaa ccgcaacgac ggccggctgc tgcacaacac ccaggcccgc 2400

gcggccgacg ccgccgacga ccggccgcac cggccggccg actggaccgt ccaccacaaa 2460

atctactatt acgtgctggt gccggccttc tcgcgggggc gctgctgcac cgcgggggtc 2520

cgcttcgacc gcgtgtacgc cacgctgcag aacatggtgg tcccggagat cgcccccggc 2580

gaggagtgcc cgagcgatcc cgtgaccgac cccgcccacc cgctgcatcc cgccaatctg 2640

gtggccaaca cggtcaacgc catgttccac aacgggcgcg tcgtcgtcga cgggcccgcc 2700

atgctcacgc tgcaggtgct ggcgcacaac atggccgagc gcacgacggc gctgctgtgc 2760

tccgcggcgc ccgacgcggg cgccaacacc gcgtcgacgg ccaacatgcg catcttcgac 2820

ggggcgctgc acgccggcgt gctgctcatg gccccccagc acctggacca caccatccaa 2880

aatggcgaat acttctacgt cctgcccgtc cacgcgctgt ttgcgggcgc cgaccacgtg 2940

gccaacgcgc ccaacttccc cccggccctg cgcgacctgg cgcgccacgt ccccctggtc 3000

cccccggccc tgggggccaa ctacttctcc tccatccgcc agcccgtggt gcagcacgcc 3060

cgcgagagcg cggcggggga gaacgcgctg acctacgcgc tcatggcggg gtacttcaag 3120

atgagccccg tggccctgta tcaccagctc aagacgggcc tccaccccgg gttcgggttc 3180

accgtcgtgc ggcaggaccg cttcgtgacc gagaacgtgc tgttttccga gcgcgcgtcg 3240

gaggcgtact ttctgggcca gctccaggtg gcccgccacg aaacgggcgg gggggtcagc 3300

ttcacgctca cccagccgcg cggaaacgtg gacctgggtg tgggctacac cgccgtcgcg 3360

gccacggcca ccgtccgcaa ccccgttacg gacatgggca acctccccca aaacttttac 3420

ctcggccgcg gggccccccc gctgctagac aacgcggccg ccgtgtacct gcgcaacgcg 3480

gtcgtggcgg gaaaccggct ggggccggcc cagcccctcc cggtctttgg ctgcgcccag 3540

gtgccgcggc gcgccggcat ggaccacggg caggatgccg tgtgtgagtt catcgccacc 3600

cccgtggcca cggacatcaa ctactttcgc cggccctgca acccgcgggg acgcgcggcc 3660

ggcggcgtgt acgcggggga caaggagggg gacgtcatag ccctcatgta cgaccacggc 3720

cagagcgacc cggcgcggcc cttcgcggcc acggccaacc cgtgggcgtc gcagcggttc 3780

tcgtacgggg acctgctgta caacggggcc tatcacctca acggggcctc gcccgtcctc 3840

agcccctgct tcaagttctt caccgcggcc gacatcacgg ccaaacatcg ctgcctggag 3900

cgtcttatcg tggaaacggg atcggcggta tccacggcca ccgctgccag cgacgtgcag 3960

tttaagcgcc cgccggggtg ccgcgagctc gtggaagacc cgtgcggcct gtttcaggaa 4020

gcctacccga tcacctgcgc cagcgacccc gccctgctac gcagcgcccg cgatggggag 4080

gcccacgcgc gagagaccca ctttacgcag tatctcatct acgacgcctc cccgctaaag 4140

ggcctgtctc tgtaa 4155

<210> 121
<211> 1200
<212> DNA
<213> Artificial Sequence

<220>
<223> Description of Artificial Sequence: Synthetic
      polynucleotide

<400> 121
atgagtgccg aacagcgtaa aaagaaaaaa accaccacca cgacccaagg acgtggagct      60

gaagttgcta tggcggatga ggatggaggc cgcttgagag ctgctgctga gactactgga     120

ggacctggat caccggaccc tgccgatgga ccccccccta caccaaaccc cgatcgtaga     180

ccggctgcta gacctggatt cggatggcat ggaggacccg aggaaaacga ggacgaggcg     240

gacgacgccg ctgccgacgc cgacgccgat gaggctgccc ctgcttctgg agaggcggta     300

gacgaacctg ctgccgatgg agttgttagc cctaggcaat tggctttgtt ggcgagcatg     360

gtagacgagg ctgtgagaac aatcccttcc cctccccctg aacgtgatgg agcacaagag     420

gaggcggcta ggagtccctc accaccccgt acaccttcta tgagagcgga ttacggcgag     480

gaaaacgacg acgacgacga tgatgatgac gacgatgatc gtgatgccgg acgctgggtt     540

aggggacctg aaaccacttc tgctgtccgt ggagcatacc ccgatcctat ggcgagtttg     600

agccctagac cacctgcccc gaggagacac caccaccacc accatcatag gcgtagacgt     660

gctcctagac gtcgttctgc cgctagtgac tcttccaaat ctggctcttc ttcatctgcc     720

tcttccgctt catcttcggc ctcatcgtcc tcttcggcat ccgcttcgag tagtgatgat     780

gatgatgacg acgacgctgc tagagccccc gcttctgctg ccgaccacgc tgctggcgga     840

actttgggag ccgacgacga ggaggcggga gttcctgctc gtgccccggg agctgctccg     900

aggccttctc caccccgtgc tgaacctgct ccggctagaa caccggccgc tactgctggt     960

agactggagc gtagacgtgc ccgtgctgct gtggctggta gagatgctac tggccgcttc    1020

actgctggcc gtcctagacg tgttgaactg gacgccgatg ctgcttctgg tgctttctac    1080

gcccgttacc gtgatggtta cgtgtctggt gaaccttggc ctggcgctgg tccacctccg    1140

cccggacgtg tactctacgg tggattgggc gattctcgcc ctggtctgtg gggcgctccg    1200


<210> 122
<211> 825
<212> DNA
<213> Artificial Sequence

<220>
<223> Description of Artificial Sequence: Synthetic
      polynucleotide

<400> 122
tcgagtgccg ccgctgctgc cgccgatttg ttgttccaaa accaatccct ccgccctctg      60

ctcgccgaca ctgttgccgc tgccgattct ctggctgctc cggcttctgc cccacgtgaa 120

gctcgtaaac gtaaatcacc cgctccggct cgtgctcccc ctggtggcgc ccctagaccc 180

cctaaaaaat cccgtgccga tgcccctaga cctgctgctg ctccccccgc tggtgctgct 240

ccccccgctc cccctactcc ccccccacgc ccacctcgtc ccgctgccct cacacgccgt 300

cctgctgagg gacccgatcc acaaggcggc tggcgtagac aacctcctgg cccatcccat 360

acaccggcac catctgccgc tgctttggag gcttactgtg ctcctcgtgc tgtggctgaa 420

ctcaccgatc atccgctgtt ccctgctccc tggcgtcccg ccctcatgtt cgatcctaga 480

gctttggctt ccttggccgc tcgttgtgct gcccctcccc ctggcggtgc tccggctgct 540

ttcggtcctc tccgtgcctc tggtccactc cgccgtgccg ctgcctggat gagacaagtt 600

cccgaccctg aggatgttag agttgtgatc ttgtactcgc ccttgcctgg cgaggatttg 660

gccgctggta gagctggcgg tggcccccct cctgaatggt ctgctgaacg tggtggtttg 720

tcttgcttgt tggccgccct gggaaaccgt ctgtgtggtc ctgctactgc tgcttgggct 780

ggaaactgga ctggcgctcc cgatgtttct gctctcggtg ctcaa 825

<210> 123
<211> 933
<212> DNA
<213> Artificial Sequence

<220>
<223> Description of Artificial Sequence: Synthetic
      polynucleotide

<400> 123
tgggctggaa actggactgg cgctcccgat gtttctgctc tcggtgctca aggagttttg 60

ctgctctcta ctcgtgactt ggcattcgct ggagctgttg aattcctggg actcttggct 120

ggcgcttgtg ataggagact catcgtcgta aacgctgtga gagctgccga ttggcctgcc 180

gatggtcctg ttgtgtctcg tcaacacgct tacttggctt gtgaagtgtt gcccgctgtc 240

caatgtgctg ttcgctggcc tgctgctcgt gatctgaggc gtactgttct ggctagtggt 300

cgtgttttcg gacctggtgt tttcgctcgt gtcgaagctg ctcacgctag actgtacccc 360

gatgccccac ccctccgttt gtgtcgtgga gcaaacgttc gctaccgtgt ccgtactcgt 420

ttcggacccg atactctggt tccaatgtcc cctcgtgaat accgtcgtgc tgttctgcct 480

gccctcgatg gacgtgctgc cgcttctggc gctggtgacg ctatggctcc tggcgctccg 540

gacttctgtg aggatgaggc tcactcacat cgtgcctgtg cccgctgggg actgggcgct 600

ccattgaggc ctgtatacgt ggcactgggc cgtgatgctg ttagaggcgg acccgctgaa 660

ttgagaggcc ctcgtcgtga attctgtgct agggctctgc tcgaacccga tggagatgct 720

cctcctttgg tactccgtga cgacgccgat gctggtcctc ccccacaaat tcgctgggct 780

agtgctgctg gacgtgctgg tactgtattg gctgctgctg gcggtggcgt tgaagttgtt 840

ggtactgccg ctggactcgc tacacctccc cgccgtgaac ctgtagacat ggatgctgaa 900

ctcgaggatg atgacgacgg attgttcgga gag 933

<210> 124
<211> 1707
<212> DNA
<213> Artificial Sequence

<220>
<223> Description of Artificial Sequence: Synthetic
      polynucleotide

<400> 124
tcgagtgccg ccgctgctgc cgccgatttg ttgttccaaa accaatccct ccgccctctg 60

ctcgccgaca ctgttgccgc tgccgattct ctggctgctc cggcttctgc cccacgtgaa 120

gctcgtaaac gtaaatcacc cgctccggct cgtgctcccc ctggtggcgc ccctagaccc 180

cctaaaaaat cccgtgccga tgcccctaga cctgctgctg ctccccccgc tggtgctgct 240

ccccccgctc cccctactcc ccccccacgc ccacctcgtc ccgctgccct cacacgccgt 300

cctgctgagg gacccgatcc acaaggcggc tggcgtagac aacctcctgg cccatcccat 360

acaccggcac catctgccgc tgctttggag gcttactgtg ctcctcgtgc tgtggctgaa 420

ctcaccgatc atccgctgtt ccctgctccc tggcgtcccg ccctcatgtt cgatcctaga 480

gctttggctt ccttggccgc tcgttgtgct gcccctcccc ctggcggtgc tccggctgct 540

ttcggtcctc tccgtgcctc tggtccactc cgccgtgccg ctgcctggat gagacaagtt 600

cccgaccctg aggatgttag agttgtgatc ttgtactcgc ccttgcctgg cgaggatttg 660

gccgctggta gagctggcgg tggcccccct cctgaatggt ctgctgaacg tggtggtttg 720

tcttgcttgt tggccgccct gggaaaccgt ctgtgtggtc ctgctactgc tgcttgggct 780

ggaaactgga ctggcgctcc cgatgtttct gctctcggtg ctcaaggagt tttgctgctc 840

tctactcgtg acttggcatt cgctggagct gttgaattcc tgggactctt ggctggcgct 900

tgtgatagga gactcatcgt cgtaaacgct gtgagagctg ccgattggcc tgccgatggt 960

cctgttgtgt ctcgtcaaca cgcttacttg gcttgtgaag tgttgcccgc tgtccaatgt 1020

gctgttcgct ggcctgctgc tcgtgatctg aggcgtactg ttctggctag tggtcgtgtt 1080

ttcggacctg gtgttttcgc tcgtgtcgaa gctgctcacg ctagactgta ccccgatgcc 1140

ccacccctcc gtttgtgtcg tggagcaaac gttcgctacc gtgtccgtac tcgtttcgga 1200

cccgatactc tggttccaat gtcccctcgt gaataccgtc gtgctgttct gcctgccctc 1260

gatggacgtg ctgccgcttc tggcgctggt gacgctatgg ctcctggcgc tccggacttc 1320

tgtgaggatg aggctcactc acatcgtgcc tgtgcccgct ggggactggg cgctccattg 1380

aggcctgtat acgtggcact gggccgtgat gctgttagag gcggacccgc tgaattgaga 1440

ggccctcgtc gtgaattctg tgctagggct ctgctcgaac ccgatggaga tgctcctcct 1500

ttggtactcc gtgacgacgc cgatgctggt cctcccccac aaattcgctg ggctagtgct 1560

gctggacgtg ctggtactgt attggctgct gctggcggtg gcgttgaagt tgttggtact 1620

gccgctggac tcgctacacc tccccgccgt gaacctgtag acatggatgc tgaactcgag 1680

gatgatgacg acggattgtt cggagag 1707

<210> 125
<211> 1629
<212> DNA
<213> Artificial Sequence

<220>
<223> Description of Artificial Sequence: Synthetic
      polynucleotide

<400> 125
actgctggcc gtcctagacg tgttgaactg gacgccgatg ctgcttctgg tgctttctac 60

gcccgttacc gtgatggtta cgtgtctggt gaaccttggc ctggcgctgg tccacctccg 120

cccggacgtg tactctacgg tggattgggc gattctcgcc ctggtctgtg gggcgctccg 180

gaggctgagg aggctagagc ccgtttcgag gcttctggtg cccctgctcc tgtttgggct 240

cctgaattgg gcgacgctgc tcaacaatac gccctcatca cacgcttgct gtacactccc 300

gacgccgagg ctatgggatg gctccaaaac cctagagttg cccctggtga tgttgctctg 360

gatcaggctt gtttccgtat ctccggcgct gctcgtaact cttcttcgtt catctccggt 420

tctgtggcta gagctgtgcc tcacttggga tacgccatgg ccgctggacg tttcggctgg 480

ggactggctc atgttgctgc cgctgtagca atgtctagac gctacgaccg tgctcaaaaa 540

ggattcttgc tcacgtcact gaggcgtgct tacgcccctt tgttggcccg tgaaaacgct 600

gccctcactg gcgcccgtac ccccgatgac ggtggcgacg ccaaccgcca cgatggtgat 660

gatgctagag gcaaacccgc tgccgctgct gctcctttgc cctctgccgc cgcttcccct 720

gccgatgaac gtgctgttcc tgccggttac ggtgccgctg gtgtgttggc tgctttggga 780

cgcttgagtg ctgccccggc tagtgccccc gctggtgccg atgacgatga cgatgacgat 840

ggtgctggcg gaggcggtgg cggtagacgt gctgaggctg gacgtgttgc tgttgaatgc 900

ctggctgcct gtagaggaat cttggaggct ctggccgagg gattcgacgg agacttggcg 960

gctgtaccgg gactggcggg agcgaggcct gccgctccac ctcgccccgg tcctgctggt 1020

gctgccgctc ctcctcatgc cgacgctcct agactccgtg cttggctccg tgaactccgt 1080

ttcgttcgtg acgctttggt tctgatgaga ctgagaggcg acttgagagt ggctggagga 1140

tccgaggctg ctgttgctgc tgtccgtgct gtttctttgg ttgctggtgc tttgggccct 1200

gctttgccga gatctccccg tttgttgtcg agtgccgccg ctgctgccgc cgatttgttg 1260

ttccaaaacc aatccctccg ccctctgctc gccgacactg ttgccgctgc cgattctctg 1320

gctgctccgg cttctgcccc acgtgaagct cgtaaacgta aatcacccgc tccggctcgt 1380

gctccccctg gtggcgcccc tagaccccct aaaaaatccc gtgccgatgc ccctagacct 1440

gctgctgctc cccccgctgg tgctgctccc cccgctcccc ctactccccc cccacgccca 1500

cctcgtcccg ctgccctcac acgccgtcct gctgagggac ccgatccaca aggcggctgg 1560

cgtagacaac ctcctggccc atcccataca ccggcaccat ctgccgctgc tttggaggct 1620

tactgtgct 1629

<210> 126
<211> 1632
<212> DNA
<213> Artificial Sequence

<220>
<223> Description of Artificial Sequence: Synthetic
      polynucleotide

<400> 126
gccgctgccg attctctggc tgctccggct tctgccccac gtgaagctcg taaacgtaaa 60

tcacccgctc cggctcgtgc tccccctggt ggcgccccta gaccccctaa aaaatcccgt 120

gccgatgccc ctagacctgc tgctgctccc cccgctggtg ctgctccccc cgctccccct 180

actccccccc cacgcccacc tcgtcccgct gccctcacac gccgtcctgc tgagggaccc 240

gatccacaag gcggctggcg tagacaacct cctggcccat cccatacacc ggcaccatct 300

gccgctgctt tggaggctta ctgtgctcct cgtgctgtgg ctgaactcac cgatcatccg 360

ctgttccctg ctccctggcg tcccgccctc atgttcgatc ctagagcttt ggcttccttg 420

gccgctcgtt gtgctgcccc tccccctggc ggtgctccgg ctgctttcgg tcctctccgt 480

gcctctggtc cactccgccg tgccgctgcc tggatgagac aagttcccga ccctgaggat 540

gttagagttg tgatcttgta ctcgcccttg cctggcgagg atttggccgc tggtagagct 600

ggcggtggcc cccctcctga atggtctgct gaacgtggtg gtttgtcttg cttgttggcc 660

gccctgggaa accgtctgtg tggtcctgct actgctgctt gggctggaaa ctggactggc 720

gctcccgatg tttctgctct cggtgctcaa ggagttttgc tgctctctac tcgtgacttg 780

gcattcgctg gagctgttga attcctggga ctcttggctg gcgcttgtga taggagactc 840

atcgtcgtaa acgctgtgag agctgccgat tggcctgccg atggtcctgt tgtgtctcgt 900

caacacgctt acttggcttg tgaagtgttg cccgctgtcc aatgtgctgt tcgctggcct 960

gctgctcgtg atctgaggcg tactgttctg gctagtggtc gtgttttcgg acctggtgtt 1020

ttcgctcgtg tcgaagctgc tcacgctaga ctgtaccccg atgccccacc cctccgtttg 1080

tgtcgtggag caaacgttcg ctaccgtgtc cgtactcgtt tcggacccga tactctggtt 1140

ccaatgtccc ctcgtgaata ccgtcgtgct gttctgcctg ccctcgatgg acgtgctgcc 1200

gcttctggcg ctggtgacgc tatggctcct ggcgctccgg acttctgtga ggatgaggct 1260

cactcacatc gtgcctgtgc ccgctgggga ctgggcgctc cattgaggcc tgtatacgtg 1320

gcactgggcc gtgatgctgt tagaggcgga cccgctgaat tgagaggccc tcgtcgtgaa 1380

ttctgtgcta gggctctgct cgaacccgat ggagatgctc ctcctttggt actccgtgac 1440

gacgccgatg ctggtcctcc cccacaaatt cgctgggcta gtgctgctgg acgtgctggt 1500

actgtattgg ctgctgctgg cggtggcgtt gaagttgttg gtactgccgc tggactcgct 1560

acacctcccc gccgtgaacc tgtagacatg gatgctgaac tcgaggatga tgacgacgga 1620

ttgttcggag ag 1632

<210> 127
<211> 3330
<212> DNA
<213> Artificial Sequence

<220>
<223> Description of Artificial Sequence: Synthetic
      polynucleotide

<400> 127
caccaccacc accaccatca taggcgtaga cgtgctccta gacgtcgttc tgccgctagt 60

gactcttcca aatctggctc ttcttcatct gcctcttccg cttcatcttc ggcctcatcg 120

tcctcttcgg catccgcttc gagtagtgat gatgatgatg acgacgacgc tgctagagcc 180

cccgcttctg ctgccgacca cgctgctggc ggaactttgg gagccgacga cgaggaggcg 240

ggagttcctg ctcgtgcccc gggagctgct ccgaggcctt ctccaccccg tgctgaacct 300

gctccggcta gaacaccggc cgctactgct ggtagactgg agcgtagacg tgcccgtgct 360

gctgtggctg gtagagatgc tactggccgc ttcactgctg gccgtcctag acgtgttgaa 420

ctggacgccg atgctgcttc tggtgctttc tacgcccgtt accgtgatgg ttacgtgtct 480

ggtgaacctt ggcctggcgc tggtccacct ccgcccggac gtgtactcta cggtggattg 540

ggcgattctc gccctggtct gtggggcgct ccggaggctg aggaggctag agcccgtttc 600

gaggcttctg gtgcccctgc tcctgtttgg gctcctgaat tgggcgacgc tgctcaacaa 660

tacgccctca tcacacgctt gctgtacact cccgacgccg aggctatggg atggctccaa 720

aaccctagag ttgcccctgg tgatgttgct ctggatcagg cttgtttccg tatctccggc 780

gctgctcgta actcttcttc gttcatctcc ggttctgtgg ctagagctgt gcctcacttg 840

ggatacgcca tggccgctgg acgtttcggc tggggactgg ctcatgttgc tgccgctgta 900

gcaatgtcta gacgctacga ccgtgctcaa aaaggattct tgctcacgtc actgaggcgt 960

gcttacgccc ctttgttggc ccgtgaaaac gctgccctca ctggcgcccg tacccccgat 1020

gacggtggcg acgccaaccg ccacgatggt gatgatgcta gaggcaaacc cgctgccgct 1080

gctgctcctt tgccctctgc cgccgcttcc cctgccgatg aacgtgctgt tcctgccggt 1140

tacggtgccg ctggtgtgtt ggctgctttg ggacgcttga gtgctgcccc ggctagtgcc 1200

cccgctggtg ccgatgacga tgacgatgac gatggtgctg gcggaggcgg tggcggtaga 1260

cgtgctgagg ctggacgtgt tgctgttgaa tgcctggctg cctgtagagg aatcttggag 1320

gctctggccg agggattcga cggagacttg gcggctgtac cgggactggc gggagcgagg 1380

cctgccgctc cacctcgccc cggtcctgct ggtgctgccg ctcctcctca tgccgacgct 1440

cctagactcc gtgcttggct ccgtgaactc cgtttcgttc gtgacgcttt ggttctgatg 1500

agactgagag gcgacttgag agtggctgga ggatccgagg ctgctgttgc tgctgtccgt 1560

gctgtttctt tggttgctgg tgctttgggc cctgctttgc cgagatctcc ccgtttgttg 1620

tcgagtgccg ccgctgctgc cgccgatttg ttgttccaaa accaatccct ccgccctctg 1680

ctcgccgaca ctgttgccgc tgccgattct ctggctgctc cggcttctgc cccacgtgaa 1740

gctcgtaaac gtaaatcacc cgctccggct cgtgctcccc ctggtggcgc ccctagaccc 1800

cctaaaaaat cccgtgccga tgcccctaga cctgctgctg ctccccccgc tggtgctgct 1860

ccccccgctc cccctactcc ccccccacgc ccacctcgtc ccgctgccct cacacgccgt 1920

cctgctgagg gacccgatcc acaaggcggc tggcgtagac aacctcctgg cccatcccat 1980

acaccggcac catctgccgc tgctttggag gcttactgtg ctcctcgtgc tgtggctgaa 2040

ctcaccgatc atccgctgtt ccctgctccc tggcgtcccg ccctcatgtt cgatcctaga 2100

gctttggctt ccttggccgc tcgttgtgct gcccctcccc ctggcggtgc tccggctgct 2160

ttcggtcctc tccgtgcctc tggtccactc cgccgtgccg ctgcctggat gagacaagtt 2220

cccgaccctg aggatgttag agttgtgatc ttgtactcgc ccttgcctgg cgaggatttg 2280

gccgctggta gagctggcgg tggcccccct cctgaatggt ctgctgaacg tggtggtttg 2340

tcttgcttgt tggccgccct gggaaaccgt ctgtgtggtc ctgctactgc tgcttgggct 2400

ggaaactgga ctggcgctcc cgatgtttct gctctcggtg ctcaaggagt tttgctgctc 2460

tctactcgtg acttggcatt cgctggagct gttgaattcc tgggactctt ggctggcgct 2520

tgtgatagga gactcatcgt cgtaaacgct gtgagagctg ccgattggcc tgccgatggt 2580

cctgttgtgt ctcgtcaaca cgcttacttg gcttgtgaag tgttgcccgc tgtccaatgt 2640

gctgttcgct ggcctgctgc tcgtgatctg aggcgtactg ttctggctag tggtcgtgtt 2700

ttcggacctg gtgttttcgc tcgtgtcgaa gctgctcacg ctagactgta ccccgatgcc 2760

ccacccctcc gtttgtgtcg tggagcaaac gttcgctacc gtgtccgtac tcgtttcgga 2820

cccgatactc tggttccaat gtcccctcgt gaataccgtc gtgctgttct gcctgccctc 2880

gatggacgtg ctgccgcttc tggcgctggt gacgctatgg ctcctggcgc tccggacttc 2940

tgtgaggatg aggctcactc acatcgtgcc tgtgcccgct ggggactggg cgctccattg 3000

aggcctgtat acgtggcact gggccgtgat gctgttagag gcggacccgc tgaattgaga 3060

ggccctcgtc gtgaattctg tgctagggct ctgctcgaac ccgatggaga tgctcctcct 3120

ttggtactcc gtgacgacgc cgatgctggt cctcccccac aaattcgctg ggctagtgct 3180

gctggacgtg ctggtactgt attggctgct gctggcggtg gcgttgaagt tgttggtact 3240

gccgctggac tcgctacacc tccccgccgt gaacctgtag acatggatgc tgaactcgag 3300

gatgatgacg acggattgtt cggagagtaa 3330

<210> 128
<211> 3492
<212> DNA
<213> Artificial Sequence

<220>
<223> Description of Artificial Sequence: Synthetic
      polynucleotide

<400> 128
atgagtgccg aacagcgtaa aaagaaaaaa accaccacca cgacccaagg acgtggagct 60

gaagttgcta tggcggatga ggatggaggc cgcttgagag ctgctgctga gactactgga 120

ggacctggat caccggaccc tgccgatgga ccccccccta caccaaaccc cgatcgtaga 180

ccggctgcta gacctggatt cggatggcat ggaggacccg aggaaaacga ggacgaggcg 240

gacgacgccg ctgccgacgc cgacgccgat gaggctgccc ctgcttctgg agaggcggta 300

gacgaacctg ctgccgatgg agttgttagc cctaggcaat tggctttgtt ggcgagcatg 360

gtagacgagg ctgtgagaac aatcccttcc cctccccctg aacgtgatgg agcacaagag 420

gaggcggcta ggagtccctc accaccccgt acaccttcta tgagagcgga ttacggcgag 480

gaaaacgacg acgacgacga tgatgatgac gacgatgatc gtgatgccgg acgctgggtt 540

aggggacctg aaaccacttc tgctgtccgt ggagcatacc ccgatcctat ggcgagtttg 600

agccctagac cacctgcccc gaggagacac caccaccacc accatcatag gcgtagacgt 660

gctcctagac gtcgttctgc cgctagtgac tcttccaaat ctggctcttc ttcatctgcc 720

tcttccgctt catcttcggc ctcatcgtcc tcttcggcat ccgcttcgag tagtgatgat 780

gatgatgacg acgacgctgc tagagccccc gcttctgctg ccgaccacgc tgctggcgga 840

actttgggag ccgacgacga ggaggcggga gttcctgctc gtgccccggg agctgctccg 900

aggccttctc caccccgtgc tgaacctgct ccggctagaa caccggccgc tactgctggt 960

agactggagc gtagacgtgc ccgtgctgct gtggctggta gagatgctac tggccgcttc 1020

actgctggcc gtcctagacg tgttgaactg gacgccgatg ctgcttctgg tgctttctac 1080

gcccgttacc gtgatggtta cgtgtctggt gaaccttggc ctggcgctgg tccacctccg 1140

cccggacgtg tactctacgg tggattgggc gcccgtaccc ccgatgacgg tggcgacgcc 1200

aaccgccacg atggtgatga tgctagaggc aaacccgctg ccgctgctgc tcctttgccc 1260

tctgccgccg cttcccctgc cgatgaacgt gctgttcctg ccggttacgg tgccgctggt 1320

gtgttggctg ctttgggacg cttgagtgct gccccggcta gtgcccccgc tggtgccgat 1380

gacgatgacg atgacgatgg tgctggcgga ggcggtggcg gtagacgtgc tgaggctgga 1440

cgtgttgctg ttgaatgcct ggctgcctgt agaggaatct tggaggctct ggccgaggga 1500

ttcgacggag acttggcggc tgtaccggga ctggcgggag cgaggcctgc cgctccacct 1560

cgccccggtc ctgctggtgc tgccgctcct cctcatgccg acgctcctag actccgtgct 1620

tggctccgtg aactccgttt cgttcgtgac gctttggttc tgatgagact gagaggcgac 1680

ttgagagtgg ctggaggatc cgaggctgct gttgctgctg tccgtgctgt ttctttggtt 1740

gctggtgctt tgggccctgc tttgccgaga tctccccgtt tgttgtcgag tgccgccgct 1800

gctgccgccg atttgttgtt ccaaaaccaa tccctccgcc ctctgctcgc cgacactgtt 1860

gccgctgccg attctctggc tgctccggct tctgccccac gtgaagctcg taaacgtaaa 1920

tcacccgctc cggctcgtgc tccccctggt ggcgccccta gaccccctaa aaaatcccgt 1980

gccgatgccc ctagacctgc tgctgctccc cccgctggtg ctgctccccc cgctccccct 2040

actccccccc cacgcccacc tcgtcccgct gccctcacac gccgtcctgc tgagggaccc 2100

gatccacaag gcggctggcg tagacaacct cctggcccat cccatacacc ggcaccatct 2160

gccgctgctt tggaggctta ctgtgctcct cgtgctgtgg ctgaactcac cgatcatccg 2220

ctgttccctg ctccctggcg tcccgccctc atgttcgatc ctagagcttt ggcttccttg 2280

gccgctcgtt gtgctgcccc tccccctggc ggtgctccgg ctgctttcgg tcctctccgt 2340

gcctctggtc cactccgccg tgccgctgcc tggatgagac aagttcccga ccctgaggat 2400

gttagagttg tgatcttgta ctcgcccttg cctggcgagg atttggccgc tggtagagct 2460

ggcggtggcc cccctcctga atggtctgct gaacgtggtg gtttgtcttg cttgttggcc 2520

gccctgggaa accgtctgtg tggtcctgct actgctgctt gggctggaaa ctggactggc 2580

gctcccgatg tttctgctct cggtgctcaa ggagttttgc tgctctctac tcgtgacttg 2640

gcattcgctg gagctgttga attcctggga ctcttggctg gcgcttgtga taggagactc 2700

atcgtcgtaa acgctgtgag agctgccgat tggcctgccg atggtcctgt tgtgtctcgt 2760

caacacgctt acttggcttg tgaagtgttg cccgctgtcc aatgtgctgt tcgctggcct 2820

gctgctcgtg atctgaggcg tactgttctg gctagtggtc gtgttttcgg acctggtgtt 2880

ttcgctcgtg tcgaagctgc tcacgctaga ctgtaccccg atgccccacc cctccgtttg 2940

tgtcgtggag caaacgttcg ctaccgtgtc cgtactcgtt tcggacccga tactctggtt 3000

ccaatgtccc ctcgtgaata ccgtcgtgct gttctgcctg ccctcgatgg acgtgctgcc 3060

gcttctggcg ctggtgacgc tatggctcct ggcgctccgg acttctgtga ggatgaggct 3120

cactcacatc gtgcctgtgc ccgctgggga ctgggcgctc cattgaggcc tgtatacgtg 3180

gcactgggcc gtgatgctgt tagaggcgga cccgctgaat tgagaggccc tcgtcgtgaa 3240

ttctgtgcta gggctctgct cgaacccgat ggagatgctc ctcctttggt actccgtgac 3300

gacgccgatg ctggtcctcc cccacaaatt cgctgggcta gtgctgctgg acgtgctggt 3360

actgtattgg ctgctgctgg cggtggcgtt gaagttgttg gtactgccgc tggactcgct 3420

acacctcccc gccgtgaacc tgtagacatg gatgctgaac tcgaggatga tgacgacgga 3480

ttgttcggag ag 3492

<210> 129
<211> 3702
<212> DNA
<213> Artificial Sequence

<220>
<223> Description of Artificial Sequence: Synthetic
      polynucleotide

<400> 129
atgagtgccg aacagcgtaa aaagaaaaaa accaccacca cgacccaagg acgtggagct 60

gaagttgcta tggcggatga ggatggaggc cgcttgagag ctgctgctga gactactgga 120

ggacctggat caccggaccc tgccgatgga ccccccccta caccaaaccc cgatcgtaga 180

ccggctgcta gacctggatt cggatggcat ggaggacccg aggaaaacga ggacgaggcg 240

gacgacgccg ctgccgacgc cgacgccgat gaggctgccc ctgcttctgg agaggcggta 300

gacgaacctg ctgccgatgg agttgttagc cctaggcaat tggctttgtt ggcgagcatg 360

gtagacgagg ctgtgagaac aatcccttcc cctccccctg aacgtgatgg agcacaagag 420

gaggcggcta ggagtccctc accaccccgt acaccttcta tgagagcgga ttacggcgag 480

gaaaacgacg acgacgacga tgatgatgac gacgatgatc gtgatgccgg acgctgggtt 540

aggggacctg aaaccacttc tgctgtccgt ggagcatacc ccgatcctat ggcgagtttg 600

agccctagac cacctgcccc gaggagacac caccaccacc accatcatag gcgtagacgt 660

gctcctagac gtcgttctgc cgctagtgac tcttccaaat ctggctcttc ttcatctgcc 720

tcttccgctt catcttcggc ctcatcgtcc tcttcggcat ccgcttcgag tagtgatgat 780

gatgatgacg acgacgctgc tagagccccc gcttctgctg ccgaccacgc tgctggcgga 840

actttgggag ccgacgacga ggaggcggga gttcctgctc gtgccccggg agctgctccg 900

aggccttctc caccccgtgc tgaacctgct ccggctagaa caccggccgc tactgctggt 960

agactggagc gtagacgtgc ccgtgctgct gtggctggta gagatgctac tggccgcttc 1020

actgctggcc gtcctagacg tgttgaactg gacgccgatg ctgcttctgg tgctttctac 1080

gcccgttacc gtgatggtta cgtgtctggt gaaccttggc ctggcgctgg tccacctccg 1140

cccggacgtg tactctacgg tggattgggc gattctcgcc ctggtctgtg gggcgctccg 1200

gaggctgagg aggctagagc ccgtttcgag gcttctggtg cccctgctcc tgtttgggct 1260

cctgaattgg gcgacgctgc tcaacaatac gccctcatca cacgcttgct gtacactccc 1320

gacgccgagg ctatgggatg gctccaaaac cctagagttg cccctggtga tgttgctctg 1380

gatcaggctt gtttccgtat ctccggcgct gctcgtaact cttcttcgtt catctccggt 1440

tctgtggcta gagctgtgcc tcacttggga tacgccatgg ccgctggacg tttcggctgg 1500

ggactggctc atgttgctgc cgctgtagca atgtctagac gctacgaccg tgctcaaaaa 1560

ggattcttgc tcacgtcact gaggcgtgct tacgcccctt tgttggcccg tgaaaacgct 1620

gccctcactg gcgcccgtac ccccgatgac ggtggcgacg ccaaccgcca cgatggtgat 1680

gatgctagag gcaaacccgc tgccgctgct gctcctttgc cctctgccgc cgcttcccct 1740

gccgatgaac gtgctgttcc tgccggttac ggtgccgctg gtgtgttggc tgctttggga 1800

cgcttgagtg ctgccccggc tagtgccccc gctggtgccg atgacgatga cgatgacgat 1860

ggtgctggcg gaggcggtgg cggtagacgt gctgaggctg gacgtgttgc tgttgaatgc 1920

ctggctgcct gtagaggaat cttggaggct ctggccgagg gattcgacgg agacttggcg 1980

gctgtaccgg gactggcggg agcgaggcct gccgctccac ctcgccccgg tcctgctggt 2040

gctgccgctc ctcctcatgc cgacgctcct agactccgtg cttggctccg tgaactccgt 2100

ttcgttcgtg acgctttggt tctgatgaga ctgagaggcg acttgagagt ggctggagga 2160

tccgaggctg ctgttgctgc tgtccgtgct gtttctttgg ttgctggtgc tttgggccct 2220

gctttgccga gatctccccg tttgttgtcg agtgccgccg ctgctgccgc cgatttgttg 2280

ttccaaaacc aatccctccg ccctctgctc gccgacactg ttgccgctgc cgattctctg 2340

gctgctccgg cttctacacc ggcaccatct gccgctgctt tggaggctta ctgtgctcct 2400

cgtgctgtgg ctgaactcac cgatcatccg ctgttccctg ctccctggcg tcccgccctc 2460

atgttcgatc ctagagcttt ggcttccttg gccgctcgtt gtgctgcccc tccccctggc 2520

ggtgctccgg ctgctttcgg tcctctccgt gcctctggtc cactccgccg tgccgctgcc 2580

tggatgagac aagttcccga ccctgaggat gttagagttg tgatcttgta ctcgcccttg 2640

cctggcgagg atttggccgc tggtagagct ggcggtggcc cccctcctga atggtctgct 2700

gaacgtggtg gtttgtcttg cttgttggcc gccctgggaa accgtctgtg tggtcctgct 2760

actgctgctt gggctggaaa ctggactggc gctcccgatg tttctgctct cggtgctcaa 2820

ggagttttgc tgctctctac tcgtgacttg gcattcgctg gagctgttga attcctggga 2880

ctcttggctg gcgcttgtga taggagactc atcgtcgtaa acgctgtgag agctgccgat 2940

tggcctgccg atggtcctgt tgtgtctcgt caacacgctt acttggcttg tgaagtgttg 3000

cccgctgtcc aatgtgctgt tcgctggcct gctgctcgtg atctgaggcg tactgttctg 3060

gctagtggtc gtgttttcgg acctggtgtt ttcgctcgtg tcgaagctgc tcacgctaga 3120

ctgtaccccg atgccccacc cctccgtttg tgtcgtggag caaacgttcg ctaccgtgtc 3180

cgtactcgtt tcggacccga tactctggtt ccaatgtccc ctcgtgaata ccgtcgtgct 3240

gttctgcctg ccctcgatgg acgtgctgcc gcttctggcg ctggtgacgc tatggctcct 3300

ggcgctccgg acttctgtga ggatgaggct cactcacatc gtgcctgtgc ccgctgggga 3360

ctgggcgctc cattgaggcc tgtatacgtg gcactgggcc gtgatgctgt tagaggcgga 3420

cccgctgaat tgagaggccc tcgtcgtgaa ttctgtgcta gggctctgct cgaacccgat 3480

ggagatgctc ctcctttggt actccgtgac gacgccgatg ctggtcctcc cccacaaatt 3540

cgctgggcta gtgctgctgg acgtgctggt actgtattgg ctgctgctgg cggtggcgtt 3600

gaagttgttg gtactgccgc tggactcgct acacctcccc gccgtgaacc tgtagacatg 3660

gatgctgaac tcgaggatga tgacgacgga ttgttcggag ag 3702

<210> 130
<211> 6
<212> PRT
<213> Artificial Sequence

<220>
<223> Description of Artificial Sequence: Synthetic
      6xHis tag

<400> 130
His His His His His His
1               5


<210> 131
<211> 10
<212> PRT
<213> Artificial Sequence

<220>
<223> Description of Artificial Sequence: Synthetic
      peptide

<400> 131
Met Ser Tyr Tyr His His His His His His
1               5                   10


<210> 132
<211> 21
<212> PRT
<213> Artificial Sequence

<220>
<223> Description of Artificial Sequence: Synthetic
      peptide

<400> 132
Met Lys Phe Leu Val Asn Val Ala Leu Val Phe Met Val Val Tyr Ile
1               5                   10                  15

Ser Tyr Ile Tyr Ala
            20

<210> 133
<211> 294
<212> DNA
<213> Human herpesvirus 2

<400> 133
atgtcgtact accatcacca tcaccatcac atgacgggga aacccgcaag actgggccgc      60

tgggtggtgc tgttgttcgt cgcgctcgtc gcgggcgtgc ccggggagcc gccgaacgcg     120

gcaggcgcac gcggcgttat cggggacgcg caatgccggg gcgacagcgc cggtgtggtg     180

tccgtcccgg gggtcctggt gcccttttat ctaggcatga cctcgatggg cgtatgtatg     240

atcgcgcacg tgtatcagat atgccagcgg gcactggccg ccgggtcagc ctga           294

<210> 134
<211> 1404
<212> DNA
<213> Human herpesvirus 2

<400> 134
atgggacgcc gggcccccag gggatccccc gaggccgcgc cgggcgccga cgtcgcgccc      60

ggggcgcggg cggcgtggtg ggtctggtgt gtgcaggtgg cgacgttcat cgtctcggcc     120

atctgcgtcg tggggctcct ggtgctggcc tctgtgttcc gggacaggtt tccctgcctt     180

tacgcccccg cgacctctta tgcgaaggcg aacgccacgg tcgaggtgcg cgggggtgta     240

gccgtccccc tccggttgga cacgcagagc ctgctggcca cgtacgcaat tacgtctacg     300

ctgttgctgg cggcggccgt gtacgccgcg gtgggcgcgg tgacctcgcg ctacgagcgc     360

gcgctggatg cggcccgtcg cctggcggcg gcccgtatgg cgatgccaca cgccacgcta     420

atcgccggaa acgtctgcgc gtggctgttg cagatcacag tcctgctgct ggcccaccgc     480

atcagccagc tggcccacct tatctacgtc ctgcactttg cgtgcctcgt gtatctcgcg     540

gcccattttt gcaccagggg ggtcctgagc gggacgtacc tgcgtcaggt tcacggcctg     600

attgacccgg cgccgacgca ccatcgtatc gtcggtccgg tgcgggcagt aatgacaaac     660

gccttattac tgggcaccct cctgtgcacg gccgccgccg cggtctcgtt gaacacgatc     720

gccgccctga acttcaactt ttccgccccg agcatgctca tctgcctgac gacgctgttc     780

gccctgcttg tcgtgtcgct gttgttggtg gtcgaggggg tgctgtgtca ctacgtgcgc     840

gtgttggtgg gcccccacct cggggccatc gccgccaccg gcatcgtcgg cctggcctgc 900

gagcactacc acaccggtgg ttactacgtg gtggagcagc agtggccggg ggcccagacg 960

ggagtccgcg tcgccctggc gctcgtcgcc gcctttgccc tcgccatggc cgtgcttcgg 1020

tgcacgcgcg cctacctgta tcaccggcga caccacacta aatttttcgt gcgcatgcgc 1080

gacacccggc accgcgccca ttcggcgctt cgacgcgtac gcagctccat gcgcggttct 1140

aggcgtggcg ggccgcccgg agacccgggc tacgcggaaa ccccctacgc gagcgtgtcc 1200

caccacgccg agatcgaccg gtatggggat tccgacgggg acccgatcta cgacgaagtg 1260

gcccccgacc acgaggccga gctctacgcc cgagtgcaac gccccgggcc tgtgcccgac 1320

gccgagccca tttacgacac cgtggagggg tatgcgccaa ggtccgcggg ggagccggtg 1380

tacagcaccg ttcggcgatg gtag 1404

<210> 135
<211> 9
<212> PRT
<213> Artificial Sequence

<220>
<223> Description of Artificial Sequence: Synthetic
      peptide

<400> 135
Tyr Val Leu Arg Ser Val Ile Ala Lys
1               5

<210> 136
<211> 9
<212> PRT
<213> Artificial Sequence

<220>
<223> Description of Artificial Sequence: Synthetic
      peptide

<400> 136
Asp Ile Leu Arg Val Pro Cys Met Arg
1               5

<210> 137
<211> 9
<212> PRT
<213> Artificial Sequence

<220>
<223> Description of Artificial Sequence: Synthetic
      peptide

<400> 137
Asp Arg His Ala Gln Arg Ala Tyr Leu
1               5